# EUROPEAN PATENT APPLICATION

(11) **EP 2 952 507 A1**
(43) Date of publication of application: **09.12.2015**
(21) Application number: 14171469.1
(22) Date of filing: 06.06.2014
(51) Int. Cl.: C07D 249/08, A01N 43/653

(54) **Substituted [1,2,4]triazole compounds**

(71) Applicant: BASF SE, 67056 Ludwigshafen (DE)
(72) Inventor: GRAMMENOS, Wassilios, 67071 Ludwigshafen (DE); BOUDET, Nadege, 69493 Hirschberg (DE); MÜLLER, Bernd, 67227 Frankenthal (DE); QUINTERO PALOMAR, Maria Angelica, 68165 Mannheim (DE); ESCRIBANO CUESTA, Ana, 68161 mannheim (DE); LAUTERWASSER, Erica May Wilson, 67157 Wachenheim (DE); LOHMANN, Jan Klaas, 67245 Lambsheim (DE); GROTE, Thomas, 67157 Wachenheim (DE); KRETSCHMER, Manuel, 68165 Mannheim (DE); CRAIG, Ian Robert, 67063 Ludwigshafen (DE)
(74) Representative: Maiwald Patentanwalts GmbH

(57) **Abstract**

The present invention relates to compounds of the formula I 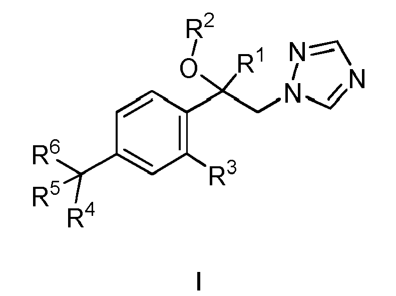 wherein the variables are defined in the description and claims, their preparation and uses thereof.

## Description

The present invention relates to substituted [1,2,4]triazole compounds and the N-oxides and the salts thereof for combating phytopathogenic fungi, and to the use and methods for combating phytopathogenic fungi and to seeds coated with at least one such compound. The invention also relates to processes for preparing these compounds, intermediates, processes for preparing such intermediates, and to compositions comprising at least one compound I.

In many cases, in particular at low application rates, the fungicidal activity of known fungicidal compounds is unsatisfactory. Based on this, it was an object of the present invention to provide compounds having improved activity and/or a broader activity spectrum against phytopathogenic harmful fungi.

Surprisingly, this object is achieved by the use of the inventive substituted [1,2,4]triazol compounds of formula I having favorable fungicidal activity against phytopathogenic fungi.

Accordingly, the present invention relates to compounds of the formula I 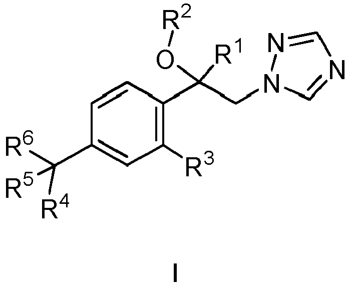 wherein
- R¹: is C₁-C₆-alkyl, C₂-C₆-alkenyl, C₂-C₆-alkynyl or C₃-C₆-cycloalkyl;
wherein the aliphatic moieties of R¹ are unsubstituted or carry one, two, three or up to the maximum possible number of identical or different groups R^{1a}:
- R^{1a}: is independently of one another selected from halogen, OH, CN, C₁-C₄-alkoxy, C₃-C₆-cycloalkyl, C₃-C₆-halogencycloalkyl and C₁-C₄-halogenalkoxy;
wherein the cycloalkyl moieties of R¹ are unsubstituted or carry one, two, three, four, five or up to the maximum number of identical or different groups R^{1b}:
- R^{1b}: is independently of one another selected from halogen, OH, CN, C₁-C₄-alkyl, C₁-C₄-alkoxy, C₁-C₄-halogenalkyl, C₃-C₆-cycloalkyl, C₃-C₆-halogencycloalkyl and C₁-C₄-halogenalkoxy;
- R²: is hydrogen, C₁-C₄-alkyl, C₂-C₄-alkenyl or C₂-C₄-alkynyl; wherein the aliphatic moieties of R² are unsubstituted or carry one, two, three or up to the maximum possible number of identical or different groups R^{2a}:
- R^{2a}: is independently of one another selected from halogen, OH, CN, C₁-C₄-alkoxy, C₃-C₆-cycloalkyl, C₃-C₆-halogencycloalkyl and C₁-C₄-halogenalkoxy;
- R³: is selected from hydrogen, halogen, CN, C₁-C₄-alkyl, C₁-C₄-alkoxy, C₂-C₄-alkenyl, C₂-C₄-alkynyl, C₃-C₆-cycloalkyl and S(O)ₚ(C₁-C₄-alkyl), wherein p is 0, 1 or 2, and wherein each of R³ is unsubstituted or further substituted by one, two, three or four R^{3a}:
- R^{3a}: is independently of one another selected from halogen, CN, OH, C₁-C₄-alkyl, C₁-C₄-halogenalkyl, C₃-C₆-cycloalkyl, C₃-C₆-halogencycloalkyl, C₁-C₄-alkoxy and C₁-C₄-halogenalkoxy;
- R⁴, R⁵, and R⁶: are independently of one another selected from hydrogen, halogen, CN, -OCN, -SCN, -S(O)ₘ, -S(O)ₒ(C₁-C₄-alkyl), C₁-C₆-alkyl, C₂-C₆-alkenyl, C₂-C₆-alkynyl, C₁-C₄-alkoxy, C₂-C₄-alkenyloxy, C₃-C₆-cycloalkyl, C₃-C₈-cycloalkyl-C₁-C₄-alkyl, C₃-C₆-halogencycloalkyl, aryl, heteroaryl and aryloxy;
- R⁴ and R⁵: together are =O, and R⁶ is as defined above;
- R⁴ and R⁵: together are =NORⁿ, and R⁶ is as defined above; or
- R⁴⁻⁶: together are ≡N
wherein the aliphatic moieties of R⁴, R⁵, and R⁶ are unsubstituted or further substituted by one, two, three or four R^{a}:
- R^{a}: is independently of one another selected from halogen, OH, CN, C₁-C₄-alkyl, C₁-C₄-halogenalkyl, C₃-C₆-cycloalkyl, C₃-C₆-halogencycloalkyl, C₁-C₄-alkoxy, C₁-C₄-halogenalkoxy and Si(R^{s})₃, wherein R^{s} is independently of one another C₁-C₄-alkyl;
wherein the cycloalkyl moieties of R⁴, R⁵, and R⁶ are unsubstituted or carry one, two, three, four, five or up to the maximum number of identical or different groups R^{b}:
- R^{b}: is independently of one another selected from halogen, OH, CN, C₁-C₄-alkyl, C₁-C₄-alkoxy, C₁-C₄-halogenalkyl, C₃-C₆-cycloalkyl, C₃-C₆-halogencycloalkyl and C₁-C₄-halogenalkoxy;
wherein the aryl, heteroaryl and aryloxy moieties of R⁴, R⁵, and R⁶ are unsubstituted or further substituted by one, two, three or four R^{c}:
- R^{c}: is independently of one another selected from halogen, OH, CN, C₁-C₄-alkyl, C₁-C₄-halogenalkyl, C₃-C₆-cycloalkyl, C₃-C₆-halogencycloalkyl, C₁-C₄-alkoxy and C₁-C₄-halogenalkoxy;
and wherein
- Rⁿ: is C₁-C₄-alkyl, C₂-C₄-alkenyl or C₂-C₄-alkynyl;
wherein m is independently 0, 1 or 2;
wherein o is independently 0, 1 or 2;
provided that at least one of R⁴, R⁵ and R⁶ is not hydrogen;
provided that when R² and R³ are both hydrogen, and R⁴⁻⁶ is each F, then R¹ is not CH(OH)CH₃, CHCH₃CH=CH₂, cyclopropanecarbonitrile, CH₂CCl₃, CF₃, CF₂CF₃,
provided that when R² and R³ are both hydrogen, -CR⁴R⁵R⁶ is CH(CH₃)₂, then R¹ is not CHCH₃CHCH₂, C(CH₃)₂CH₂OH,
provided that when R² and R³ are both hydrogen, -CR⁴R⁵R⁶ is CH(CH₃)₂, then R¹ is not C(CH₃)₃,
provided that when R² and R³ are both hydrogen, R⁴⁻⁶ is each CH₃, then R¹ is not CH₂CCl₃, C(CH₃)₃,
provided that when R² and R³ are both hydrogen, R⁴⁻⁵ is =O and R⁶ is OCH₃, then R¹ is not C(CH₃)₃,
provided that when R² and R³ are both hydrogen, and R⁴⁻⁶ is ≡N, then R¹ is not C(CH₃)₃, C(CH₃)₂C(CH₃)₂OH, or CF₂C(CH₃)₂OH; and
provided that when R² is hydrogen, R³ is Cl, and R⁴⁻⁶ is ≡N, then R¹ is not CF₂C(CH₃)₂OH;
and the N-oxides and the agriculturally acceptable salts thereof.

The inventive compounds can be prepared as follows.

Compounds of formula I can be prepared from compounds II by using an appropriate cross coupling method known to a skilled person. A suitable organometallic species, where M is preferably Mg, Zn, or Sn, is reacted with compound II in the presence of a transition metal catalyst, preferably Pd (or a salt, or a complex hereof), in the presence of a suitable ligand and, if appropriate, a base. The reaction is performed in a suitable organic solvent such as THF, DMF, MeCN.

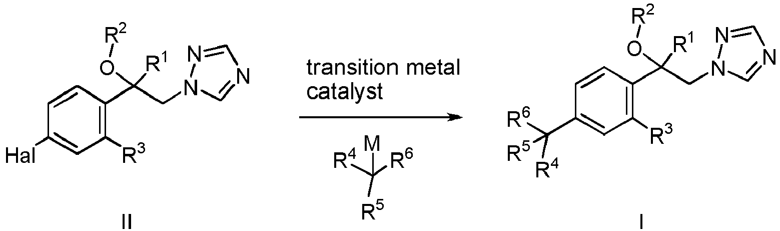

Compounds of formula III (i.e., compounds of formula I wherein R⁴ and R⁵ together form =O) can be prepared by reacting compounds II with a suitable transmetallation agent, preferably n-BuLi or iso-propylmagnesium chloride, in an organic solvent, preferably THF at low temperatures and subsequent addition of a carboxylic acid chloride to afford compound III.

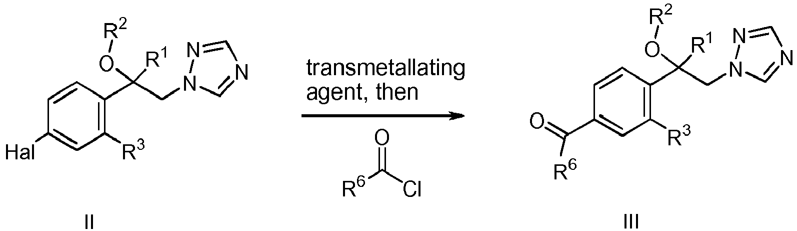

Compounds IIIa, where R⁶ is H, can be prepared by reacting the organometallic congener of compounds II (prepared as described above) with a suitable formyl transfer reagent, preferably DMF.

Alternatively, compounds IIIa can be prepared when compounds II are reacted with CO and hydrogen gas in the presence of a transition metal catalyst, preferably using palladium, rhodium, iridium or mixtures hereof in the combination with a suitable ligand (Angewandte Chemie, International Edition 2006, 45, 154-158; Journal of the American Chemical Society 2008, 130, 15549-15563). If appropriate, the reaction can be performed at elevated pressures of one or both gases and/or temperatures above room temperature.

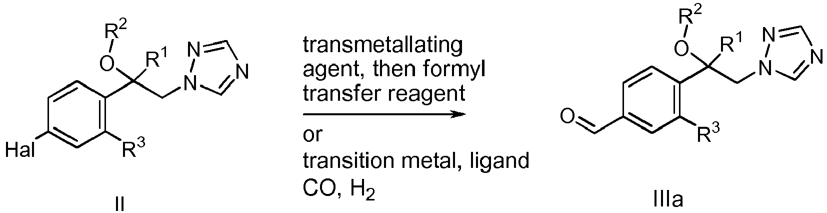

Compounds III can be reacted in a Wittig-type reaction known to a skilled person. Phosphonium salts (with X being a halogen, preferably Br or I) can be reacted with a strong base, preferably n-BuLi or potassium tert-butoxide, in a organic solvent, preferably THF and added to compound III to afford compounds IV. Compound IV can then be reacted with hydrogen either at atmospheric or elevated pressure in the presence of a transition metal catalyst known to a skilled person, preferably Pt, Pd, Pd/C, Ir, or their salts, or complexes, or mixtures hereof in an organic solvent, if required at elevated temperatures to afford compounds Ia.

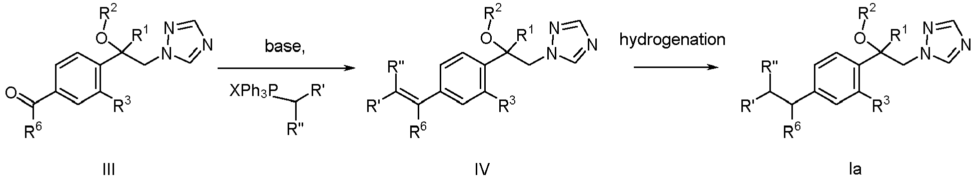

Compounds Ic can be prepared from compounds III as follows. Compounds III can be reacted with a suitable reducing agent, preferably NaBH₄ or LiAlH₄, in a suitable organic solvent such as MeOH or THF to afford compounds V. Compounds Ic can be obtained from alcohol compounds V by reacting the alcohol compound with R"'-LG, wherein LG represents a nucleophilically replaceable leaving group, such as halogen, alkylsulfonyl, alkylsulfonyloxy and arylsulfonyloxy, preferably chloro, bromo or iodo, particularly preferably bromo, preferably in the presence of a base, such as for example, NaH in a suitable solvent such as THF.

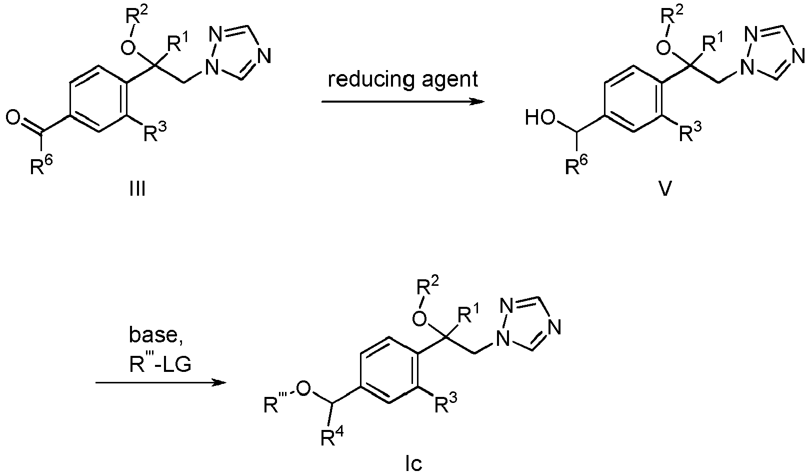

In the definitions of the variables given above, collective terms are used which are generally representative for the substituents in question. The term "Cₙ-Cₘ" indicates the number of carbon atoms possible in each case in the substituent or substituent moiety in question.

The term "halogen" refers to fluorine, chlorine, bromine and iodine.

The term "C₁-C₆-alkyl" refers to a straight-chained or branched saturated hydrocarbon group having 1 to 6 carbon atoms, e.g. methyl, ethyl, propyl, 1-methylethyl, butyl, 1-methylpropyl, 2-methylpropyl, 1,1-dimethylethyl, pentyl, 1-methylbutyl, 2-methylbutyl, 3-methylbutyl, 2,2-dimethylpropyl, 1-ethylpropyl, 1,1-dimethylpropyl, 1,2-dimethylpropyl, hexyl, 1-methylpentyl, 2-methylpentyl, 3-methylpentyl, 4-methylpentyl, 1,1-dimethylbutyl, 1,2-dimethylbutyl, 1,3-dimethylbutyl, 2,2-dimethylbutyl, 2,3-dimethylbutyl, 3,3-dimethylbutyl, 1-ethylbutyl, 2-ethylbutyl, 1,1,2-trimethylpropyl, 1,2,2-trimethylpropyl, 1-ethyl-1-methylpropyl and 1-ethyl-2-methylpropyl. A preferred embodiment of a C₁-C₆-alkyl is a C₂-C₄-alkyl. Likewise, the term "C₂-C₄-alkyl" refers to a straight-chained or branched alkyl group having 2 to 4 carbon atoms, such as ethyl, propyl (n-propyl), 1-methylethyl (iso-propoyl), butyl, 1-methylpropyl (sec.-butyl), 2-methylpropyl (iso-butyl), 1,1-dimethylethyl (tert.-butyl).

The term "C₁-C₆-haloalkyl" refers to an alkyl group having 1 or 6 carbon atoms as defined above, wherein some or all of the hydrogen atoms in these groups may be replaced by halogen atoms as mentioned above. A preferred embodiment of a C₁-C₆-haloalkyl is a C₁-C₂-haloalkyl. Representative C₁-C₂-haloalkyl groups include chloromethyl, bromomethyl, dichloromethyl, trichloromethyl, fluoromethyl, difluoromethyl, trifluoromethyl, chlorofluoromethyl, dichlorofluoromethyl, chlorodifluoromethyl, 1-chloroethyl, 1-bromoethyl, 1-fluoroethyl, 2-fluoroethyl, 2,2-difluoroethyl, 2,2,2-trifluoroethyl, 2-chloro-2-fluoroethyl, 2-chloro-2,2-difluoroethyl, 2,2-dichloro-2-fluoroethyl, 2,2,2-trichloroethyl or pentafluoroethyl.

The term "C₁-C₆-hydroxyalkyl" refers to an alkyl group having 1 or 6 carbon atoms as defined above, wherein one or more of the hydrogen atoms in said alkyl group is replaced by an OH group. Representative C₁-C₆-hydroxyalkyl groups include hydroxymethyl, hydroxyethyl, hydroxypropyl and hydroxybutyl groups, and especially hydroxymethyl, 1-hydroxyethyl, 2-hydroxyethyl, 1,2-dihydroxyethyl, 2-hydroxypropyl, 3-hydroxypropyl, 3-hydroxybutyl, 4-hydroxybutyl, 2-hydroxy-1-methylpropyl, and 1,3-dihydroxyprop-2-yl.

The term "C₂-C₆-alkenyl" refers to a straight-chain or branched unsaturated hydrocarbon radical having 2 to 6 carbon atoms and at least one double bond in any position. A preferred embodiment of a C₂-C₆-alkenyl is a C₂-C₄-alkenyl, such as ethenyl, 1-propenyl, 2-propenyl (allyl), 1-methylethenyl, 1-butenyl, 2-butenyl, 3-butenyl, 1-methyl-1-propenyl, 2-methyl-1-propenyl, 1-methyl-2-propenyl, 2-methyl-2-propenyl.

The term "C₂-C₆-alkynyl" refers to a straight-chain or branched unsaturated hydrocarbon radical having 2 to 6 carbon atoms and containing at least one triple bond. A preferred embodiment of a C₂-C₆-alkynyl is a C₂-C₄-alkynyl, such as ethynyl, prop-1-ynyl (-C≡C-CH₃), prop-2-ynyl (propargyl), but-1-ynyl, but-2-ynyl, but-3-ynyl, 1-methyl-prop-2-ynyl.

The term "C₃-C₈-cycloalkyl" refers to monocyclic saturated hydrocarbon radicals having 3 to 8 carbon ring members, such as cyclopropyl, cyclobutyl, cyclopentyl, cyclohexyl, cycloheptyl or cyclooctyl.

The term "C₃-C₈-cycloalkyl-C₁-C₄-alkyl" refers to alkyl having 1 to 4 carbon atoms (as defined above), wherein one hydrogen atom of the alkyl radical is replaced by a cycloalkyl radical having 3 to 8 carbon atoms (as defined above).

The term "C₃-C₈-halogencycloalkyl" refers to monocyclic saturated hydrocarbon radicals having 3 to 8 carbon ring members as defined above, wherein some or all of the hydrogen atoms in these groups may be replaced by halogen atoms as mentioned above.

The term "C₃-C₈-cycloalkyloxy" refers to monocyclic saturated hydrocarbon radicals having 3 to 8 carbon ring members as defined above attached to a terminal oxygen atom, i.e., the moiety -O-C₃-C₈-cycloalkyl.

The term "C₁-C₄-alkoxy" refers to a straight-chain or branched alkyl group having 1 to 4 carbon atoms which is bonded via an oxygen at any position in the alkyl group. Examples of "C₁-C₄-alkoxy" groups are methoxy, ethoxy, n-propoxy, 1-methylethoxy, butoxy, 1-methyl¬propoxy, 2-methylpropoxy or 1,1-dimethylethoxy.

The term "C₁-C₄-haloalkoxy" or "C₁-C₄-halogenalkoxy" refers to a C₁-C₄-alkoxy radical as defined above, wherein some or all of the hydrogen atoms in these groups may be replaced by halogen atoms as mentioned above. Examples of C₁-C₄-haloalkoxy groups include substituents such as OCH₂F, OCHF₂, OCF₃, OCH₂Cl, OCHCl₂, OCCl₃, chlorofluoromethoxy, dichlorofluoromethoxy, chlorodifluoromethoxy, 2-fluoroethoxy, 2-chloroethoxy, 2-bromoethoxy, 2-iodoethoxy, 2,2-difluoroethoxy, 2,2,2-trifluoroethoxy, 2-chloro-2-fluoroethoxy, 2-chloro-2,2-difluoroethoxy, 2,2-dichloro-2-fluoroethoxy, 2,2,2-trichloro¬ethoxy, OC₂F₅, 2-fluoropropoxy, 3-fluoropropoxy, 2,2-difluoropropoxy, 2,3-difluoro¬propoxy, 2 chloropropoxy, 3-chloropropoxy, 2,3-dichloropropoxy, 2-bromo¬propoxy, 3 bromopropoxy, 3,3,3-trifluoropropoxy, 3,3,3-trichloropropoxy, OCH₂-C₂F₅, OCF₂-C₂F₅, 1-fluoromethyl-2-fluoroethoxy, 1-chloromethyl-2-chloroethoxy, 1-bromomethyl-2-bromo¬ethoxy, 4-fluorobutoxy, 4-chlorobutoxy, 4-bromobutoxy or nonafluorobutoxy.

The term "C₂-C₄-alkenyloxy" refers to a straight-chain or branched alkenyl group having 2 to 4 carbon atoms which is bonded via an oxygen at any position in the alkenyl group. Examples of "C₂-C₄-alkenyloxy" groups are ethenyloxy, 1-propenyloxy, 2-propenyloxy, 1-methylethenyloxy, 1-butenyloxy, 2-butenyloxy, 3-butenyloxy, 1-methyl-1-propenyloxy, 2-methyl-1-propenyloxy, 1-methyl-2-propenyloxy, or 2-methyl-2-propenyloxy.

The term "C₁-C₄-alkoxy-C₁-C₄-alkyl" refers to alkyl having 1 to 4 carbon atoms (as defined above), wherein one hydrogen atom of the alkyl radical is replaced by a C₁-C₄-alkoxy group (as defined above).

The term "C(=O)-O-C₁-C₄-alkyl" refers to an ester radical which is attached through the carbon atom of the group C(=O).

The term "aliphatic" or "aliphatic group" is to be understood to refer to a non-cyclic compound, substituent or residue composed of hydrogen and carbon atoms only, and it may be saturated or unsaturated, as well as linear or branched. An aliphatic compound, substituent or residue is non-aromatic and does not comprise any possibly given substitutions of the hydrogen atoms, however, it may be optionally substituted where indicated. Examples of an aliphatic compound, substituent or residue comprises alkyl, alkenyl, and alkynyl, all with a variable number of carbon atoms, but does not include hydrogen itself.

The term "cyclo-aliphatic" or "cyclo-aliphatic group" is to be understood to refer to a cyclic compound, substituent or residue composed of hydrogen and carbon atoms only, and it may be saturated or unsaturated. A cyclo-aliphatic compound, substituent or residue is non-aromatic and does not comprise any possibly given substitutions of the hydrogen atoms, however, it may be optionally substituted where indicated. Examples of a cyclo-aliphatic compound, substituent or residue comprises cycloalkyl, cycloalkenyl and cycloalkynyl, all with a variable number of carbon atoms, but does not include hydrogen itself.

The term "aryl" is to be understood to include mono-, bi- or tricyclic aromatic radicals having usually from 6 to 14, preferably 6, 10 or 14 carbon atoms. Exemplary aryl groups include phenyl, naphthyl, phenanthryl, anthracenyl, indenyl, azulenyl, biphenyl, biphenylenyl, and fluorenyl groups, more preferably phenyl, naphthyl, and biphenyl groups. Phenyl is preferred as aryl group.

The term "heteroaryl" is to be understood to include monocyclic and bicyclic aromatic ring systems having 5, 6, 7, 8, 9, 10, 11, 12, 13 or 14 ring atoms (*i.e.*, C₅-C₁₄ heteroaryl) and 1, 2, 3, or 4 heteroatoms independently chosen from oxygen, nitrogen and sulfur. In one embodiment, the heteroaryl has three heteroatoms. In another embodiment, the heteroaryl has two heteroatoms. In another embodiment, the heteroaryl has one heteroatom. In one embodiment, the heteroaryl is a C₅ heteroaryl. In another embodiment, the heteroaryl is a C₆ heteroaryl. Non-limiting exemplary heteroaryl groups include thienyl, benzo[b]thienyl, naphtho[2,3-b]thienyl, thianthrenyl, furyl, benzofuryl, pyranyl, isobenzofuranyl, benzooxazonyl, chromenyl, xanthenyl, 2*H*-pyrrolyl, pyrrolyl, imidazolyl, pyrazolyl, pyridyl, pyrazinyl, pyrimidinyl, pyridazinyl, isoindolyl, 3*H*-indolyl, indolyl, indazolyl, purinyl, isoquinolyl, quinolyl, phthalazinyl, naphthyridinyl, cinnolinyl, quinazolinyl, pteridinyl, 4a*H*-carbazolyl, carbazolyl, β-carbolinyl, phenanthridinyl, acridinyl, pyrimidinyl, phenanthrolinyl, phenazinyl, thiazolyl, isothiazolyl, phenothiazolyl, isoxazolyl, furazanyl, and phenoxazinyl. In one embodiment, the heteroaryl is chosen from thienyl (e.g., thien-2-yl and thien-3-yl), furyl *(e.g.,* 2-furyl and 3-furyl), pyrrolyl *(e.g.,* 1 H-pyrrol-2-yl and 1 H-pyrrol-3-yl), imidazolyl *(e.g.,* 2H-imidazol-2-yl and 2H-imidazol-4-yl), pyrazolyl *(e.g.,* 1 H-pyrazol-3-yl, 1 H-pyrazol-4-yl, and 1 H-pyrazol-5-yl), pyridyl (*e.g*., pyridin-2-yl, pyridin-3-yl, and pyridin-4-yl), pyrimidinyl *(e.g.,* pyrimidin-2-yl, pyrimidin-4-yl, and pyrimidin-5-yl), thiazolyl *(e.g.,* thiazol-2-yl, thiazol-4-yl, and thiazol-5-yl), isothiazolyl (*e.g*., isothiazol-3-yl, isothiazol-4-yl, and isothiazol-5-yl), oxazolyl *(e.g.,* oxazol-2-yl, oxazol-4-yl, and oxazol-5-yl) and isoxazolyl *(e.g.,* isoxazol-3-yl, isoxazol-4-yl, and isoxazol-5-yl). The term "heteroaryl" is also meant to include possible N-oxides. Exemplary N-oxides include pyridyl N-oxide and the like.

If any of the variables is optionally substituted, it is understood that this applies to moieties containing carbon-hydrogen bonds, wherein the hydrogen atom is substituted by the corresponding substituent, however, not to moieties such as hydrogen, halogen, CN or the like. As an exemplary embodiment, if methyl is substituted by OH, a hydroxymethyl group is generated.

Agriculturally acceptable salts of the inventive compounds encompass especially the salts of those cations or the acid addition salts of those acids whose cations and anions, respectively, have no adverse effect on the fungicidal action of said compounds. Suitable cations are thus in particular the ions of the alkali metals, preferably sodium and potassium, of the alkaline earth metals, preferably calcium, magnesium and barium, of the transition metals, preferably manganese, copper, zinc and iron, and also the ammonium ion which, if desired, may carry one to four C₁-C₄-alkyl substituents and/or one phenyl or benzyl substituent, preferably diisopropylammonium, tetramethylammonium, tetrabutylammonium, trimethylbenzylammonium, furthermore phosphonium ions, sulfonium ions, preferably tri(C₁-C₄-alkyl)sulfonium, and sulfoxonium ions, preferably tri(C₁-C₄-alkyl)sulfoxonium. Anions of useful acid addition salts are primarily chloride, bromide, fluoride, hydrogensulfate, sulfate, dihydrogenphosphate, hydrogenphosphate, phosphate, nitrate, bicarbonate, carbonate, hexafluorosilicate, hexafluorophosphate, benzoate, and the anions of C₁-C₄-alkanoic acids, preferably formate, acetate, propionate and butyrate. They can be formed by reacting such inventive compound with an acid of the corresponding anion, preferably of hydrochloric acid, hydrobromic acid, sulfuric acid, phosphoric acid or nitric acid.

The inventive compounds can be present in atropisomers arising from restricted rotation about a single bond of asymmetric groups. They also form part of the subject matter of the present invention.

If the synthesis yields mixtures of isomers, a separation is generally not necessarily required since in some cases the individual isomers can be interconverted during work-up for use or during application (e. g. under the action of light, acids or bases). Such conversions may also take place after use, e. g. in the treatment of plants in the treated plant, or in the harmful fungus to be controlled. All different types of isomers are comprised by the compounds of formula I, in particular enantiomers, diasteriomers or geometric isomers, and they all form part of the subject matter of the present invention.

Depending on the substitution pattern, the compounds of formula I and their N-oxides may have one or more centers of chirality, in which case they are present as pure enantiomers or pure diastereomers or as enantiomer or diastereomer mixtures. Both, the pure enantiomers or diastereomers and their mixtures are subject matter of the present invention.

In the following, particular embodiments of the inventive compounds are described. Therein, specific meanings of the respective substituents are further detailled, wherein the meanings are in each case on their own but also in any combination with one another, particular embodiments of the present invention.

Furthermore, in respect of the variables, generally, the embodiments of the compounds of formula I also apply to the intermediates.

R¹ according to the invention is C₁-C₆-alkyl, C₂-C₆-alkenyl, C₂-C₆-alkynyl or C₃-C₆-cycloalkyl; wherein the aliphatic moieties of R¹ are not further substituted or carry one, two, three or up to the maximum possible number of identical or different groups R^{1a} which independently of one another are selected from halogen, OH, CN, C₁-C₄-alkoxy, C₃-C₆-cycloalkyl, C₃-C₈-halogencycloalkyl and C₁-C₄-halogenalkoxy; and wherein the cycloalkyl moieties of R¹ are not further substituted or carry one, two, three, four, five or up to the maximum number of identical or different groups R^{1b} which independently of one another are selected from halogen, OH, CN, C₁-C₄-alkyl, C₁-C₄-alkoxy, C₁-C₄-halogenalkyl, C₃-C₆-cycloalkyl, C₃-C₆-halogencycloalkyl and C₁-C₄-halogenalkoxy.

According to one particular embodiment, R¹ is C₁-C₆-alkyl, in particular C₁-C₄-alkyl, such as CH₃ (methyl), C₂H₅ (ethyl), CH₂CH₂CH₃ (n-propyl), CH(CH₃)₂ (iso-propyl), CH₂CH(CH₃)₂ (iso-butly) or C(CH₃)₃ (tert-butyl). A further embodiment relates to compounds, wherein R¹ is C₁-C₃-alkyl, in particular CH₃, C₂H₅ or n-C₃H₇. A further embodiment relates to compounds, wherein R¹ is C₁-C₆-alkyl, in particular C₁-C₄-alkyl or C₁-C₃-alkyl, that is substituted by one, two or three or up to the maximum possible number of identical or different groups R^{1a}, as defined and preferably defined herein. According to a specific embodiment thereof, R¹ is C₁-C₆-halogenalkyl, in particular C₁-C₄-halogenalkyl or C₁-C₃-halogenalkyl, more particularly C₁-C₂-halogenalkyl such as CF₃ or CHF₂, CF₂CH₃, CH₂CF₃ or CF₂CF₃. According to a further specific embodiment thereof, R¹ is C₁-C₄-alkoxy-C₁-C₆-alkyl, in particular C₁-C₄-alkoxy-C₁-C₄-alkyl, such as CH₂-OCH₃. Further specific embodiments thereof can be found in the below Table P1.

According to still another embodiment, R¹ is C₃-C₆-cycloalkyl-C₁-C₆-alkyl, in particular C₃-C₆-cycloalkyl-C₁-C₄-alkyl, i.e., C₁-C₆-alkyl substituted by R^{1a} selected as C₃-C₆-cycloalkyl. A further embodiment relates to compounds, wherein R¹ is C₃-C₆-cycloalkyl-C₁-C₆-alkyl, in particular C₃-C₆-cycloalkyl-C₁-C₄-alkyl, that is substituted by one, two or three or up to the maximum possible number of identical or different groups R^{1a} in the alkyl moiety and/or substituted by one, two, three four or five or up to the maximum possible number of identical or different groups R^{1b} in the cycloalkyl moiety. R^{1a} are in each case as defined and preferably defined herein. Specific embodiments thereof can be found in the below Table P1.

According to another embodiment, R¹ is C₂-C₆-alkenyl, in particular C₂-C₄-alkenyl, such as CH=CH₂, CH₂CH=CH₂, CH=CHCH₃ or C(CH₃)=CH₂. A further embodiment relates to compounds, wherein R¹ is C₂-C₆-alkenyl, in particular C₂-C₄-alkenyl, that is substituted by one, two or three or up to the maximum possible number of identical or different groups R^{1a} as defined and preferably defined herein. According to a specific embodiment thereof, R¹ is C₂-C₆-halogenalkenyl, in particular C₂-C₄-halogenalkenyl. Further specific embodiments thereof can be found in the below Table P1.

According to still another embodiment, R¹ is C₂-C₆-alkynyl, in particular C₂-C₄-alkynyl, such as C≡CH, C≡CCH₃, CH₂-C≡C-H or CH₂-C≡C-CH₃.

A further embodiment relates to compounds, wherein R¹ is C₂-C₆-alkynyl, in particular C₂-C₄-alkynyl, that is substituted by one, two or three or up to the maximum possible number of identical or different groups R^{1a}, as defined and preferably defined herein. According to a specific embodiment thereof, R¹ is C₂-C₆-halogenalkynyl, in particular C₂-C₄-halogenalkynyl. According to a further specific embodiment thereof, R¹ is C₃-C₆-cycloalkyl-C₂-C₆-alkynyl or C₃-C₆-halogen-cycloalkyl-C₂-C₆-alkynyl, in particular C₃-C₆-cycloalkyl-C₂-C₄-alkynyl or C₃-C₆-halogencycloalkyl-C₂-C₄-alkynyl. Further specific embodiments thereof can be found in the below Table P1.

According to still another embodiment, R¹ is C₃-C₆-cycloalkyl, such as C₃H₅ (cyclopropyl), C₄H₇ (cyclobutyl), cyclopentyl or cyclohexyl. A further embodiment relates to compounds, wherein R¹ is C₃-C₆-cycloalkyl, such as C₃H₅ (cyclopropyl) or C₄H₇ (cyclobutyl), that is substituted by one, two, three four or five or up to the maximum possible number of identical or different groups R^{1b} as defined and preferably defined herein. According to a specific embodiment thereof, R¹ is C₃-C₆-halogencycloalkyl, such as halogencyclopropyl, in particular 1-F-cyclopropyl or 1-Cl-cyclopropyl. According to a further specific embodiment thereof, R¹ C₃-C₆-cycloalkyl-C₃-C₆-cycloalkyl, wherein each of said cycloalkyl-cycloalkyl moieties is unsubstituted or carries one, two or three R^{1b} as defined and preferably defined herein, such as 1-cyclopropyl-cyclopropyl or 2-cyclopropyl-cyclopropyl. Specific embodiments thereof can be found in the below Table P1. Specifically, it may be preferred, according to one particular embodiment, if R¹ is selected from C₁-C₄-alkyl, such as methyl, ethyl, n-propyl, iso-propyl, tert-butyl, CH₂C(CH₃)₃ and CH₂CH(CH₃)₂ more particularly methyl, ethyl, n-propyl, CH₂C(CH₃)₃ and CH₂CH(CH₃)₂, C₁-C₄-halogenalkyl, such as CF₃, C₂-C₆-alkenyl, C₂-C₆-alkynyl, such as -C≡CCH₃, and unsubstituted C₃-C₆-cycloalkyl, such as cyclopropyl, or substituted C₃-C₆-cycloalkyl, such as 1-fluor-cyclopropyl and 1-chloro-cyclopropyl.

In one further particular embodiment, R¹ is selected from methyl, ethyl, n-propyl, iso-propyl, CH₂C(CH₃)₃, CH₂CH(CH₃)₂, CF₃, C₂-C₆-alkenyl, C₂-C₆-alkynyl, in particular -C≡C-CH₃, unsubstituted C₃-C₆-cycloalkyl, in particular cyclopropyl, and substituted C₃-C₆-cycloalkyl, in particular 1-F-cyclopropyl and 1-Cl-cyclopropyl.

Specifically, it may further be preferred, according to a further particular embodiment, if R¹ is selected from C₁-C₃-alkyl, such as methyl, ethyl, n-propyl and iso-propyl, more specifically methyl, ethyl and n-propyl, C₁-C₃-halogenalkyl, such as CF₃, C₂-C₄-alkenyl, C₂-C₄-alkynyl, such as -C≡CCH₃, and C₃-C₆-cycloalkyl, such as cyclopropyl.

More specifically, it may be preferred, according to a further particular embodiment, if R¹ is selected from C₁-C₃-alkyl, selected from methyl, ethyl and n-propyl, C₁-C₃-halogenalkyl, C₂-C₄-alkenyl, C₂-C₄-alkynyl and C₃-C₆-cycloalkyl.

In another specific embodiment, R¹ is not cyclopropyl, or R¹ is not C₃-C₆-cycloalkyl.

Particularly preferred embodiments of R¹ according to the invention are in Table P1 below, wherein each line of lines P1-1 to P1-139 corresponds to one particular embodiment of the invention, wherein P1-1 to P1-139 are also in any combination a preferred embodiment of the present invention.

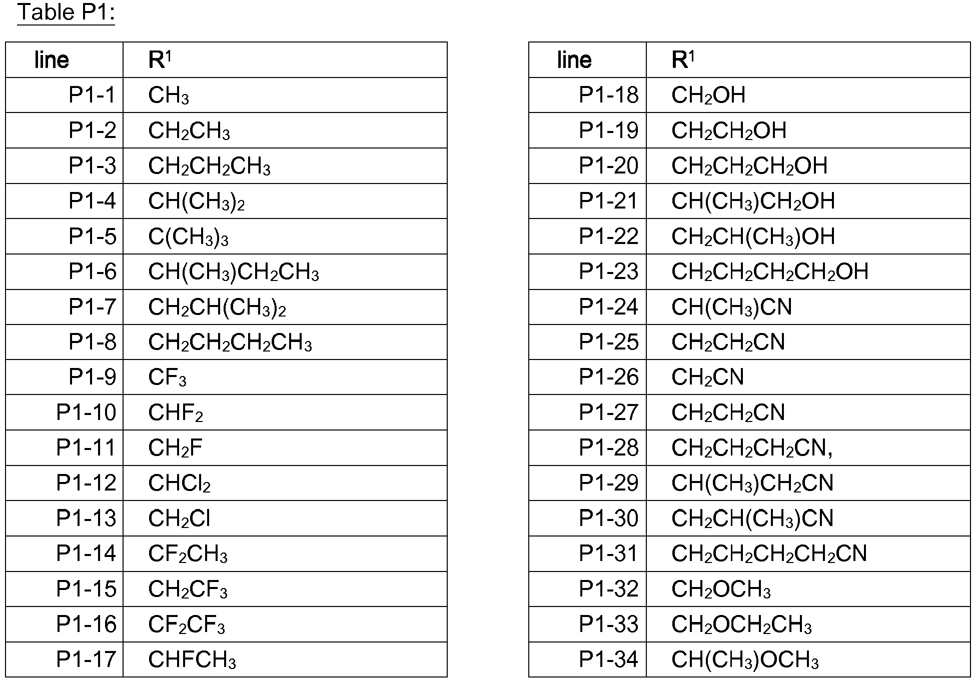

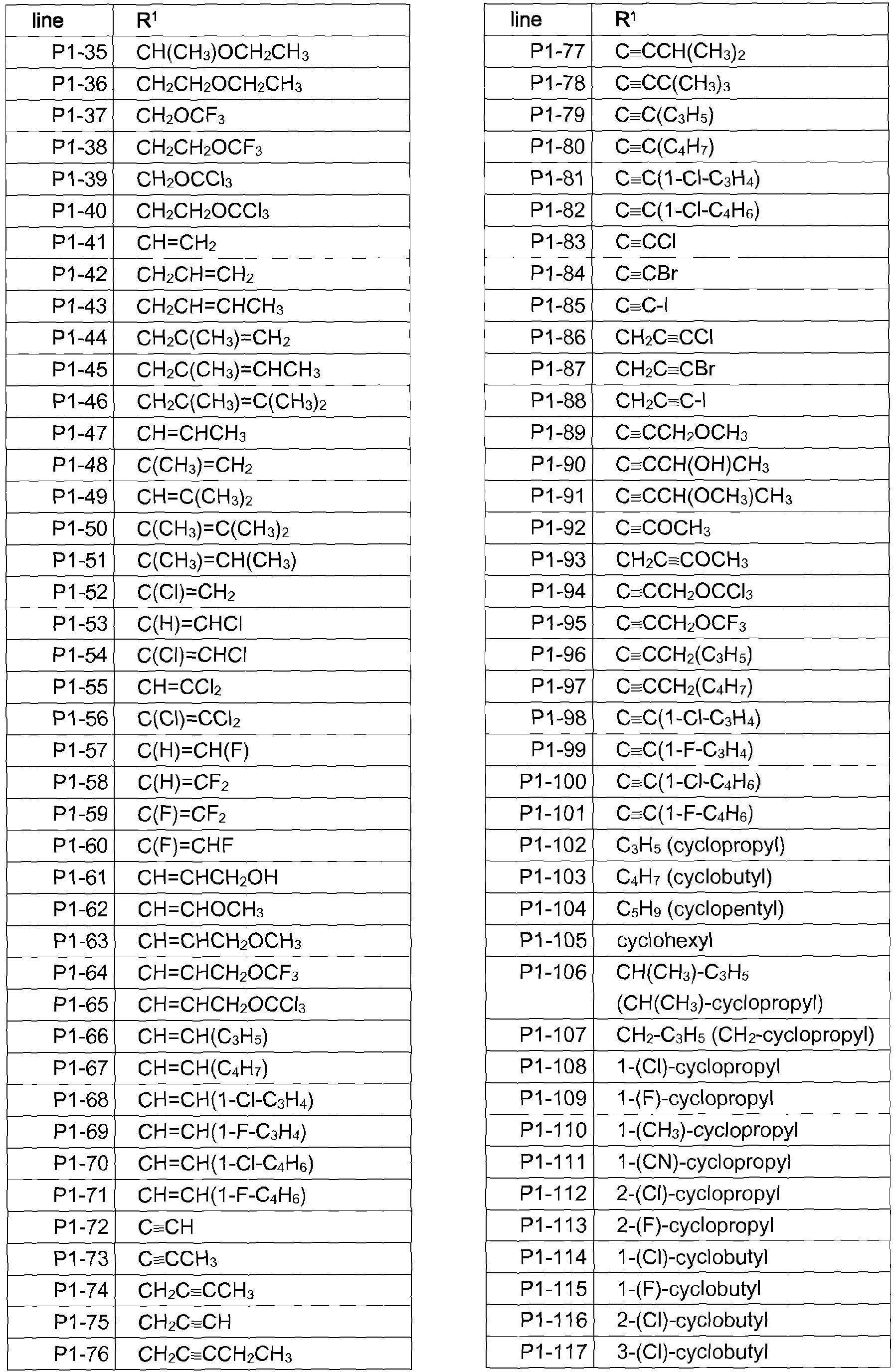

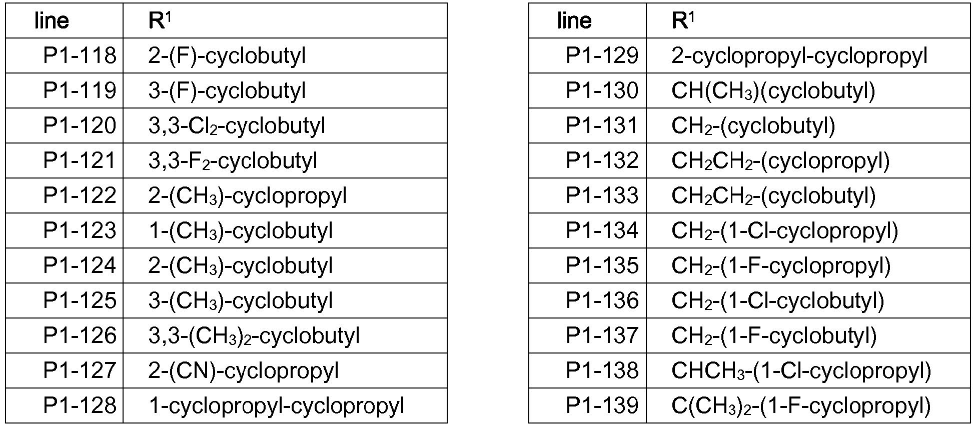

R^{1a} are the possible substituents for the aliphatic moieties of R¹.

R^{1a} according to the invention is independently selected from halogen, OH, CN, C₁-C₄-alkoxy, C₃-C₆-cycloalkyl, C₃-C₆-halocycloalkyl and C₁-C₄-halogenalkoxy.

According to one embodiment R^{1a} is independently selected from halogen, OH, CN, C₁-C₂-alkoxy, C₃-C₆-cycloalkyl, C₃-C₆-halocycloalkyl and C₁-C₂-halogenalkoxy. Specifically, R^{1a} is independently selected from F, Cl, OH, CN, C₁-C₂-alkoxy, cyclopropyl, 1-F-cyclopropyl, 1-Cl-cyclopropyl and C₁-C₂-halogenalkoxy.

R^{1b} are the possible substituents for the cycloalkyl moieties of R¹.

R^{1b} according to the invention is independently selected from halogen, OH, CN, C₁-C₄-alkyl, C₁-C₄-alkoxy, C₁-C₄-halogenalkyl, C₃-C₆-cycloalkyl, C₃-C₆-halocycloalkyl and C₁-C₄-halogenalkoxy.

According to one embodiment thereof R^{1b} is independently selected from halogen, CN, C₁-C₂-alkyl, C₁-C₂-alkoxy, C₁-C₂-halogenalkyl, C₃-C₆-cycloalkyl, C₃-C₆-halocycloalkyl and C₁-C₂-halogenalkoxy. Specifically, R^{1b} is independently selected from F, Cl, OH, CN, CH₃, OCH₃, cyclopropyl, 1-F-cyclopropyl, 1-Cl-cyclopropyl and halogenmethoxy.

According to the invention, R² is hydrogen, C₁-C₄-alkyl, C₂-C₄-alkenyl, C₂-C₄-alkynyl, wherein the aliphatic moieties of R² are not further substituted or carry one, two, three or up to the maximum possible number of identical or different groups R^{2a} which independently of one another are selected from halogen, OH, CN, C₁-C₄-alkoxy, C₃-C₆-cycloalkyl, C₃-C₆-halocycloalkyl and C₁-C₄-halogenalkoxy.

According to one embodiment, R² is H.

According to a further embodiment of the invention, R² is selected from C₁-C₄-alkyl, C₂-C₄-alkenyl, C₂-C₄-alkynyl.

According to a further embodiment of the invention, R² is selected from H, C₁-C₄-alkyl, in particular methyl or ethyl, C₂-C₄-alkenyl, in particular CH₂CH=CH₂, and C₂-C₄-alkynyl, in particular CH₂C≡CH. Specific embodiments thereof can be found in the below Table P2.

According to one particular embodiment, R² is C₁-C₄-alkyl, such as CH₃, C₂H₅, CH(CH₃)₂, CH₂CH₂CH₃, CH₂CH₂CH₂CH₃, CH₂CH(CH₃)₂. A further embodiment relates to compounds, wherein R² is C₁-C₄-alkyl, that is substituted by one, two or three or up to the maximum possible number of identical or different groups R^{2a}, as defined and preferably defined herein. According to a specific embodiment thereof, R² is C₁-C₄-halogenalkyl, more particularly C₁-C₂-halogenalkyl. According to a further specific embodiment thereof, R² is C₁-C₄-alkoxy-C₁-C₄-alkyl, such as CH₂OCH₃ or CH₂CH₂OCH₃. According to still a further specific embodiment thereof, R² is hydroxyl-C₁-C₄-alkyl, such as CH₂CH₂OH. Further specific embodiments thereof can be found in the below Table P2.

According to still another embodiment, R² is C₃-C₆-cycloalkyl-C₁-C₄-alkyl. A further embodiment relates to compounds, wherein R² is C₃-C₆-cycloalkyl-C₁-C₄-alkyl, more particularly C₃-C₆-cycloalkyl-C₁-C₂-alkyl, that is substituted by one, two or three or up to the maximum possible number of identical or different groups R^{2a}. A further embodiment relates to compounds, wherein R² is C₃-C₆-halocycloalkyl-C₁-C₄-alkyl, more particularly C₃-C₆-halocycloalkyl-C₁-C₂-alkyl. Specific embodiments thereof can be found in the below Table P2.

According to another embodiment, R² is C₂-C₄-alkenyl, such as CH₂CH=CH₂, CH₂C(CH₃)=CH₂ or CH₂CH=CHCH₃. A further embodiment relates to compounds, wherein R² is C₂-C₄-alkenyl that is substituted by one, two or three or up to the maximum possible number of identical or different groups R^{2a} as defined and preferably defined herein. According to a specific embodiment thereof, R² is C₂-C₄-halogenalkenyl, such as CH₂C(Cl)=CH₂ and CH₂C(H)=CHCl. According to a further specific embodiment thereof, R² is C₃-C₆-cycloalkyl-C₂-C₄-alkenyl or C₃-C₆-halocycloalkyl-C₂-C₄-alkenyl. Further specific embodiments thereof can be found in the below Table P2.

According to still another embodiment, R² is C₂-C₄-alkynyl, such as CH₂C≡CH or CH₂C≡CCH₃. A further embodiment relates to compounds, wherein R² is C₂-C₄-alkynyl that is substituted by one, two or three or up to the maximum possible number of identical or different groups R^{2a}, as defined and preferably defined herein. According to a specific embodiment thereof, R² is C₂-C₄-halogenalkynyl. According to a further specific embodiment thereof, R² is C₃-C₆-cycloalkyl-C₂-C₄-alkynyl or C₃-C₆-halocycloalkyl-C₂-C₄-alkynyl. Specific embodiments thereof can be found in the below Table P2.

Particularly preferred embodiments of R² according to the invention are in Table P2 below, wherein each line of lines P2-1 to P2-79 corresponds to one particular embodiment of the invention, wherein P2-1 to P2-79 are also in any combination a preferred embodiment of the present invention.

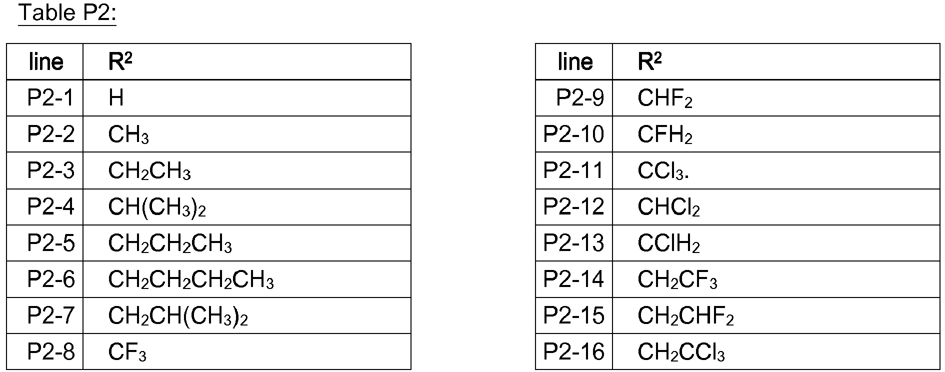

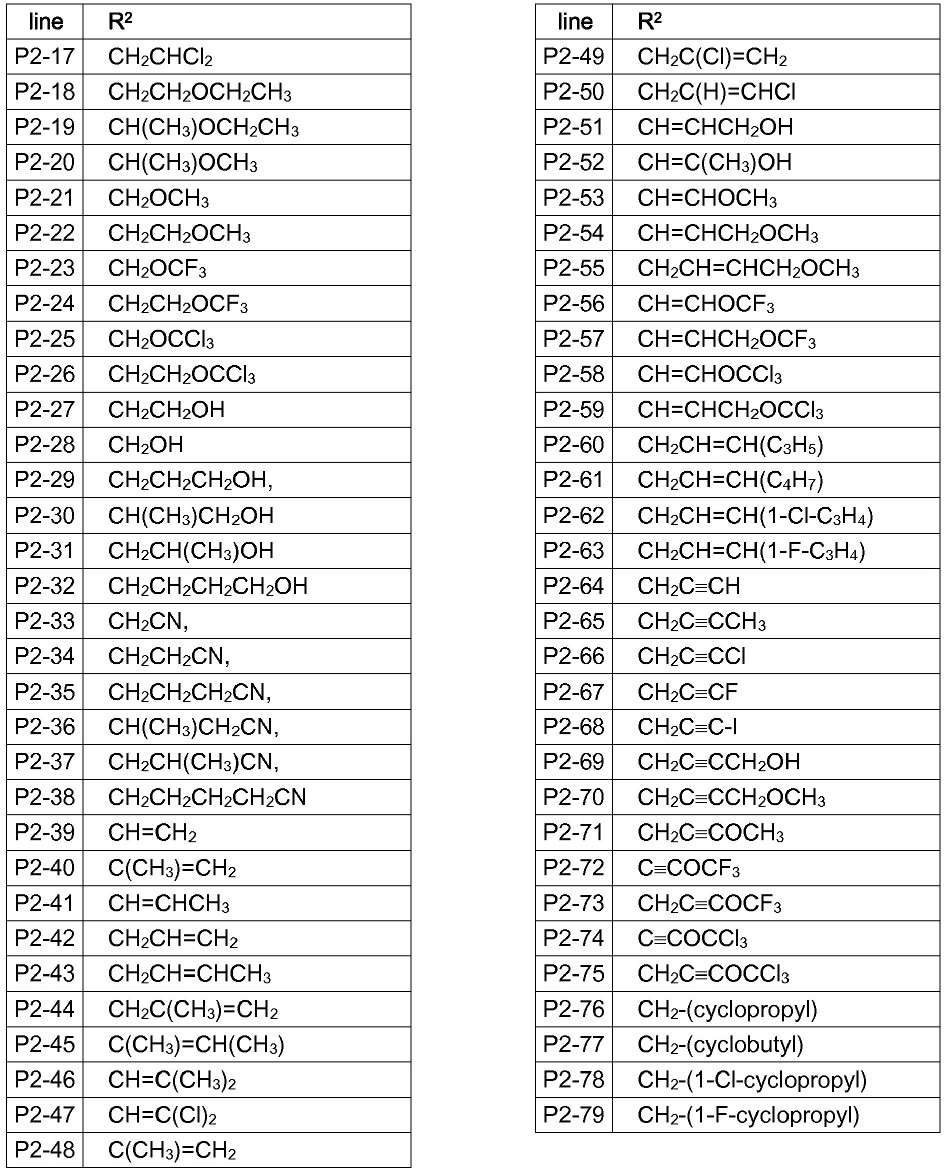

R³ according to the present invention is selected from hydrogen, halogen, CN, C₁-C₄-alkyl, C₁-C₄-alkoxy, C₂-C₄-alkenyl, C₂-C₄-alkynyl, C₃-C₆-cycloalkyl and S(O)ₚ(C₁-C₄-alkyl), wherein each of R³ is unsubstituted or further substituted by one, two, three or four R^{3a}; wherein R^{3a} is independently selected from halogen, CN, OH, C₁-C₄-alkyl, C₁-C₄-halogenalkyl, C₃-C₆-cycloalkyl, C₃-C₆-halogencycloalkyl, C₁-C₄-alkoxy and C₁-C₄-halogenalkoxy, and wherein p is 0, 1 or 2.

R³ according one embodiment is hydrogen.

R³ according to a further embodiment is selected from halogen, CN, C₁-C₄-alkyl, C₁-C₄-alkoxy, C₂-C₄-alkenyl, C₂-C₄-alkynyl, C₃-C₆-cycloalkyl and S(O)ₚ(C₁-C₄-alkyl), wherein each of R³ is unsubstituted or further substituted by one, two, three or four R^{3a}; wherein R^{3a} is independently selected from halogen, CN, OH, C₁-C₄-alkyl, C₁-C₄-halogenalkyl, C₃-C₆-cycloalkyl, C₃-C₆-halogencycloalkyl, C₁-C₄-alkoxy and C₁-C₄-halogenalkoxy, and wherein p is 0, 1 or 2.

According to a further embodiment, R³ is selected from H, F, Cl, Br, CN, C₁-C₄-alkyl, C₁-C₄-halogenalkyl, C₁-C₄-alkoxy, C₁-C₄-halogenalkoxy, S(C₁-C₄-alkyl), S(O)(C₁-C₄-alkyl) and S(O)₂(C₁-C₄-alkyl).

According to still a further embodiment, R³ is selected from F, Cl, Br, CN, C₁-C₄-alkyl, C₁-C₄-halogenalkyl, C₁-C₄-alkoxy, C₁-C₄-halogenalkoxy, S(C₁-C₄-alkyl), S(O)(C₁-C₄-alkyl) and S(O)₂(C₁-C₄-alkyl).

According to a further embodiment, R³ is selected from H, Cl, F, Br, CN, C₁-C₂-alkyl, in particular H, CH₃, C₁-C₂-halogenalkyl, in particular H, CF₃, C₁-C₂-alkoxy, in particular OCH₃, and C₁-C₂-halogenalkoxy, in particular OCF₃.

According to still a further embodiment, R³ is selected from Cl, F, Br, C₁-C₂-alkyl, in particular CH₃, C₁-C₂-halogenalkyl, in particular CF₃, C₁-C₂-alkoxy, in particular OCH₃, and C₁-C₂-halogenalkoxy, in particular OCF₃.

According to a further embodiment, R³ is selected from H, C₂-C₄-alkenyl, C₂-C₄-halogenalkenyl, C₂-C₄-alkynyl and C₂-C₄-halogenalkynyl. According to one particular embodiment, R³ is H, C₂-C₄-alkenyl or C₂-C₄-halogenalkenyl, such as H or CH=CH₂. According to a further particular embodiment, R³ is H, C₂-C₄-alkynyl or C₂-C₄-halogenalkynyl, such as H or CΞCH

According to still a further embodiment, R³ is selected from C₂-C₄-alkenyl, C₂-C₄-halogenalkenyl, C₂-C₄-alkynyl and C₂-C₄-halogenalkynyl. According to one particular embodiment, R³ is C₂-C₄-alkenyl or C₂-C₄-halogenalkenyl, such as CH=CH₂. According to a further particular embodiment, R³ is C₂-C₄-alkynyl or C₂-C₄-halogenalkynyl, such as CΞCH.

According to a further embodiment, R³ is selected from H, C₃-C₆-cycloalkyl and C₃-C₆-halogencycloalkyl.

According to still a further embodiment, R³ is selected from C₃-C₆-cycloalkyl and C₃-C₆-halogencycloalkyl.

According to a further embodiment, R³ is selected from H, S(C₁-C₂-alkyl), S(O)(C₁-C₂-alkyl) and S(O)₂(C₁-C₂-alkyl). According to a particular embodiment thereof, R³ is selected from H, SCH₃, S(O)(CH₃) and S(O)₂(CH₃).

According to still a further embodiment, R³ is selected from S(C₁-C₂-alkyl), S(O)(C₁-C₂-alkyl) and S(O)₂(C₁-C₂-alkyl). According to a particular embodiment thereof, R³ is selected from SCH₃, S(O)(CH₃) and S(O)₂(CH₃).

According to one specific embodiment, R³ is H or halogen, in particular H, Br, F or Cl, more specifically H, F or Cl.

According to a further specific embodiment, R³ is halogen, in particular Br, F or Cl, more specifically F or Cl.

According to a further specific embodiment, R³ is H or CN.

According to still a further specific embodiment, R³ is CN.

According to still a further specific embodiment, R³ is H, C₁-C₄-alkyl, such as CH₃, or C₁-C₄-halogenalkyl, such as CF₃, CHF₂, CH₂F, CCl₃, CHCl₂ or CH₂Cl.

According to still a further specific embodiment, R³ is C₁-C₄-alkyl, such as CH₃, or C₁-C₄-halogenalkyl, such as CF₃, CHF₂, CH₂F, CCl₃, CHCl₂ or CH₂Cl.

According to a further specific embodiment, R³ is H, C₁-C₄-alkoxy, more specifically C₁-C₂-alkoxy such as OCH₃ or OCH₂CH₃, or C₁-C₄-halogenalkoxy, more specifically C₁-C₂-halogenalkoxy such as OCF₃, OCHF₂, OCH₂F, OCCl₃, OCHCl₂ or OCH₂Cl, in particular OCF₃, OCHF₂, OCCl₃ or OCHCl₂.

According to a further specific embodiment, R³ is C₁-C₄-alkoxy, more specifically C₁-C₂-alkoxy such as OCH₃ or OCH₂CH₃, or C₁-C₄-halogenalkoxy, more specifically C₁-C₂-halogenalkoxy such as OCF₃, OCHF₂, OCH₂F, OCCl₃, OCHCl₂ or OCH₂Cl, in particular OCF₃, OCHF₂, OCCl₃ or OCHCl₂.

In another specific embodiment, R³ is not H.

R^{3a} is selected from halogen, CN, OH, C₁-C₄-alkyl, C₁-C₄-halogenalkyl, C₃-C₆-cycloalkyl, C₃-C₈-halogencycloalkyl, C₁-C₄-alkoxy and C₁-C₄-halogenalkoxy, in particular selected from halogen, CN, C₁-C₂-alkyl, C₁-C₂-halogenalkyl, C₃-C₆-cycloalkyl, C₃-C₆-halogencycloalkyl, C₁-C₂-alkoxy and C₁-C₂-halogenalkoxy. Specifically, R^{3a} is independently selected from F, Cl, CN, OH, CH₃, halogenmethyl, cyclopropyl, halogencyclopropyl, OCH₃ and halogenmethoxy.

Particularly preferred embodiments of R³ according to the invention are in Table P3 below, wherein each line of lines P3-1 to P3-16 corresponds to one particular embodiment of the invention, wherein P3-1 to P3-16 are also in any combination with one another a preferred embodiment of the present invention. Thereby, for every R³ that is present in the inventive compounds, these specific embodiments and preferences apply independently of the meaning of any other R³ that may be present in the phenyl ring:

**Table P3:**

| No. | R³ | | No. | R³ | | No. | R³ |
|---|---|---|---|---|---|---|---|
| P3-1 | H | | P3-7 | CF₃ | | P3-13 | SCH₃ |
| P3-2 | Cl | | P3-8 | CHF₂ | | P3-14 | SOCH₃ |
| P3-3 | F | | P3-9 | OCH₃ | | P3-15 | SO₂CH₃ |
| P3-4 | CN | | P3-10 | OCH₂CH₃ | | P3-16 | Br |
| P3-5 | CH₃ | | P3-11 | OCF₃ | | | |
| P3-6 | CH₂CH₃ | | P3-12 | OCHF₂ | | | |

R⁴, R⁵, and R⁶ according to the invention are independently of one another selected from hydrogen, halogen, CN, -OCN, -SCN, -S(O)ₘ, -S(O)ₒ(C₁-C₄-alkyl), C₁-C₆-alkyl, C₂-C₆-alkenyl, C₂-C₆-alkynyl, C₁-C₄-alkoxy, C₂-C₄-alkenyloxy, C₃-C₆-cycloalkyl, C₃-C₈-cycloalkyl-C₁-C₄-alkyl, C₃-C₆-halogencycloalkyl, aryl, heteroaryl and aryloxy; or R⁴ and R⁵ together are =O, and R⁶ is as defined above; or R⁴ and R⁵ together are =NORⁿ, and R⁶ is as defined above; or R⁴⁻⁶ together are ≡N; wherein Rⁿ is C₁-C₄-alkyl, C₂-C₄-alkenyl or C₂-C₄-alkynyl; wherein m is independently 0, 1 or 2; wherein o is independently 0, 1 or 2; wherein the aliphatic moieties of R⁴, R⁵, and R⁶ are unsubstituted or further substituted by one, two, three, or up to the maximum possible number of identical or different groups R^{a} which independently of one another are selected from halogen, OH, CN, C₁-C₄-alkyl, C₁-C₄-halogenalkyl, C₃-C₆-cycloalkyl, C₃-C₆-halogencycloalkyl, C₁-C₄-alkoxy, C₁-C₄-halogenalkoxy and Si(R^{s})₃, wherein R^{s} is independently of one another C₁-C₄-alkyl; wherein the cycloalkyl moieties of R⁴, R⁵, and R⁶ are not further substituted or carry one, two, three, four, five or up to the maximum number of identical or different groups R^{b} which independently of one another are selected from halogen, OH, CN, C₁-C₄-alkyl, C₁-C₄-alkoxy, C₁-C₄-halogenalkyl, C₃-C₆-cycloalkyl, C₃-C₆-halogencycloalkyl and C₁-C₄-halogenalkoxy; and wherein the aryl, heteroaryl and aryloxy moieties of R⁴, R⁵, and R⁶ are not further substituted or carry one, two, three, four, or up to the maximum number of identical or different groups R^{c} which independently of one another are selected from halogen, OH, CN, C₁-C₄-alkyl, C₁-C₄-halogenalkyl, C₃-C₆-cycloalkyl, C₃-C₆-halogencycloalkyl, C₁-C₄-alkoxy and C₁-C₄-halogenalkoxy.

It should be noted that the selection of each of the three variables R⁴, R⁵ and R⁶ is made independent of each other, and R⁴, R⁵ and R⁶ may be identical or different. However, certain provisos also apply to the selection of R⁴, R⁵ and R⁶:
at least one of R⁴, R⁵ and R⁶ is not hydrogen;
when R² and R³ are both hydrogen, and each of R⁴, R⁵ and R⁶ is F, then R¹ is not CH(OH)CH₃, CHCH₃CH=CH₂, cyclopropanecarbonitrile, CH₂CCl₃, CF₃, CF₂CF₃;
when R² and R³ are both hydrogen, -CR⁴R⁵R⁶ is CH(CH₃)₂, then R¹ is not CHCH₃CHCH₂, C(CH₃)₂CH₂OH;
when R² and R³ are both hydrogen, -CR⁴R⁵R⁶ is CH(CH₃)₂, then R¹ is not C(CH₃)₃;
when R² and R³ are both hydrogen, each of R⁴, R⁵ and R⁶ is CH₃, then R¹ is not CH₂CCl₃, C(CH₃)₃;
when R² and R³ are both hydrogen, R⁴⁻⁵ is =O and R⁶ is OCH₃, then R¹ is not C(CH₃)₃;
when R² and R³ are both hydrogen, and R⁴⁻⁶ is ≡N, then R¹ is not C(CH₃)₃, C(CH₃)₂C(CH₃)₂OH, or CF₂C(CH₃)₂OH; and
when R² is hydrogen, R³ is Cl, and R⁴⁻⁶ is ≡N, then R¹ is not CF₂C(CH₃)₂OH.

According to one particular embodiment, R⁴, R⁵, and/or R⁶ is C₁-C₆-alkyl, in particular C₁-C₄-alkyl, such as CH₃ (methyl), C₂H₅ (ethyl), CH₂CH₂CH₃ (n-propyl), CH(CH₃)₂ (iso-propyl), CH₂CH(CH₃)₂ (iso-butly) or C(CH₃)₃ (tert-butyl). A further embodiment relates to compounds, wherein R⁴, R⁵, and/or R⁶ is C₁-C₃-alkyl, in particular CH₃, C₂H₅ or n-C₃H₇. A further embodiment relates to compounds, wherein R⁴, R⁵, and/or R⁶ is C₁-C₆-alkyl, in particular C₁-C₄-alkyl or C₁-C₃-alkyl, that is substituted by one, two or three or up to the maximum possible number of identical or different groups R^{a}, as defined and preferably defined herein. According to a specific embodiment thereof, R⁴, R⁵, and/or R⁶ is C₁-C₆-halogenalkyl, in particular C₁-C₄-halogenalkyl or C₁-C₃-halogenalkyl, more particularly C₁-C₂-halogenalkyl such as CF₃ or CHF₂, CF₂CH₃, CH₂CF₃ or CF₂CF₃. According to a further specific embodiment thereof, R⁴, R⁵, and/or R⁶ is C₁-C₄-alkoxy-C₁-C₆-alkyl, in particular C₁-C₄-alkoxy-C₁-C₄-alkyl, such as CH₂-OCH₃. Further specific embodiments thereof can be found in the below Table P4.

According to a particular embodiment, one of R⁴, R⁵, and R⁶ is methyl, ethyl or iso-propyl, preferably methyl. In another embodiment, one of R⁴, R⁵, and R⁶ is methoxy or CF₃. In still another embodiment, two of R⁴, R⁵, and R⁶ are methyl, and in another embodiment, all three residues R4, R⁵, and R⁶ are methyl.

According to still another embodiment, R⁴, R⁵, and/or R⁶ is C₃-C₆-cycloalkyl-C₁-C₆-alkyl, in particular C₃-C₆-cycloalkyl-C₁-C₄-alkyl, i.e., C₁-C₆-alkyl substituted by R^{a} selected as C₃-C₆-cycloalkyl. A further embodiment relates to compounds, wherein R⁴, R⁵, and/or R⁶ is C₃-C₆-cycloalkyl-C₁-C₆-alkyl, in particular C₃-C₆-cycloalkyl-C₁-C₄-alkyl, that is substituted by one, two or three or up to the maximum possible number of identical or different groups R^{a} in the alkyl moiety and/or substituted by one, two, three four or five or up to the maximum possible number of identical or different groups R^{b} in the cycloalkyl moiety. R^{a} and R^{b} are in each case as defined and preferably defined herein. Specific embodiments thereof can be found in the below Table P4.

According to another embodiment, R⁴, R⁵, and/or R⁶ is C₂-C₆-alkenyl, in particular C₂-C₄-alkenyl, such as CH=CH₂, CH₂CH=CH₂, CH=CHCH₃ or C(CH₃)=CH₂. A further embodiment relates to compounds, wherein R⁴, R⁵, and/or R⁶ is C₂-C₆-alkenyl, in particular C₂-C₄-alkenyl, that is substituted by one, two or three or up to the maximum possible number of identical or different groups R^{a} as defined and preferably defined herein. According to a specific embodiment thereof, R⁴, R⁵, and/or R⁶ is C₂-C₆-halogenalkenyl, in particular C₂-C₄-halogenalkenyl. Further specific embodiments thereof can be found in the below Table P4.

According to still another embodiment, R⁴, R⁵, and/or R⁶ is C₂-C₆-alkynyl, in particular C₂-C₄-alkynyl, such as C≡CH, C≡CCH₃, CH₂-C≡C-H or CH₂-C≡C-CH₃.

A further embodiment relates to compounds, wherein R⁴, R⁵, and/or R⁶ is C₂-C₆-alkynyl, in particular C₂-C₄-alkynyl, that is substituted by one, two or three or up to the maximum possible number of identical or different groups R^{a}, as defined and preferably defined herein. According to a specific embodiment thereof, R⁴, R⁵, and/or R⁶ is C₂-C₆-halogenalkynyl, in particular C₂-C₄-halogenalkynyl. According to a further specific embodiment thereof, R⁴, R⁵, and/or R⁶ is C₃-C₆-cycloalkyl-C₂-C₆-alkynyl or C₃-C₆-halogencycloalkyl-C₂-C₆-alkynyl, in particular C₃-C₆-cycloalkyl-C₂-C₄-alkynyl or C₃-C₆-halogencycloalkyl-C₂-C₄-alkynyl. In particular, R⁴, R⁵, and/or R⁶ is substituted C₂-C₄-alkynyl, such as C≡CCl, C≡CBr, C≡CSi(CH₃)₃. Further specific embodiments thereof can be found in the below Table P4.

According to still another embodiment, R⁴, R⁵, and/or R⁶ is C₃-C₆-cycloalkyl, such as C₃H₅ (cyclopropyl), C₄H₇ (cyclobutyl), cyclopentyl or cyclohexyl. In a particular embodiment, one of R⁴, R⁵, and R⁶ is cyclopropyl. A further embodiment relates to compounds, wherein R⁴, R⁵, and/or R⁶ is C₃-C₆-cycloalkyl, such as C₃H₅ (cyclopropyl) or C₄H₇ (cyclobutyl), that is substituted by one, two, three four or five or up to the maximum possible number of identical or different groups R^{b} as defined and preferably defined herein. According to a specific embodiment thereof, R⁴, R⁵, and/or R⁶ is C₃-C₆-halogencycloalkyl, such as halogencyclopropyl, in particular 1-F-cyclopropyl or 1-Cl-cyclopropyl. According to a further specific embodiment thereof, R⁴, R⁵, and R⁶ is C₃-C₆-cycloalkyl-C₃-C₆-cycloalkyl, wherein each of said cycloalkyl-cycloalkyl moieties is unsubstituted or carries one, two or three R^{b} as defined and preferably defined herein, such as 1-cyclopropyl-cyclopropyl or 2-cyclopropyl-cyclopropyl. Specific embodiments thereof can be found in the below Table P4.

In another embodiment, R⁴, R⁵, and/or R⁶ is aryl, such as phenyl, naphthyl, and or a biphenyl group. In a particular embodiment, one or two of R⁴, R⁵, and/or R⁶ is phenyl, preferably one of

R⁴, R⁵, and/or R⁶ is phenyl. A further embodiment relates to compounds, wherein R⁴, R⁵, and/or R⁶ is aryl, such as phenyl, that is substituted by one, two, three four or five or up to the maximum possible number of identical or different groups R^{c} as defined and preferably defined herein, such as phenyl substituted by F, Cl, CH₃, CF₃, CN, CO₂CH₃ or CHF₂. Specific embodiments thereof can be found in the below Table P4.

In another embodiment, R⁴, R⁵, and/or R⁶ is aryloxy, such as phenoxy, or naphthoxy. In a particular embodiment, one or two of R⁴, R⁵, and/or R⁶ is phenoxy, preferably one of R⁴, R⁵, and/or R⁶ is phenoxy. A further embodiment relates to compounds, wherein R⁴, R⁵, and/or R⁶ is aryloxy, such as phenoxy, that is substituted by one, two, three four or five or up to the maximum possible number of identical or different groups R^{c} as defined and preferably defined herein, such as phenyl substituted by F, Cl, CH₃, CF₃, CN, CO₂CH₃ or CHF₂. Specific embodiments thereof can be found in the below Table P4.

In another embodiment, R⁴, R⁵, and/or R⁶ is heteroaryl, such as pyridyl, preferably 2-pyridyl, 3-pyridly, or 4-pyridyl, oxazolyl, thiazolyl, isoxazolyl, thienyl, preferably 2-thienyl, pyrazolyl, and imidazolyl. In a particular embodiment, one or two of R⁴, R⁵, and/or R⁶ is pyridyl, preferably one of R⁴, R⁵, and/or R⁶ is pyridyl. A further embodiment relates to compounds, wherein R⁴, R⁵, and/or R⁶ is heteroaryl, such as pyridyl, preferably 2-pyridyl, 3-pyridly, or 4-pyridyl, oxazolyl, thiazolyl, isoxazolyl, thienyl, preferably 2-thienyl, pyrazolyl, and imidazolyl, that is substituted by one, two, three four or five or up to the maximum possible number of identical or different groups R^{c} as defined and preferably defined herein, such as chloro-pyridyl, fluoro-pyridyl, methyl-pyridyl. Specific embodiments thereof can be found in the below Table P4.

According to another embodiment, R⁴ and R⁵ together are =0, and R⁶ is as defined above. In the case when R⁶ is hydrogen, an aldehyde is formed, and in other cases, a ketone or ester is formed.

According to still another embodiment, R⁴ and R⁵ together are =NORⁿ, and R⁶ is as defined above, wherein Rⁿ is C₁-C₄-alkyl, C₂-C₄-alkenyl or C₂-C₄-alkynyl, preferably wherein Rⁿ is C₁-C₂-alkyl, C₂-C₃-alkenyl or C₂-C₃-alkynyl.

In a still further embodiment, R⁴⁻⁶ together are ≡N, i.e., the phenyl is substituted by a nitrile.

Specifically, it may be preferred, according to one particular embodiment, if R⁴, R⁵, and/or R⁶ is independently selected from hydrogen, halogen, such as F, Cl or Br, CN, -OCN, -SCN, -S(O)ₙ, such as S, SO or SO₂, -S(O)ₒ(C₁-C₄-alkyl), such as S-methyl, S(O)-methyl, or S(O)₂-methyl, C₁-C₄-alkyl, such as methyl, ethyl, n-propyl, iso-propyl, tert-butyl, CH₂C(CH₃)₃ and CH₂CH(CH₃)₂ more particularly methyl, ethyl, n-propyl, CH₂C(CH₃)₃ and CH₂CH(CH₃)₂, C₁-C₄-halogenalkyl, such as CF₃, C₂-C₆-alkenyl, C₂-C₆-alkynyl, such as -C≡CCH₃, unsubstituted C₃-C₆-cycloalkyl, such as cyclopropyl, substituted C₃-C₆-cycloalkyl, such as 1-fluor-cyclopropyl and 1-chloro-cyclopropyl, aryl, such as phenyl, aryloxy, such as phenoxy, or heteroaryl, such as pyridyl or furyl.

In a further preferred embodiment, R⁴, R⁵, and/or R⁶ is independently selected from hydrogen, halogen, CN, C₁-C₄-alkyl, C₁-C₄-halogenalkyl, C₁-C₄-alkoxy, C₂-C₄-alkenyl, C₂-C₄-alkynyl, unsubstituted and substituted C₃-C₆-cycloalkyl, aryl, aryloxy, or heteroaryl.

In one embodiment, (only) one of R⁴, R⁵, and/or R⁶ is hydrogen.

In another embodiment, at least one of R⁴, R⁵, and/or R⁶ is methyl, preferably only one of R⁴, R⁵, and/or R⁶ is methyl.

In one further particular embodiment, R⁴, R⁵, and/or R⁶ is independently selected from hydrogen, F, Cl, Br, CN, -OCN, -SCN, S, SO or SO₂, S-methyl, S(O)-methyl, or S(O)₂-methyl, methyl, ethyl, n-propyl, iso-propyl, CH₂C(CH₃)₃, CH₂CH(CH₃)₂, methoxy, CF₃, C₂-C₆-alkenyl, C₂-C₆-alkynyl, in particular -C≡C-CH₃, unsubstituted C₃-C₆-cycloalkyl, in particular cyclopropyl, and substituted C₃-C₆-cycloalkyl, in particular 1-fluor-cyclopropyl and 1-chloro-cyclopropyl, phenyl, phenoxy, pyridyl or furyl.

Specifically, it may further be preferred, according to a further particular embodiment, if R⁴, R⁵, and/or R⁶ is independently selected from hydrogen, halogen, such as F or Cl, CN, C₁-C₃-alkyl, such as methyl, ethyl, n-propyl and iso-propyl, more specifically methyl, ethyl and iso-propyl, C₁-C₃-alkoxy, such as methoxy, C₁-C₃-halogenalkyl, such as CF₃, C₂-C₄-alkenyl, C₂-C₄-alkynyl, such as -C≡CCH₃, C₃-C₆-cycloalkyl, such as cyclopropyl, phenyl, phenoxy, p-chlorophenoxy, pyridyl or furyl.

More specifically, it may be preferred, according to a further particular embodiment, if R⁴, R⁵, and/or R⁶ is independently selected from hydrogen, F, Cl, CN, C₁-C₃-alkyl, selected from methyl, ethyl and n-propyl, C₁-C₃-halogenalkyl, C₂-C₄-alkenyl, C₂-C₄-alkynyl and C₃-C₆-cycloalkyl, phenyl, p-chlorophenoxy or phenoxy.

According to another embodiment, at least one of R⁴, R⁵, and R⁶ is not F.

In another embodiment, at least one of R⁴, R⁵, and R⁶ is not CH₃, or at least two of R⁴, R⁵, and R⁶ is not CH₃. In still another embodiment, none of R⁴, R⁵, and R⁶ is CH₃.

In a further embodiment, when R⁴ and R⁵ together are =O, R⁶ is not OH.

Particularly preferred embodiments of R⁴, R⁵, and/or R⁶ according to the invention are in Table P4 below, wherein each line of lines P4-1 to P4-169 corresponds to one particular embodiment of the invention, wherein P4-1 to P4-169 are also in any combination a preferred embodiment of the present invention.

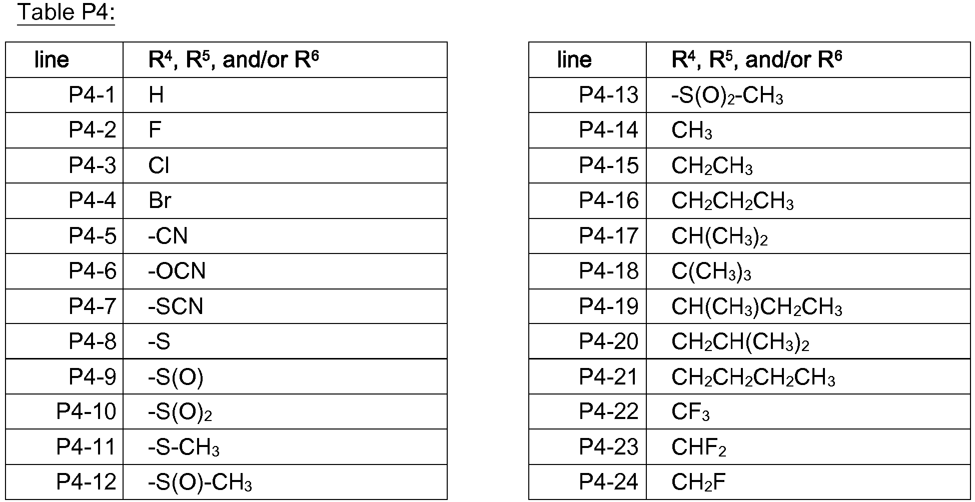

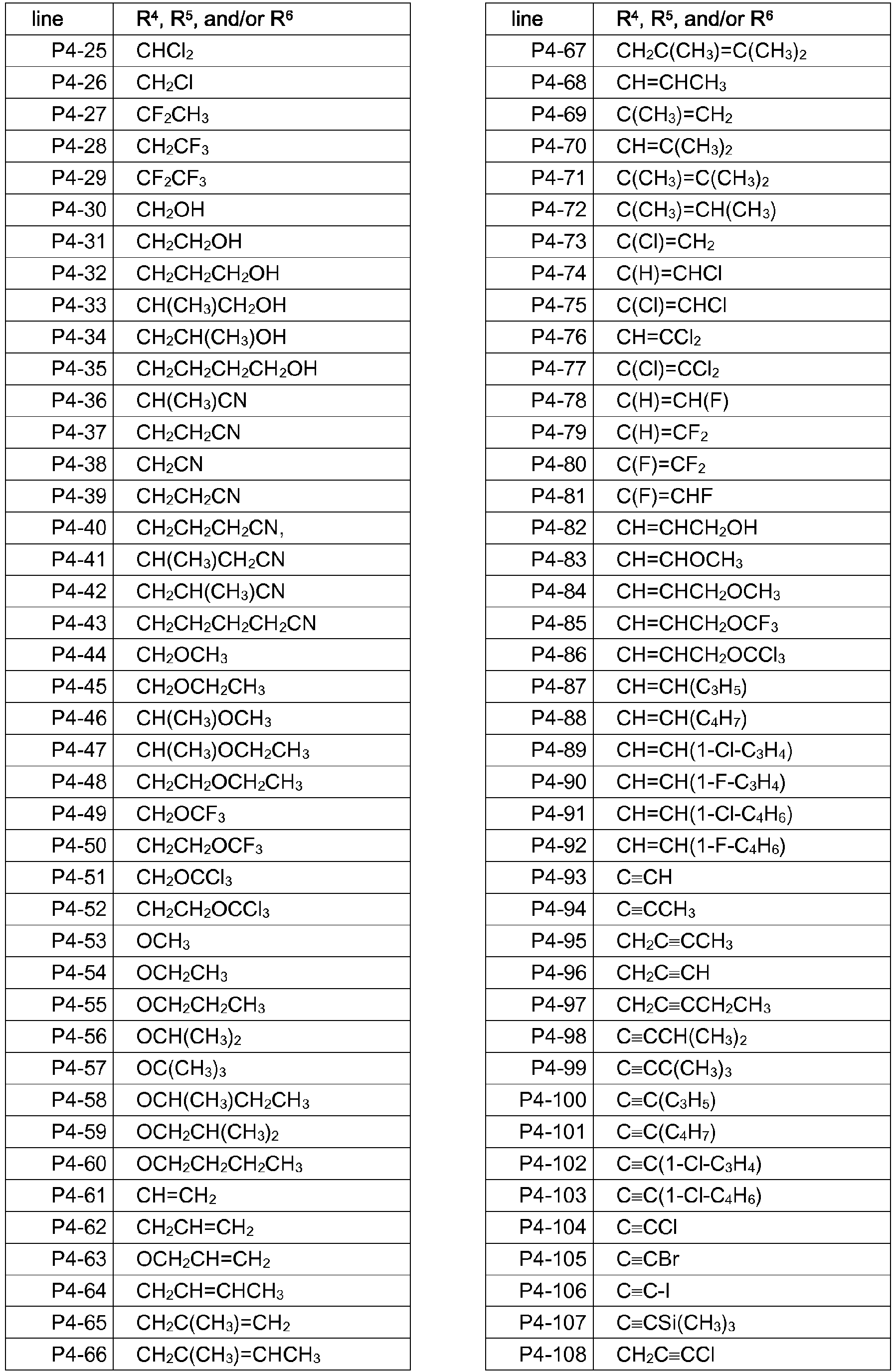

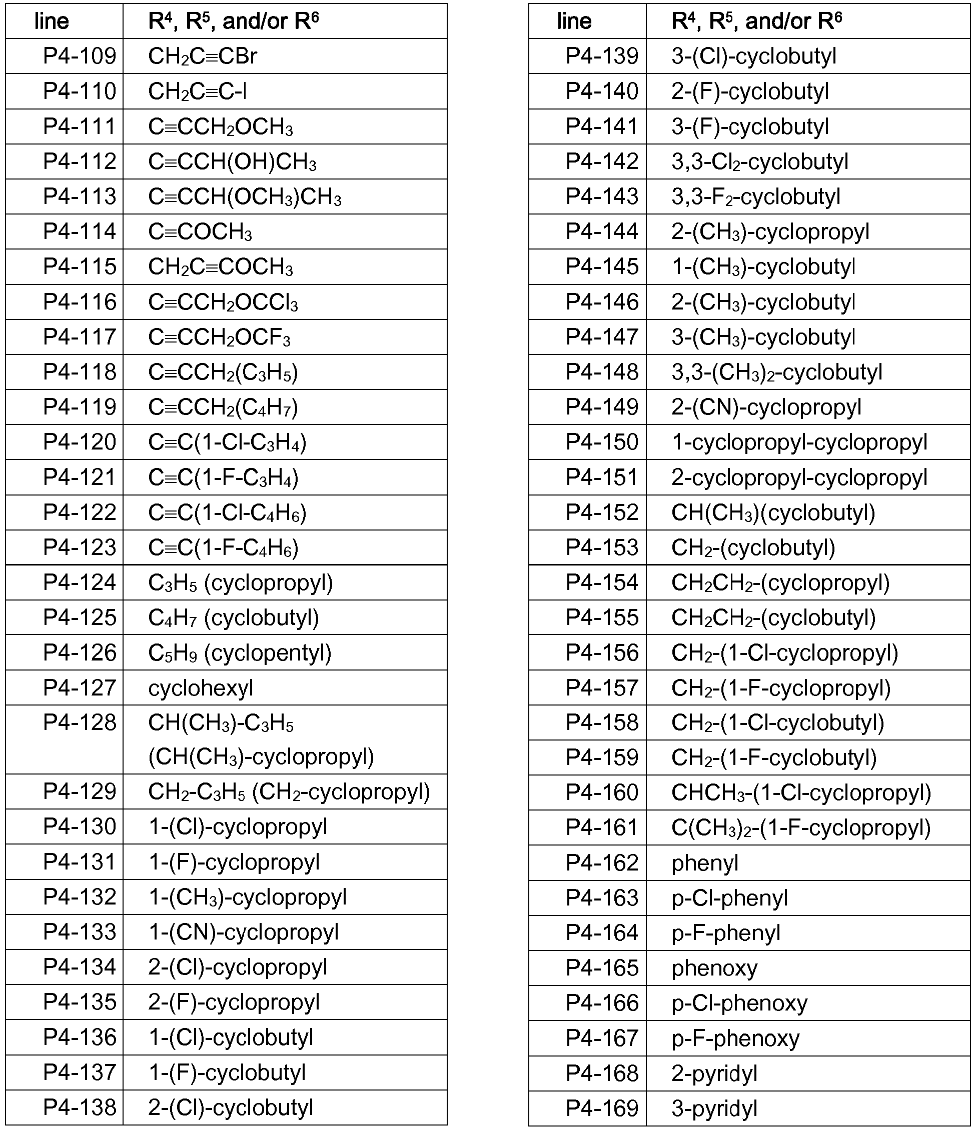

R^{a} are the possible substituents for the aliphatic moieties of R⁴, R⁵, and/or R⁶.

R^{a} according to the invention is independently selected from halogen, OH, CN, C₁-C₄-alkyl, C₁-C₄-halogenalkyl, C₃-C₆-cycloalkyl, C₃-C₆-halogencycloalkyl, C₁-C₄-alkoxy, C₁-C₄-halogenalkoxy and Si(R^{s})₃, wherein R^{s} is independently of one another C₁-C₄-alkyl.

According to one embodiment R^{a} is independently selected from halogen, OH, CN, C₁-C₂-alkyl, C₁-C₂-alkoxy, C₃-C₆-cycloalkyl, C₃-C₆-halocycloalkyl and C₁-C₂-halogenalkoxy. Specifically, R^{a} is independently selected from F, Cl, OH, CN, C₁-C₂-alkyl, C₁-C₂-alkoxy, cyclopropyl, 1-F-cyclopropyl, 1-Cl-cyclopropyl, C₁-C₂-halogenalkoxy and Si(CH₃)₃.

R^{b} are the possible substituents for the cycloalkyl moieties of R⁴, R⁵, and/or R⁶.

R^{b} according to the invention is independently selected from halogen, OH, CN, C₁-C₄-alkyl, C₁-C₄-alkoxy, C₁-C₄-halogenalkyl, C₃-C₆-cycloalkyl, C₃-C₆-halogencycloalkyl and C₁-C₄-halogenalkoxy.

According to one embodiment thereof R^{b} is independently selected from halogen, CN, C₁-C₂-alkyl, C₁-C₂-alkoxy, C₁-C₂-halogenalkyl, C₃-C₆-cycloalkyl, C₃-C₆-halocycloalkyl and C₁-C₂-halogenalkoxy. Specifically, R^{b} is independently selected from F, Cl, OH, CN, CH₃, OCH₃, cyclopropyl, 1-F-cyclopropyl, 1-Cl-cyclopropyl and halogenmethoxy.

R^{c} are the possible substituents for the aryl, heteroaryl and aryloxy moieties of R⁴, R⁵, and/or R⁶.

R^{c} according to the invention is independently selected from halogen, OH, CN, C₁-C₄-alkyl, C₁-C₄-alkoxy, C₁-C₄-halogenalkyl, C₃-C₆-cycloalkyl, C₃-C₆-halogencycloalkyl and C₁-C₄-halogenalkoxy.

According to one embodiment thereof R^{c} is independently selected from halogen, CN, C₁-C₂-alkyl, C₁-C₂-alkoxy, C₁-C₂-halogenalkyl, C₃-C₆-cycloalkyl, C₃-C₆-halocycloalkyl and C₁-C₂-halogenalkoxy. Specifically, R^{c} is independently selected from F, Cl, OH, CN, CH₃, OCH₃, cyclopropyl, 1-F-cyclopropyl, 1-Cl-cyclopropyl and halogenmethoxy.

In a preferred embodiment, R^{a}, R^{b}, and R^{c} are independently selected from halogen, CN, and OH.

Particularly preferred embodiments of the combination of R⁴, R⁵ and R⁶ according to the invention are in Table P5 below, wherein each line of lines P5-1 to P5-78 corresponds to one particular embodiment of the invention, wherein P5-1 to P5-78 are also in any combination a preferred embodiment of the present invention.

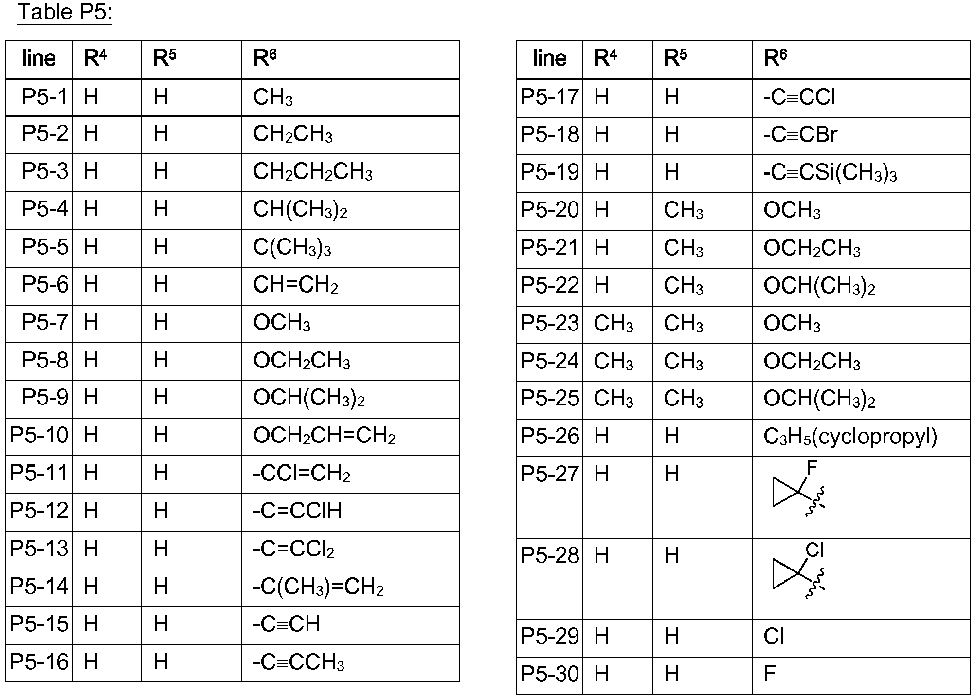

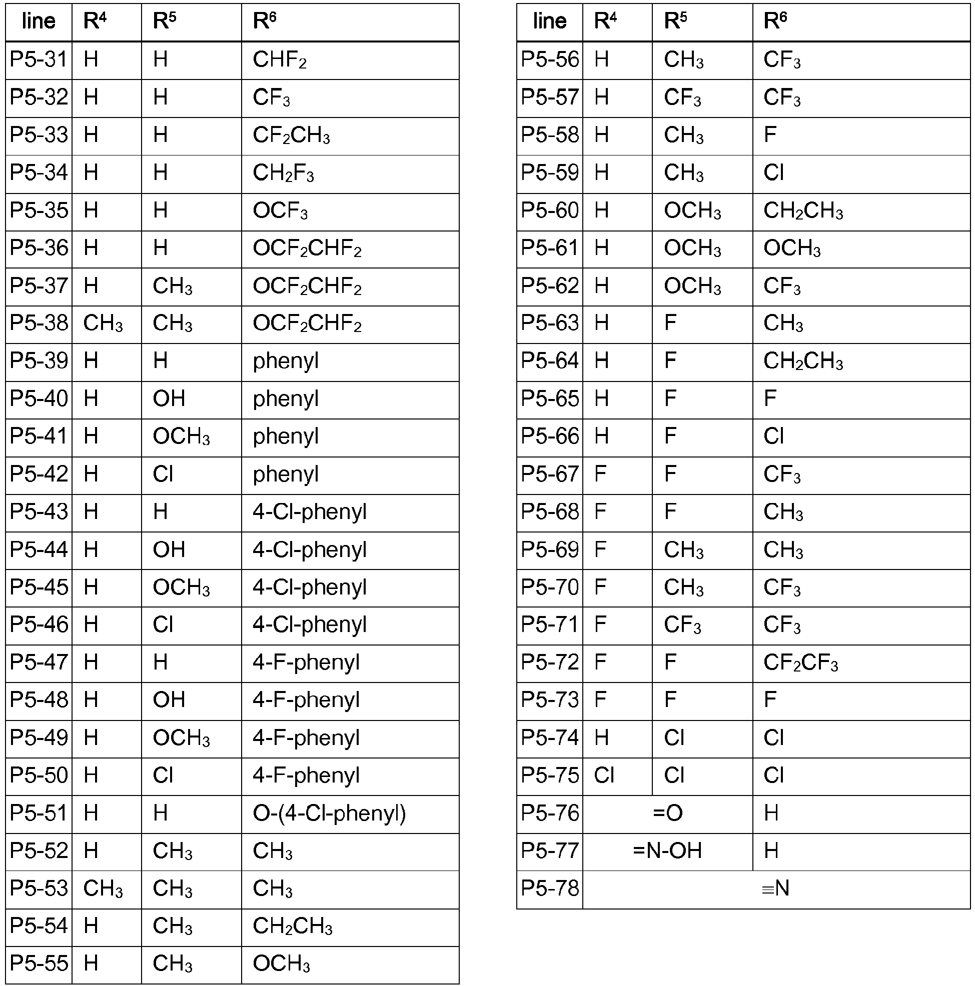

One embodiment relates to compounds of formula I, wherein R⁴ is hydrogen (compounds I.A):

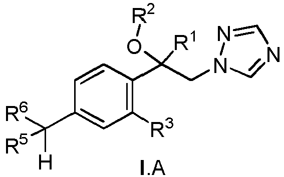

A further specific embodiment relates to compounds of formula I, wherein R⁴ and R⁵ are both methyl (compounds I.B):

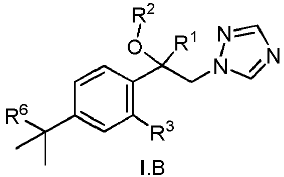

Another embodiment relates to compounds of formula I, wherein R⁴ is F (compounds I.C):

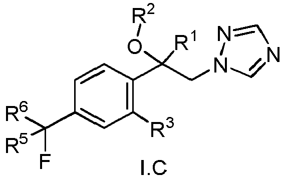

A further specific embodiment relates to compounds of formula I, wherein R⁴, R⁵ and R⁶ are all Cl (compounds I.D):

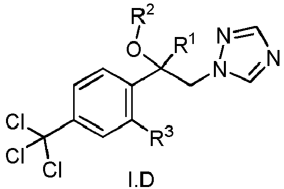

A still further specific embodiment relates to compounds of formula I, wherein R⁴, R⁵ and R⁶ together are ≡N (compounds I.E):

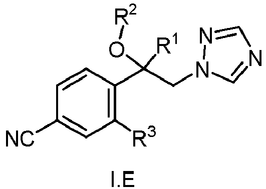

In particular with a view to their use, according to one embodiment, preference is given to the compounds of the formula I that are compiled in the Tables 1 a to 248a, Tables 1 b to 20b, Tables 1 c to 28c, Tables 1 d to 4d and Tables 1 e to 4e below. Each of the groups mentioned for a substituent in the tables is furthermore per se, independently of the combination in which it is mentioned, a particularly preferred aspect of the substituent in question.

### Table 1a

Compounds of the formula I.A in which the combination of R³, R⁵ and R⁶ corresponds to line D1-1 of Table D1 and the combination of R¹ and R² for each individual compound corresponds in each case to one line of Table B (compounds I.A.D1-1.B-1 to I.A.D1-1.B-460).

### Table 2a

Compounds of the formula I.A in which the combination of R³, R⁵ and R⁶ corresponds to line D1-2 of Table D1 and the combination of R¹ and R² for each individual compound corresponds in each case to one line of Table B (compounds I.A.D1-2.B-1 to I.A.D1-2.B-460).

### Table 3a

Compounds of the formula I.A in which the combination of R³, R⁵ and R⁶ corresponds to line D1-3 of Table D1 and the combination of R¹ and R² for each individual compound corresponds in each case to one line of Table B (compounds I.A.D1-3.B-1 to I.A.D1-3.B-460).

### Table 4a

Compounds of the formula I.A in which the combination of R³, R⁵ and R⁶ corresponds to line D1-4 of Table D1 and the combination of R¹ and R² for each individual compound corresponds in each case to one line of Table B (compounds I.A.D1-4.B-1 to I.A.D1-4.B-460).

### Table 5a

Compounds of the formula I.A in which the combination of R³, R⁵ and R⁶ corresponds to line D1-5 of Table D1 and the combination of R¹ and R² for each individual compound corresponds in each case to one line of Table B (compounds I.A.D1-5.B-1 to I.A.D1-5.B-460).

### Table 6a

Compounds of the formula I.A in which the combination of R³, R⁵ and R⁶ corresponds to line D1-6 of Table D1 and the combination of R¹ and R² for each individual compound corresponds in each case to one line of Table B (compounds I.A.D1-6.B-1 to I.A.D1-6.B-460).

### Table 7a

Compounds of the formula I.A in which the combination of R³, R⁵ and R⁶ corresponds to line D1-7 of Table D1 and the combination of R¹ and R² for each individual compound corresponds in each case to one line of Table B (compounds I.A.D1-7.B-1 to I.A.D1-7.B-460).

### Table 8a

Compounds of the formula I.A in which the combination of R³, R⁵ and R⁶ corresponds to line D1-8 of Table D1 and the combination of R¹ and R² for each individual compound corresponds in each case to one line of Table B (compounds I.A.D1-8.B-1 to I.A.D1-8.B-460).

### Table 9a

Compounds of the formula I.A in which the combination of R³, R⁵ and R⁶ corresponds to line D1-9 of Table D1 and the combination of R¹ and R² for each individual compound corresponds in each case to one line of Table B (compounds I.A.D1-9.B-1 to I.A.D1-9.B-460).

### Table 10a

Compounds of the formula I.A in which the combination of R³, R⁵ and R⁶ corresponds to line D1-10 of Table D1 and the combination of R¹ and R² for each individual compound corresponds in each case to one line of Table B (compounds I.A.D1-10.B-1 to I.A.D1-10.B-460).

### Table 11 a

Compounds of the formula I.A in which the combination of R³, R⁵ and R⁶ corresponds to line D1-11 of Table D1 and the combination of R¹ and R² for each individual compound corresponds in each case to one line of Table B (compounds I.A.D1-11.B-1 to I.A.D1-11.B-460).

### Table 12a

Compounds of the formula I.A in which the combination of R³, R⁵ and R⁶ corresponds to line D1-12 of Table D1 and the combination of R¹ and R² for each individual compound corresponds in each case to one line of Table B (compounds I.A.D1-12.B-1 to I.A.D1-12.B-460).

### Table 13a

Compounds of the formula I.A in which the combination of R³, R⁵ and R⁶ corresponds to line

D1-13 of Table D1 and the combination of R¹ and R² for each individual compound corresponds in each case to one line of Table B (compounds I.A.D1-13.B-1 to I.A.D1-13.B-460).

### Table 14a

Compounds of the formula I.A in which the combination of R³, R⁵ and R⁶ corresponds to line D1-14 of Table D1 and the combination of R¹ and R² for each individual compound corresponds in each case to one line of Table B (compounds I.A.D1-14.B-1 to I.A.D1-14.B-460).

### Table 15a

Compounds of the formula I.A in which the combination of R³, R⁵ and R⁶ corresponds to line D1-15 of Table D1 and the combination of R¹ and R² for each individual compound corresponds in each case to one line of Table B (compounds I.A.D1-15.B-1 to I.A.D1-15.B-460).

### Table 16a

Compounds of the formula I.A in which the combination of R³, R⁵ and R⁶ corresponds to line D1-16 of Table D1 and the combination of R¹ and R² for each individual compound corresponds in each case to one line of Table B (compounds I.A.D1-16.B-1 to I.A.D1-16.B-460).

### Table 17a

Compounds of the formula I.A in which the combination of R³, R⁵ and R⁶ corresponds to line D1-17 of Table D1 and the combination of R¹ and R² for each individual compound corresponds in each case to one line of Table B (compounds I.A.D1-17.B-1 to I.A.D1-17.B-460).

### Table 18a

Compounds of the formula I.A in which the combination of R³, R⁵ and R⁶ corresponds to line D1-18 of Table D1 and the combination of R¹ and R² for each individual compound corresponds in each case to one line of Table B (compounds I.A.D1-18.B-1 to I.A.D1-18.B-460).

### Table 19a

Compounds of the formula I.A in which the combination of R³, R⁵ and R⁶ corresponds to line D1-19 of Table D1 and the combination of R¹ and R² for each individual compound corresponds in each case to one line of Table B (compounds I.A.D1-19.B-1 to I.A.D1-19.B-460).

### Table 20a

Compounds of the formula I.A in which the combination of R³, R⁵ and R⁶ corresponds to line D1-20 of Table D1 and the combination of R¹ and R² for each individual compound corresponds in each case to one line of Table B (compounds I.A.D1-20.B-1 to I.A.D1-20.B-460).

### Table 21 a

Compounds of the formula I.A in which the combination of R³, R⁵ and R⁶ corresponds to line D1-21 of Table D1 and the combination of R¹ and R² for each individual compound corresponds in each case to one line of Table B (compounds I.A.D1-21.B-1 to I.A.D1-21.B-460).

### Table 22a

Compounds of the formula I.A in which the combination of R³, R⁵ and R⁶ corresponds to line D1-22 of Table D1 and the combination of R¹ and R² for each individual compound corresponds in each case to one line of Table B (compounds I.A.D1-22.B-1 to I.A.D1-22.B-460).

### Table 23a

Compounds of the formula I.A in which the combination of R³, R⁵ and R⁶ corresponds to line D1-23 of Table D1 and the combination of R¹ and R² for each individual compound corresponds in each case to one line of Table B (compounds I.A.D1-23.B-1 to I.A.D1-23.B-460).

### Table 24a

Compounds of the formula I.A in which the combination of R³, R⁵ and R⁶ corresponds to line D1-24 of Table D1 and the combination of R¹ and R² for each individual compound corresponds in each case to one line of Table B (compounds I.A.D1-24.B-1 to I.A.D1-24.B-460).

### Table 25a

Compounds of the formula I.A in which the combination of R³, R⁵ and R⁶ corresponds to line D1-25 of Table D1 and the combination of R¹ and R² for each individual compound corresponds in each case to one line of Table B (compounds I.A.D1-25.B-1 to I.A.D1-25.B-460).

### Table 26a

Compounds of the formula I.A in which the combination of R³, R⁵ and R⁶ corresponds to line D1-26 of Table D1 and the combination of R¹ and R² for each individual compound corresponds in each case to one line of Table B (compounds I.A.D1-26.B-1 to I.A.D1-26.B-460).

### Table 27a

Compounds of the formula I.A in which the combination of R³, R⁵ and R⁶ corresponds to line D1-27 of Table D1 and the combination of R¹ and R² for each individual compound corresponds in each case to one line of Table B (compounds I.A.D1-27.B-1 to I.A.D1-27.B-460).

### Table 28a

Compounds of the formula I.A in which the combination of R³, R⁵ and R⁶ corresponds to line D1-28 of Table D1 and the combination of R¹ and R² for each individual compound corresponds in each case to one line of Table B (compounds I.A.D1-28.B-1 to I.A.D1-28.B-460).

### Table 29a

Compounds of the formula I.A in which the combination of R³, R⁵ and R⁶ corresponds to line D1-29 of Table D1 and the combination of R¹ and R² for each individual compound corresponds in each case to one line of Table B (compounds I.A.D1-29.B-1 to I.A.D1-29.B-460).

### Table 30a

Compounds of the formula I.A in which the combination of R³, R⁵ and R⁶ corresponds to line D1-30 of Table D1 and the combination of R¹ and R² for each individual compound corresponds in each case to one line of Table B (compounds I.A.D1-30.B-1 to I.A.D1-30.B-460).

### Table 31 a

Compounds of the formula I.A in which the combination of R³, R⁵ and R⁶ corresponds to line D1-31 of Table D1 and the combination of R¹ and R² for each individual compound corresponds in each case to one line of Table B (compounds I.A.D1-31.B-1 to I.A.D1-31.B-460).

### Table 32a

Compounds of the formula I.A in which the combination of R³, R⁵ and R⁶ corresponds to line D1-32 of Table D1 and the combination of R¹ and R² for each individual compound corresponds in each case to one line of Table B (compounds I.A.D1-32.B-1 to I.A.D1-32.B-460).

### Table 33a

Compounds of the formula I.A in which the combination of R³, R⁵ and R⁶ corresponds to line D1-33 of Table D1 and the combination of R¹ and R² for each individual compound corresponds in each case to one line of Table B (compounds I.A.D1-33.B-1 to I.A.D1-33.B-460).

### Table 34a

Compounds of the formula I.A in which the combination of R³, R⁵ and R⁶ corresponds to line D1-34 of Table D1 and the combination of R¹ and R² for each individual compound corresponds in each case to one line of Table B (compounds I.A.D1-34.B-1 to I.A.D1-34.B-460).

### Table 35a

Compounds of the formula I.A in which the combination of R³, R⁵ and R⁶ corresponds to line D1-35 of Table D1 and the combination of R¹ and R² for each individual compound corresponds in each case to one line of Table B (compounds I.A.D1-35.B-1 to I.A.D1-35.B-460).

### Table 36a

Compounds of the formula I.A in which the combination of R³, R⁵ and R⁶ corresponds to line D1-36 of Table D1 and the combination of R¹ and R² for each individual compound corresponds in each case to one line of Table B (compounds I.A.D1-36.B-1 to I.A.D1-36.B-460).

### Table 37a

Compounds of the formula I.A in which the combination of R³, R⁵ and R⁶ corresponds to line D1-37 of Table D1 and the combination of R¹ and R² for each individual compound corresponds in each case to one line of Table B (compounds I.A.D1-37.B-1 to I.A.D1-37.B-460).

### Table 38a

Compounds of the formula I.A in which the combination of R³, R⁵ and R⁶ corresponds to line D1-38 of Table D1 and the combination of R¹ and R² for each individual compound corresponds in each case to one line of Table B (compounds I.A.D1-38.B-1 to I.A.D1-38.B-460).

### Table 39a

Compounds of the formula I.A in which the combination of R³, R⁵ and R⁶ corresponds to line D1-39 of Table D1 and the combination of R¹ and R² for each individual compound corresponds in each case to one line of Table B (compounds I.A.D1-39.B-1 to I.A.D1-39.B-460).

### Table 40a

Compounds of the formula I.A in which the combination of R³, R⁵ and R⁶ corresponds to line D1-40 of Table D1 and the combination of R¹ and R² for each individual compound corresponds in each case to one line of Table B (compounds I.A.D1-40.B-1 to I.A.D1-40.B-460).

### Table 41 a

Compounds of the formula I.A in which the combination of R³, R⁵ and R⁶ corresponds to line D1-41 of Table D1 and the combination of R¹ and R² for each individual compound corresponds in each case to one line of Table B (compounds I.A.D1-41.B-1 to I.A.D1-41.B-460).

### Table 42a

Compounds of the formula I.A in which the combination of R³, R⁵ and R⁶ corresponds to line D1-42 of Table D1 and the combination of R¹ and R² for each individual compound corresponds in each case to one line of Table B (compounds I.A.D1-42.B-1 to I.A.D1-42.B-460).

### Table 43a

Compounds of the formula I.A in which the combination of R³, R⁵ and R⁶ corresponds to line D1-43 of Table D1 and the combination of R¹ and R² for each individual compound corresponds in each case to one line of Table B (compounds I.A.D1-43.B-1 to I.A.D1-43.B-460).

### Table 44a

Compounds of the formula I.A in which the combination of R³, R⁵ and R⁶ corresponds to line D1-44 of Table D1 and the combination of R¹ and R² for each individual compound corresponds in each case to one line of Table B (compounds I.A.D1-44.B-1 to I.A.D1-44.B-460).

### Table 45a

Compounds of the formula I.A in which the combination of R³, R⁵ and R⁶ corresponds to line D1-45 of Table D1 and the combination of R¹ and R² for each individual compound corresponds in each case to one line of Table B (compounds I.A.D1-45.B-1 to I.A.D1-45.B-460).

### Table 46a

Compounds of the formula I.A in which the combination of R³, R⁵ and R⁶ corresponds to line D1-46 of Table D1 and the combination of R¹ and R² for each individual compound corresponds in each case to one line of Table B (compounds I.A.D1-46.B-1 to I.A.D1-46.B-460).

### Table 47a

Compounds of the formula I.A in which the combination of R³, R⁵ and R⁶ corresponds to line D1-47 of Table D1 and the combination of R¹ and R² for each individual compound corresponds in each case to one line of Table B (compounds I.A.D1-47.B-1 to I.A.D1-47.B-460).

### Table 48a

Compounds of the formula I.A in which the combination of R³, R⁵ and R⁶ corresponds to line D1-48 of Table D1 and the combination of R¹ and R² for each individual compound corresponds in each case to one line of Table B (compounds I.A.D1-48.B-1 to I.A.D1-48.B-460).

### Table 49a

Compounds of the formula I.A in which the combination of R³, R⁵ and R⁶ corresponds to line D1-49 of Table D1 and the combination of R¹ and R² for each individual compound corresponds in each case to one line of Table B (compounds I.A.D1-49.B-1 to I.A.D1-49.B-460).

### Table 50a

Compounds of the formula I.A in which the combination of R³, R⁵ and R⁶ corresponds to line D1-50 of Table D1 and the combination of R¹ and R² for each individual compound corresponds in each case to one line of Table B (compounds I.A.D1-50.B-1 to I.A.D1-50.B-460).

### Table 51 a

Compounds of the formula I.A in which the combination of R³, R⁵ and R⁶ corresponds to line D1-51 of Table D1 and the combination of R¹ and R² for each individual compound corresponds in each case to one line of Table B (compounds I.A.D1-51.B-1 to I.A.D1-51.B-460).

### Table 52a

Compounds of the formula I.A in which the combination of R³, R⁵ and R⁶ corresponds to line D1-52 of Table D1 and the combination of R¹ and R² for each individual compound corresponds in each case to one line of Table B (compounds I.A.D1-52.B-1 to I.A.D1-52.B-460).

### Table 53a

Compounds of the formula I.A in which the combination of R³, R⁵ and R⁶ corresponds to line D1-53 of Table D1 and the combination of R¹ and R² for each individual compound corresponds in each case to one line of Table B (compounds I.A.D1-53.B-1 to I.A.D1-53.B-460).

### Table 54a

Compounds of the formula I.A in which the combination of R³, R⁵ and R⁶ corresponds to line

D1-54 of Table D1 and the combination of R¹ and R² for each individual compound corresponds in each case to one line of Table B (compounds I.A.D1-54.B-1 to I.A.D1-54.B-460).

### Table 55a

Compounds of the formula I.A in which the combination of R³, R⁵ and R⁶ corresponds to line D1-55 of Table D1 and the combination of R¹ and R² for each individual compound corresponds in each case to one line of Table B (compounds I.A.D1-55.B-1 to I.A.D1-55.B-460).

### Table 56a

Compounds of the formula I.A in which the combination of R³, R⁵ and R⁶ corresponds to line D1-56 of Table D1 and the combination of R¹ and R² for each individual compound corresponds in each case to one line of Table B (compounds I.A.D1-56.B-1 to I.A.D1-56.B-460).

### Table 57a

Compounds of the formula I.A in which the combination of R³, R⁵ and R⁶ corresponds to line D1-57 of Table D1 and the combination of R¹ and R² for each individual compound corresponds in each case to one line of Table B (compounds I.A.D1-57.B-1 to I.A.D1-57.B-460).

### Table 58a

Compounds of the formula I.A in which the combination of R³, R⁵ and R⁶ corresponds to line D1-58 of Table D1 and the combination of R¹ and R² for each individual compound corresponds in each case to one line of Table B (compounds I.A.D1-58.B-1 to I.A.D1-58.B-460).

### Table 59a

Compounds of the formula I.A in which the combination of R³, R⁵ and R⁶ corresponds to line D1-59 of Table D1 and the combination of R¹ and R² for each individual compound corresponds in each case to one line of Table B (compounds I.A.D1-59.B-1 to I.A.D1-59.B-460).

### Table 60a

Compounds of the formula I.A in which the combination of R³, R⁵ and R⁶ corresponds to line D1-60 of Table D1 and the combination of R¹ and R² for each individual compound corresponds in each case to one line of Table B (compounds I.A.D1-60.B-1 to I.A.D1-60.B-460).

### Table 61 a

Compounds of the formula I.A in which the combination of R³, R⁵ and R⁶ corresponds to line D1-61 of Table D1 and the combination of R¹ and R² for each individual compound corresponds in each case to one line of Table B (compounds I.A.D1-61.B-1 to I.A.D1-61.B-460).

### Table 62a

Compounds of the formula I.A in which the combination of R³, R⁵ and R⁶ corresponds to line D1-62 of Table D1 and the combination of R¹ and R² for each individual compound corresponds in each case to one line of Table B (compounds I.A.D1-62.B-1 to I.A.D1-62.B-460).

### Table 63a

Compounds of the formula I.A in which the combination of R³, R⁵ and R⁶ corresponds to line D1-63 of Table D1 and the combination of R¹ and R² for each individual compound corresponds in each case to one line of Table B (compounds I.A.D1-63.B-1 to I.A.D1-63.B-460).

### Table 64a

Compounds of the formula I.A in which the combination of R³, R⁵ and R⁶ corresponds to line D1-64 of Table D1 and the combination of R¹ and R² for each individual compound corresponds in each case to one line of Table B (compounds I.A.D1-64.B-1 to I.A.D1-64.B-460).

### Table 65a

Compounds of the formula I.A in which the combination of R³, R⁵ and R⁶ corresponds to line D1-65 of Table D1 and the combination of R¹ and R² for each individual compound corresponds in each case to one line of Table B (compounds I.A.D1-65.B-1 to I.A.D1-65.B-460).

### Table 66a

Compounds of the formula I.A in which the combination of R³, R⁵ and R⁶ corresponds to line D1-66 of Table D1 and the combination of R¹ and R² for each individual compound corresponds in each case to one line of Table B (compounds I.A.D1-66.B-1 to I.A.D1-66.B-460).

### Table 67a

Compounds of the formula I.A in which the combination of R³, R⁵ and R⁶ corresponds to line D1-67 of Table D1 and the combination of R¹ and R² for each individual compound corresponds in each case to one line of Table B (compounds I.A.D1-67.B-1 to I.A.D1-67.B-460).

### Table 68a

Compounds of the formula I.A in which the combination of R³, R⁵ and R⁶ corresponds to line D1-68 of Table D1 and the combination of R¹ and R² for each individual compound corresponds in each case to one line of Table B (compounds I.A.D1-68.B-1 to I.A.D1-68.B-460).

### Table 69a

Compounds of the formula I.A in which the combination of R³, R⁵ and R⁶ corresponds to line D1-69 of Table D1 and the combination of R¹ and R² for each individual compound corresponds in each case to one line of Table B (compounds I.A.D1-69.B-1 to I.A.D1-69.B-460).

### Table 70a

Compounds of the formula I.A in which the combination of R³, R⁵ and R⁶ corresponds to line D1-70 of Table D1 and the combination of R¹ and R² for each individual compound corresponds in each case to one line of Table B (compounds I.A.D1-70.B-1 to I.A.D1-70.B-460).

### Table 71 a

Compounds of the formula I.A in which the combination of R³, R⁵ and R⁶ corresponds to line D1-71 of Table D1 and the combination of R¹ and R² for each individual compound corresponds in each case to one line of Table B (compounds I.A.D1-71.B-1 to I.A.D1-71.B-460).

### Table 72a

Compounds of the formula I.A in which the combination of R³, R⁵ and R⁶ corresponds to line D1-72 of Table D1 and the combination of R¹ and R² for each individual compound corresponds in each case to one line of Table B (compounds I.A.D1-72.B-1 to I.A.D1-72.B-460).

### Table 73a

Compounds of the formula I.A in which the combination of R³, R⁵ and R⁶ corresponds to line D1-73 of Table D1 and the combination of R¹ and R² for each individual compound corresponds in each case to one line of Table B (compounds I.A.D1-73.B-1 to I.A.D1-73.B-460).

### Table 74a

Compounds of the formula I.A in which the combination of R³, R⁵ and R⁶ corresponds to line D1-74 of Table D1 and the combination of R¹ and R² for each individual compound corresponds in each case to one line of Table B (compounds I.A.D1-74.B-1 to I.A.D1-74.B-460).

### Table 75a

Compounds of the formula I.A in which the combination of R³, R⁵ and R⁶ corresponds to line D1-75 of Table D1 and the combination of R¹ and R² for each individual compound corresponds in each case to one line of Table B (compounds I.A.D1-75.B-1 to I.A.D1-75.B-460).

### Table 76a

Compounds of the formula I.A in which the combination of R³, R⁵ and R⁶ corresponds to line D1-76 of Table D1 and the combination of R¹ and R² for each individual compound corresponds in each case to one line of Table B (compounds I.A.D1-76.B-1 to I.A.D1-76.B-460).

### Table 77a

Compounds of the formula I.A in which the combination of R³, R⁵ and R⁶ corresponds to line D1-77 of Table D1 and the combination of R¹ and R² for each individual compound corresponds in each case to one line of Table B (compounds I.A.D1-77.B-1 to I.A.D1-77.B-460).

### Table 78a

Compounds of the formula I.A in which the combination of R³, R⁵ and R⁶ corresponds to line D1-78 of Table D1 and the combination of R¹ and R² for each individual compound corresponds in each case to one line of Table B (compounds I.A.D1-78.B-1 to I.A.D1-78.B-460).

### Table 79a

Compounds of the formula I.A in which the combination of R³, R⁵ and R⁶ corresponds to line D1-79 of Table D1 and the combination of R¹ and R² for each individual compound corresponds in each case to one line of Table B (compounds I.A.D1-79.B-1 to I.A.D1-79.B-460).

### Table 80a

Compounds of the formula I.A in which the combination of R³, R⁵ and R⁶ corresponds to line D1-80 of Table D1 and the combination of R¹ and R² for each individual compound corresponds in each case to one line of Table B (compounds I.A.D1-80.B-1 to I.A.D1-80.B-460).

### Table 81 a

Compounds of the formula I.A in which the combination of R³, R⁵ and R⁶ corresponds to line D1-81 of Table D1 and the combination of R¹ and R² for each individual compound corresponds in each case to one line of Table B (compounds I.A.D1-81.B-1 to I.A.D1-81.B-460).

### Table 82a

Compounds of the formula I.A in which the combination of R³, R⁵ and R⁶ corresponds to line D1-82 of Table D1 and the combination of R¹ and R² for each individual compound corresponds in each case to one line of Table B (compounds I.A.D1-82.B-1 to I.A.D1-82.B-460).

### Table 83a

Compounds of the formula I.A in which the combination of R³, R⁵ and R⁶ corresponds to line D1-83 of Table D1 and the combination of R¹ and R² for each individual compound corresponds in each case to one line of Table B (compounds I.A.D1-83.B-1 to I.A.D1-83.B-460).

### Table 84a

Compounds of the formula I.A in which the combination of R³, R⁵ and R⁶ corresponds to line D1-84 of Table D1 and the combination of R¹ and R² for each individual compound corresponds in each case to one line of Table B (compounds I.A.D1-84.B-1 to I.A.D1-84.B-460).

### Table 85a

Compounds of the formula I.A in which the combination of R³, R⁵ and R⁶ corresponds to line D1-85 of Table D1 and the combination of R¹ and R² for each individual compound corresponds in each case to one line of Table B (compounds I.A.D1-85.B-1 to I.A.D1-85.B-460).

### Table 86a

Compounds of the formula I.A in which the combination of R³, R⁵ and R⁶ corresponds to line D1-86 of Table D1 and the combination of R¹ and R² for each individual compound corresponds in each case to one line of Table B (compounds I.A.D1-86.B-1 to I.A.D1-86.B-460).

### Table 87a

Compounds of the formula I.A in which the combination of R³, R⁵ and R⁶ corresponds to line D1-87 of Table D1 and the combination of R¹ and R² for each individual compound corresponds in each case to one line of Table B (compounds I.A.D1-87.B-1 to I.A.D1-87.B-460).

### Table 88a

Compounds of the formula I.A in which the combination of R³, R⁵ and R⁶ corresponds to line D1-88 of Table D1 and the combination of R¹ and R² for each individual compound corresponds in each case to one line of Table B (compounds I.A.D1-88.B-1 to I.A.D1-88.B-460).

### Table 89a

Compounds of the formula I.A in which the combination of R³, R⁵ and R⁶ corresponds to line D1-89 of Table D1 and the combination of R¹ and R² for each individual compound corresponds in each case to one line of Table B (compounds I.A.D1-89.B-1 to I.A.D1-89.B-460).

### Table 90a

Compounds of the formula I.A in which the combination of R³, R⁵ and R⁶ corresponds to line D1-90 of Table D1 and the combination of R¹ and R² for each individual compound corresponds in each case to one line of Table B (compounds I.A.D1-90.B-1 to I.A.D1-90.B-460).

### Table 91 a

Compounds of the formula I.A in which the combination of R³, R⁵ and R⁶ corresponds to line D1-91 of Table D1 and the combination of R¹ and R² for each individual compound corresponds in each case to one line of Table B (compounds I.A.D1-91.B-1 to I.A.D1-91.B-460).

### Table 92a

Compounds of the formula I.A in which the combination of R³, R⁵ and R⁶ corresponds to line D1-92 of Table D1 and the combination of R¹ and R² for each individual compound corresponds in each case to one line of Table B (compounds I.A.D1-92.B-1 to I.A.D1-92.B-460).

### Table 93a

Compounds of the formula I.A in which the combination of R³, R⁵ and R⁶ corresponds to line D1-93 of Table D1 and the combination of R¹ and R² for each individual compound corresponds in each case to one line of Table B (compounds I.A.D1-93.B-1 to I.A.D1-93.B-460).

### Table 94a

Compounds of the formula I.A in which the combination of R³, R⁵ and R⁶ corresponds to line D1-94 of Table D1 and the combination of R¹ and R² for each individual compound corresponds in each case to one line of Table B (compounds I.A.D1-94.B-1 to I.A.D1-94.B-460).

### Table 95a

Compounds of the formula I.A in which the combination of R³, R⁵ and R⁶ corresponds to line

D1-95 of Table D1 and the combination of R¹ and R² for each individual compound corresponds in each case to one line of Table B (compounds I.A.D1-95.B-1 to I.A.D1-95.B-460).

### Table 96a

Compounds of the formula I.A in which the combination of R³, R⁵ and R⁶ corresponds to line D1-96 of Table D1 and the combination of R¹ and R² for each individual compound corresponds in each case to one line of Table B (compounds I.A.D1-96.B-1 to I.A.D1-96.B-460).

### Table 97a

Compounds of the formula I.A in which the combination of R³, R⁵ and R⁶ corresponds to line D1-97 of Table D1 and the combination of R¹ and R² for each individual compound corresponds in each case to one line of Table B (compounds I.A.D1-97.B-1 to I.A.D1-97.B-460).

### Table 98a

Compounds of the formula I.A in which the combination of R³, R⁵ and R⁶ corresponds to line D1-98 of Table D1 and the combination of R¹ and R² for each individual compound corresponds in each case to one line of Table B (compounds I.A.D1-98.B-1 to I.A.D1-98.B-460).

### Table 99a

Compounds of the formula I.A in which the combination of R³, R⁵ and R⁶ corresponds to line D1-99 of Table D1 and the combination of R¹ and R² for each individual compound corresponds in each case to one line of Table B (compounds I.A.D1-99.B-1 to I.A.D1-99.B-460).

### Table 100a

Compounds of the formula I.A in which the combination of R³, R⁵ and R⁶ corresponds to line D1-100 of Table D1 and the combination of R¹ and R² for each individual compound corresponds in each case to one line of Table B (compounds I.A.D1-100.B-1 to I.A.D1-100.B-460).

### Table 101a

Compounds of the formula I.A in which the combination of R³, R⁵ and R⁶ corresponds to line D1-101 of Table D1 and the combination of R¹ and R² for each individual compound corresponds in each case to one line of Table B (compounds I.A.D1-101.B-1 to I.A.D1-101.B-460).

### Table 102a

Compounds of the formula I.A in which the combination of R³, R⁵ and R⁶ corresponds to line D1-102 of Table D1 and the combination of R¹ and R² for each individual compound corresponds in each case to one line of Table B (compounds I.A.D1-102.B-1 to I.A.D1-102.B-460).

### Table 103a

Compounds of the formula I.A in which the combination of R³, R⁵ and R⁶ corresponds to line D1-103 of Table D1 and the combination of R¹ and R² for each individual compound corresponds in each case to one line of Table B (compounds I.A.D1-103.B-1 to I.A.D1-103.B-460).

### Table 104a

Compounds of the formula I.A in which the combination of R³, R⁵ and R⁶ corresponds to line D1-104 of Table D1 and the combination of R¹ and R² for each individual compound corresponds in each case to one line of Table B (compounds I.A.D1-104.B-1 to I.A.D1-104.B-460).

### Table 105a

Compounds of the formula I.A in which the combination of R³, R⁵ and R⁶ corresponds to line D1-105 of Table D1 and the combination of R¹ and R² for each individual compound corresponds in each case to one line of Table B (compounds I.A.D1-105.B-1 to I.A.D1-105.B-460).

### Table 106a

Compounds of the formula I.A in which the combination of R³, R⁵ and R⁶ corresponds to line D1-106 of Table D1 and the combination of R¹ and R² for each individual compound corresponds in each case to one line of Table B (compounds I.A.D1-106.B-1 to I.A.D1-106.B-460).

### Table 107a

Compounds of the formula I.A in which the combination of R³, R⁵ and R⁶ corresponds to line D1-107 of Table D1 and the combination of R¹ and R² for each individual compound corresponds in each case to one line of Table B (compounds I.A.D1-107.B-1 to I.A.D1-107.B-460).

### Table 108a

Compounds of the formula I.A in which the combination of R³, R⁵ and R⁶ corresponds to line D1-108 of Table D1 and the combination of R¹ and R² for each individual compound corresponds in each case to one line of Table B (compounds I.A.D1-108.B-1 to I.A.D1-108.B-460).

### Table 109a

Compounds of the formula I.A in which the combination of R³, R⁵ and R⁶ corresponds to line D1-109 of Table D1 and the combination of R¹ and R² for each individual compound corresponds in each case to one line of Table B (compounds I.A.D1-109.B-1 to I.A.D1-109.B-460).

### Table 110a

Compounds of the formula I.A in which the combination of R³, R⁵ and R⁶ corresponds to line D1-110 of Table D1 and the combination of R¹ and R² for each individual compound corresponds in each case to one line of Table B (compounds I.A.D1-110.B-1 to I.A.D1-110.B-460).

### Table 111 a

Compounds of the formula I.A in which the combination of R³, R⁵ and R⁶ corresponds to line D1-111 of Table D1 and the combination of R¹ and R² for each individual compound corresponds in each case to one line of Table B (compounds I.A.D1-111.B-1 to I.A.D1-111.B-460).

### Table 112a

Compounds of the formula I.A in which the combination of R³, R⁵ and R⁶ corresponds to line D1-112 of Table D1 and the combination of R¹ and R² for each individual compound corresponds in each case to one line of Table B (compounds I.A.D1-112.B-1 to I.A.D1-112.B-460).

### Table 113a

Compounds of the formula I.A in which the combination of R³, R⁵ and R⁶ corresponds to line D1-113 of Table D1 and the combination of R¹ and R² for each individual compound corresponds in each case to one line of Table B (compounds I.A.D1-113.B-1 to I.A.D1-113.B-460).

### Table 114a

Compounds of the formula I.A in which the combination of R³, R⁵ and R⁶ corresponds to line D1-114 of Table D1 and the combination of R¹ and R² for each individual compound corresponds in each case to one line of Table B (compounds I.A.D1-114.B-1 to I.A.D1-114.B-460).

### Table 115a

Compounds of the formula I.A in which the combination of R³, R⁵ and R⁶ corresponds to line D1-115 of Table D1 and the combination of R¹ and R² for each individual compound corresponds in each case to one line of Table B (compounds I.A.D1-115.B-1 to I.A.D1-115.B-460).

### Table 116a

Compounds of the formula I.A in which the combination of R³, R⁵ and R⁶ corresponds to line D1-116 of Table D1 and the combination of R¹ and R² for each individual compound corresponds in each case to one line of Table B (compounds I.A.D1-116.B-1 to I.A.D1-116.B-460).

### Table 117a

Compounds of the formula I.A in which the combination of R³, R⁵ and R⁶ corresponds to line D1-117 of Table D1 and the combination of R¹ and R² for each individual compound corresponds in each case to one line of Table B (compounds I.A.D1-117.B-1 to I.A.D1-117.B-460).

### Table 118a

Compounds of the formula I.A in which the combination of R³, R⁵ and R⁶ corresponds to line D1-118 of Table D1 and the combination of R¹ and R² for each individual compound corresponds in each case to one line of Table B (compounds I.A.D1-118.B-1 to I.A.D1-118.B-460).

### Table 119a

Compounds of the formula I.A in which the combination of R³, R⁵ and R⁶ corresponds to line D1-119 of Table D1 and the combination of R¹ and R² for each individual compound corresponds in each case to one line of Table B (compounds I.A.D1-119.B-1 to I.A.D1-119.B-460).

### Table 120a

Compounds of the formula I.A in which the combination of R³, R⁵ and R⁶ corresponds to line D1-120 of Table D1 and the combination of R¹ and R² for each individual compound corresponds in each case to one line of Table B (compounds I.A.D1-120.B-1 to I.A.D1-120.B-460).

### Table 121a

Compounds of the formula I.A in which the combination of R³, R⁵ and R⁶ corresponds to line D1-121 of Table D1 and the combination of R¹ and R² for each individual compound corresponds in each case to one line of Table B (compounds I.A.D1-121.B-1 to I.A.D1-121.B-460).

### Table 122a

Compounds of the formula I.A in which the combination of R³, R⁵ and R⁶ corresponds to line D1-122 of Table D1 and the combination of R¹ and R² for each individual compound corresponds in each case to one line of Table B (compounds I.A.D1-122.B-1 to I.A.D1-122.B-460).

### Table 123a

Compounds of the formula I.A in which the combination of R³, R⁵ and R⁶ corresponds to line D1-123 of Table D1 and the combination of R¹ and R² for each individual compound corresponds in each case to one line of Table B (compounds I.A.D1-123.B-1 to I.A.D1-123.B-460).

### Table 124a

Compounds of the formula I.A in which the combination of R³, R⁵ and R⁶ corresponds to line D1-124 of Table D1 and the combination of R¹ and R² for each individual compound corresponds in each case to one line of Table B (compounds I.A.D1-124.B-1 to I.A.D1-124.B-460).

### Table 125a

Compounds of the formula I.A in which the combination of R³, R⁵ and R⁶ corresponds to line D1-125 of Table D1 and the combination of R¹ and R² for each individual compound corresponds in each case to one line of Table B (compounds I.A.D1-125.B-1 to I.A.D1-125.B-460).

### Table 126a

Compounds of the formula I.A in which the combination of R³, R⁵ and R⁶ corresponds to line D1-126 of Table D1 and the combination of R¹ and R² for each individual compound corresponds in each case to one line of Table B (compounds I.A.D1-126.B-1 to I.A.D1-126.B-460).

### Table 127a

Compounds of the formula I.A in which the combination of R³, R⁵ and R⁶ corresponds to line D1-127 of Table D1 and the combination of R¹ and R² for each individual compound corresponds in each case to one line of Table B (compounds I.A.D1-127.B-1 to I.A.D1-127.B-460).

### Table 128a

Compounds of the formula I.A in which the combination of R³, R⁵ and R⁶ corresponds to line D1-128 of Table D1 and the combination of R¹ and R² for each individual compound corresponds in each case to one line of Table B (compounds I.A.D1-128.B-1 to I.A.D1-128.B-460).

### Table 129a

Compounds of the formula I.A in which the combination of R³, R⁵ and R⁶ corresponds to line D1-129 of Table D1 and the combination of R¹ and R² for each individual compound corresponds in each case to one line of Table B (compounds I.A.D1-129.B-1 to I.A.D1-129.B-460).

### Table 130a

Compounds of the formula I.A in which the combination of R³, R⁵ and R⁶ corresponds to line D1-130 of Table D1 and the combination of R¹ and R² for each individual compound corresponds in each case to one line of Table B (compounds I.A.D1-130.B-1 to I.A.D1-130.B-460).

### Table 131a

Compounds of the formula I.A in which the combination of R³, R⁵ and R⁶ corresponds to line D1-131 of Table D1 and the combination of R¹ and R² for each individual compound corresponds in each case to one line of Table B (compounds I.A.D1-131.B-1 to I.A.D1-131.B-460).

### Table 132a

Compounds of the formula I.A in which the combination of R³, R⁵ and R⁶ corresponds to line D1-132 of Table D1 and the combination of R¹ and R² for each individual compound corresponds in each case to one line of Table B (compounds I.A.D1-132.B-1 to I.A.D1-132.B-460).

### Table 133a

Compounds of the formula I.A in which the combination of R³, R⁵ and R⁶ corresponds to line D1-133 of Table D1 and the combination of R¹ and R² for each individual compound corresponds in each case to one line of Table B (compounds I.A.D1-133.B-1 to I.A.D1-133.B-460).

### Table 134a

Compounds of the formula I.A in which the combination of R³, R⁵ and R⁶ corresponds to line D1-134 of Table D1 and the combination of R¹ and R² for each individual compound corresponds in each case to one line of Table B (compounds I.A.D1-134.B-1 to I.A.D1-134.B-460).

### Table 135a

Compounds of the formula I.A in which the combination of R³, R⁵ and R⁶ corresponds to line D1-135 of Table D1 and the combination of R¹ and R² for each individual compound corresponds in each case to one line of Table B (compounds I.A.D1-135.B-1 to I.A.D1-135.B-460).

### Table 136a

Compounds of the formula I.A in which the combination of R³, R⁵ and R⁶ corresponds to line

D1-136 of Table D1 and the combination of R¹ and R² for each individual compound corresponds in each case to one line of Table B (compounds I.A.D1-136.B-1 to I.A.D1-136.B-460).

### Table 137a

Compounds of the formula I.A in which the combination of R³, R⁵ and R⁶ corresponds to line D1-137 of Table D1 and the combination of R¹ and R² for each individual compound corresponds in each case to one line of Table B (compounds I.A.D1-137.B-1 to I.A.D1-137.B-460).

### Table 138a

Compounds of the formula I.A in which the combination of R³, R⁵ and R⁶ corresponds to line D1-138 of Table D1 and the combination of R¹ and R² for each individual compound corresponds in each case to one line of Table B (compounds I.A.D1-138.B-1 to I.A.D1-138.B-460).

### Table 139a

Compounds of the formula I.A in which the combination of R³, R⁵ and R⁶ corresponds to line D1-139 of Table D1 and the combination of R¹ and R² for each individual compound corresponds in each case to one line of Table B (compounds I.A.D1-139.B-1 to I.A.D1-139.B-460).

### Table 140a

Compounds of the formula I.A in which the combination of R³, R⁵ and R⁶ corresponds to line D1-140 of Table D1 and the combination of R¹ and R² for each individual compound corresponds in each case to one line of Table B (compounds I.A.D1-140.B-1 to I.A.D1-140.B-460).

### Table 141a

Compounds of the formula I.A in which the combination of R³, R⁵ and R⁶ corresponds to line D1-141 of Table D1 and the combination of R¹ and R² for each individual compound corresponds in each case to one line of Table B (compounds I.A.D1-141.B-1 to I.A.D1-141.B-460).

### Table 142a

Compounds of the formula I.A in which the combination of R³, R⁵ and R⁶ corresponds to line D1-142 of Table D1 and the combination of R¹ and R² for each individual compound corresponds in each case to one line of Table B (compounds I.A.D1-142.B-1 to I.A.D1-142.B-460).

### Table 143a

Compounds of the formula I.A in which the combination of R³, R⁵ and R⁶ corresponds to line D1-143 of Table D1 and the combination of R¹ and R² for each individual compound corresponds in each case to one line of Table B (compounds I.A.D1-143.B-1 to I.A.D1-143.B-460).

### Table 144a

Compounds of the formula I.A in which the combination of R³, R⁵ and R⁶ corresponds to line D1-144 of Table D1 and the combination of R¹ and R² for each individual compound corresponds in each case to one line of Table B (compounds I.A.D1-144.B-1 to I.A.D1-144.B-460).

### Table 145a

Compounds of the formula I.A in which the combination of R³, R⁵ and R⁶ corresponds to line D1-145 of Table D1 and the combination of R¹ and R² for each individual compound corresponds in each case to one line of Table B (compounds I.A.D1-145.B-1 to I.A.D1-145.B-460).

### Table 146a

Compounds of the formula I.A in which the combination of R³, R⁵ and R⁶ corresponds to line D1-146 of Table D1 and the combination of R¹ and R² for each individual compound corre-sponds in each case to one line of Table B (compounds I.A.D1-146.B-1 to I.A.D1-146.B-460).

### Table 147a

Compounds of the formula I.A in which the combination of R³, R⁵ and R⁶ corresponds to line D1-147 of Table D1 and the combination of R¹ and R² for each individual compound corresponds in each case to one line of Table B (compounds I.A.D1-147.B-1 to I.A.D1-147.B-460).

### Table 148a

Compounds of the formula I.A in which the combination of R³, R⁵ and R⁶ corresponds to line D1-148 of Table D1 and the combination of R¹ and R² for each individual compound corresponds in each case to one line of Table B (compounds I.A.D1-148.B-1 to I.A.D1-148.B-460).

### Table 149a

Compounds of the formula I.A in which the combination of R³, R⁵ and R⁶ corresponds to line D1-149 of Table D1 and the combination of R¹ and R² for each individual compound corresponds in each case to one line of Table B (compounds I.A.D1-149.B-1 to I.A.D1-149.B-460).

### Table 150a

Compounds of the formula I.A in which the combination of R³, R⁵ and R⁶ corresponds to line D1-150 of Table D1 and the combination of R¹ and R² for each individual compound corresponds in each case to one line of Table B (compounds I.A.D1-150.B-1 to I.A.D1-150.B-460).

### Table 151 a

Compounds of the formula I.A in which the combination of R³, R⁵ and R⁶ corresponds to line D1-151 of Table D1 and the combination of R¹ and R² for each individual compound corresponds in each case to one line of Table B (compounds I.A.D1-151.B-1 to I.A.D1-151.B-460).

### Table 152a

Compounds of the formula I.A in which the combination of R³, R⁵ and R⁶ corresponds to line D1-152 of Table D1 and the combination of R¹ and R² for each individual compound corresponds in each case to one line of Table B (compounds I.A.D1-152.B-1 to I.A.D1-152.B-460).

### Table 153a

Compounds of the formula I.A in which the combination of R³, R⁵ and R⁶ corresponds to line D1-153 of Table D1 and the combination of R¹ and R² for each individual compound corresponds in each case to one line of Table B (compounds I.A.D1-153.B-1 to I.A.D1-153.B-460).

### Table 154a

Compounds of the formula I.A in which the combination of R³, R⁵ and R⁶ corresponds to line D1-154 of Table D1 and the combination of R¹ and R² for each individual compound corresponds in each case to one line of Table B (compounds I.A.D1-154.B-1 to I.A.D1-154.B-460).

### Table 155a

Compounds of the formula I.A in which the combination of R³, R⁵ and R⁶ corresponds to line D1-155 of Table D1 and the combination of R¹ and R² for each individual compound corresponds in each case to one line of Table B (compounds I.A.D1-155.B-1 to I.A.D1-155.B-460).

### Table 156a

Compounds of the formula I.A in which the combination of R³, R⁵ and R⁶ corresponds to line D1-156 of Table D1 and the combination of R¹ and R² for each individual compound corresponds in each case to one line of Table B (compounds I.A.D1-156.B-1 to I.A.D1-156.B-460).

### Table 157a

Compounds of the formula I.A in which the combination of R³, R⁵ and R⁶ corresponds to line D1-157 of Table D1 and the combination of R¹ and R² for each individual compound corresponds in each case to one line of Table B (compounds I.A.D1-157.B-1 to I.A.D1-157.B-460).

### Table 158a

Compounds of the formula I.A in which the combination of R³, R⁵ and R⁶ corresponds to line D1-158 of Table D1 and the combination of R¹ and R² for each individual compound corresponds in each case to one line of Table B (compounds I.A.D1-158.B-1 to I.A.D1-158.B-460).

### Table 159a

Compounds of the formula I.A in which the combination of R³, R⁵ and R⁶ corresponds to line D1-159 of Table D1 and the combination of R¹ and R² for each individual compound corresponds in each case to one line of Table B (compounds I.A.D1-159.B-1 to I.A.D1-159.B-460).

### Table 160a

Compounds of the formula I.A in which the combination of R³, R⁵ and R⁶ corresponds to line D1-160 of Table D1 and the combination of R¹ and R² for each individual compound corresponds in each case to one line of Table B (compounds I.A.D1-160.B-1 to I.A.D1-160.B-460).

### Table 161 a

Compounds of the formula I.A in which the combination of R³, R⁵ and R⁶ corresponds to line D1-161 of Table D1 and the combination of R¹ and R² for each individual compound corresponds in each case to one line of Table B (compounds I.A.D1-161.B-1 to I.A.D1-161.B-460).

### Table 162a

Compounds of the formula I.A in which the combination of R³, R⁵ and R⁶ corresponds to line D1-162 of Table D1 and the combination of R¹ and R² for each individual compound corresponds in each case to one line of Table B (compounds I.A.D1-162.B-1 to I.A.D1-162.B-460).

### Table 163a

Compounds of the formula I.A in which the combination of R³, R⁵ and R⁶ corresponds to line D1-163 of Table D1 and the combination of R¹ and R² for each individual compound corresponds in each case to one line of Table B (compounds I.A.D1-163.B-1 to I.A.D1-163.B-460).

### Table 164a

Compounds of the formula I.A in which the combination of R³, R⁵ and R⁶ corresponds to line D1-164 of Table D1 and the combination of R¹ and R² for each individual compound corresponds in each case to one line of Table B (compounds I.A.D1-164.B-1 to I.A.D1-164.B-460).

### Table 165a

Compounds of the formula I.A in which the combination of R³, R⁵ and R⁶ corresponds to line D1-165 of Table D1 and the combination of R¹ and R² for each individual compound corresponds in each case to one line of Table B (compounds I.A.D1-165.B-1 to I.A.D1-165.B-460).

### Table 166a

Compounds of the formula I.A in which the combination of R³, R⁵ and R⁶ corresponds to line D1-166 of Table D1 and the combination of R¹ and R² for each individual compound corresponds in each case to one line of Table B (compounds I.A.D1-166.B-1 to I.A.D1-166.B-460).

### Table 167a

Compounds of the formula I.A in which the combination of R³, R⁵ and R⁶ corresponds to line D1-167 of Table D1 and the combination of R¹ and R² for each individual compound corresponds in each case to one line of Table B (compounds I.A.D1-167.B-1 to I.A.D1-167.B-460).

### Table 168a

Compounds of the formula I.A in which the combination of R³, R⁵ and R⁶ corresponds to line D1-168 of Table D1 and the combination of R¹ and R² for each individual compound corresponds in each case to one line of Table B (compounds I.A.D1-168.B-1 to I.A.D1-168.B-460).

### Table 169a

Compounds of the formula I.A in which the combination of R³, R⁵ and R⁶ corresponds to line D1-169 of Table D1 and the combination of R¹ and R² for each individual compound corresponds in each case to one line of Table B (compounds I.A.D1-169.B-1 to I.A.D1-169.B-460).

### Table 170a

Compounds of the formula I.A in which the combination of R³, R⁵ and R⁶ corresponds to line D1-170 of Table D1 and the combination of R¹ and R² for each individual compound corresponds in each case to one line of Table B (compounds I.A.D1-170.B-1 to I.A.D1-170.B-460).

### Table 171 a

Compounds of the formula I.A in which the combination of R³, R⁵ and R⁶ corresponds to line D1-171 of Table D1 and the combination of R¹ and R² for each individual compound corresponds in each case to one line of Table B (compounds I.A.D1-171.B-1 to I.A.D1-171.B-460).

### Table 172a

Compounds of the formula I.A in which the combination of R³, R⁵ and R⁶ corresponds to line D1-172 of Table D1 and the combination of R¹ and R² for each individual compound corresponds in each case to one line of Table B (compounds I.A.D1-172.B-1 to I.A.D1-172.B-460).

### Table 173a

Compounds of the formula I.A in which the combination of R³, R⁵ and R⁶ corresponds to line D1-173 of Table D1 and the combination of R¹ and R² for each individual compound corresponds in each case to one line of Table B (compounds I.A.D1-173.B-1 to I.A.D1-173.B-460).

### Table 174a

Compounds of the formula I.A in which the combination of R³, R⁵ and R⁶ corresponds to line D1-174 of Table D1 and the combination of R¹ and R² for each individual compound corresponds in each case to one line of Table B (compounds I.A.D1-174.B-1 to I.A.D1-174.B-460).

### Table 175a

Compounds of the formula I.A in which the combination of R³, R⁵ and R⁶ corresponds to line D1-175 of Table D1 and the combination of R¹ and R² for each individual compound corresponds in each case to one line of Table B (compounds I.A.D1-175.B-1 to I.A.D1-175.B-460).

### Table 176a

Compounds of the formula I.A in which the combination of R³, R⁵ and R⁶ corresponds to line D1-176 of Table D1 and the combination of R¹ and R² for each individual compound corresponds in each case to one line of Table B (compounds I.A.D1-176.B-1 to I.A.D1-176.B-460).

### Table 177a

Compounds of the formula I.A in which the combination of R³, R⁵ and R⁶ corresponds to line

D1-177 of Table D1 and the combination of R¹ and R² for each individual compound corresponds in each case to one line of Table B (compounds I.A.D1-177.B-1 to I.A.D1-177.B-460).

### Table 178a

Compounds of the formula I.A in which the combination of R³, R⁵ and R⁶ corresponds to line D1-178 of Table D1 and the combination of R¹ and R² for each individual compound corresponds in each case to one line of Table B (compounds I.A.D1-178.B-1 to I.A.D1-178.B-460).

### Table 179a

Compounds of the formula I.A in which the combination of R³, R⁵ and R⁶ corresponds to line D1-179 of Table D1 and the combination of R¹ and R² for each individual compound corresponds in each case to one line of Table B (compounds I.A.D1-179.B-1 to I.A.D1-179.B-460).

### Table 180a

Compounds of the formula I.A in which the combination of R³, R⁵ and R⁶ corresponds to line D1-180 of Table D1 and the combination of R¹ and R² for each individual compound corresponds in each case to one line of Table B (compounds I.A.D1-180.B-1 to I.A.D1-180.B-460).

### Table 181 a

Compounds of the formula I.A in which the combination of R³, R⁵ and R⁶ corresponds to line D1-181 of Table D1 and the combination of R¹ and R² for each individual compound corresponds in each case to one line of Table B (compounds I.A.D1-181.B-1 to I.A.D1-181.B-460).

### Table 182a

Compounds of the formula I.A in which the combination of R³, R⁵ and R⁶ corresponds to line D1-182 of Table D1 and the combination of R¹ and R² for each individual compound corresponds in each case to one line of Table B (compounds I.A.D1-182.B-1 to I.A.D1-182.B-460).

### Table 183a

Compounds of the formula I.A in which the combination of R³, R⁵ and R⁶ corresponds to line D1-183 of Table D1 and the combination of R¹ and R² for each individual compound corresponds in each case to one line of Table B (compounds I.A.D1-183.B-1 to I.A.D1-183.B-460).

### Table 184a

Compounds of the formula I.A in which the combination of R³, R⁵ and R⁶ corresponds to line D1-184 of Table D1 and the combination of R¹ and R² for each individual compound corresponds in each case to one line of Table B (compounds I.A.D1-184.B-1 to I.A.D1-184.B-460).

### Table 185a

Compounds of the formula I.A in which the combination of R³, R⁵ and R⁶ corresponds to line D1-185 of Table D1 and the combination of R¹ and R² for each individual compound corresponds in each case to one line of Table B (compounds I.A.D1-185.B-1 to I.A.D1-185.B-460).

### Table 186a

Compounds of the formula I.A in which the combination of R³, R⁵ and R⁶ corresponds to line D1-186 of Table D1 and the combination of R¹ and R² for each individual compound corresponds in each case to one line of Table B (compounds I.A.D1-186.B-1 to I.A.D1-186.B-460).

### Table 187a

Compounds of the formula I.A in which the combination of R³, R⁵ and R⁶ corresponds to line D1-187 of Table D1 and the combination of R¹ and R² for each individual compound corresponds in each case to one line of Table B (compounds I.A.D1-187.B-1 to I.A.D1-187.B-460).

### Table 188a

Compounds of the formula I.A in which the combination of R³, R⁵ and R⁶ corresponds to line D1-188 of Table D1 and the combination of R¹ and R² for each individual compound corresponds in each case to one line of Table B (compounds I.A.D1-188.B-1 to I.A.D1-188.B-460).

### Table 189a

Compounds of the formula I.A in which the combination of R³, R⁵ and R⁶ corresponds to line D1-189 of Table D1 and the combination of R¹ and R² for each individual compound corresponds in each case to one line of Table B (compounds I.A.D1-189.B-1 to I.A.D1-189.B-460).

### Table 190a

Compounds of the formula I.A in which the combination of R³, R⁵ and R⁶ corresponds to line D1-190 of Table D1 and the combination of R¹ and R² for each individual compound corresponds in each case to one line of Table B (compounds I.A.D1-190.B-1 to I.A.D1-190.B-460).

### Table 191a

Compounds of the formula I.A in which the combination of R³, R⁵ and R⁶ corresponds to line D1-191 of Table D1 and the combination of R¹ and R² for each individual compound corresponds in each case to one line of Table B (compounds I.A.D1-191.B-1 to I.A.D1-191.B-460).

### Table 192a

Compounds of the formula I.A in which the combination of R³, R⁵ and R⁶ corresponds to line D1-192 of Table D1 and the combination of R¹ and R² for each individual compound corresponds in each case to one line of Table B (compounds I.A.D1-192.B-1 to I.A.D1-192.B-460).

### Table 193a

Compounds of the formula I.A in which the combination of R³, R⁵ and R⁶ corresponds to line D1-193 of Table D1 and the combination of R¹ and R² for each individual compound corresponds in each case to one line of Table B (compounds I.A.D1-193.B-1 to I.A.D1-193.B-460).

### Table 194a

Compounds of the formula I.A in which the combination of R³, R⁵ and R⁶ corresponds to line D1-194 of Table D1 and the combination of R¹ and R² for each individual compound corresponds in each case to one line of Table B (compounds I.A.D1-194.B-1 to I.A.D1-194.B-460).

### Table 195a

Compounds of the formula I.A in which the combination of R³, R⁵ and R⁶ corresponds to line D1-195 of Table D1 and the combination of R¹ and R² for each individual compound corresponds in each case to one line of Table B (compounds I.A.D1-195.B-1 to I.A.D1-195.B-460).

### Table 196a

Compounds of the formula I.A in which the combination of R³, R⁵ and R⁶ corresponds to line D1-196 of Table D1 and the combination of R¹ and R² for each individual compound corresponds in each case to one line of Table B (compounds I.A.D1-196.B-1 to I.A.D1-196.B-460).

### Table 197a

Compounds of the formula I.A in which the combination of R³, R⁵ and R⁶ corresponds to line D1-197 of Table D1 and the combination of R¹ and R² for each individual compound corresponds in each case to one line of Table B (compounds I.A.D1-197.B-1 to I.A.D1-197.B-460).

### Table 198a

Compounds of the formula I.A in which the combination of R³, R⁵ and R⁶ corresponds to line D1-198 of Table D1 and the combination of R¹ and R² for each individual compound corresponds in each case to one line of Table B (compounds I.A.D1-198.B-1 to I.A.D1-198.B-460).

### Table 199a

Compounds of the formula I.A in which the combination of R³, R⁵ and R⁶ corresponds to line D1-199 of Table D1 and the combination of R¹ and R² for each individual compound corresponds in each case to one line of Table B (compounds I.A.D1-199.B-1 to I.A.D1-199.B-460).

### Table 200a

Compounds of the formula I.A in which the combination of R³, R⁵ and R⁶ corresponds to line D1-200 of Table D1 and the combination of R¹ and R² for each individual compound corresponds in each case to one line of Table B (compounds I.A.D1-200.B-1 to I.A.D1-200.B-460).

### Table 201 a

Compounds of the formula I.A in which the combination of R³, R⁵ and R⁶ corresponds to line D1-201 of Table D1 and the combination of R¹ and R² for each individual compound corresponds in each case to one line of Table B (compounds I.A.D1-201.B-1 to I.A.D1-201.B-460).

### Table 202a

Compounds of the formula I.A in which the combination of R³, R⁵ and R⁶ corresponds to line D1-202 of Table D1 and the combination of R¹ and R² for each individual compound corresponds in each case to one line of Table B (compounds I.A.D1-202.B-1 to I.A.D1-202.B-460).

### Table 203a

Compounds of the formula I.A in which the combination of R³, R⁵ and R⁶ corresponds to line D1-203 of Table D1 and the combination of R¹ and R² for each individual compound corresponds in each case to one line of Table B (compounds I.A.D1-203.B-1 to I.A.D1-203.B-460).

### Table 204a

Compounds of the formula I.A in which the combination of R³, R⁵ and R⁶ corresponds to line D1-204 of Table D1 and the combination of R¹ and R² for each individual compound corresponds in each case to one line of Table B (compounds I.A.D1-204.B-1 to I.A.D1-204.B-460).

### Table 205a

Compounds of the formula I.A in which the combination of R³, R⁵ and R⁶ corresponds to line D1-205 of Table D1 and the combination of R¹ and R² for each individual compound corresponds in each case to one line of Table B (compounds I.A.D1-205.B-1 to I.A.D1-205.B-460).

### Table 206a

Compounds of the formula I.A in which the combination of R³, R⁵ and R⁶ corresponds to line D1-206 of Table D1 and the combination of R¹ and R² for each individual compound corresponds in each case to one line of Table B (compounds I.A.D1-206.B-1 to I.A.D1-206.B-460).

### Table 207a

Compounds of the formula I.A in which the combination of R³, R⁵ and R⁶ corresponds to line D1-207 of Table D1 and the combination of R¹ and R² for each individual compound corresponds in each case to one line of Table B (compounds I.A.D1-207.B-1 to I.A.D1-207.B-460).

### Table 208a

Compounds of the formula I.A in which the combination of R³, R⁵ and R⁶ corresponds to line D1-208 of Table D1 and the combination of R¹ and R² for each individual compound corresponds in each case to one line of Table B (compounds I.A.D1-208.B-1 to I.A.D1-208.B-460).

### Table 209a

Compounds of the formula I.A in which the combination of R³, R⁵ and R⁶ corresponds to line D1-209 of Table D1 and the combination of R¹ and R² for each individual compound corresponds in each case to one line of Table B (compounds I.A.D1-209.B-1 to I.A.D1-209.B-460).

### Table 210a

Compounds of the formula I.A in which the combination of R³, R⁵ and R⁶ corresponds to line D1-210 of Table D1 and the combination of R¹ and R² for each individual compound corresponds in each case to one line of Table B (compounds I.A.D1-210.B-1 to I.A.D1-210.B-460).

### Table 211 a

Compounds of the formula I.A in which the combination of R³, R⁵ and R⁶ corresponds to line D1-211 of Table D1 and the combination of R¹ and R² for each individual compound corresponds in each case to one line of Table B (compounds I.A.D1-211.B-1 to I.A.D1-211.B-460).

### Table 212a

Compounds of the formula I.A in which the combination of R³, R⁵ and R⁶ corresponds to line D1-212 of Table D1 and the combination of R¹ and R² for each individual compound corresponds in each case to one line of Table B (compounds I.A.D1-212.B-1 to I.A.D1-212.B-460).

### Table 213a

Compounds of the formula I.A in which the combination of R³, R⁵ and R⁶ corresponds to line D1-213 of Table D1 and the combination of R¹ and R² for each individual compound corresponds in each case to one line of Table B (compounds I.A.D1-213.B-1 to I.A.D1-213.B-460).

### Table 214a

Compounds of the formula I.A in which the combination of R³, R⁵ and R⁶ corresponds to line D1-214 of Table D1 and the combination of R¹ and R² for each individual compound corresponds in each case to one line of Table B (compounds I.A.D1-214.B-1 to I.A.D1-214.B-460).

### Table 215a

Compounds of the formula I.A in which the combination of R³, R⁵ and R⁶ corresponds to line D1-215 of Table D1 and the combination of R¹ and R² for each individual compound corresponds in each case to one line of Table B (compounds I.A.D1-215.B-1 to I.A.D1-215.B-460).

### Table 216a

Compounds of the formula I.A in which the combination of R³, R⁵ and R⁶ corresponds to line D1-216 of Table D1 and the combination of R¹ and R² for each individual compound corresponds in each case to one line of Table B (compounds I.A.D1-216.B-1 to I.A.D1-216.B-460).

### Table 217a

Compounds of the formula I.A in which the combination of R³, R⁵ and R⁶ corresponds to line D1-217 of Table D1 and the combination of R¹ and R² for each individual compound corresponds in each case to one line of Table B (compounds I.A.D1-217.B-1 to I.A.D1-217.B-460).

### Table 218a

Compounds of the formula I.A in which the combination of R³, R⁵ and R⁶ corresponds to line

D1-218 of Table D1 and the combination of R¹ and R² for each individual compound corresponds in each case to one line of Table B (compounds I.A.D1-218.B-1 to I.A.D1-218.B-460).

### Table 219a

Compounds of the formula I.A in which the combination of R³, R⁵ and R⁶ corresponds to line D1-219 of Table D1 and the combination of R¹ and R² for each individual compound corresponds in each case to one line of Table B (compounds I.A.D1-219.B-1 to I.A.D1-219.B-460).

### Table 220a

Compounds of the formula I.A in which the combination of R³, R⁵ and R⁶ corresponds to line D1-220 of Table D1 and the combination of R¹ and R² for each individual compound corresponds in each case to one line of Table B (compounds I.A.D1-220.B-1 to I.A.D1-220.B-460).

### Table 221 a

Compounds of the formula I.A in which the combination of R³, R⁵ and R⁶ corresponds to line D1-221 of Table D1 and the combination of R¹ and R² for each individual compound corresponds in each case to one line of Table B (compounds I.A.D1-221.B-1 to I.A.D1-221.B-460).

### Table 222a

Compounds of the formula I.A in which the combination of R³, R⁵ and R⁶ corresponds to line D1-222 of Table D1 and the combination of R¹ and R² for each individual compound corresponds in each case to one line of Table B (compounds I.A.D1-222.B-1 to I.A.D1-222.B-460).

### Table 223a

Compounds of the formula I.A in which the combination of R³, R⁵ and R⁶ corresponds to line D1-223 of Table D1 and the combination of R¹ and R² for each individual compound corresponds in each case to one line of Table B (compounds I.A.D1-223.B-1 to I.A.D1-223.B-460).

### Table 224a

Compounds of the formula I.A in which the combination of R³, R⁵ and R⁶ corresponds to line D1-224 of Table D1 and the combination of R¹ and R² for each individual compound corresponds in each case to one line of Table B (compounds I.A.D1-224.B-1 to I.A.D1-224.B-460).

### Table 225a

Compounds of the formula I.A in which the combination of R³, R⁵ and R⁶ corresponds to line D1-225 of Table D1 and the combination of R¹ and R² for each individual compound corresponds in each case to one line of Table B (compounds I.A.D1-225.B-1 to I.A.D1-225.B-460).

### Table 226a

Compounds of the formula I.A in which the combination of R³, R⁵ and R⁶ corresponds to line D1-226 of Table D1 and the combination of R¹ and R² for each individual compound corresponds in each case to one line of Table B (compounds I.A.D1-226.B-1 to I.A.D1-226.B-460).

### Table 227a

Compounds of the formula I.A in which the combination of R³, R⁵ and R⁶ corresponds to line D1-227 of Table D1 and the combination of R¹ and R² for each individual compound corresponds in each case to one line of Table B (compounds I.A.D1-227.B-1 to I.A.D1-227.B-460).

### Table 228a

Compounds of the formula I.A in which the combination of R³, R⁵ and R⁶ corresponds to line D1-228 of Table D1 and the combination of R¹ and R² for each individual compound corresponds in each case to one line of Table B (compounds I.A.D1-228.B-1 to I.A.D1-228.B-460).

### Table 229a

Compounds of the formula I.A in which the combination of R³, R⁵ and R⁶ corresponds to line D1-229 of Table D1 and the combination of R¹ and R² for each individual compound corresponds in each case to one line of Table B (compounds I.A.D1-229.B-1 to I.A.D1-229.B-460).

### Table 230a

Compounds of the formula I.A in which the combination of R³, R⁵ and R⁶ corresponds to line D1-230 of Table D1 and the combination of R¹ and R² for each individual compound corresponds in each case to one line of Table B (compounds I.A.D1-230.B-1 to I.A.D1-230.B-460).

### Table 231 a

Compounds of the formula I.A in which the combination of R³, R⁵ and R⁶ corresponds to line D1-231 of Table D1 and the combination of R¹ and R² for each individual compound corresponds in each case to one line of Table B (compounds I.A.D1-231.B-1 to I.A.D1-231.B-460).

### Table 232a

Compounds of the formula I.A in which the combination of R³, R⁵ and R⁶ corresponds to line D1-232 of Table D1 and the combination of R¹ and R² for each individual compound corresponds in each case to one line of Table B (compounds I.A.D1-232.B-1 to I.A.D1-232.B-460).

### Table 233a

Compounds of the formula I.A in which the combination of R³, R⁵ and R⁶ corresponds to line D1-233 of Table D1 and the combination of R¹ and R² for each individual compound corresponds in each case to one line of Table B (compounds I.A.D1-233.B-1 to I.A.D1-233.B-460).

### Table 234a

Compounds of the formula I.A in which the combination of R³, R⁵ and R⁶ corresponds to line D1-234 of Table D1 and the combination of R¹ and R² for each individual compound corresponds in each case to one line of Table B (compounds I.A.D1-234.B-1 to I.A.D1-234.B-460).

### Table 235a

Compounds of the formula I.A in which the combination of R³, R⁵ and R⁶ corresponds to line D1-235 of Table D1 and the combination of R¹ and R² for each individual compound corresponds in each case to one line of Table B (compounds I.A.D1-235.B-1 to I.A.D1-235.B-460).

### Table 236a

Compounds of the formula I.A in which the combination of R³, R⁵ and R⁶ corresponds to line D1-236 of Table D1 and the combination of R¹ and R² for each individual compound corresponds in each case to one line of Table B (compounds I.A.D1-236.B-1 to I.A.D1-236.B-460).

### Table 237a

Compounds of the formula I.A in which the combination of R³, R⁵ and R⁶ corresponds to line D1-237 of Table D1 and the combination of R¹ and R² for each individual compound corresponds in each case to one line of Table B (compounds I.A.D1-237.B-1 to I.A.D1-237.B-460).

### Table 238a

Compounds of the formula I.A in which the combination of R³, R⁵ and R⁶ corresponds to line D1-238 of Table D1 and the combination of R¹ and R² for each individual compound corresponds in each case to one line of Table B (compounds I.A.D1-238.B-1 to I.A.D1-238.B-460).

### Table 239a

Compounds of the formula I.A in which the combination of R³, R⁵ and R⁶ corresponds to line D1-239 of Table D1 and the combination of R¹ and R² for each individual compound corresponds in each case to one line of Table B (compounds I.A.D1-239.B-1 to I.A.D1-239.B-460).

### Table 240a

Compounds of the formula I.A in which the combination of R³, R⁵ and R⁶ corresponds to line D1-240 of Table D1 and the combination of R¹ and R² for each individual compound corresponds in each case to one line of Table B (compounds I.A.D1-240.B-1 to I.A.D1-240.B-460).

### Table 241 a

Compounds of the formula I.A in which the combination of R³, R⁵ and R⁶ corresponds to line D1-241 of Table D1 and the combination of R¹ and R² for each individual compound corresponds in each case to one line of Table B (compounds I.A.D1-241.B-1 to I.A.D1-241.B-460).

### Table 242a

Compounds of the formula I.A in which the combination of R³, R⁵ and R⁶ corresponds to line D1-242 of Table D1 and the combination of R¹ and R² for each individual compound corresponds in each case to one line of Table B (compounds I.A.D1-242.B-1 to I.A.D1-242.B-460).

### Table 243a

Compounds of the formula I.A in which the combination of R³, R⁵ and R⁶ corresponds to line D1-243 of Table D1 and the combination of R¹ and R² for each individual compound corresponds in each case to one line of Table B (compounds I.A.D1-243.B-1 to I.A.D1-243.B-460).

### Table 244a

Compounds of the formula I.A in which the combination of R³, R⁵ and R⁶ corresponds to line D1-244 of Table D1 and the combination of R¹ and R² for each individual compound corresponds in each case to one line of Table B (compounds I.A.D1-244.B-1 to I.A.D1-244.B-460).

### Table 245a

Compounds of the formula I.A in which the combination of R³, R⁵ and R⁶ corresponds to line D1-245 of Table D1 and the combination of R¹ and R² for each individual compound corresponds in each case to one line of Table B (compounds I.A.D1-245.B-1 to I.A.D1-245.B-460).

### Table 246a

Compounds of the formula I.A in which the combination of R³, R⁵ and R⁶ corresponds to line D1-246 of Table D1 and the combination of R¹ and R² for each individual compound corresponds in each case to one line of Table B (compounds I.A.D1-246.B-1 to I.A.D1-246.B-460).

### Table 247a

Compounds of the formula I.A in which the combination of R³, R⁵ and R⁶ corresponds to line D1-247 of Table D1 and the combination of R¹ and R² for each individual compound corresponds in each case to one line of Table B (compounds I.A.D1-247.B-1 to I.A.D1-247.B-460).

### Table 248a

Compounds of the formula I.A in which the combination of R³, R⁵ and R⁶ corresponds to line D1-248 of Table D1 and the combination of R¹ and R² for each individual compound corresponds in each case to one line of Table B (compounds I.A.D1-248.B-1 to I.A.D1-248.B-460).

### Table 1 b

Compounds of the formula I.B in which the combination of R³ and R⁶ corresponds to line D2-1 of Table D2 and the combination of R¹ and R² for each individual compound corresponds in each case to one line of Table B (compounds I.B.D2-1.B-1 to I.B.D2-1.B-460).

### Table 2b

Compounds of the formula I.B in which the combination of R³ and R⁶ corresponds to line D2-2 of Table D2 and the combination of R¹ and R² for each individual compound corresponds in each case to one line of Table B (compounds I.B.D2-2.B-1 to I.B.D2-2.B-460).

### Table 3b

Compounds of the formula I.B in which the combination of R³ and R⁶ corresponds to line D2-3 of Table D2 and the combination of R¹ and R² for each individual compound corresponds in each case to one line of Table B (compounds I.B.D2-3.B-1 to I.B.D2-3.B-460).

### Table 4b

Compounds of the formula I.B in which the combination of R³ and R⁶ corresponds to line D2-4 of Table D2 and the combination of R¹ and R² for each individual compound corresponds in each case to one line of Table B (compounds I.B.D2-4.B-1 to I.B.D2-4.B-460).

### Table 5b

Compounds of the formula I.B in which the combination of R³ and R⁶ corresponds to line D2-5 of Table D2 and the combination of R¹ and R² for each individual compound corresponds in each case to one line of Table B (compounds I.B.D2-5.B-1 to I.B.D2-5.B-460).

### Table 6b

Compounds of the formula I.B in which the combination of R³ and R⁶ corresponds to line D2-6 of Table D2 and the combination of R¹ and R² for each individual compound corresponds in each case to one line of Table B (compounds I.B.D2-6.B-1 to I.B.D2-6.B-460).

### Table 7b

Compounds of the formula I.B in which the combination of R³ and R⁶ corresponds to line D2-7 of Table D2 and the combination of R¹ and R² for each individual compound corresponds in each case to one line of Table B (compounds I.B.D2-7.B-1 to I.B.D2-7.B-460).

### Table 8b

Compounds of the formula I.B in which the combination of R³ and R⁶ corresponds to line D2-8 of Table D2 and the combination of R¹ and R² for each individual compound corresponds in each case to one line of Table B (compounds I.B.D2-8.B-1 to I.B.D2-8.B-460).

### Table 9b

Compounds of the formula I.B in which the combination of R³ and R⁶ corresponds to line D2-9 of Table D2 and the combination of R¹ and R² for each individual compound corresponds in each case to one line of Table B (compounds I.B.D2-9.B-1 to I.B.D2-9.B-460).

### Table 10b

Compounds of the formula I.B in which the combination of R³ and R⁶ corresponds to line D2-10 of Table D2 and the combination of R¹ and R² for each individual compound corresponds in each case to one line of Table B (compounds I.B.D2-10.B-1 to I.B.D2-10.B-460).

### Table 11b

Compounds of the formula I.B in which the combination of R³ and R⁶ corresponds to line D2-11 of Table D2 and the combination of R¹ and R² for each individual compound corresponds in each case to one line of Table B (compounds I.B.D2-11.B-1 to I.B.D2-11.B-460).

### Table 12b

Compounds of the formula I.B in which the combination of R³ and R⁶ corresponds to line D2-12 of Table D2 and the combination of R¹ and R² for each individual compound corresponds in each case to one line of Table B (compounds I.B.D2-12.B-1 to I.B.D2-12.B-460).

### Table 13b

Compounds of the formula I.B in which the combination of R³ and R⁶ corresponds to line D2-13 of Table D2 and the combination of R¹ and R² for each individual compound corresponds in each case to one line of Table B (compounds I.B.D2-13.B-1 to I.B.D2-13.B-460).

### Table 14b

Compounds of the formula I.B in which the combination of R³ and R⁶ corresponds to line D2-14 of Table D2 and the combination of R¹ and R² for each individual compound corresponds in each case to one line of Table B (compounds I.B.D2-14.B-1 to I.B.D2-14.B-460).

### Table 15b

Compounds of the formula I.B in which the combination of R³ and R⁶ corresponds to line D2-15 of Table D2 and the combination of R¹ and R² for each individual compound corresponds in each case to one line of Table B (compounds I.B.D2-15.B-1 to I.B.D2-15.B-460).

### Table 16b

Compounds of the formula I.B in which the combination of R³ and R⁶ corresponds to line D2-16 of Table D2 and the combination of R¹ and R² for each individual compound corresponds in each case to one line of Table B (compounds I.B.D2-16.B-1 to I.B.D2-16.B-460).

### Table 17b

Compounds of the formula I.B in which the combination of R³ and R⁶ corresponds to line D2-17 of Table D2 and the combination of R¹ and R² for each individual compound corresponds in each case to one line of Table B (compounds I.B.D2-17.B-1 to I.B.D2-17.B-460).

### Table 18b

Compounds of the formula I.B in which the combination of R³ and R⁶ corresponds to line D2-18 of Table D2 and the combination of R¹ and R² for each individual compound corresponds in each case to one line of Table B (compounds I.B.D2-18.B-1 to I.B.D2-18.B-460).

### Table 19b

Compounds of the formula I.B in which the combination of R³ and R⁶ corresponds to line D2-19 of Table D2 and the combination of R¹ and R² for each individual compound corresponds in each case to one line of Table B (compounds I.B.D2-19.B-1 to I.B.D2-19.B-460).

### Table 20b

Compounds of the formula I.B in which the combination of R³ and R⁶ corresponds to line D2-20 of Table D2 and the combination of R¹ and R² for each individual compound corresponds in each case to one line of Table B (compounds I.B.D2-20.B-1 to I.B.D2-20.B-460).

### Table 1 c

Compounds of the formula I.C in which the combination of R³, R⁵ and R⁶ corresponds to line D3-1 of Table D3 and the combination of R¹ and R² for each individual compound corresponds in each case to one line of Table B (compounds I.C.D3-1.B-1 to I.C.D3-1.B-460).

### Table 2c

Compounds of the formula I.C in which the combination of R³, R⁵ and R⁶ corresponds to line D3-2 of Table D3 and the combination of R¹ and R² for each individual compound corresponds in each case to one line of Table B (compounds I.C.D3-2.B-1 to I.C.D3-2.B-460).

### Table 3c

Compounds of the formula I.C in which the combination of R³, R⁵ and R⁶ corresponds to line D3-3 of Table D3 and the combination of R¹ and R² for each individual compound corresponds in each case to one line of Table B (compounds I.C.D3-3.B-1 to I.C.D3-3.B-460).

### Table 4c

Compounds of the formula I.C in which the combination of R³, R⁵ and R⁶ corresponds to line D3-4 of Table D3 and the combination of R¹ and R² for each individual compound corresponds in each case to one line of Table B (compounds I.C.D3-4.B-1 to I.C.D3-4.B-460).

### Table 5c

Compounds of the formula I.C in which the combination of R³, R⁵ and R⁶ corresponds to line D3-5 of Table D3 and the combination of R¹ and R² for each individual compound corresponds in each case to one line of Table B (compounds I.C.D3-5.B-1 to I.C.D3-5.B-460).

### Table 6c

Compounds of the formula I.C in which the combination of R³, R⁵ and R⁶ corresponds to line D3-6 of Table D3 and the combination of R¹ and R² for each individual compound corresponds in each case to one line of Table B (compounds I.C.D3-6.B-1 to I.C.D3-6.B-460).

### Table 7c

Compounds of the formula I.C in which the combination of R³, R⁵ and R⁶ corresponds to line D3-7 of Table D3 and the combination of R¹ and R² for each individual compound corresponds in each case to one line of Table B (compounds I.C.D3-7.B-1 to I.C.D3-7.B-460).

### Table 8c

Compounds of the formula I.C in which the combination of R³, R⁵ and R⁶ corresponds to line D3-8 of Table D3 and the combination of R¹ and R² for each individual compound corresponds in each case to one line of Table B (compounds I.C.D3-8.B-1 to I.C.D3-8.B-460).

### Table 9c

Compounds of the formula I.C in which the combination of R³, R⁵ and R⁶ corresponds to line D3-9 of Table D3 and the combination of R¹ and R² for each individual compound corresponds in each case to one line of Table B (compounds I.C.D3-9.B-1 to I.C.D3-9.B-460).

### Table 10c

Compounds of the formula I.C in which the combination of R³, R⁵ and R⁶ corresponds to line D3-10 of Table D3 and the combination of R¹ and R² for each individual compound corresponds in each case to one line of Table B (compounds I.C.D3-10.B-1 to I.C.D3-10.B-460).

### Table 11c

Compounds of the formula I.C in which the combination of R³, R⁵ and R⁶ corresponds to line D3-11 of Table D3 and the combination of R¹ and R² for each individual compound corresponds in each case to one line of Table B (compounds I.C.D3-11.B-1 to I.C.D3-11.B-460).

### Table 12c

Compounds of the formula I.C in which the combination of R³, R⁵ and R⁶ corresponds to line D3-12 of Table D3 and the combination of R¹ and R² for each individual compound corresponds in each case to one line of Table B (compounds I.C.D3-12.B-1 to I.C.D3-12.B-460).

### Table 13c

Compounds of the formula I.C in which the combination of R³, R⁵ and R⁶ corresponds to line D3-13 of Table D3 and the combination of R¹ and R² for each individual compound corresponds in each case to one line of Table B (compounds I.C.D3-13.B-1 to I.C.D3-13.B-460).

### Table 14c

Compounds of the formula I.C in which the combination of R³, R⁵ and R⁶ corresponds to line D3-14 of Table D3 and the combination of R¹ and R² for each individual compound corresponds in each case to one line of Table B (compounds I.C.D3-14.B-1 to I.C.D3-14.B-460).

### Table 15c

Compounds of the formula I.C in which the combination of R³, R⁵ and R⁶ corresponds to line D3-15 of Table D3 and the combination of R¹ and R² for each individual compound corresponds in each case to one line of Table B (compounds I.C.D3-15.B-1 to I.C.D3-15.B-460).

### Table 16c

Compounds of the formula I.C in which the combination of R³, R⁵ and R⁶ corresponds to line D3-16 of Table D3 and the combination of R¹ and R² for each individual compound corresponds in each case to one line of Table B (compounds I.C.D3-16.B-1 to I.C.D3-16.B-460).

### Table 17c

Compounds of the formula I.C in which the combination of R³, R⁵ and R⁶ corresponds to line D3-17 of Table D3 and the combination of R¹ and R² for each individual compound corresponds in each case to one line of Table B (compounds I.C.D3-17.B-1 to I.C.D3-17.B-460).

### Table 18c

Compounds of the formula I.C in which the combination of R³, R⁵ and R⁶ corresponds to line D3-18 of Table D3 and the combination of R¹ and R² for each individual compound corresponds in each case to one line of Table B (compounds I.C.D3-18.B-1 to I.C.D3-18.B-460).

### Table 19c

Compounds of the formula I.C in which the combination of R³, R⁵ and R⁶ corresponds to line D3-19 of Table D3 and the combination of R¹ and R² for each individual compound corresponds in each case to one line of Table B (compounds I.C.D3-19.B-1 to I.C.D3-19.B-460).

### Table 20c

Compounds of the formula I.C in which the combination of R³, R⁵ and R⁶ corresponds to line D3-20 of Table D3 and the combination of R¹ and R² for each individual compound corresponds in each case to one line of Table B (compounds I.C.D3-20.B-1 to I.C.D3-20.B-460).

### Table 21 c

Compounds of the formula I.C in which the combination of R³, R⁵ and R⁶ corresponds to line D3-21 of Table D3 and the combination of R¹ and R² for each individual compound corresponds in each case to one line of Table B (compounds I.C.D3-21.B-1 to I.C.D3-21.B-460).

### Table 22c

Compounds of the formula I.C in which the combination of R³, R⁵ and R⁶ corresponds to line D3-22 of Table D3 and the combination of R¹ and R² for each individual compound corresponds in each case to one line of Table B (compounds I.C.D3-22.B-1 to I.C.D3-22.B-460).

### Table 23c

Compounds of the formula I.C in which the combination of R³, R⁵ and R⁶ corresponds to line D3-23 of Table D3 and the combination of R¹ and R² for each individual compound corresponds in each case to one line of Table B (compounds I.C.D3-23.B-1 to I.C.D3-23.B-460).

### Table 24c

Compounds of the formula I.C in which the combination of R³, R⁵ and R⁶ corresponds to line D3-24 of Table D3 and the combination of R¹ and R² for each individual compound corresponds in each case to one line of Table B (compounds I.C.D3-24.B-1 to I.C.D3-24.B-460).

### Table 25c

Compounds of the formula I.C in which the combination of R³, R⁵ and R⁶ corresponds to line D3-25 of Table D3 and the combination of R¹ and R² for each individual compound corresponds in each case to one line of Table B (compounds I.C.D3-25.B-1 to I.C.D3-25.B-460).

### Table 26c

Compounds of the formula I.C in which the combination of R³, R⁵ and R⁶ corresponds to line D3-26 of Table D3 and the combination of R¹ and R² for each individual compound corresponds in each case to one line of Table B (compounds I.C.D3-26.B-1 to I.C.D3-26.B-460).

### Table 27c

Compounds of the formula I.C in which the combination of R³, R⁵ and R⁶ corresponds to line D3-27 of Table D3 and the combination of R¹ and R² for each individual compound corresponds in each case to one line of Table B (compounds I.C.D3-27.B-1 to I.C.D3-27.B-460).

### Table 28c

Compounds of the formula I.C in which the combination of R³, R⁵ and R⁶ corresponds to line D3-28 of Table D3 and the combination of R¹ and R² for each individual compound corresponds in each case to one line of Table B (compounds I.C.D3-28.B-1 to I.C.D3-28.B-460).

### Table 1d

Compounds of the formula I.D in which R³ corresponds to line D4-1 of Table D4 and the combination of R¹ and R² for each individual compound corresponds in each case to one line of Table B (compounds I.D.D4-1.B-1 to I.D.D4-1.B-460).

### Table 2d

Compounds of the formula I.D in which R³ corresponds to line D4-2 of Table D4 and the combination of R¹ and R² for each individual compound corresponds in each case to one line of Table B (compounds I.D.D4-2.B-1 to I.D.D4-2.B-460).

### Table 3d

Compounds of the formula I.D in which R³ corresponds to line D4-3 of Table D4 and the combination of R¹ and R² for each individual compound corresponds in each case to one line of Table B (compounds I.D.D4-3.B-1 to I.D.D4-3.B-460).

### Table 4d

Compounds of the formula I.D in which R³ corresponds to line D4-4 of Table D4 and the combination of R¹ and R² for each individual compound corresponds in each case to one line of Table B (compounds I.D.D4-4.B-1 to I.D.D4-4.B-460).

### Table 1e

Compounds of the formula I.E in which R³ corresponds to line D4-1 of Table D4 and the combination of R¹ and R² for each individual compound corresponds in each case to one line of Table B (compounds I.E.D4-1.B-1 to I.E.D4-1.B-460).

### Table 2e

Compounds of the formula I.E in which R³ corresponds to line D4-2 of Table D4 and the combination of R¹ and R² for each individual compound corresponds in each case to one line of Table B (compounds I.E.D4-2.B-1 to I.E.D4-2.B-460).

### Table 3e

Compounds of the formula I.E in which R³ corresponds to line D4-3 of Table D4 and the combination of R¹ and R² for each individual compound corresponds in each case to one line of Table B (compounds I.E.D4-3.B-1 to I.E.D4-3.B-460).

### Table 4e

Compounds of the formula I.E in which R³ corresponds to line D4-4 of Table D4 and the combination of R¹ and R² for each individual compound corresponds in each case to one line of Table B (compounds I.E.D4-4.B-1 to I.E.D4-4.B-460).

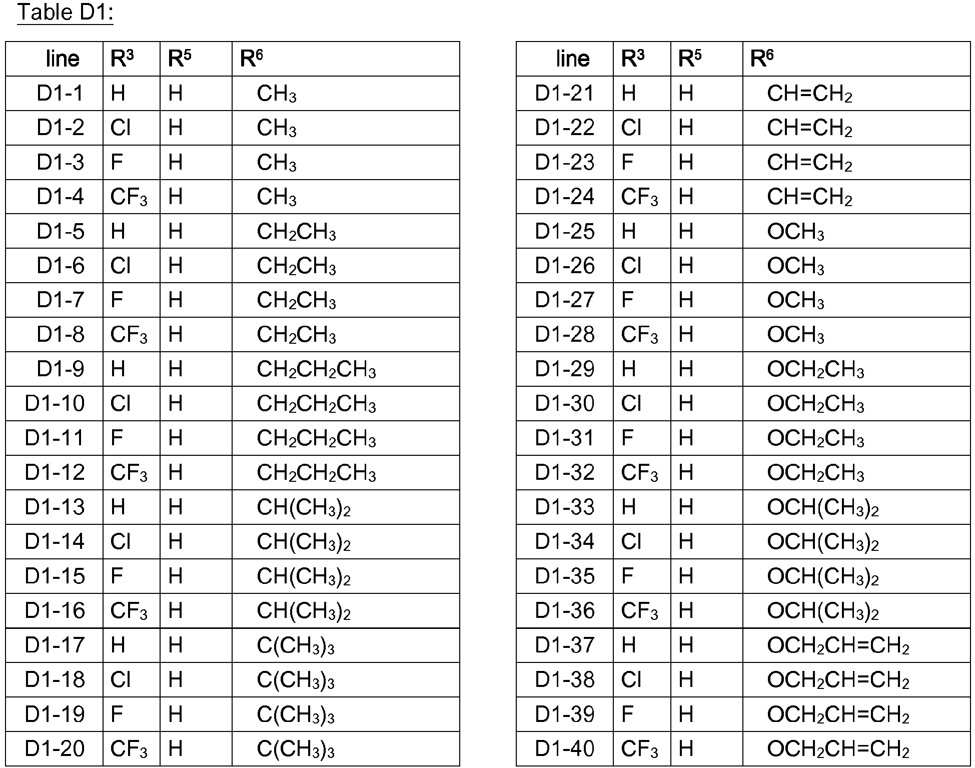

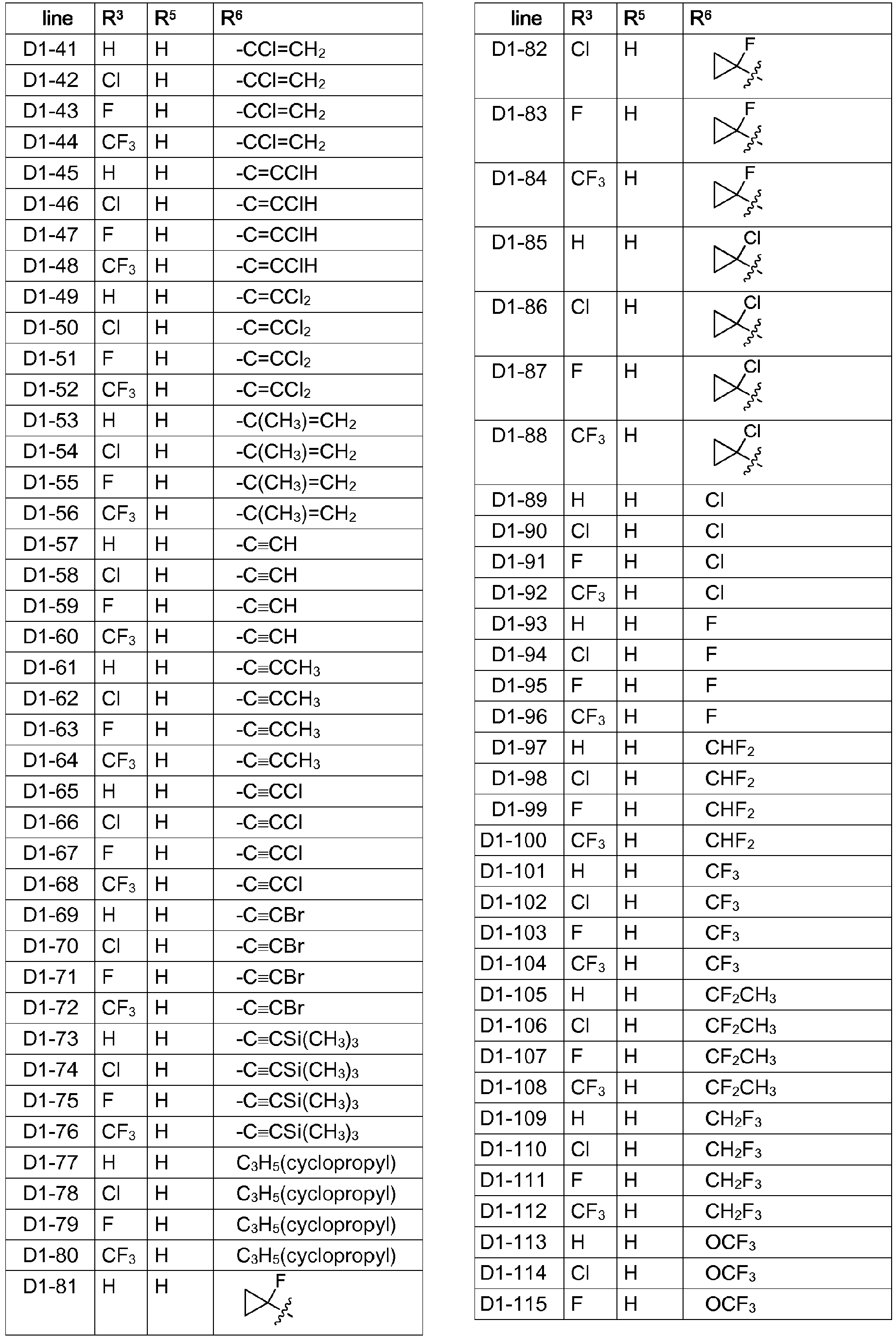

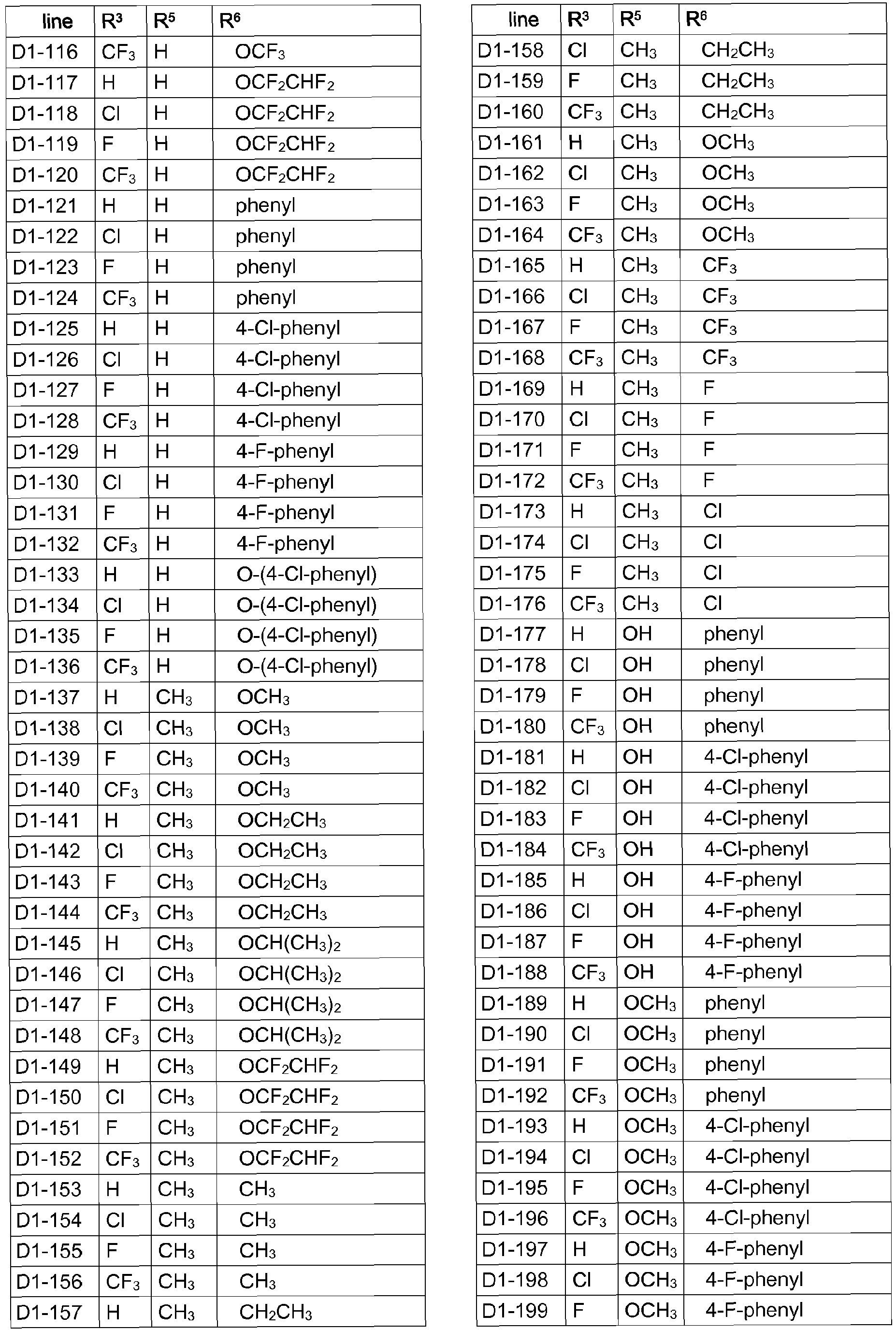

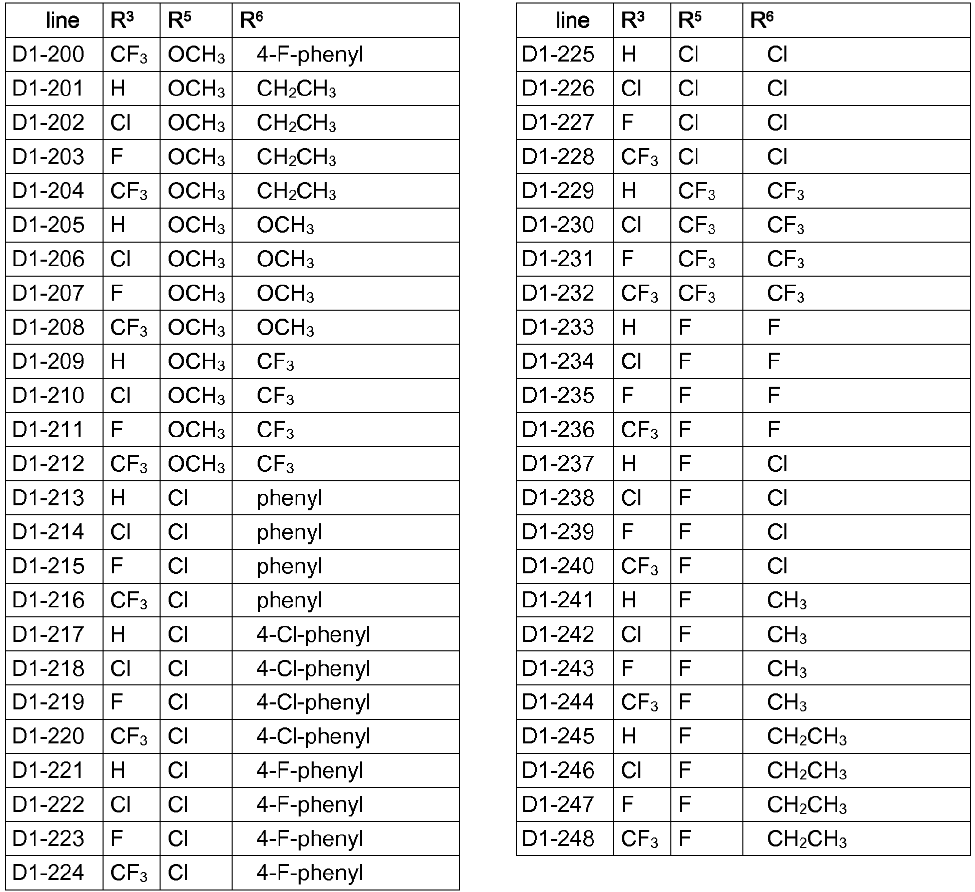

**Table D2:**

| **line** | **R³** | **R⁶** | | **line** | **R³** | **R⁶** |
|---|---|---|---|---|---|---|
| D2-1 | H | OCH₃ | | D2-11 | F | OCH(CH₃)₂ |
| D2-2 | Cl | OCH₃ | | D2-12 | CF₃ | OCH(CH₃)₂ |
| D2-3 | F | OCH₃ | | D2-13 | H | OCF₂CHF₂ |
| D2-4 | CF₃ | OCH₃ | | D2-14 | Cl | OCF₂CHF₂ |
| D2-5 | H | OCH₂CH₃ | | D2-15 | F | OCF₂CHF₂ |
| D2-6 | Cl | OCH₂CH₃ | | D2-16 | CF₃ | OCF₂CHF₂ |
| D2-7 | F | OCH₂CH₃ | | D2-17 | H | CH₃ |
| D2-8 | CF₃ | OCH₂CH₃ | | D2-18 | Cl | CH₃ |
| D2-9 | H | OCH(CH₃)₂ | | D2-19 | F | CH₃ |
| D2-10 | Cl | OCH(CH₃)₂ | | D2-20 | CF₃ | CH₃ |

**Table D3:**

| **line** | **R³** | **R⁵** | **R⁶** | | **line** | **R³** | **R⁵** | **R⁶** |
|---|---|---|---|---|---|---|---|---|
| D3-1 | H | F | CH₃ | | D3-15 | F | F | F |
| D3-2 | Cl | F | CH₃ | | D3-16 | CF₃ | F | F |
| D3-3 | F | F | CH₃ | | D3-17 | H | CH₃ | CH₃ |
| D3-4 | CF₃ | F | CH₃ | | D3-18 | Cl | CH₃ | CH₃ |
| D3-5 | H | F | CF₃ | | D3-19 | F | CH₃ | CH₃ |
| D3-6 | Cl | F | CF₃ | | D3-20 | CF₃ | CH₃ | CH₃ |
| D3-7 | F | F | CF₃ | | D3-21 | H | CH₃ | CF₃ |
| D3-8 | CF₃ | F | CF₃ | | D3-22 | Cl | CH₃ | CF₃ |
| D3-9 | H | F | CF₂CF₃ | | D3-23 | F | CH₃ | CF₃ |
| D3-10 | Cl | F | CF₂CF₃ | | D3-24 | CF₃ | CH₃ | CF₃ |
| D3-11 | F | F | CF₂CF₃ | | D3-25 | H | CF₃ | CF₃ |
| D3-12 | CF₃ | F | CF₂CF₃ | | D3-26 | Cl | CF₃ | CF₃ |
| D3-13 | H | F | F | | D3-27 | F | CF₃ | CF₃ |
| D3-14 | Cl | F | F | | D3-28 | CF₃ | CF₃ | CF₃ |

**Table D4:**

| **line** | **R³** | | **line** | **R³** |
|---|---|---|---|---|
| D4-1 | H | | D4-3 | F |
| D4-2 | Cl | | D4-4 | CF₃ |

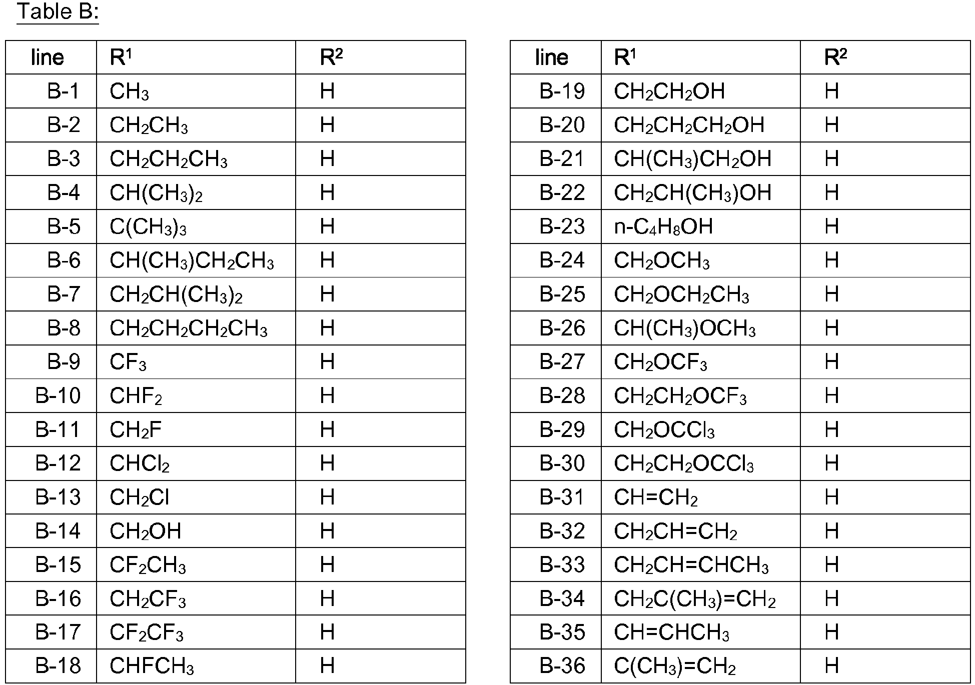

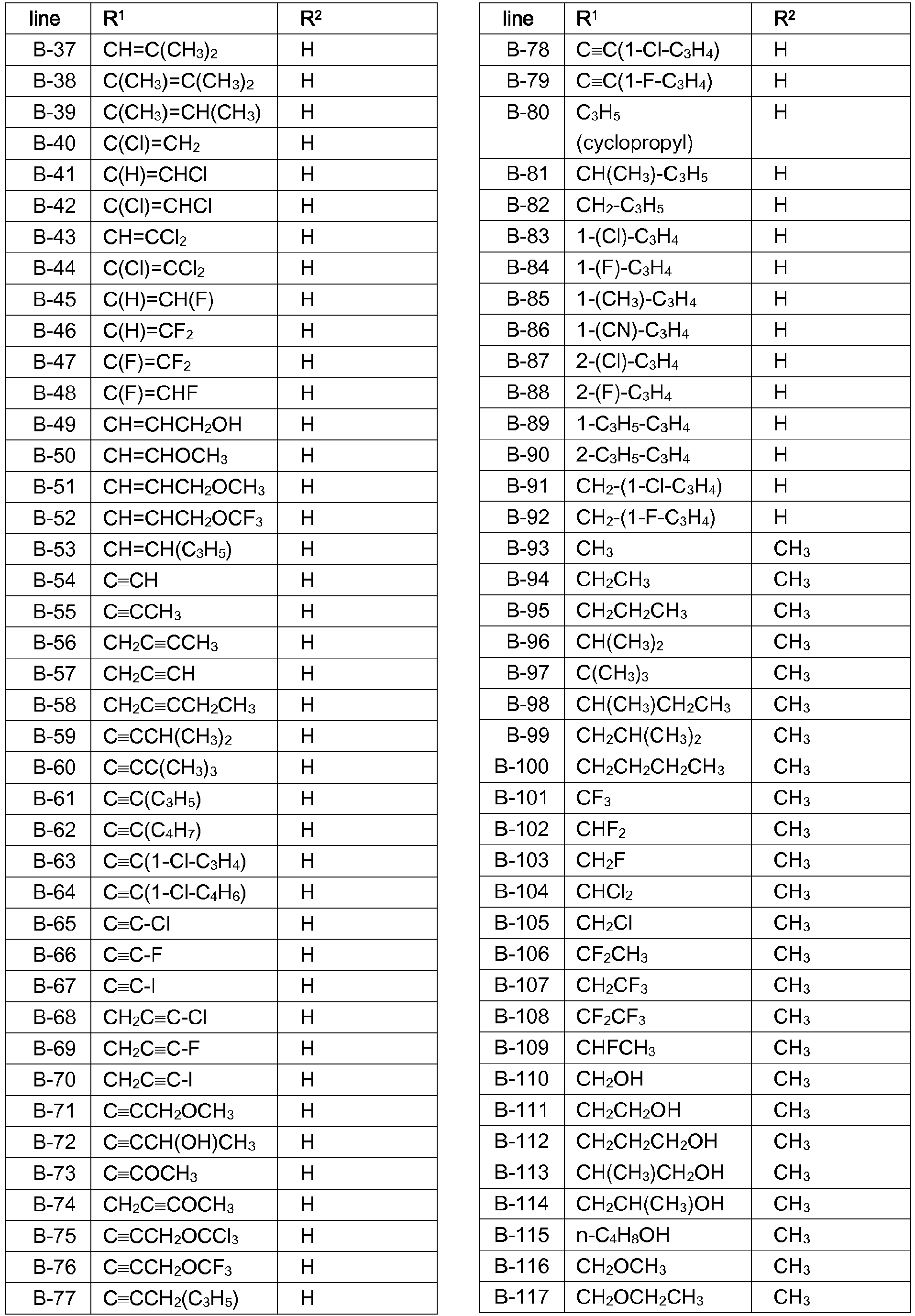

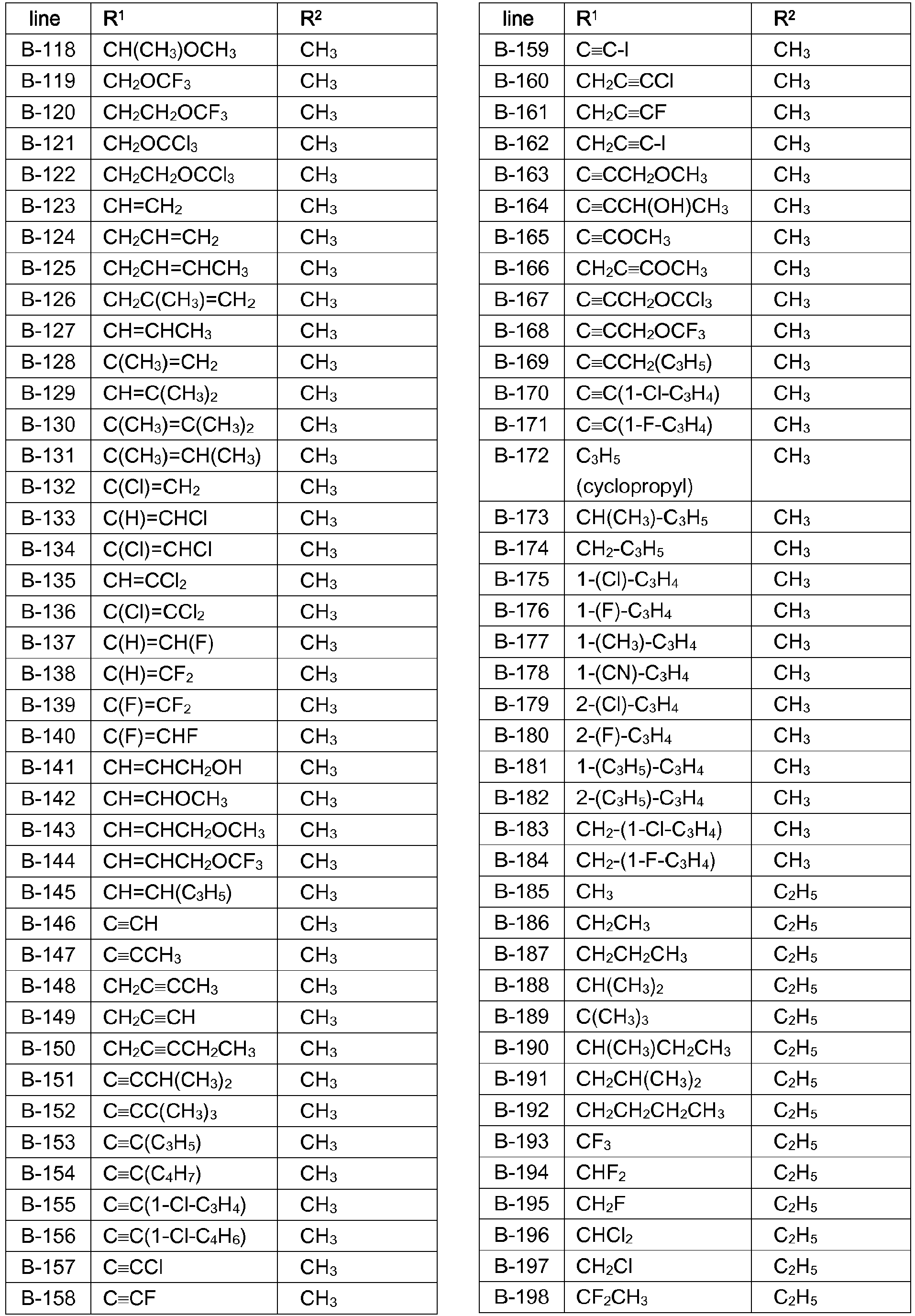

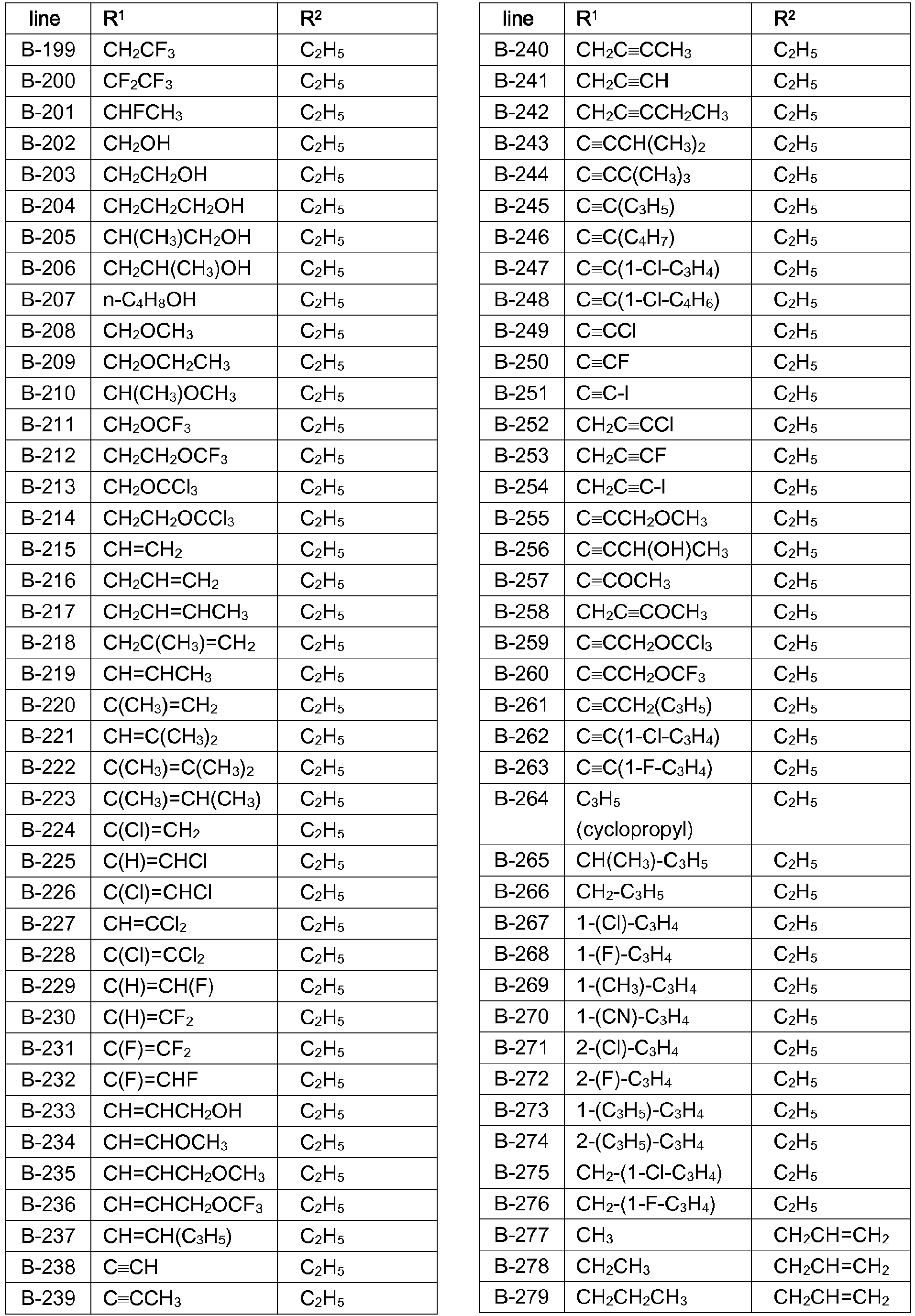

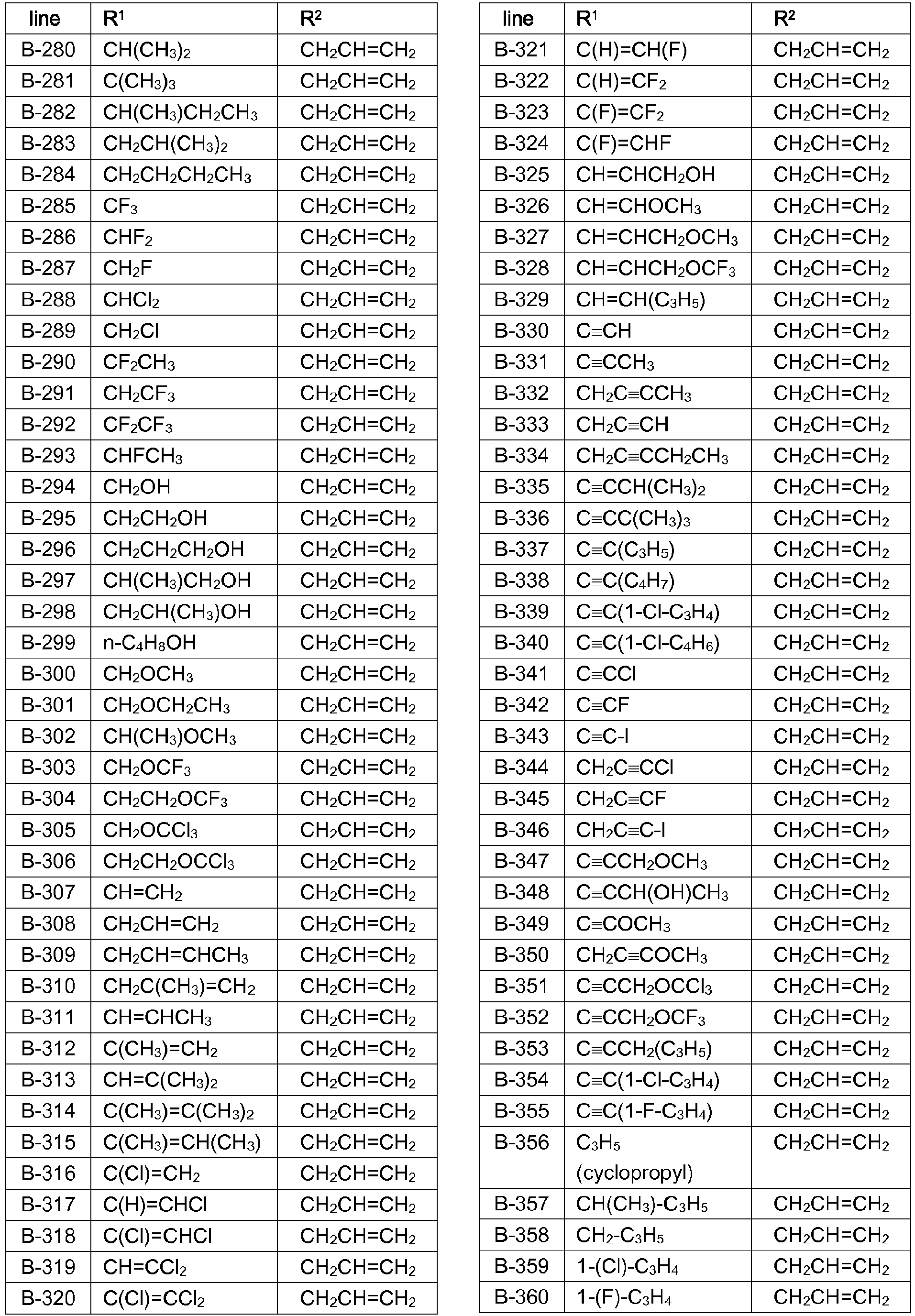

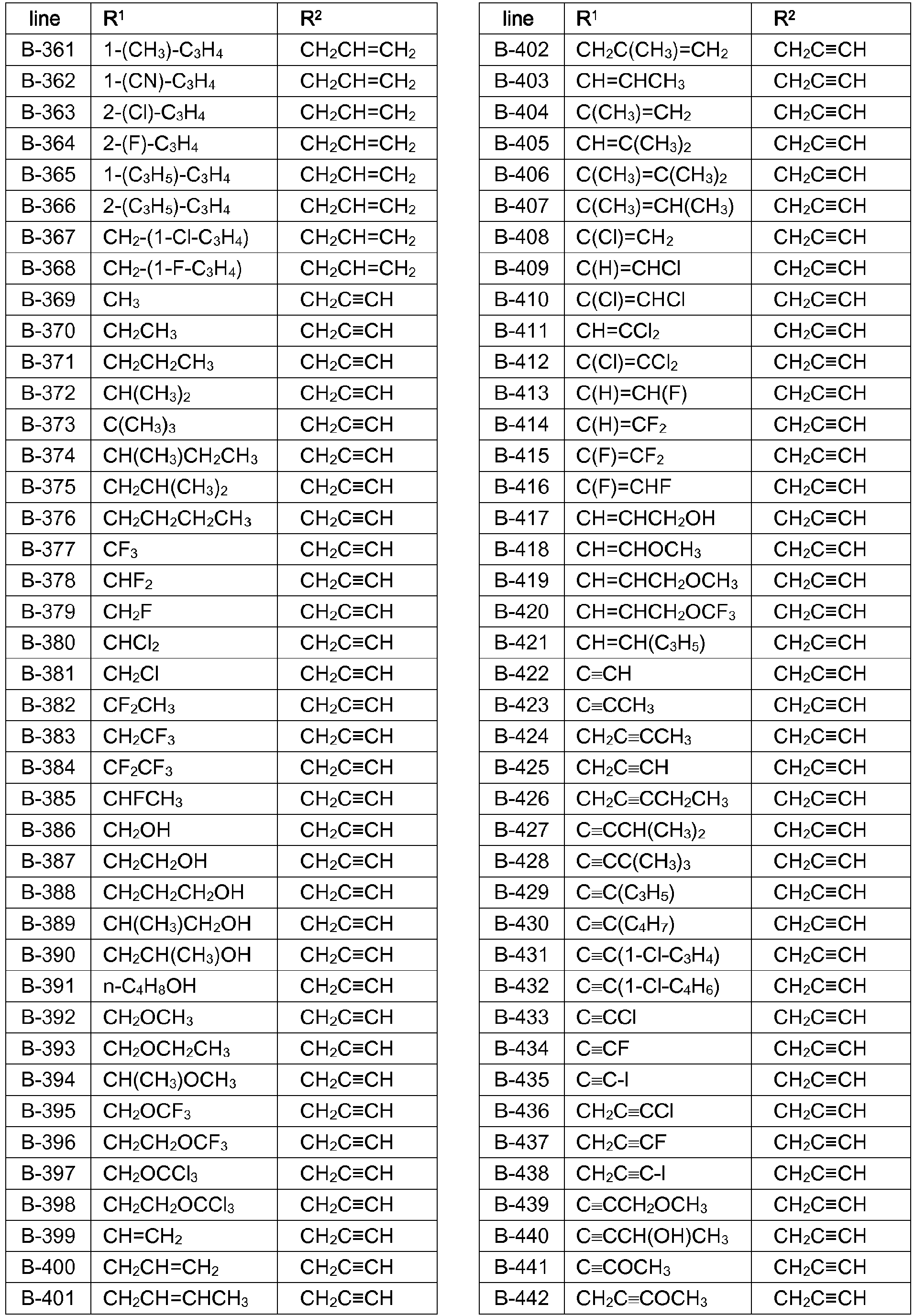

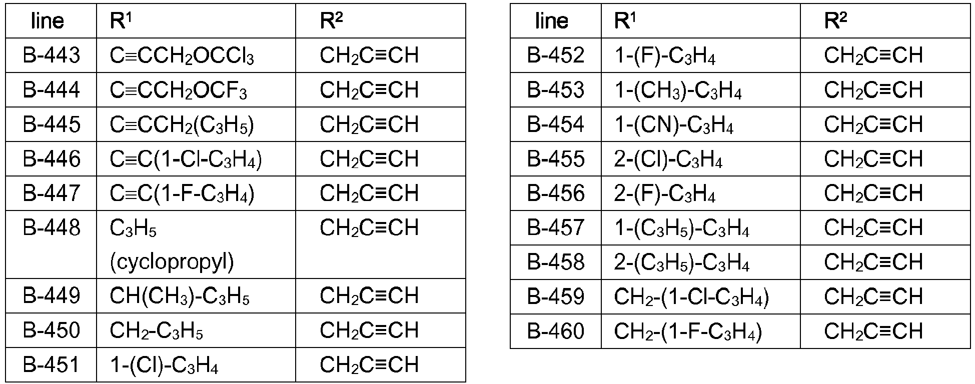

The compounds of formula I, also termed compounds I, and the compositions according to the invention, respectively, are suitable as fungicides. They are distinguished by an outstanding effectiveness against a broad spectrum of phytopathogenic fungi, including soil-borne fungi, which derive especially from the classes of the Plasmodiophoromycetes, Peronosporomycetes (syn. Oomycetes), Chytridiomycetes, Zygomycetes, Ascomycetes, Basidiomycetes and Deuteromycetes (syn. Fungi imperfecti). Some are systemically effective and they can be used in crop protection as foliar fungicides, fungicides for seed dressing and soil fungicides. Moreover, they are suitable for controlling harmful fungi, which inter alia occur in wood or roots of plants.

The compounds I and the compositions according to the invention are particularly important in the control of a multitude of phytopathogenic fungi on various cultivated plants, such as cereals, e. g. wheat, rye, barley, triticale, oats or rice; beet, e. g. sugar beet or fodder beet; fruits, such as pomes, stone fruits or soft fruits, e. g. apples, pears, plums, peaches, almonds, cherries, strawberries, raspberries, blackberries or gooseberries; leguminous plants, such as lentils, peas, alfalfa or soybeans; oil plants, such as rape, mustard, olives, sunflowers, coconut, cocoa beans, castor oil plants, oil palms, ground nuts or soybeans; cucurbits, such as squashes, cucumber or melons; fiber plants, such as cotton, flax, hemp or jute; citrus fruit, such as oranges, lemons, grapefruits or mandarins; vegetables, such as spinach, lettuce, asparagus, cabbages, carrots, onions, tomatoes, potatoes, cucurbits or paprika; lauraceous plants, such as avocados, cinnamon or camphor; energy and raw material plants, such as corn, soybean, rape, sugar cane or oil palm; corn; tobacco; nuts; coffee; tea; bananas; vines (table grapes and grape juice grape vines); hop; turf; sweet leaf (also called Stevia); natural rubber plants or ornamental and forestry plants, such as flowers, shrubs, broad-leaved trees or evergreens, e. g. conifers; and on the plant propagation material, such as seeds, and the crop material of these plants.

Preferably, compounds I and compositions thereof, respectively are used for controlling a multitude of fungi on field crops, such as potatoes sugar beets, tobacco, wheat, rye, barley, oats, rice, corn, cotton, soybeans, rape, legumes, sunflowers, coffee or sugar cane; fruits; vines; ornamentals; or vegetables, such as cucumbers, tomatoes, beans or squashes.

The term "plant propagation material" is to be understood to denote all the generative parts of the plant such as seeds and vegetative plant material such as cuttings and tubers (e. g. potatoes), which can be used for the multiplication of the plant. This includes seeds, roots, fruits, tubers, bulbs, rhizomes, shoots, sprouts and other parts of plants, including seedlings and young plants, which are to be transplanted after germination or after emergence from soil. These young plants may also be protected before transplantation by a total or partial treatment by immersion or pouring.

Preferably, treatment of plant propagation materials with compounds I and compositions thereof, respectively, is used for controlling a multitude of fungi on cereals, such as wheat, rye, barley and oats; rice, corn, cotton and soybeans.

The term "cultivated plants" is to be understood as including plants which have been modified by breeding, mutagenesis or genetic engineering including but not limiting to agricultural biotech products on the market or in development (cf. http://cera-gmc.org/, see GM crop database therein). Genetically modified plants are plants, which genetic material has been so modified by the use of recombinant DNA techniques that under natural circumstances cannot readily be obtained by cross breeding, mutations or natural recombination. Typically, one or more genes have been integrated into the genetic material of a genetically modified plant in order to improve certain properties of the plant. Such genetic modifications also include but are not limited to targeted post-translational modification of protein(s), oligo- or polypeptides e. g. by glycosylation or polymer additions such as prenylated, acetylated or farnesylated moieties or PEG moieties.

Plants that have been modified by breeding, mutagenesis or genetic engineering, e. g. have been rendered tolerant to applications of specific classes of herbicides, such as auxin herbicides such as dicamba or 2,4-D; bleacher herbicides such as hydroxylphenylpyruvate dioxygenase (HPPD) inhibitors or phytoene desaturase (PDS) inhibitors; acetolactate synthase (ALS) inhibitors such as sulfonyl ureas or imidazolinones; enolpyruvylshikimate-3-phosphate synthase (EPSPS) inhibitors, such as glyphosate; glutamine synthetase (GS) inhibitors such as glufosinate; protoporphyrinogen-IX oxidase inhibitors; lipid biosynthesis inhibitors such as acetyl CoA carboxylase (ACCase) inhibitors; or oxynil (i. e. bromoxynil or ioxynil) herbicides as a result of conventional methods of breeding or genetic engineering. Furthermore, plants have been made resistant to multiple classes of herbicides through multiple genetic modifications, such as resistance to both glyphosate and glufosinate or to both glyphosate and a herbicide from another class such as ALS inhibitors, H PPD inhibitors, auxin herbicides, or ACCase inhibitors. These herbicide resistance technologies are e. g. described in Pest Managem. Sci. 61, 2005, 246; 61, 2005, 258; 61, 2005, 277; 61, 2005, 269; 61, 2005, 286; 64, 2008, 326; 64, 2008, 332; Weed Sci. 57, 2009, 108; Austral. J. Agricult. Res. 58, 2007, 708; Science 316, 2007, 1185; and references quoted therein. Several cultivated plants have been rendered tolerant to herbicides by conventional methods of breeding (mutagenesis), e. g. Clearfield^{®} summer rape (Canola, BASF SE, Germany) being tolerant to imidazolinones, e. g. imazamox, or ExpressSun^{®} sunflowers (DuPont, USA) being tolerant to sulfonyl ureas, e. g. tribenuron. Genetic engineering methods have been used to render cultivated plants such as soybean, cotton, corn, beets and rape, tolerant to herbicides such as glyphosate and glufosinate, some of which are commercially available under the trade names RoundupReady^{®} (glyphosate-tolerant, Monsanto, U.S.A.), Cultivance^{®} (imidazolinone tolerant, BASF SE, Germany) and LibertyLink^{®} (glufosinate-tolerant, Bayer CropScience, Germany).

Furthermore, plants are also covered that are by the use of recombinant DNA techniques capable to synthesize one or more insecticidal proteins, especially those known from the bacterial genus *Bacillus,* particularly from *Bacillus thuringiensis,* such as δ-endotoxins, e. g. CryIA(b), CryIA(c), CryIF, CryIF(a2), CryIIA(b), CryIIIA, CryIIIB(b1) or Cry9c; vegetative insecticidal proteins (VIP), e. g. VIP1, VIP2, VIP3 or VIP3A; insecticidal proteins of bacteria colonizing nematodes, e. g. *Photorhabdus* spp. or *Xenorhabdus* spp.; toxins produced by animals, such as scorpion toxins, arachnid toxins, wasp toxins, or other insect-specific neurotoxins; toxins produced by fungi, such Streptomycetes toxins, plant lectins, such as pea or barley lectins; agglutinins; proteinase inhibitors, such as trypsin inhibitors, serine protease inhibitors, patatin, cystatin or papain inhibitors; ribosome-inactivating proteins (RIP), such as ricin, maize-RIP, abrin, luffin, saporin or bryodin; steroid metabolism enzymes, such as 3-hydroxysteroid oxidase, ecdysteroid-IDP-glycosyl-transferase, cholesterol oxidases, ecdysone inhibitors or HMG-CoA-reductase; ion channel blockers, such as blockers of sodium or calcium channels; juvenile hormone esterase; diuretic hormone receptors (helicokinin receptors); stilbene synthase, bibenzyl synthase, chitinases or glucanases. In the context of the present invention these insecticidal proteins or toxins are to be understood expressly also as pre-toxins, hybrid proteins, truncated or otherwise modified proteins. Hybrid proteins are characterized by a new combination of protein domains, (see, e. g. WO 02/015701). Further examples of such toxins or genetically modified plants capable of synthesizing such toxins are disclosed, e. g., in EP-A 374 753, WO 93/007278, WO 95/34656, EP-A 427 529, EP-A 451 878, WO 03/18810 und WO 03/52073. The methods for producing such genetically modified plants are generally known to the person skilled in the art and are described, e. g. in the publications mentioned above. These insecticidal proteins contained in the genetically modified plants impart to the plants producing these proteins tolerance to harmful pests from all taxonomic groups of arthropods, especially to beetles (Coeloptera), two-winged insects (Diptera), and moths (Lepidoptera) and to nematodes (Nematoda). Genetically modified plants capable to synthesize one or more insecticidal proteins are, e. g., described in the publications mentioned above, and some of which are commercially available such as YieldGard^{®} (corn cultivars producing the Cry1Ab toxin), YieldGard^{®} Plus (corn cultivars producing Cry1Ab and Cry3Bb1 toxins), Starlink^{®} (corn cultivars producing the Cry9c toxin), Herculex^{®} RW (corn cultivars producing Cry34Ab1, Cry35Ab1 and the enzyme phosphinothricin-N-acetyltransferase [PAT]); NuCOTN^{®} 33B (cotton cultivars producing the Cry1Ac toxin), Bollgard^{®} I (cotton cultivars producing the Cry1Ac toxin), Bollgard^{®} II (cotton cultivars producing Cry1Ac and Cry2Ab2 toxins); VIPCOT^{®} (cotton cultivars producing a VIP-toxin); NewLeaf^{®} (potato cultivars producing the Cry3A toxin); Bt-Xtra^{®}, NatureGard^{®}, KnockOut^{®}, BiteGard^{®}, Protecta^{®}, Bt11 (e. g. Agrisure^{®} CB) and Bt176 from Syngenta Seeds SAS, France, (corn cultivars producing the Cry1Ab toxin and PAT enyzme), MIR604 from Syngenta Seeds SAS, France (corn cultivars producing a modified version of the Cry3A toxin, c.f. WO 03/018810), MON 863 from Monsanto Europe S.A., Belgium (corn cultivars producing the Cry3Bb1 toxin), IPC 531 from Monsanto Europe S.A., Belgium (cotton cultivars producing a modified version of the Cry1Ac toxin) and 1507 from Pioneer Overseas Corporation, Belgium (corn cultivars producing the Cry1 F toxin and PAT enzyme).

Furthermore, plants are also covered that are by the use of recombinant DNA techniques capable to synthesize one or more proteins to increase the resistance or tolerance of those plants to bacterial, viral or fungal pathogens. Examples of such proteins are the so-called "pathogenesis-related proteins" (PR proteins, see, e. g. EP-A 392 225), plant disease resistance genes (e. g. potato cultivars, which express resistance genes acting against *Phytophthora infestans* derived from the Mexican wild potato *Solanum bulbocastanum*) or T4-lysozym (e. g. potato cultivars capable of synthesizing these proteins with increased resistance against bacteria such as *Erwinia amylvora*). The methods for producing such genetically modified plants are generally known to the person skilled in the art and are described, e. g. in the publications mentioned above.

Furthermore, plants are also covered that are by the use of recombinant DNA techniques capable to synthesize one or more proteins to increase the productivity (e. g. bio mass production, grain yield, starch content, oil content or protein content), tolerance to drought, salinity or other growth-limiting environmental factors or tolerance to pests and fungal, bacterial or viral pathogens of those plants.

Furthermore, plants are also covered that contain by the use of recombinant DNA techniques a modified amount of substances of content or new substances of content, specifically to improve human or animal nutrition, e. g. oil crops that produce health-promoting long-chain omega-3 fatty acids or unsaturated omega-9 fatty acids (e. g. Nexera^{®} rape, DOW Agro Sciences, Canada).

Furthermore, plants are also covered that contain by the use of recombinant DNA techniques a modified amount of substances of content or new substances of content, specifically to improve raw material production, e. g. potatoes that produce increased amounts of amylopectin (e. g. Amflora^{®} potato, BASF SE, Germany).

The compounds I and compositions thereof, respectively, are particularly suitable for controlling the following plant diseases:
*Albugo* spp. (white rust) on ornamentals, vegetables (e. g. *A*. *candida*) and sunflowers (e. g. *A*. *tragopogonis*); *Alternaria* spp. (Alternaria leaf spot) on vegetables, rape (*A*. *brassicola* or *brassicae),* sugar beets (*A*. *tenuis*), fruits, rice, soybeans, potatoes (e. g. *A*. *solani* or *A*. *alternata*), tomatoes (e. g. *A*. *solani* or *A*. *alternata*) and wheat; *Aphanomyces* spp. on sugar beets and vegetables; *Ascochyta* spp. on cereals and vegetables, e. g. *A*. *tritici* (anthracnose) on wheat and *A*. *hordei* on barley; *Bipolaris* and *Drechslera* spp. (teleomorph: *Cochliobolus* spp.), e. g. Southern leaf blight (*D. maydis*) or Northern leaf blight (*B. zeicola*) on corn, e. g. spot blotch (*B. sorokiniana*) on cereals and e. g. *B. oryzae* on rice and turfs; *Blumeria* (formerly *Erysiphe*) *graminis* (powdery mildew) on cereals (e. g. on wheat or barley); *Botrytis cinerea* (teleomorph: *Botryotinia fuckeliana*: grey mold) on fruits and berries (e. g. strawberries), vegetables (e. g. lettuce, carrots, celery and cabbages), rape, flowers, vines, forestry plants and wheat; *Bremia lactucae* (downy mildew) on lettuce; *Ceratocystis* (syn. *Ophiostoma*) spp. (rot or wilt) on broad-leaved trees and evergreens, e. g. *C. ulmi* (Dutch elm disease) on elms; *Cercospora* spp. (Cercospora leaf spots) on corn (e. g. Gray leaf spot: *C. zeae-maydis*), rice, sugar beets (e. g. *C. beticola*), sugar cane, vegetables, coffee, soybeans (e. g. *C. sojina* or *C*. *kikuchii*) and rice; *Cladosporium* spp. on tomatoes (e. g. *C. fulvum*: leaf mold) and cereals, e. g. *C. herbarum* (black ear) on wheat; *Claviceps purpurea* (ergot) on cereals; *Cochliobolus* (anamorph: *Helminthosporium* of *Bipolaris*) spp. (leaf spots) on corn (*C*. *carbonum*), cereals (e. g. *C. sativus,* anamorph: *B. sorokiniana*) and rice (e. g. *C. miyabeanus,* anamorph: *H*. *oryzae*); *Colletotrichum* (teleomorph: *Glomerella*) spp. (anthracnose) on cotton (e. g. *C*. *gossypii*), corn (e. g. *C. graminicola:* Anthracnose stalk rot), soft fruits, potatoes (e. g. *C*. *coccodes*: black dot), beans (e. g. *C. lindemuthianum*) and soybeans (e. g. *C. truncatum* or *C*. *gloeosporioides*); *Corticium* spp., e. g. *C. sasakii* (sheath blight) on rice; *Corynespora cassiicola* (leaf spots) on soybeans and ornamentals; *Cycloconium* spp., e. g. *C. oleaginum* on olive trees; *Cylindrocarpon* spp. (e. g. fruit tree canker or young vine decline, teleomorph: *Nectria* or *Neonectria* spp.) on fruit trees, vines (e. g. *C. liriodendri,* teleomorph: *Neonectria liriodendri*: Black Foot Disease) and ornamentals; *Dematophora* (teleomorph: *Rosellinia*) *necatrix* (root and stem rot) on soybeans; *Diaporthe* spp., e. g. *D. phaseolorum* (damping off) on soybeans; *Drechslera* (syn. *Helminthosporium,* teleomorph: *Pyrenophora*) spp. on corn, cereals, such as barley (e. g. *D. teres,* net blotch) and wheat (e. g. *D*. *tritici-repentis*: tan spot), rice and turf; Esca (dieback, apoplexy) on vines, caused by *Formitiporia* (syn. *Phellinus*) *punctata, F. mediterranea, Phaeomoniella chlamydospora* (earlier *Phaeoacremonium chlamydosporum*), *Phaeoacremonium aleophilum* and/or *Botryosphaeria obtusa*; *Elsinoe* spp. on pome fruits (*E. pyri*)*,* soft fruits (*E*. *veneta*: anthracnose) and vines (*E. ampelina*: anthracnose); *Entyloma oryzae* (leaf smut) on rice; *Epicoccum* spp. (black mold) on wheat; *Erysiphe* spp. (powdery mildew) on sugar beets (*E. betae*), vegetables (e. g. *E. pisi*), such as cucurbits (e. g. *E. cichoracearum*), cabbages, rape (e. g. *E. cruciferarum*); *Eutypa lata* (Eutypa canker or dieback, anamorph: *Cytosporina lata,* syn. *Libertella blepharis*) on fruit trees, vines and ornamental woods; *Exserohilum* (syn. *Helminthosporium*) spp. on corn (e. g. *E. turcicum*); *Fusarium* (teleomorph: *Gibberella*) spp. (wilt, root or stem rot) on various plants, such as *F. graminearum* or *F. culmorum* (root rot, scab or head blight) on cereals (e. g. wheat or barley), *F. oxysporum* on tomatoes, *F. solani* (f. sp. *glycines* now syn. *F. virguliforme*) and *F. tucumaniae* and *F*. *brasiliense* each causing sudden death syndrome on soybeans, and *F. verticillioides* on corn; *Gaeumannomyces graminis* (take-all) on cereals (e. g. wheat or barley) and corn; *Gibberella* spp. on cereals (e. g. *G. zeae*) and rice (e. g. *G. fujikuroi*: Bakanae disease); *Glomerella cingulata* on vines, pome fruits and other plants and *G. gossypii* on cotton; Grainstaining complex on rice; *Guignardia bidwellii* (black rot) on vines; *Gymnosporangium* spp. on rosaceous plants and junipers, e. g. *G. sabinae* (rust) on pears; *Helminthosporium* spp. (syn. *Drechslera,* teleomorph: *Cochliobolus*) on corn, cereals and rice; *Hemileia* spp., e. g. *H. vastatrix* (coffee leaf rust) on coffee; *Isariopsis clavispora* (syn. *Cladosporium vitis*) on vines; *Macrophomina phaseolina* (syn. *phaseoli*) (root and stem rot) on soybeans and cotton; *Microdochium* (syn. *Fusarium*) *nivale* (pink snow mold) on cereals (e. g. wheat or barley); *Microsphaera diffusa* (powdery mildew) on soybeans; *Monilinia* spp., e. g. *M. laxa, M. fructicola* and *M. fructigena* (bloom and twig blight, brown rot) on stone fruits and other rosaceous plants; *Mycosphaerella* spp. on cereals, bananas, soft fruits and ground nuts, such as e. g. *M. graminicola* (anamorph: *Septoria tritici,* Septoria blotch) on wheat or *M*. *fijiensis* (black Sigatoka disease) on bananas; *Peronospora* spp. (downy mildew) on cabbage (e. g. *P. brassicae*), rape (e. g. *P. parasitica*), onions (e. g. *P. destructor*), tobacco (*P*. *tabacina*) and soybeans (e. g. *P. manshurica*); *Phakopsora pachyrhizi* and *P. meibomiae* (soybean rust) on soybeans; *Phialophora* spp. e. g. on vines (e. g. *P. tracheiphila* and *P. tetraspora*) and soybeans (e. g. *P. gregata*: stem rot); *Phoma lingam* (root and stem rot) on rape and cabbage and *P. betae* (root rot, leaf spot and damping-off) on sugar beets; *Phomopsis* spp. on sunflowers, vines (e. g. *P. viticola*: can and leaf spot) and soybeans (e. g. stem rot: *P. phaseoli,* teleomorph: *Diaporthe phaseolorum*); *Physoderma maydis* (brown spots) on corn; *Phytophthora* spp. (wilt, root, leaf, fruit and stem root) on various plants, such as paprika and cucurbits (e. g. *P. capsici*), soybeans (e. g. *P*. *megasperma,* syn. *P. sojae*), potatoes and tomatoes (e. g. *P. infestans*: late blight) and broad-leaved trees (e. g. *P. ramorum*: sudden oak death); *Plasmodiophora brassicae* (club root) on cabbage, rape, radish and other plants; *Plasmopara* spp., e. g. *P. viticola* (grapevine downy mildew) on vines and *P. halstedii* on sunflowers; *Podosphaera* spp. (powdery mildew) on rosaceous plants, hop, pome and soft fruits, e. g. *P. leucotricha* on apples; *Polymyxa* spp., e. g. on cereals, such as barley and wheat (*P. graminis*) and sugar beets (*P. betae*) and thereby transmitted viral diseases; *Pseudocercosporella herpotrichoides* (eyespot, teleomorph: *Tapesia yallundae*) on cereals, e. g. wheat or barley; *Pseudoperonospora* (downy mildew) on various plants, e. g. *P*. *cubensis* on cucurbits or *P*. *humili* on hop; *Pseudopezicula tracheiphila* (red fire disease or 'rotbrenner', anamorph: *Phialophora*) on vines; *Puccinia* spp. (rusts) on various plants, e. g. *P*. *triticina* (brown or leaf rust), *P*. *striiformis* (stripe or yellow rust), *P*. *hordei* (dwarf rust), *P*. *graminis* (stem or black rust) or *P*. *recondita* (brown or leaf rust) on cereals, such as e. g. wheat, barley or rye, *P*. *kuehnii* (orange rust) on sugar cane and *P*. *asparagi* on asparagus; *Pyrenophora* (anamorph: *Drechslera*) *tritici-repentis* (tan spot) on wheat or *P*. *teres* (net blotch) on barley; *Pyricularia* spp., e. g. *P*. *oryzae* (teleomorph: *Magnaporthe grisea,* rice blast) on rice and *P*. *grisea* on turf and cereals; *Pythium* spp. (damping-off) on turf, rice, corn, wheat, cotton, rape, sunflowers, soybeans, sugar beets, vegetables and various other plants (e. g. *P*. *ultimum* or *P*. *aphanidermatum*); *Ramularia* spp., e. g. *R. collo-cygni* (Ramularia leaf spots, Physiological leaf spots) on barley and *R. beticola* on sugar beets; *Rhizoctonia* spp. on cotton, rice, potatoes, turf, corn, rape, potatoes, sugar beets, vegetables and various other plants, e. g. *R. solani* (root and stem rot) on soybeans, *R. solani* (sheath blight) on rice or *R*. *cerealis* (Rhizoctonia spring blight) on wheat or barley; *Rhizopus stolonifer* (black mold, soft rot) on strawberries, carrots, cabbage, vines and tomatoes; *Rhynchosporium secalis* (scald) on barley, rye and triticale; *Sarocladium oryzae* and *S*. *attenuatum* (sheath rot) on rice; *Sclerotinia* spp. (stem rot or white mold) on vegetables and field crops, such as rape, sunflowers (e. g. S. *sclerotiorum*) and soybeans (e. g. *S*. *rolfsii* or *S*. *sclerotiorum*); *Septoria* spp. on various plants, e. g. *S*. *glycines* (brown spot) on soybeans, *S*. *tritici* (Septoria blotch) on wheat and *S*. (syn. *Stagonospora*) nodorum (Stagonospora blotch) on cereals; *Uncinula* (syn. *Erysiphe*) *necator* (powdery mildew, anamorph: *Oidium tuckeri*) on vines; *Setospaeria* spp. (leaf blight) on corn (e. g. *S*. *turcicum,* syn. *Helminthosporium turcicum*) and turf; *Sphacelotheca* spp. (smut) on corn, (e. g. *S*. *reiliana*: head smut), sorghum und sugar cane; *Sphaerotheca fuliginea* (powdery mildew) on cucurbits; *Spongospora subterranea* (powdery scab) on potatoes and thereby transmitted viral diseases; *Stagonospora* spp. on cereals, e. g. *S*. *nodorum* (Stagonospora blotch, teleomorph: *Leptosphaeria* [syn. *Phaeosphaeria*] *nodorum*) on wheat; *Synchytrium endobioticum* on potatoes (potato wart disease); *Taphrina* spp., e. g. *T. deformans* (leaf curl disease) on peaches and *T. pruni* (plum pocket) on plums; *Thielaviopsis spp.* (black root rot) on tobacco, pome fruits, vegetables, soybeans and cotton, e. g. *T. basicola* (syn. *Chalara elegans*); *Tilletia* spp. (common bunt or stinking smut) on cereals, such as e. g. *T. tritici* (syn. *T. caries,* wheat bunt) and *T. controversa* (dwarf bunt) on wheat; *Typhula incarnata* (grey snow mold) on barley or wheat; *Urocystis* spp., e. g. *U. occulta* (stem smut) on rye; *Uromyces* spp. (rust) on vegetables, such as beans (e. g. *U. appendiculatus,* syn. *U. phaseoli*) and sugar beets (e. g. *U. betae*); *Ustilago* spp. (loose smut) on cereals (e. g. *U. nuda* and *U. avaenae*), corn (e. g. *U. maydis*: corn smut) and sugar cane; *Venturia* spp. (scab) on apples (e. g. *V. inaequalis*) and pears; and *Verticillium* spp. (wilt) on various plants, such as fruits and ornamentals, vines, soft fruits, vegetables and field crops, e. g. *V*. *dahliae* on strawberries, rape, potatoes and tomatoes.

The compounds I and compositions thereof, respectively, are also suitable for controlling harmful fungi in the protection of stored products or harvest and in the protection of materials.

The term "protection of materials" is to be understood to denote the protection of technical and non-living materials, such as adhesives, glues, wood, paper and paperboard, textiles, leather, paint dispersions, plastics, cooling lubricants, fiber or fabrics, against the infestation and destruction by harmful microorganisms, such as fungi and bacteria. As to the protection of wood and other materials, the particular attention is paid to the following harmful fungi: Ascomycetes such as *Ophiostoma* spp., *Ceratocystis* spp., *Aureobasidium pullulans, Sclerophoma* spp., *Chaetomium* spp., *Humicola* spp., *Petriella* spp., *Trichurus* spp.; Basidiomycetes such as *Coniophora* spp., *Coriolus* spp., *Gloeophyllum* spp., *Lentinus* spp., *Pleurotus* spp., *Poria* spp., *Serpula* spp. and *Tyromyces* spp., Deuteromycetes such as *Aspergillus* spp., *Cladosporium* spp., *Penicillium* spp., *Trichoderma* spp., *Alternaria* spp., *Paecilomyces* spp. and Zygomycetes such as *Mucor* spp., and in addition in the protection of stored products and harvest the following yeast fungi are worthy of note: *Candida* spp. and *Saccharomyces cerevisae.*

The method of treatment according to the invention can also be used in the field of protecting stored products or harvest against attack of fungi and microorganisms. According to the present invention, the term "stored products" is understood to denote natural substances of plant or animal origin and their processed forms, which have been taken from the natural life cycle and for which long-term protection is desired. Stored products of crop plant origin, such as plants or parts thereof, for example stalks, leafs, tubers, seeds, fruits or grains, can be protected in the freshly harvested state or in processed form, such as pre-dried, moistened, comminuted, ground, pressed or roasted, which process is also known as post-harvest treatment. Also falling under the definition of stored products is timber, whether in the form of crude timber, such as construction timber, electricity pylons and barriers, or in the form of finished articles, such as furniture or objects made from wood. Stored products of animal origin are hides, leather, furs, hairs and the like. The combinations according the present invention can prevent disadvantageous effects such as decay, discoloration or mold. Preferably "stored products" is understood to denote natural substances of plant origin and their processed forms, more preferably fruits and their processed forms, such as pomes, stone fruits, soft fruits and citrus fruits and their processed forms.

The compounds I and compositions thereof, respectively, may be used for improving the health of a plant. The invention also relates to a method for improving plant health by treating a plant, its propagation material and/or the locus where the plant is growing or is to grow with an effective amount of compounds I and compositions thereof, respectively.

The term "plant health" is to be understood to denote a condition of the plant and/or its products which is determined by several indicators alone or in combination with each other such as yield (e. g. increased biomass and/or increased content of valuable ingredients), plant vigor (e. g. improved plant growth and/or greener leaves ("greening effect")), quality (e. g. improved content or composition of certain ingredients) and tolerance to abiotic and/or biotic stress. The above identified indicators for the health condition of a plant may be interdependent or may result from each other.

The compounds of formula I can be present in different crystal modifications whose biological activity may differ. They are likewise subject matter of the present invention.

The compounds I are employed as such or in form of compositions by treating the fungi or the plants, plant propagation materials, such as seeds, soil, surfaces, materials or rooms to be protected from fungal attack with a fungicidally effective amount of the active substances. The application can be carried out both before and after the infection of the plants, plant propagation materials, such as seeds, soil, surfaces, materials or rooms by the fungi.

Plant propagation materials may be treated with compounds I as such or a composition comprising at least one compound I prophylactically either at or before planting or transplanting.

The invention also relates to agrochemical compositions comprising an auxiliary and at least one compound I according to the invention.

An agrochemical composition comprises a fungicidally effective amount of a compound I. The term "effective amount" denotes an amount of the composition or of the compounds I, which is sufficient for controlling harmful fungi on cultivated plants or in the protection of materials and which does not result in a substantial damage to the treated plants. Such an amount can vary in a broad range and is dependent on various factors, such as the fungal species to be controlled, the treated cultivated plant or material, the climatic conditions and the specific compound I used.

The compounds I, their N-oxides and salts can be converted into customary types of agrochemical compositions, e. g. solutions, emulsions, suspensions, dusts, powders, pastes, granules, pressings, capsules, and mixtures thereof. Examples for composition types are suspensions (e. g. SC, OD, FS), emulsifiable concentrates (e. g. EC), emulsions (e. g. EW, EO, ES, ME), capsules (e. g. CS, ZC), pastes, pastilles, wettable powders or dusts (e. g. WP, SP, WS, DP, DS), pressings (e. g. BR, TB, DT), granules (e. g. WG, SG, GR, FG, GG, MG), insecticidal articles (e. g. LN), as well as gel formulations for the treatment of plant propagation materials such as seeds (e. g. GF). These and further compositions types are defined in the "Catalogue of pesticide formulation types and international coding system", Technical Monograph No. 2, 6th Ed. May 2008, CropLife International.

The compositions are prepared in a known manner, such as described by Mollet and Grubemann, Formulation technology, Wiley VCH, Weinheim, 2001; or Knowles, New developments in crop protection product formulation, Agrow Reports DS243, T&F Informa, London, 2005.

Suitable auxiliaries are solvents, liquid carriers, solid carriers or fillers, surfactants, dispersants, emulsifiers, wetters, adjuvants, solubilizers, penetration enhancers, protective colloids, adhesion agents, thickeners, humectants, repellents, attractants, feeding stimulants, compatibilizers, bactericides, anti-freezing agents, anti-foaming agents, colorants, tackifiers and binders.

Suitable solvents and liquid carriers are water and organic solvents, such as mineral oil fractions of medium to high boiling point, e. g. kerosene, diesel oil; oils of vegetable or animal origin; aliphatic, cyclic and aromatic hydrocarbons, e. g. toluene, paraffin, tetrahydronaphthalene, al-kylated naphthalenes; alcohols, e. g. ethanol, propanol, butanol, benzyl alcohol, cyclohexanol; glycols; DMSO; ketones, e. g. cyclohexanone; esters, e. g. lactates, carbonates, fatty acid esters, gamma-butyrolactone; fatty acids; phosphonates; amines; amides, e. g. N-methyl pyrrolidone, fatty acid dimethyl amides; and mixtures thereof.

Suitable solid carriers or fillers are mineral earths, e. g. silicates, silica gels, talc, kaolins, limestone, lime, chalk, clays, dolomite, diatomaceous earth, bentonite, calcium sulfate, magnesium sulfate, magnesium oxide; polysaccharides, e. g. cellulose, starch; fertilizers, e. g. ammonium sulfate, ammonium phosphate, ammonium nitrate, ureas; products of vegetable origin, e. g. cereal meal, tree bark meal, wood meal, nutshell meal, and mixtures thereof.

Suitable surfactants are surface-active compounds, such as anionic, cationic, nonionic and amphoteric surfactants, block polymers, polyelectrolytes, and mixtures thereof. Such surfactants can be used as emulsifier, dispersant, solubilizer, wetter, penetration enhancer, protective colloid, or adjuvant. Examples of surfactants are listed in McCutcheon's, Vol.1: Emulsifiers & Detergents, McCutcheon's Directories, Glen Rock, USA, 2008 (International Ed. or North American Ed.).

Suitable anionic surfactants are alkali, alkaline earth or ammonium salts of sulfonates, sulfates, phosphates, carboxylates, and mixtures thereof. Examples of sulfonates are alkylaryl sulfonates, diphenyl sulfonates, alpha-olefin sulfonates, lignin sulfonates, sulfonates of fatty acids and oils, sulfonates of ethoxylated alkylphenols, sulfonates of alkoxylated arylphenols, sulfonates of condensed naphthalenes, sulfonates of dodecyl- and tridecylbenzenes, sulfonates of naphthalenes and alkyl naphthalenes, sulfosuccinates or sulfosuccinamates. Examples of sulfates are sulfates of fatty acids and oils, of ethoxylated alkylphenols, of alcohols, of ethoxylated alcohols, or of fatty acid esters. Examples of phosphates are phosphate esters. Examples of carboxylates are alkyl carboxylates, and carboxylated alcohol or alkylphenol ethoxylates.

Suitable nonionic surfactants are alkoxylates, N-substituted fatty acid amides, amine oxides, esters, sugar-based surfactants, polymeric surfactants, and mixtures thereof. Examples of alkoxylates are compounds such as alcohols, alkylphenols, amines, amides, arylphenols, fatty acids or fatty acid esters which have been alkoxylated with 1 to 50 equivalents. Ethylene oxide and/or propylene oxide may be employed for the alkoxylation, preferably ethylene oxide. Examples of N-substituted fatty acid amides are fatty acid glucamides or fatty acid alkanolamides. Examples of esters are fatty acid esters, glycerol esters or monoglycerides. Examples of sugar-based surfactants are sorbitans, ethoxylated sorbitans, sucrose and glucose esters or alkyl-polyglucosides. Examples of polymeric surfactants are home- or copolymers of vinyl pyrrolidone, vinyl alcohols, or vinyl acetate.

Suitable cationic surfactants are quaternary surfactants, for example quaternary ammonium compounds with one or two hydrophobic groups, or salts of long-chain primary amines. Suitable amphoteric surfactants are alkylbetains and imidazolines. Suitable block polymers are block polymers of the A-B or A-B-A type comprising blocks of polyethylene oxide and polypropylene oxide, or of the A-B-C type comprising alkanol, polyethylene oxide and polypropylene oxide. Suitable polyelectrolytes are polyacids or polybases. Examples of polyacids are alkali salts of polyacrylic acid or polyacid comb polymers. Examples of polybases are polyvinyl amines or polyethylene amines.

Suitable adjuvants are compounds, which have a negligible or even no pesticidal activity themselves, and which improve the biological performance of the compound I on the target. Examples are surfactants, mineral or vegetable oils, and other auxiliaries. Further examples are listed by Knowles, Adjuvants and additives, Agrow Reports DS256, T&F Informa UK, 2006, chapter 5.

Suitable thickeners are polysaccharides (e. g. xanthan gum, carboxymethyl cellulose), inorganic clays (organically modified or unmodified), polycarboxylates, and silicates.

Suitable bactericides are bronopol and isothiazolinone derivatives such as alkylisothiazolinones and benzisothiazolinones.

Suitable anti-freezing agents are ethylene glycol, propylene glycol, urea and glycerin. Suitable anti-foaming agents are silicones, long chain alcohols, and salts of fatty acids.

Suitable colorants (e. g. in red, blue, or green) are pigments of low water solubility and water-soluble dyes. Examples are inorganic colorants (e. g. iron oxide, titan oxide, iron hexacyanoferrate) and organic colorants (e. g. alizarin-, azo- and phthalocyanine colorants).

Suitable tackifiers or binders are polyvinyl pyrrolidones, polyvinyl acetates, polyvinyl alcohols, polyacrylates, biological or synthetic waxes, and cellulose ethers.

Examples for composition types and their preparation are:

### i) Water-soluble concentrates (SL, LS)

10-60 wt% of a compound I and 5-15 wt% wetting agent (e. g. alcohol alkoxylates) are dissolved in water and/or in a water-soluble solvent (e. g. alcohols) ad 100 wt%. The active substance dissolves upon dilution with water.

### ii) Dispersible concentrates (DC)

5-25 wt% of a compound I and 1-10 wt% dispersant (e. g. polyvinyl pyrrolidone) are dissolved in organic solvent (e. g. cyclohexanone) ad 100 wt%. Dilution with water gives a dispersion.

### iii) Emulsifiable concentrates (EC)

15-70 wt% of a compound I and 5-10 wt% emulsifiers (e. g. calcium dodecylbenzenesulfonate and castor oil ethoxylate) are dissolved in water-insoluble organic solvent (e. g. aromatic hydrocarbon) ad 100 wt%. Dilution with water gives an emulsion.

### iv) Emulsions (EW, EO, ES)

5-40 wt% of a compound I and 1-10 wt% emulsifiers (e. g. calcium dodecylbenzenesulfonate and castor oil ethoxylate) are dissolved in 20-40 wt% water-insoluble organic solvent (e. g. aromatic hydrocarbon). This mixture is introduced into water ad 100 wt% by means of an emulsifying machine and made into a homogeneous emulsion. Dilution with water gives an emulsion.

### v) Suspensions (SC, OD, FS)

In an agitated ball mill, 20-60 wt% of a compound I are comminuted with addition of 2-10 wt% dispersants and wetting agents (e. g. sodium lignosulfonate and alcohol ethoxylate), 0.1-2 wt% thickener (e. g. xanthan gum) and water ad 100 wt% to give a fine active substance suspension. Dilution with water gives a stable suspension of the active substance. For FS type composition up to 40 wt% binder (e. g. polyvinyl alcohol) is added.

### vi) Water-dispersible granules and water-soluble granules (WG, SG)

50-80 wt% of a compound I are ground finely with addition of dispersants and wetting agents (e. g. sodium lignosulfonate and alcohol ethoxylate) ad 100 wt% and prepared as water-dispersible or water-soluble granules by means of technical appliances (e. g. extrusion, spray tower, fluidized bed). Dilution with water gives a stable dispersion or solution of the active substance.

### vii) Water-dispersible powders and water-soluble powders (WP, SP, WS)

50-80 wt% of a compound I are ground in a rotor-stator mill with addition of 1-5 wt% dispersants (e. g. sodium lignosulfonate), 1-3 wt% wetting agents (e. g. alcohol ethoxylate) and solid carrier (e. g. silica gel) ad 100 wt%. Dilution with water gives a stable dispersion or solution of the active substance.

### viii) Gel (GW, GF)

In an agitated ball mill, 5-25 wt% of a compound I are comminuted with addition of 3-10 wt% dispersants (e. g. sodium lignosulfonate), 1-5 wt% thickener (e. g. carboxymethyl cellulose) and water ad 100 wt% to give a fine suspension of the active substance. Dilution with water gives a stable suspension of the active substance.

### ix) Microemulsion (ME)

5-20 wt% of a compound I are added to 5-30 wt% organic solvent blend (e. g. fatty acid dimethyl amide and cyclohexanone), 10-25 wt% surfactant blend (e. g. alcohol ethoxylate and arylphenol ethoxylate), and water ad 100 %. This mixture is stirred for 1 h to produce spontaneously a thermodynamically stable microemulsion.

### x) Microcapsules (CS)

An oil phase comprising 5-50 wt% of a compound I, 0-40 wt% water insoluble organic solvent (e. g. aromatic hydrocarbon), 2-15 wt% acrylic monomers (e. g. methylmethacrylate, methacrylic acid and a di- or triacrylate) are dispersed into an aqueous solution of a protective colloid (e. g. polyvinyl alcohol). Radical polymerization results in the formation of poly(meth)acrylate microcapsules. Alternatively, an oil phase comprising 5-50 wt% of a compound I according to the invention, 0-40 wt% water insoluble organic solvent (e. g. aromatic hydrocarbon), and an isocyanate monomer (e. g. diphenylmethene-4,4'-diisocyanatae) are dispersed into an aqueous solution of a protective colloid (e. g. polyvinyl alcohol). The addition of a polyamine (e. g. hexamethylenediamine) results in the formation of polyurea microcapsules. The monomers amount to 1-10 wt%. The wt% relate to the total CS composition.

### xi) Dustable powders (DP, DS)

1-10 wt% of a compound I are ground finely and mixed intimately with solid carrier (e. g. finely divided kaolin) ad 100 wt%.

### xii) Granules (GR, FG)

0.5-30 wt% of a compound I is ground finely and associated with solid carrier (e. g. silicate) ad 100 wt%. Granulation is achieved by extrusion, spray-drying or fluidized bed.

### xiii) Ultra-low volume liquids (UL)

1-50 wt% of a compound I are dissolved in organic solvent (e. g. aromatic hydrocarbon) ad 100 wt%.

The compositions types i) to xiii) may optionally comprise further auxiliaries, such as 0.1-1 wt% bactericides, 5-15 wt% anti-freezing agents, 0.1-1 wt% anti-foaming agents, and 0.1-1 wt% colorants.

The agrochemical compositions generally comprise between 0.01 and 95%, preferably between 0.1 and 90%, and in particular between 0.5 and 75%, by weight of active substance. The active substances are employed in a purity of from 90% to 100%, preferably from 95% to 100% (according to NMR spectrum).

For the purposes of treatment of plant propagation materials, particularly seeds, solutions for seed treatment (LS), Suspoemulsions (SE), flowable concentrates (FS), powders for dry treatment (DS), water-dispersible powders for slurry treatment (WS), water-soluble powders (SS), emulsions (ES), emulsifiable concentrates (EC), and gels (GF) are usually employed. The compositions in question give, after two-to-tenfold dilution, active substance concentrations of from 0.01 to 60% by weight, preferably from 0.1 to 40%, in the ready-to-use preparations. Application can be carried out before or during sowing. Methods for applying compound I and compositions thereof, respectively, onto plant propagation material, especially seeds, include dressing, coating, pelleting, dusting, and soaking as well as in-furrow application methods. Preferably, compound I or the compositions thereof, respectively, are applied on to the plant propagation material by a method such that germination is not induced, e. g. by seed dressing, pelleting, coating and dusting.

When employed in plant protection, the amounts of active substances applied are, depending on the kind of effect desired, from 0.001 to 2 kg per ha, preferably from 0.005 to 2 kg per ha, more preferably from 0.05 to 0.9 kg per ha, and in particular from 0.1 to 0.75 kg per ha.

In treatment of plant propagation materials such as seeds, e. g. by dusting, coating or drenching seed, amounts of active substance of from 0.1 to 1000 g, preferably from 1 to 1000 g, more preferably from 1 to 100 g and most preferably from 5 to 100 g, per 100 kilogram of plant propagation material (preferably seeds) are generally required.

When used in the protection of materials or stored products, the amount of active substance applied depends on the kind of application area and on the desired effect. Amounts customarily applied in the protection of materials are 0.001 g to 2 kg, preferably 0.005 g to 1 kg, of active substance per cubic meter of treated material.

Various types of oils, wetters, adjuvants, fertilizer, or micronutrients, and further pesticides (e. g. herbicides, insecticides, fungicides, growth regulators, safeners, biopesticides) may be added to the active substances or the compositions comprising them as premix or, if appropriate not until immediately prior to use (tank mix). These agents can be admixed with the compositions according to the invention in a weight ratio of 1:100 to 100:1, preferably 1:10 to 10:1.

A pesticide is generally a chemical or biological agent (such as pestidal active ingredient, compound, composition, virus, bacterium, antimicrobial or disinfectant) that through its effect deters, incapacitates, kills or otherwise discourages pests. Target pests can include insects, plant pathogens, weeds, mollusks, birds, mammals, fish, nematodes (roundworms), and microbes that destroy property, cause nuisance, spread disease or are vectors for disease. The term "pesticide" includes also plant growth regulators that alter the expected growth, flowering, or reproduction rate of plants; defoliants that cause leaves or other foliage to drop from a plant, usually to facilitate harvest; desiccants that promote drying of living tissues, such as unwanted plant tops; plant activators that activate plant physiology for defense of against certain pests; safeners that reduce unwanted herbicidal action of pesticides on crop plants; and plant growth promoters that affect plant physiology e.g. to increase plant growth, biomass, yield or any other quality parameter of the harvestable goods of a crop plant.

Biopesticides have been defined as a form of pesticides based on micro-organisms (bacteria, fungi, viruses, nematodes, etc.) or natural products (compounds, such as metabolites, proteins, or extracts from biological or other natural sources) (U.S. Environmental Protection Agency: http://www.epa.gov/pesticides/biopesticides/). Biopesticides fall into two major classes, microbial and biochemical pesticides:
(1) Microbial pesticides consist of bacteria, fungi or viruses (and often include the metabolites that bacteria and fungi produce). Entomopathogenic nematodes are also classed as microbial pesticides, even though they are multi-cellular.
(2) Biochemical pesticides are naturally occurring substances that control pests or provide other crop protection uses as defined below, but are relatively non-toxic to mammals.

The user applies the composition according to the invention usually from a predosage device, a knapsack sprayer, a spray tank, a spray plane, or an irrigation system. Usually, the agrochemical composition is made up with water, buffer, and/or further auxiliaries to the desired application concentration and the ready-to-use spray liquor or the agrochemical composition according to the invention is thus obtained. Usually, 20 to 2000 liters, preferably 50 to 400 liters, of the ready-to-use spray liquor are applied per hectare of agricultural useful area.

According to one embodiment, individual components of the composition according to the invention such as parts of a kit or parts of a binary or ternary mixture may be mixed by the user himself in a spray tank or any other kind of vessel used for applications (e. g. seed treater drums, seed pelleting machinery, knapsack sprayer) and further auxiliaries may be added, if appropriate.

When living microorganisms, such as microbial pesticides from groups L1), L3) and L5), form part of such kit, it must be taken care that choice and amounts of the components (e. g. chemical pesticides) and of the further auxiliaries should not influence the viability of the microbial pesticides in the composition mixed by the user. Especially for bactericides and solvents, compatibility with the respective microbial pesticide has to be taken into account.

Consequently, one embodiment of the invention is a kit for preparing a usable pesticidal composition, the kit comprising a) a composition comprising component 1) as defined herein and at least one auxiliary; and b) a composition comprising component 2) as defined herein and at least one auxiliary; and optionally c) a composition comprising at least one auxiliary and optionally a further active component 3) as defined herein.

Mixing the compounds I or the compositions comprising them in the use form as fungicides with other fungicides results in many cases in an expansion of the fungicidal spectrum of activity being obtained or in a prevention of fungicide resistance development. Furthermore, in many cases, synergistic effects are obtained.

The following list of pesticides II (e. g. pesticidally-active substances and biopesticides), in conjunction with which the compounds I can be used, is intended to illustrate the possible combinations but does not limit them:
A) Respiration inhibitors
   - Inhibitors of complex III at Qₒ site (e. g. strobilurins): azoxystrobin (A.1.1), coumethoxystrobin (A.1.2), coumoxystrobin (A.1.3), dimoxystrobin (A.1.4), enestroburin (A.1.5), fenaminstrobin (A.1.6), fenoxystrobin/flufenoxystrobin (A.1.7), fluoxastrobin (A.1.8), kresoxim-methyl (A.1.9), mandestrobin (A.1.10), metominostrobin (A.1.11), orysastrobin (A.1.12), picoxystrobin (A.1.13), pyraclostrobin (A.1.14), pyrametostrobin (A.1.15), pyraoxystrobin (A.1.16), trifloxystrobin (A.1.17), 2-(2-(3-(2,6-dichlorophenyl)-1-methyl-allylideneaminooxymethyl)-phenyl)-2-methoxyimino-N-methyl-acetamide (A.1.18), pyribencarb (A.1.19), triclopyricarb/chlorodincarb (A.1.20), famoxadone (A.1.21), fenamidone (A.1.21), methyl-*N*-[2-[(1,4-dimethyl-5-phenyl-pyrazol-3-yl)oxylmethyl]phenyl]-N-methoxy-carbamate (A.1.22), 1-[3-chloro-2-[[[1-(4-chlorophenyl)-1H-pyrazol-3-yl]oxy]methyl]-phenyl]-1,4-dihydro-4-methyl-5H-tetrazol-5-one (A.1.23), (2*E*,3*Z*)-5-[[1-(2,4-dich)orophenyl)-1*H*-pyrazol-3-yl]oxy]-2-(methoxyimino)-*N*,3-dimethyl-pent-3-enamide (A.1.24), (2*E*,3Z)-5-[[1-(4-chlorophenyl)-1*H*-pyrazol-3-yl]oxy]-2-(methoxyimino)-*N*,3-dimethyl-pent-3-enamide (A.1.25);
   - inhibitors of complex III at Qᵢ site: cyazofamid (A.2.1), amisulbrom (A.2.2), [(3S,6S,7R,8R)-8-benzyl-3-[(3-acetoxy-4-methoxy-pyridine-2-carbonyl)amino]-6-methyl-4,9-dioxo-1,5-di-oxonan-7-yl] 2-methylpropanoate (A.2.3), [(3S,6S,7R,8R)-8-benzyl-3-[[3-(acetoxymethoxy)-4-methoxy-pyridine-2-carbonyl]amino]-6-methyl-4,9-dioxo-1,5-dioxonan-7-yl] 2-methylpropanoate (A.2.4), [(3S,6S,7R,8R)-8-benzyl-3-[(3-isobutoxycarbonyloxy-4-meth-oxy-pyridine-2-carbonyl)amino]-6-methyl-4,9-dioxo-1,5-dioxonan-7-yl] 2-methylpropanoate (A.2.5), [(3S,6S,7R,8R)-8-benzyl-3-[[3-(1,3-benzodioxol-5-ylmethoxy)-4-methoxy-pyridine-2-carbonyl]amino]-6-methyl-4,9-dioxo-1,5-dioxonan-7-yl] 2-methylpropanoate (A.2.6); (3S,6S,7R,8R)-3-[[(3-hydroxy-4-methoxy-2-pyridinyl)carbonyl]amino]-6-methyl-4,9-dioxo-8-(phenylmethyl)-1,5-dioxonan-7-yl 2-methylpropanoate (A.2.7);
   - inhibitors of complex II (e. g. carboxamides): benodanil (A.3.1), benzovindiflupyr (A.3.2), bixafen (A.3.3), boscalid (A.3.4), carboxin (A.3.5), fenfuram (A.3.6), fluopyram (A.3.7), flutolanil (A.3.8), fluxapyroxad (A.3.9), furametpyr (A.3.10), isofetamid (A.3.11), isopyrazam (A.3.12), mepronil (A.3.13), oxycarboxin (A.3.14), penflufen (A.3.14), penthiopyrad (A.3.15), sedaxane (A.3.16), tecloftalam (A.3.17), thifluzamide (A.3.18), N-(4'-trifluoromethylthiobiphenyl-2-yl)-3-difluoromethyl-1-methyl-1 H-pyrazole-4-carboxamide (A.3.19), N-(2-(1,3,3-trimethyl-butyl)-phenyl)-1,3-dimethyl-5-fluoro-1 H-pyrazole-4-carboxamide (A.3.20), 3-(difluoromethyl)-1-methyl-N-(1,1,3-trimethylindan-4-yl)pyrazole-4-carboxamide (A.3.21), 3-(trifluoromethyl)-1-methyl-N-(1,1,3-trimethylindan-4-yl)pyrazole-4-carboxamide (A.3.22), 1,3-dimethyl-N-(1,1,3-trimethylindan-4-yl)pyrazole-4-carboxamide (A.3.23), 3-(trifluoromethyl)-1,5-dimethyl-N-(1,1,3-trimethylindan-4-yl)pyrazole-4-carboxamide (A.3.24), 1,3,5-trimethyl-N-(1,1,3-trimethylindan-4-yl)pyrazole-4-carboxamide (A.3.25), N-(7-fluoro-1,1,3-trimethyl-indan-4-yl)-1,3-dimethyl-pyrazole-4-carboxamide (A.3.26), N-[2-(2,4-dichlorophenyl)-2-methoxy-1-methyl-ethyl]-3-(difluoromethyl)-1-methyl-pyrazole-4-carboxamide (A.3.27);
   - other respiration inhibitors (e. g. complex I, uncouplers): diflumetorim (A.4.1), (5,8-difluoro-quinazolin-4-yl)-{2-[2-fluoro-4-(4-trifluoromethylpyridin-2-yloxy)-phenyl]-ethyl}-amine (A.4.2); nitrophenyl derivates: binapacryl (A.4.3), dinobuton (A.4.4), dinocap (A.4.5), fluazinam (A.4.6); ferimzone (A.4.7); organometal compounds: fentin salts, such as fentin-acetate (A.4.8), fentin chloride (A.4.9) or fentin hydroxide (A.4.10); ametoctradin (A.4.11); and silthiofam (A.4.12);
B) Sterol biosynthesis inhibitors (SBI fungicides)
   - C14 demethylase inhibitors (DMI fungicides): triazoles: azaconazole (B.1.1), bitertanol (B.1.2), bromuconazole (B.1.3), cyproconazole (B.1.4), difenoconazole (B.1.5), diniconazole (B.1.6), diniconazole-M (B.1.7), epoxiconazole (B.1.8), fenbuconazole (B.1.9), fluquinconazole (B.1.10), flusilazole (B.1.11), flutriafol (B.1.12), hexaconazole (B.1.13), imibenconazole (B.1.14), ipconazole (B.1.15), metconazole (B.1.17), myclobutanil (B.1.18), oxpoconazole (B.1.19), paclobutrazole (B.1.20), penconazole (B.1.21), propiconazole (B.1.22), prothioconazole (B.1.23), simeconazole (B.1.24), tebuconazole (B.1.25), tetraconazole (B.1.26), triadimefon (B.1.27), triadimenol (B.1.28), triticonazole (B.1.29), uniconazole (B.1.30), 1-[*rel*-(2S;3R)-3-(2-chlorophenyl)-2-(2,4-difluorophenyl)-oxiranylmethyl]-5-thiocyanato-1 H-[1,2,4]triazolo (B.1.31), 2-[*rel*-(2S;3R)-3-(2-chlorophenyl)-2-(2,4-difluorophenyl)-oxiranylmethyl]-2H-[1,2,4]triazole-3-thiol (B.1.32), 2-[2-chloro-4-(4-chlorophenoxy)phenyl]-1-(1,2,4-triazol-1-yl)pentan-2-ol (B.1.33), 1-[4-(4-chlorophenoxy)-2-(trifluoromethyl)phenyl]-1-cyclopropyl-2-(1,2,4-triazol-1-yl)ethanol (B.1.34), 2-[4-(4-chloro-phenoxy)-2-(trifluoromethyl)phenyl]-1-(1,2,4-triazol-1-yl)butan-2-ol (B.1.35), 2-[2-chloro-4-(4-chlorophenoxy)phenyl]-1-(1,2,4-triazol-1-yl)butan-2-ol (B.1.36), 2-[4-(4-chlorophenoxy)-2-(trifluoromethyl)phenyl]-3-methyl-1-(1,2,4-triazol-1-yl)butan-2-ol (B.1.37), 2-[4-(4-chlorophenoxy)-2-(trifluoromethyl)phenyl]-1-(1,2,4-triazol-1-yl)propan-2-ol (B.1.38), 2-[2-chloro-4-(4-chlorophenoxy)phenyl]-3-methyl-1-(1,2,4-triazol-1-yl)butan-2-ol (B.1.39), 2-[4-(4-chlorophenoxy)-2-(trifluoromethyl)phenyl]-1-(1,2,4-triazol-1-yl)pentan-2-ol (B.1.40), 2-[4-(4-fluorophenoxy)-2-(trifluoromethyl)phenyl]-1-(1,2,4-triazol-1-yl)propan-2-ol (B.1.41), 2-[2-chloro-4-(4-chlorophenoxy)phenyl]-1-(1,2,4-triazol-1-yl)pent-3-yn-2-ol (B.1.51); imidazoles: imazalil (B.1.42), pefurazoate (B.1.43), prochloraz (B.1.44), triflumizol (B.1.45); pyrimidines, pyridines and piperazines: fenarimol (B.1.46), nuarimol (B.1.47), pyrifenox (B.1.48), triforine (B.1.49), [3-(4-chloro-2-fluoro-phenyl)-5-(2,4-difluorophenyl)isoxazol-4-yl]-(3-pyridyl)methanol (B.1.50);
   - Delta14-reductase inhibitors: aldimorph (B.2.1), dodemorph (B.2.2), dodemorph-acetate (B.2.3), fenpropimorph (B.2.4), tridemorph (B.2.5), fenpropidin (B.2.6), piperalin (B.2.7), spiroxamine (B.2.8);
   - Inhibitors of 3-keto reductase: fenhexamid (B.3.1);
C) Nucleic acid synthesis inhibitors
   - phenylamides or acyl amino acid fungicides: benalaxyl (C.1.1), benalaxyl-M (C.1.2), kiralaxyl (C.1.3), metalaxyl (C.1.4), metalaxyl-M (mefenoxam, C.1.5), ofurace (C.1.6), oxadixyl (C.1.7);
   - others: hymexazole (C.2.1), octhilinone (C.2.2), oxolinic acid (C.2.3), bupirimate (C.2.4), 5-fluorocytosine (C.2.5), 5-fluoro-2-(p-tolylmethoxy)pyrimidin-4-amine (C.2.6), 5-fluoro-2-(4-fluorophenylmethoxy)pyrimidin-4-amine (C.2.7);
D) Inhibitors of cell division and cytoskeleton
   - tubulin inhibitors, such as benzimidazoles, thiophanates: benomyl (D1.1), carbendazim (D1.2), fuberidazole (D1.3), thiabendazole (D1.4), thiophanate-methyl (D1.5); triazolopyrimidines: 5-chloro-7-(4-methylpiperidin-1-yl)-6-(2,4,6-trifluorophenyl)-[1,2,4]tri-azolo[1,5-a]pyrimidine (D1.6);
   - other cell division inhibitors: diethofencarb (D2.1), ethaboxam (D2.2), pencycuron (D2.3), fluopicolide (D2.4), zoxamide (D2.5), metrafenone (D2.6), pyriofenone (D2.7);
E) Inhibitors of amino acid and protein synthesis
   - methionine synthesis inhibitors (anilino-pyrimidines): cyprodinil (E.1.1), mepanipyrim (E.1.2), pyrimethanil (E.1.3);
   - protein synthesis inhibitors: blasticidin-S (E.2.1), kasugamycin (E.2.2), kasugamycin hydrochloride-hydrate (E.2.3), mildiomycin (E.2.4), streptomycin (E.2.5), oxytetracyclin (E.2.6), polyoxine (E.2.7), validamycin A (E.2.8);
F) Signal transduction inhibitors
   - MAP / histidine kinase inhibitors: fluoroimid (F.1.1), iprodione (F.1.2), procymidone (F.1.3), vinclozolin (F.1.4), fenpiclonil (F.1.5), fludioxonil (F.1.6);
   - G protein inhibitors: quinoxyfen (F.2.1);
G) Lipid and membrane synthesis inhibitors
   - Phospholipid biosynthesis inhibitors: edifenphos (G.1.1), iprobenfos (G.1.2), pyrazophos (G.1.3), isoprothiolane (G.1.4);
   - lipid peroxidation: dicloran (G.2.1), quintozene (G.2.2), tecnazene (G.2.3), tolclofos-methyl (G.2.4), biphenyl (G.2.5), chloroneb (G.2.6), etridiazole (G.2.7);
   - phospholipid biosynthesis and cell wall deposition: dimethomorph (G.3.1), flumorph (G.3.2), mandipropamid (G.3.3), pyrimorph (G.3.4), benthiavalicarb (G.3.5), iprovalicarb (G.3.6), valifenalate (G.3.7) and N-(1-(1-(4-cyano-phenyl)ethanesulfonyl)-but-2-yl) carbamic acid-(4-fluorophenyl) ester (G.3.8);
   - compounds affecting cell membrane permeability and fatty acides: propamocarb (G.4.1);
   - fatty acid amide hydrolase inhibitors: oxathiapiprolin (G.5.1), 2-{3-[2-(1-{[3,5-bis(difluoromethyl-1 H-pyrazol-1-yl]acetyl}piperidin-4-yl)-1,3-thiazol-4-yl]-4,5-dihydro-1,2-oxazol-5-yl}phenyl methanesulfonate (G.5.2), 2-{3-[2-(1-{[3,5-bis(difluoromethyl)-1H-pyrazol-1-yl]acetyl}piperidin-4-yl) 1,3-thiazol-4-yl]-4,5-dihydro-1,2-oxazol-5-yl}-3-chlorophenyl methanesulfonate (G.5.3);
H) Inhibitors with Multi Site Action
   - inorganic active substances: Bordeaux mixture (H.1.1), copper acetate (H.1.2), copper hydroxide (H.1.3), copper oxychloride (H.1.4), basic copper sulfate (H.1.5), sulfur (H.1.6);
   - thio- and dithiocarbamates: ferbam (H.2.1), mancozeb (H.2.2), maneb (H.2.3), metam (H.2.4), metiram (H.2.5), propineb (H.2.6), thiram (H.2.7), zineb (H.2.8), ziram (H.2.9);
   - organochlorine compounds (e. g. phthalimides, sulfamides, chloronitriles): anilazine (H.3.1), chlorothalonil (H.3.2), captafol (H.3.3), captan (H.3.4), folpet (H.3.5), dichlofluanid (H.3.6), dichlorophen (H.3.7), hexachlorobenzene (H.3.8), pentachlorphenole (H.3.9) and its salts, phthalide (H.3.10), tolylfluanid (H.3.11), N-(4-chloro-2-nitro-phenyl)-N-ethyl-4-methyl-benzenesulfonamide (H.3.12);
   - guanidines and others: guanidine (H.4.1), dodine (H.4.2), dodine free base (H.4.3), guazatine (H.4.4), guazatine-acetate (H.4.5), iminoctadine (H.4.6), iminoctadine-triacetate (H.4.7), iminoctadine-tris(albesilate) (H.4.8), dithianon (H.4.9), 2,6-dimethyl-1 H,5H-[1,4]dithiino[2,3-c:5,6-c']dipyrrole-1,3,5,7(2H,6H)-tetraone (H.4.10);
I) Cell wall synthesis inhibitors
   - inhibitors of glucan synthesis: validamycin (1.1.1), polyoxin B (I.1.2);
   - melanin synthesis inhibitors: pyroquilon (1.2.1), tricyclazole (1.2.2), carpropamid (1.2.3), dicyclomet (1.2.4), fenoxanil (I.2.5);
J) Plant defence inducers
   - acibenzolar-S-methyl (J.1.1), probenazole (J.1.2), isotianil (J.1.3), tiadinil (J.1.4), prohexadione-calcium (J.1.5); phosphonates: fosetyl (J.1.6), fosetyl-aluminum (J.1.7), phosphorous acid and its salts (J.1.8), potassium or sodium bicarbonate (J.1.9);
K) Unknown mode of action
   - bronopol (K.1.1), chinomethionat (K.1.2), cyflufenamid (K.1.3), cymoxanil (K.1.4), dazomet (K.1.5), debacarb (K.1.6), diclomezine (K.1.7), difenzoquat (K.1.8), difenzoquat-methylsulfate (K.1.9), diphenylamin (K.1.10), fenpyrazamine (K.1.11), flumetover (K.1.12), flusulfamide (K.1.13), flutianil (K.1.14), methasulfocarb (K.1.15), nitrapyrin (K.1.16), nitrothal-isopropyl (K.1.18), oxathiapiprolin (K.1.19), tolprocarb (K.1.20), oxin-copper (K.1.21), proquinazid (K.1.22), tebufloquin (K.1.23), tecloftalam (K.1.24), triazoxide (K.1.25), 2-butoxy-6-iodo-3-propylchromen-4-one (K.1.26), 2-[3,5-bis(difluoromethyl)-1 H-pyrazol-1-yl]-1-[4-(4-{5-[2-(prop-2-yn-1-yloxy)phenyl]-4,5-dihydro-1,2-oxazol-3-yl}-1,3-thiazol-2-yl)piperidin-1-yl]ethanone (K.1.27), 2-[3,5-bis(difluoromethyl)-1H-pyrazol-1-yl]-1-[4-(4-{5-[2-fluoro-6-(prop-2-yn-1-yloxy)phenyl]-4,5-dihydro-1,2-oxazol-3-yl}-1,3-thiazol-2-yl)piperidin-1-yl]ethanone (K.1.28), 2-[3,5-bis(difluoromethyl)-1H-pyrazol-1-yl]-1-[4-(4-{5-[2-chloro-6-(prop-2-yn-1-yl-oxy)phenyl]-4,5-dihydro-1,2-oxazol-3-yl}-1,3-thiazol-2-yl)piperidin-1-yl]ethanone (K.1.29), N-(cyclopropylmethoxyimino-(6-difluoro-methoxy-2,3-difluoro-phenyl)-methyl)-2-phenyl acetamide (K.1.30), N'-(4-(4-chloro-3-trifluoromethyl-phenoxy)-2,5-dimethyl-phenyl)-N-ethyl-N-methyl formamidine (K.1.31), N'-(4-(4-fluoro-3-trifluoromethyl-phenoxy)-2,5-dimethylphenyl)-N-ethyl-N-methyl formamidine (K.1.32), N'-(2-methyl-5-trifluoromethyl-4-(3-trimethylsilanyl-propoxy)-phenyl)-N-ethyl-N-methyl formamidine (K.1.33), N'-(5-difluoromethyl2-methyl-4-(3-trimethylsilanyl-propoxy)-phenyl)-N-ethyl-N-methyl formamidine (K.1.34), methoxy-acetic acid 6-tert-butyl-8-fluoro-2,3-dimethyl-quinolin-4-yl ester (K.1.35), 3-[5-(4-methylphenyl)-2,3-dimethyl-isoxazolidin-3-yl]-pyridine (K.1.36), 3-[5-(4-chloro-phenyl)-2,3-dimethyl-isoxazolidin-3-yl]-pyridine (pyrisoxazole) (K.1.37), N-(6-methoxy-pyridin-3-yl) cyclopropanecarboxylic acid amide (K.1.38), 5-chloro-1-(4,6-dimethoxy-pyrimidin-2-yl)-2-methyl-1 H-benzoimidazole (K.1.39), 2-(4-chloro-phenyl)-N-[4-(3,4-dimethoxy-phenyl)-isoxazol-5-yl]-2-prop-2-ynyloxy-acetamide, ethyl (Z)-3-amino-2-cyano-3-phenyl-prop-2-enoate (K.1.40), picarbutrazox (K.1.41), pentyl N-[6-[[(Z)-[(1-methyltetrazol-5-yl)-phenyl-methylene]amino]oxymethyl]-2-pyridyl]carbamate (K.1.42), 2-[2-[(7,8-difluoro-2-methyl-3-quinolyl)oxy]-6-fluoro-phenyl]propan-2-ol (K.1.43), 2-[2-fluoro-6-[(8-fluoro-2-methyl-3-quinolyl)oxy]phen-yl]propan-2-ol (K.1.44), 3-(5-fluoro-3,3,4,4-tetramethyl-3,4-dihydroisoquinolin-1-yl)quinoline (K.1.45), 3-(4,4-difluoro-3,3-dimethyl-3,4-dihydroisoquinolin-1-yl)quinoline (K.1.46), 3-(4,4,5-trifluoro-3,3-dimethyl-3,4-dihydroisoquinolin-1-yl)quinoline (K.1.47), 9-fluoro-2,2-dimethyl-5-(3-quinolyl)-3H-1,4-benzoxazepine (K.1.48);
L) Biopesticides
   L1) Microbial pesticides with fungicidal, bactericidal, viricidal and/or plant defense activator activity: *Ampelomyces quisqualis, Aspergillus flavus, Aureobasidium pullulans, Bacillus altitudinis, B. amyloliquefaciens, B. megaterium, B. mojavensis, B. mycoides, B. pumilus, B. simplex, B. solisalsi, B. subtilis, B. subtilis* var. *amyloliquefaciens, Candida oleophila, C. saitoana, Clavibacter michiganensis* (bacteriophages), *Coniothyrium minitans, Cryphonectria parasitica, Cryptococcus albidus, Dilophosphora alopecuri, Fusarium oxysporum, Clonostachys rosea* f. *catenulate* (also named *Gliocladium catenulatum), Gliocladium roseum, Lysobacter antibioticus, L. enzymogenes, Metschnikowia fructicola, Microdochium dimerum, Microsphaeropsis ochracea, Muscodor albus, Paenibacillus alvei, Paenibacillus polymyxa, Pantoea vagans, Penicillium bilaiae, Phlebiopsis gigantea, Pseudomonas* sp., *Pseudomonas chloraphis, Pseudozyma flocculosa, Pichia anomala, Pythium oligandrum, Sphaerodes mycoparasitica, Streptomyces griseoviridis, S. lydicus, S. violaceusniger, Talaromyces flavus, Trichoderma asperellum, T. atroviride, T. fertile, T. gamsii, T. harmatum, T. harzianum, T. polysporum, T. stromaticum, T. virens, T. viride, Typhula phacorrhiza, Ulocladium oudemansii, Verticillium dahlia,* zucchini yellow mosaic virus (avirulent strain);
   L2) Biochemical pesticides with fungicidal, bactericidal, viricidal and/or plant defense activator activity: chitosan (hydrolysate), harpin protein, laminarin, Menhaden fish oil, natamycin, Plum pox virus coat protein, potassium or sodium bicarbonate, *Reynoutria sachalinensis* extract, salicylic acid, tea tree oil;
   L3) Microbial pesticides with insecticidal, acaricidal, molluscidal and/or nematicidal activity: *Agrobacterium radiobacter, Bacillus cereus, B. firmus, B. thuringiensis, B. thuringiensis* ssp. *aizawai, B. t.* ssp. *israelensis, B. t.* ssp. *galleriae, B. t.* ssp. *kurstaki, B. t.* ssp. *tenebrionis, Beauveria bassiana, B. brongniartii, Burkholderia* spp., *Chromobacterium subtsugae, Cydia pomonella* granulovirus (CpGV), *Cryptophlebia leucotreta* granulovirus (CrIeGV), *Flavobacterium* spp., *Helicoverpa armigera* nucleopolyhedrovirus (HearNPV), *Heterorhabditis bacteriophora, Isaria fumosorosea, Lecanicillium longisporum, L. muscarium, Metarhizium anisopliae, Metarhizium anisopliae* var. *anisopliae, M. anisopliae* var. *acridum, Nomuraea rileyi, Paecilomyces lilacinus, Paenibacillus popilliae, Pasteuria* spp., *P. nishizawae, P. penetrans, P. ramosa, P. thornea, P. usgae, Pseudomonas fluorescens, Spodoptera littoralis* nucleopolyhedrovirus (SpliNPV), *Steinernema carpocapsae, S. feltiae, S. kraussei, Streptomyces galbus, S. microflavus;*
   L4) Biochemical pesticides with insecticidal, acaricidal, molluscidal, pheromone and/or nematicidal activity: L-carvone, citral, (E,Z)-7,9-dodecadien-1-yl acetate, ethyl formate, (E,Z)-2,4-ethyl decadienoate (pear ester), (Z,Z,E)-7,11,13-hexadecatrienal, heptyl butyrate, isopropyl myristate, lavanulyl senecioate, cis-jasmone, 2-methyl 1-butanol, methyl eugenol, methyl jasmonate, (E,Z)-2,13-octadecadien-1-ol, (E,Z)-2,13-octadecadien-1-ol acetate, (E,Z)-3,13-octadecadien-1-ol, R-1-octen-3-ol, pentatermanone, potassium silicate, sorbitol actanoate, (E,Z,Z)-3,8,11-tetradecatrienyl acetate, (Z,E)-9,12-tetradecadien-1-yl acetate, Z-7-tetradecen-2-one, Z-9-tetradecen-1-yl acetate, Z-11-tetradecenal, Z-11-tetradecen-1-ol, Acacia negra extract, extract of grapefruit seeds and pulp, extract of *Chenopodium ambrosiodes,* Catnip oil, Neem oil, Quillay extract, Tagetes oil;
   L5) Microbial pesticides with plant stress reducing, plant growth regulator, plant growth promoting and/or yield enhancing activity: *Azospirillum amazonense, A. brasilense, A. lipoferum, A. irakense, A. halopraeferens, Bradyrhizobium* spp., *B. elkanii, B. japonicum, B. liaoningense, B. lupini, Delftia acidovorans, Glomus intraradices, Mesorhizobium* spp., *Rhizobium leguminosarum* bv. *phaseoli, R*. *I.* bv. *trifolii, R*. *I.* bv. *viciae, R. tropici, Sinorhizobium meliloti*;
   L6) Biochemical pesticides with plant stress reducing, plant growth regulator and/or plant yield enhancing activity: abscisic acid, aluminium silicate (kaolin), 3-decen-2-one, formononetin, genistein, hesperetin, homobrassinolide, humates, jasmonic acid and its salts or derivatives thereof, lysophosphatidyl ethanolamine, naringenin, polymeric polyhydroxy acid, *Ascophyllum nodosum* (Norwegian kelp, Brown kelp) extract and *Ecklonia maxima* (kelp) extract;
M) Growth regulators
   - abscisic acid (M.1.1), amidochlor, ancymidol, 6-benzylaminopurine, brassinolide, butralin, chlormequat, chlormequat chloride, choline chloride, cyclanilide, daminozide, dikegulac, dimethipin, 2,6-dimethylpuridine, ethephon, flumetralin, flurprimidol, fluthiacet, forchlorfenuron, gibberellic acid, inabenfide, indole-3-acetic acid , maleic hydrazide, mefluidide, mepiquat, mepiquat chloride, naphthaleneacetic acid, N-6-benzyladenine, paclobutrazol, prohexadione, prohexadione-calcium, prohydrojasmon, thidiazuron, triapenthenol, tributyl phosphorotrithioate, 2,3,5-tri-iodobenzoic acid , trinexapac-ethyl and uniconazole;
N) Herbicides
   - acetamides: acetochlor (N.1.1), alachlor, butachlor, dimethachlor, dimethenamid (N.1.2), flufenacet (N.1.3), mefenacet (N.1.4), metolachlor (N.1.5), metazachlor (N.1.6), napropamide, naproanilide, pethoxamid, pretilachlor, propachlor, thenylchlor;
   - amino acid derivatives: bilanafos, glyphosate (N.2.1), glufosinate (N.2.2), sulfosate (N.2.3);
   - aryloxyphenoxypropionates: clodinafop (N.3.1), cyhalofop-butyl, fenoxaprop (N.3.2), fluazifop (N.3.3), haloxyfop (N.3.4), metamifop, propaquizafop, quizalofop, quizalofop-P-tefuryl;
   - Bipyridyls: diquat, paraquat (N.4.1);
   - (thio)carbamates: asulam, butylate, carbetamide, desmedipham, dimepiperate, eptam (EPTC), esprocarb, molinate, orbencarb, phenmedipham (N.5.1), prosulfocarb, pyributicarb, thiobencarb, triallate;
   - cyclohexanediones: butroxydim, clethodim (N.6.1), cycloxydim (N.6.2), profoxydim (N.6.3), sethoxydim (N.6.4), tepraloxydim (N.6.5), tralkoxydim;
   - dinitroanilines: benfluralin, ethalfluralin, oryzalin, pendimethalin (N.7.1), prodiamine (N.7.2), trifluralin (N.7.3);
   - diphenyl ethers: acifluorfen (N.8.1), aclonifen, bifenox, diclofop, ethoxyfen, fomesafen, lactofen, oxyfluorfen;
   - hydroxybenzonitriles: bomoxynil (N.9.1), dichlobenil, ioxynil;
   - imidazolinones: imazamethabenz, imazamox (N.10.1), imazapic (N.10.2), imazapyr (N.10.3), imazaquin (N.10.4), imazethapyr (N.10.5);
   - phenoxy acetic acids: clomeprop, 2,4-dichlorophenoxyacetic acid (2,4-D) (N.11.1), 2,4-DB, dichlorprop, MCPA, MCPA-thioethyl, MCPB, Mecoprop;
   - pyrazines: chloridazon (N.11.1), flufenpyr-ethyl, fluthiacet, norflurazon, pyridate;
   - pyridines: aminopyralid, clopyralid (N.12.1), diflufenican, dithiopyr, fluridone, fluroxypyr (N.12.2), picloram (N.12.3), picolinafen (N.12.4), thiazopyr;
   - sulfonyl ureas: amidosulfuron, azimsulfuron, bensulfuron (N.13.1), chlorimuron-ethyl (N.13.2), chlorsulfuron, cinosulfuron, cyclosulfamuron (N.13.3), ethoxysulfuron, flazasulfuron, flucetosulfuron, flupyrsulfuron, foramsulfuron, halosulfuron, imazosulfuron, iodosulfuron (N.13.4), mesosulfuron (N.13.5), metazosulfuron, metsulfuron-methyl (N.13.6), nicosulfuron (N.13.7), oxasulfuron, primisulfuron, prosulfuron, pyrazosulfuron, rimsulfuron (N.13.8), sulfometuron, sulfosulfuron, thifensulfuron, triasulfuron, tribenuron, trifloxysulfuron, triflusulfuron (N.13.9), tritosulfuron, 1-((2-chloro-6-propyl-imidazo[1,2-b]pyridazin-3-yl)sulfonyl)-3-(4,6-dimethoxy-pyrimidin-2-yl)urea;
   - triazines: ametryn, atrazine (N.14.1), cyanazine, dimethametryn, ethiozin, hexazinone (N.14.2), metamitron, metribuzin, prometryn, simazine, terbuthylazine, terbutryn, triaziflam, trifludimoxazin (N14.3);
   - ureas: chlorotoluron, daimuron, diuron (N.15.1), fluometuron, isoproturon, linuron, methabenzthiazuron, tebuthiuron;
   - other acetolactate synthase inhibitors: bispyribac-sodium, cloransulam-methyl, diclosulam, florasulam (N.16.1), flucarbazone, flumetsulam, metosulam, ortho-sulfamuron, penoxsulam, propoxycarbazone, pyribambenz-propyl, pyribenzoxim, pyriftalid, pyriminobac-methyl, pyrimisulfan, pyrithiobac, pyroxasulfone (N.16.2), pyroxsulam;
   - others: amicarbazone, aminotriazole, anilofos, beflubutamid, benazolin, bencarbazone,benfluresate, benzofenap, bentazone (N.17.1), benzobicyclon, bicyclopyrone, bromacil, bromobutide, butafenacil, butamifos, cafenstrole, carfentrazone, cinidon-ethyl (N.17.2), chlorthal, cinmethylin (N.17.3), clomazone (N.17.4), cumyluron, cyprosulfamide, dicamba (N.17.5), difenzoquat, diflufenzopyr (N.17.6), *Drechslera monoceras,* endothal, ethofumesate, etobenzanid, fenoxasulfone, fentrazamide, flumiclorac-pentyl, flumioxazin, flupoxam, flurochloridone, flurtamone, indanofan, isoxaben, isoxaflutole, lenacil, propanil, propyzamide, quinclorac (N.17.7), quinmerac (N.17.8), mesotrione (N.17.9), methyl arsonic acid, naptalam, oxadiargyl, oxadiazon, oxaziclomefone, pentoxazone, pinoxaden, pyraclonil, pyraflufen-ethyl, pyrasulfotole, pyrazoxyfen, pyrazolynate, quinoclamine, saflufenacil (N.17.10), sulcotrione (N.17.11), sulfentrazone, terbacil, tefuryltrione, tembotrione, thiencarbazone, topramezone (N.17.12), (3-[2-chloro-4-fluoro-5-(3-methyl-2,6-dioxo-4-trifluoromethyl-3,6-dihydro-2H-pyrimidin-1-yl)-phenoxy]-pyridin-2-yloxy)-acetic acid ethyl ester, 6-amino-5-chloro-2-cyclopropyl-pyrimidine-4-carboxylic acid methyl ester, 6-chloro-3-(2-cyclopropyl-6-methyl-phenoxy)-pyridazin-4-ol, 4-amino-3-chloro-6-(4-chloro-phenyl)-5-fluoropyridine-2-carboxylic acid, 4-amino-3-chloro-6-(4-chloro-2-fluoro-3-methoxy-phenyl)-pyridine-2-carboxylic acid methyl ester, and 4-amino-3-chloro-6-(4-chloro-3-dimethylamino-2-fluoro-phenyl)-pyridine-2-carboxylic acid methyl ester;
O) Insecticides
   - organo(thio)phosphates: acephate (O.1.1), azamethiphos (0.1.2), azinphos-methyl (0.1.3), chlorpyrifos (0.1.4), chlorpyrifos-methyl (0.1.5), chlorfenvinphos (0.1.6), diazinon (0.1.7), dichlorvos (0.1.8), dicrotophos (0.1.9), dimethoate (O.1.10), disulfoton (O.1.11), ethion (0.1.12), fenitrothion (0.1.13), fenthion (0.1.14), isoxathion (0.1.15), malathion (0.1.16), methamidophos (0.1.17), methidathion (0.1.18), methyl-parathion (0.1.19), mevinphos (0.1.20), monocrotophos (0.1.21), oxydemeton-methyl (0.1.22), paraoxon (0.1.23), parathion (0.1.24), phenthoate (0.1.25), phosalone (0.1.26), phosmet (0.1.27), phosphamidon (0.1.28), phorate (0.1.29), phoxim (0.1.30), pirimiphos-methyl (0.1.31), profenofos (0.1.32), prothiofos (0.1.33), sulprophos (0.1.34), tetrachlorvinphos (0.1.35), terbufos (0.1.36), triazophos (0.1.37), trichlorfon (0.1.38);
   - carbamates: alanycarb (0.2.1), aldicarb (0.2.2), bendiocarb (0.2.3), benfuracarb (O.2.4), carbaryl (0.2.5), carbofuran (0.2.6), carbosulfan (0.2.7), fenoxycarb (0.2.8), furathiocarb (0.2.9), methiocarb (0.2.10), methomyl (0.2.11), oxamyl (0.2.12), pirimicarb (0.2.13), propoxur (0.2.14), thiodicarb (0.2.15), triazamate (0.2.16);
   - pyrethroids: allethrin (0.3.1), bifenthrin (0.3.2), cyfluthrin (0.3.3), cyhalothrin (O.3.4), cyphenothrin (0.3.5), cypermethrin (0.3.6), alpha-cypermethrin (0.3.7), beta-cypermethrin (0.3.8), zeta-cypermethrin (0.3.9), deltamethrin (0.3.10), esfenvalerate (0.3.11), etofenprox (0.3.11), fenpropathrin (0.3.12), fenvalerate (0.3.13), imiprothrin (0.3.14), lambda-cyhalothrin (0.3.15), permethrin (0.3.16), prallethrin (0.3.17), pyrethrin I and II (0.3.18), resmethrin (0.3.19), silafluofen (0.3.20), tau-fluvalinate (0.3.21), tefluthrin (0.3.22), tetramethrin (0.3.23), tralomethrin (0.3.24), transfluthrin (0.3.25), profluthrin (0.3.26), dimefluthrin (0.3.27);
   - insect growth regulators: a) chitin synthesis inhibitors: benzoylureas: chlorfluazuron (O.4.1), cyramazin (O.4.2), diflubenzuron (O.4.3), flucycloxuron (O.4.4), flufenoxuron (O.4.5), hexaflumuron (O.4.6), lufenuron (O.4.7), novaluron (O.4.8), teflubenzuron (O.4.9), triflumuron (O.4.10); buprofezin (O.4.11), diofenolan (O.4.12), hexythiazox (O.4.13), etoxazole (O.4.14), clofentazine (O.4.15); b) ecdysone antagonists: halofenozide (O.4.16), methoxyfenozide (O.4.17), tebufenozide (O.4.18), azadirachtin (O.4.19); c) juvenoids: pyriproxyfen (O.4.20), methoprene (O.4.21), fenoxycarb (O.4.22); d) lipid biosynthesis inhibitors: spirodiclofen (O.4.23), spiromesifen (O.4.24), spirotetramat (O.4.24);
   - nicotinic receptor agonists/antagonists compounds: clothianidin (0.5.1), dinotefuran (0.5.2), flupyradifurone (0.5.3), imidacloprid (O.5.4), thiamethoxam (0.5.5), nitenpyram (0.5.6), acetamiprid (0.5.7), thiacloprid (0.5.8), 1-2-chloro-thiazol-5-ylmethyl)-2-nitrimino-3,5-dimethyl-[1,3,5]triazinane (0.5.9);
   - GABA antagonist compounds: endosulfan (0.6.19, ethiprole (0.6.2), fipronil (0.6.3), vaniliprole (O.6.4), pyrafluprole (0.6.5), pyriprole (0.6.6), 5-amino-1-(2,6-dichloro-4-methyl-phenyl)-4-sulfinamoyl-1 H-pyrazole-3-carbothioic acid amide (0.6.7);
   - macrocyclic lactone insecticides: abamectin (0.7.1), emamectin (0.7.2), milbemectin (0.7.3), lepimectin (0.7.4), spinosad (0.7.5), spinetoram (0.7.6);
   - mitochondrial electron transport inhibitor (METI) I acaricides: fenazaquin (0.8.1), pyridaben (0.8.2), tebufenpyrad (0.8.3), tolfenpyrad (O.8.4), flufenerim (0.8.5);
   - METI II and III compounds: acequinocyl (0.9.1), fluacyprim (0.9.2), hydramethylnon (0.9.3);
   - Uncouplers: chlorfenapyr (O.10.1);
   - oxidative phosphorylation inhibitors: cyhexatin (O.11.1), diafenthiuron (0.11.2), fenbutatin oxide (0.11.3), propargite (O.11.4);
   - moulting disruptor compounds: cryomazine (0.12.1);
   - mixed function oxidase inhibitors: piperonyl butoxide (0.13.1);
   - sodium channel blockers: indoxacarb (0.14.1), metaflumizone (0.14.2);
   - ryanodine receptor inhibitors: chlorantraniliprole (O.15.1), cyantraniliprole (0.15.2), flubendiamide (0.15.3), N-[4,6-dichloro-2-[(diethyl-lambda-4-sulfanylidene)carbamoyl]-phenyl]-2-(3-chloro-2-pyridyl)-5-(trifluoromethyl)pyrazole-3-carboxamide (0.15.4); N-[4-chloro-2-[(di-ethyl-lambda-4-sulfanylidene)carbamoyl]-6-methyl-phenyl]-2-(3-chloro-2-pyridyl)-5-(trifluoromethyl)pyrazole-3-carboxamide (0.15.5); N-[4-chloro-2-[(di-2-propyl-lambda-4-sulfanylidene)carbamoyl]-6-methyl-phenyl]-2-(3-chloro-2-pyridyl)-5-(trifluoromethyl)pyrazole-3-carboxamide (0.15.6); N-[4,6-dichloro-2-[(di-2-propyl-lambda-4-sulfanylidene)carbamoyl]phenyl]-2-(3-chloro-2-pyridyl)-5-(trifluoromethyl)pyrazole-3-carboxamide (0.15.7); N-[4,6-dichloro-2-[(diethyl-lambda-4-sulfanylidene)carbamoyl]-phenyl]-2-(3-chloro-2-pyridyl)-5-(difluoromethyl)pyrazole-3-carboxamide (0.15.8); N-[4,6-dibromo-2-[(di-2-propyl-lambda-4-sulfanylidene)carbamoyl]-phenyl]-2-(3-chloro-2-pyridyl)-5-(trifluoromethyl)pyrazole-3-carboxamide (0.15.9); N-[4-chloro-2-[(di-2-propyl-lambda-4-sulfanylidene)carbamoyl]-6-cyanophenyl]-2-(3-chloro-2-pyridyl)-5-(trifluoromethyl)pyrazole-3-carboxamide (0.15.10); N-[4,6-dibromo-2-[(diethyl-lambda-4-sulfanylidene)carbamoyl]-phenyl]-2-(3-chloro-2-pyridyl)-5-(trifluoromethyl)pyrazole-3-carboxamide (O.15.11);
   - others: benclothiaz (O.16.1), bifenazate (0.16.2), artap (0.16.3), flonicamid (0.16.4), pyridalyl (0.16.5), pymetrozine (0.16.6), sulfur (0.16.7), thiocyclam (0.16.8), cyenopyrafen (0.16.9), flupyrazofos (0.16.10), cyflumetofen (O.16.11), amidoflumet (0.16.12), imicyafos (0.16.13), bistrifluron (0.16.14), pyrifluquinazon (0.16.15) and 1,1 '-[(3S,4R,4aR,6S,6aS, 12R, 12aS, 12bS)-4-[[(2-cyclopropylacetyl)oxy]methyl]-1,3,4,4a,5,6,6a,12,12a,12b-decahydro-12-hydroxy-4,6a,12b-trimethyl-11-oxo-9-(3-pyridinyl)-2H,11H-naphtho[2,1-b]pyrano[3,4-e]pyran-3,6-diyl] cyclopropaneacetic acid ester (0.16.16).

The present invention furthermore relates to agrochemical compositions comprising a mixture of at least one compound I (component 1) and at least one further active substance useful for plant protection, e. g. selected from the groups A) to O) (component 2), in particular one further fungicide, e. g. one or more fungicide from the groups A) to K), as described above, and if desired one suitable solvent or solid carrier. Those mixtures are of particular interest, since many of them at the same application rate show higher efficiencies against harmful fungi. Furthermore, combating harmful fungi with a mixture of compounds I and at least one fungicide from groups A) to K), as described above, is more efficient than combating those fungi with individual compounds I or individual fungicides from groups A) to K).

By applying compounds I together with at least one active substance from groups A) to O) a synergistic effect can be obtained, i.e. more then simple addition of the individual effects is obtained (synergistic mixtures).

This can be obtained by applying the compounds I and at least one further active substance simultaneously, either jointly (e. g. as tank-mix) or seperately, or in succession, wherein the time interval between the individual applications is selected to ensure that the active substance applied first still occurs at the site of action in a sufficient amount at the time of application of the further active substance(s). The order of application is not essential for working of the present invention.

When applying compound I and a pesticide II sequentially the time between both applications may vary e. g. between 2 hours to 7 days. Also a broader range is possible ranging from 0.25 hour to 30 days, preferably from 0.5 hour to 14 days, particularly from 1 hour to 7 days or from 1.5 hours to 5 days, even more preferred from 2 hours to 1 day. In case of a mixture comprising a pesticide II selected from group L), it is preferred that the pesticide II is applied as last treatment.

According to the invention, the solid material (dry matter) of the biopesticides (with the exception of oils such as Neem oil, Tagetes oil, etc.) are considered as active components (e. g. to be obtained after drying or evaporation of the extraction medium or the suspension medium in case of liquid formulations of the microbial pesticides).

In accordance with the present invention, the weight ratios and percentages used herein for a biological extract such as Quillay extract are based on the total weight of the dry content (solid material) of the respective extract(s).

The total weight ratios of compositions comprising at least one microbial pesticide in the form of viable microbial cells including dormant forms, can be determined using the amount of CFU of the respective microorganism to calclulate the total weight of the respective active component with the following equation that 1 x 10⁹ CFU equals one gram of total weight of the respective active component. Colony forming unit is measure of viable microbial cells, in particular fungal and bacterial cells. In addition, here "CFU" may also be understood as the number of (juvenile) individual nematodes in case of (entomopathogenic) nematode biopesticides, such as Steinernema feltiae.

In the binary mixtures and compositions according to the invention the weight ratio of the component 1) and the component 2) generally depends from the properties of the active components used, usually it is in the range of from 1:100 to 100:1, regularly in the range of from 1:50 to 50:1, preferably in the range of from 1:20 to 20:1, more preferably in the range of from 1:10 to 10:1, even more preferably in the range of from 1:4 to 4:1 and in particular in the range of from 1:2 to 2:1.

According to a further embodiment of the binary mixtures and compositions, the weight ratio of the component 1) and the component 2) usually is in the range of from 1000:1 to 1:1, often in the range of from 100: 1 to 1:1, regularly in the range of from 50:1 to 1:1, preferably in the range of from 20:1 to 1:1, more preferably in the range of from 10:1 to 1:1, even more preferably in the range of from 4:1 to 1:1 and in particular in the range of from 2:1 to 1:1.

According to a further embodiment of the binary mixtures and compositions, the weight ratio of the component 1) and the component 2) usually is in the range of from 1:1 to 1:1000, often in the range of from 1:1 to 1:100, regularly in the range of from 1:1 to 1:50, preferably in the range of from 1:1 to 1:20, more preferably in the range of from 1:1 to 1:10, even more preferably in the range of from 1:1 to 1:4 and in particular in the range of from 1:1 to 1:2.

In the ternary mixtures, i.e. compositions according to the invention comprising the component 1) and component 2) and a compound III (component 3), the weight ratio of component 1) and component 2) depends from the properties of the active substances used, usually it is in the range of from 1:100 to 100:1, regularly in the range of from 1:50 to 50:1, preferably in the range of from 1:20 to 20:1, more preferably in the range of from 1:10 to 10:1 and in particular in the range of from 1:4 to 4:1, and the weight ratio of component 1) and component 3) usually it is in the range of from 1:100 to 100:1, regularly in the range of from 1:50 to 50:1, preferably in the range of from 1:20 to 20:1, more preferably in the range of from 1:10 to 10:1 and in particular in the range of from 1:4 to 4:1.

Any further active components are, if desired, added in a ratio of from 20:1 to 1:20 to the component 1).

These ratios are also suitable for inventive mixtures applied by seed treatment.

When mixtures comprising microbial pesticides are employed in crop protection, the application rates preferably range from about 1 x 10⁶ to 5 x 10¹⁵ (or more) CFU/ha. Preferably, the spore concentration is about 1 x 10⁷ to about 1 x 10¹¹ CFU/ha. In the case of (entomopathogenic) nematodes as microbial pesticides (e. g. Steinernema feltiae), the application rates preferably range inform about 1 x 10⁵ to 1 x 10¹² (or more), more preferably from 1 x 10⁸ to 1 x 10¹¹, even more preferably from 5 x 10⁸ to 1 x 10¹⁰ individuals (e. g. in the form of eggs, juvenile or any other live stages, preferably in an infetive juvenile stage) per ha.

When mixtures comprising microbial pesticides are employed in seed treatment, the application rates with respect to plant propagation material preferably range from about 1 x 10⁶ to 1 x 10¹² (or more) CFU/seed. Preferably, the concentration is about 1 x 10⁶ to about 1 x 10¹¹ CFU/seed. In the case of the microbial pesticides II, the application rates with respect to plant propagation material also preferably range from about 1 x 10⁷ to 1 x 10¹⁴ (or more) CFU per 100 kg of seed, preferably from 1 x 10⁹ to about 1 x 10¹¹ CFU per 100 kg of seed.

Preference is also given to mixtures comprising as component 2) at least one active substance selected from group A), which is particularly selected from (A.1.1), (A.1.4), (A.1.8), (A.1.9), (A.1.12), (A.1.13), (A.1.14), (A.1.17), (A.1.19), (A.1.21), (A.2.1), (A.2.2), (A.3.2), (A.3.3), (A.3.4), (A.3.7), (A.3.8), (A.3.9), (A.3.12), (A.3.14), (A.3.15), (A.3.16), (A.3.19), (A.3.20), (A.3.21), (A.3.22), (A.3.23), (A.3.24), (A.3.25), (A.3.26), (A.3.27); (A.4.5), (A.4.6), (A.4.8), (A.4.9), (A.4.11), (A.1.23), (A.1.24) and (A.1.25).

Preference is given to mixtures as component 2) at least one active substance selected from group B), which is particularly selected from (B.1.4), (B.1.5), diniconazole (B.1.6), (B.1.8), (B.1.10), (B.1.11), (B.1.12), (B.1.17), (B.1.18), (B.1.21), (B.1.22), (B.1.23), (B.1.25), (B.1.26), (B.1.27), (B.1.28), (B.1.29), uni (B.1.31), (B.1.32), (B.1.33), (B.1.34), (B.1.35), (B.1.36), (B.1.37), (B.1.38), (B.1.39), (B.1.40), (B.1.41), (B.1.42), (B.1.44), (B.1.46), (B.1.49) and (B.1.50; (B.2.2), (B.2.4), (B.2.5), (B.2.6), piperalin (B.2.7), (B.2.8); and (B.3.1).

Preference is given to mixtures comprising as component 2) at least one active substance selected from group C), which is particularly selected from (C.1.4), C.1.5), (C.1.6), and (C.2.4).

Preference is given to mixtures comprising as component 2) at least one active substance selected from group D), which is particularly selected from (D1.1), (D1.2), (D1.4), (D1.5); (D2.2), (D2.4), (D2.5), (D2.6) and (D2.7);

Preference is also given to mixtures comprising as component 2) at least one active substance selected from group E), which is particularly selected from (E.1.1), (E.1.2), and (E.1.3);

Preference is also given to mixtures comprising as component 2) at least one active substance selected from group F), which is particularly selected from (F.1.2), (F.1.4), (F.1.5), (F.1.6) and (F.2.1).

Preference is also given to mixtures as component 2) at least one active substance selected from group G), which is particularly selected from (G.3.1), (G.3.2), (G.3.3), (G.3.4), (G.3.5), (G.3.6), (G.4.1) and (G.5.1).

Preference is also given to mixtures comprising as component 2) at least one active substance selected from group H), which is and particularly selected from (H.1.2), (H.1.3), copper oxychloride (H.1.4), (H.1.5), (H.1.6); (H.2.2), (H.2.5), (H.2.7), (H.3.2), (H.3.3), (H.3.4), (H.3.5), (H.3.6), (H.3.12); (H.4.2), (H.4.6), dithianon (H.4.9) and (H.4.10).

Preference is also given to mixtures comprising as component 2) at least one active substance selected from group I), which is particularly selected from (I.2.3) and (I.2.5).

Preference is also given to mixtures comprising as component 2) at least one active substance selected from group J), which is particularly selected from (J.1.1), (J.1.2), (J.1.3), (J.1.4), (J.1.6), (J.1.7), (J.1.8) and (J.1.9).

Preference is also given to mixtures comprising as component 2) at least one active substance selected from group K), which is particularly selected from (K.1.4), (K.1.5), (K.1.8), (K.1.12), (K.1.14), (K.1.15), (K.1.19) and(K.1.22).

The biopesticides from group L1) and/or L2) may also have insecticidal, acaricidal, molluscidal, pheromone, nematicidal, plant stress reducing, plant growth regulator, plant growth promoting and/or yield enhancing activity. The biopesticides from group L3) and/or L4) may also have fungicidal, bactericidal, viricidal, plant defense activator, plant stress reducing, plant growth regulator, plant growth promoting and/or yield enhancing activity. The biopesticides from group L5) and/or L6) may also have fungicidal, bactericidal, viricidal, plant defense activator, insecticidal, acaricidal, molluscidal, pheromone and/or nematicidal activity.

Many of these biopesticides have been deposited under deposition numbers mentioned herein (the prefices refer to the acronym of the respective culture collection), are referred to in literature, registered and/or are commercially available: aluminium silicate (Screen™ Duo from Certis LLC, USA), *Agrobacterium radiobacter* K1026 (e. g. NoGall® from BASF Agricultural Specialties Pty Ltd, Australia), A. *radiobacter* K84 (Nature 280, 697-699, 1979; e. g. GallTroll® from AG Biochem, Inc., C, USA), *Ampelomyces quisqualis* M-10 (e. g. AQ 10® from Intrachem Bio GmbH & Co. KG, Germany), *Ascophyllum nodosum* (Norwegian kelp, Brown kelp) extract or filtrate (e. g. ORKA GOLD from BASF Agricultural Specialities (Pty) Ltd., South Africa; or Goemar® from Laboratoires Goemar, France), *Aspergillus flavus* NRRL 21882 isolated from a peanut in Georgia in 1991 by USDA, National Peanut Research Laboratory (e. g. in AflaGuard® from Syngenta, CH), mixtures of *Aureobasidium pullulans* DSM 14940 and DSM 14941 (e. g. blastospores in Blossom Protect® from bio-ferm GmbH, Germany), *Azospirillum amazonense* SpY2 (DN: BR 11140; Proc. 9th Int. and 1st Latin American PGPR meeting, Quimara, Medellín, Colombia 2012, p. 60, ISBN 978-958-46-0908-3), A. *brasilense* AZ39 (also called Az 39; INTA Az-39; Eur. J. Soil Biol 45(1), 28-35, 2009), A. *brasilense* XOH (e. g. AZOS from Xtreme Gardening, USA or RTI Reforestation Technologies International; USA), A. *brasilense* BR 11002 (Proc. 9th Int. and 1st Latin American PGPR meeting, Quimara, Medellín, Colombia 2012, p. 60, ISBN 978-958-46-0908-3), A. *brasilense* Sp245 (BR 11005; e. g. in GELFIX Gramíneas from BASF Agricultural Specialties Ltd., Brazil), A. *brasilense* strains Ab-V5 and Ab-V6 (e. g. in AzoMax from Novozymes BioAg Produtos papra Agricultura Ltda., Quattro Barras, Brazil or SimbioseMaíz® from Simbiose-Agro, Cruz Alta, RS, Brazil; Plant Soil 331, 413-425, 2010), *A. lipoferum* BR 11646 (Sp31) (Proc. 9th Int. and 1st Latin American PGPR meeting, Quimara, Medellín, Colombia 2012, p. 60), *Bacillus altitudinis* 41 KF2b (DSM 21631; Int. J. Syst. Evol. Microbiol. 56(7), 1465-1473, 2006), *Bacillus amyloliquefaciens* strains AP-136 (NRRL B-50614 and B-50330), AP-188 (NRRL B-50615 and B-50331), AP-218 (NRRL B-50618), AP-219 (NRRL B-50619 and B-50332), and AP-295 (NRRL B-50620 and B-50333) all known from US 8,445,255; *B. amyloliquefaciens* IT-45 (CNCM I-3800) (e. g. Rhizocell C from ITHEC, France), *B. amyloliquefaciens* IN937a (J. Microbiol. Biotechnol. 17(2), 280-286, 2007; e. g. BioYield® from Gustafson LLC, TX, USA), *B. amyloliquefaciens* spp. *plantarum* D747 (US 20130236522 A1; FERM BP-8234; e. g. Double Nickel™ 55 WDG or Double Nickel™ LC from Certis LLC, USA), *B. amyloliquefaciens* spp. *plantarum* FZB24 isolated from plant pathogen-infested soil of a sugar beet field in Brandenburg, Germany (also called SB3615; DSM ID 96-2; J. Plant Dis. Prot. 105, 181-197, 1998; e. g. Taegro® from Novozyme Biologicals, Inc., USA),), *B. amyloliquefaciens* spp. *plantarum* SB3615vPPI being a phage-resistant variant of FZB24 (MRRL B-50349; US 2011/023045 A1; from Novozyme Biologicals, Inc., USA), *B. amyloliquefaciens* ssp. *plantarum* FZB42 isolated from plant pathogen-infested soil of a sugar beet field in Brandenburg, Germany (J. Plant Dis. Prot. 105, 181-197, 1998; DSM 23117; e. g. RhizoVital® 42 from AbiTEP GmbH, Berlin, Germany), *B. amyloliquefaciens* ssp. *plantarum* GB03 (also called GBO3; ATCC SD-1397; Phytopathol. 86(11), S36, 1996; e. g. Kodiak® or BioYield® from Gustafson, Inc., USA; or Companion® from Growth Products, Ltd., White Plains, NY 10603, USA), *B. amyloliquefaciens* ssp. *plantarum* MBI600 also referred to as 1430 (NRRL B-50595; Int. J. Microbiol. Res. 3(2) (2011), 120-130; US 2012/0149571 A1; e. g. Integral®, Subtilex® NG from BASF Corp., USA), *B. amyloliquefaciens* spp. *plantarum* TJ1000 (also called 1 BE; CA 2471555 A1; ATCC BAA-390; e. g. QuickRoots™ from TJ Technologies, Watertown, SD, USA), *B. cereus* CNCM I-1562 (US 6,406,690), *B. chitinosporus* AQ746 isolated from roots in Saskatchewan, Canada (NRRL B-21618; US 5,733,544; AgraQuest now Bayer CropScience LP, USA), *B. firmus* CNCM I-1582 (WO 2009/126473, WO 2009/124707, US 6,406,690; e. g. Votivo® from Bayer CropScience LP, USA), *B. megaterium* strains H491 (NRRL B-50769), M018 (NRRL B-50770) and J142 (NRRL B-50771) all known from US 2014/0051571 A1 from Marrone Biolnnovations, Inc., USA; *B. mojavensis* AP-209 (NRRL B-50616; US 8,445,255), *B. mycoides* AQ726 (NRRL B-21664; US 5,906,818; from Bayer Crop Science, Germany), *B. mycoides* strain J (e.g. BmJ WG from Certis, USA against potato virus Y), *B. pumilus* GB34 (ATCC 700814; e. g. YieldShield® from Gustafson LLC, TX, USA), *B. pumilus* GHA 180 isolated from apple tree rhizosphere in Mexico (IDAC 260707-01; e. g. in PRO-MIX® BX from Premier Horticulture, 1, avenue Premier, Rivie're-du-Loup, Quebec, Canada G5R6C1), *B. pumilus* KFP9F (NRRL B-50754; WO 2014/029697; e. g. BAC-UP or FUSION-P from BASF Agricultural Specialities (Pty) Ltd., South Africa), *B. pumilus* INR-7 otherwise referred to as BU-F22 and BU-F33 (NRRL B-50185, NRRL B-50153; US 8,445,255), *B. pumilus* QST 2808 (NRRL B-30087; e. g. Sonata® or Ballad® Plus from AgraQuest Inc., USA), *B. solisalsiAP-217* (NRRL B-50617; US 8,445,255), *B. subtilis* CX-9060 (Federal Register 77(7), 1633-1637; by Certis U.S.A., L.L.C.), *B. subtilis* FB17 also called UD 1022 or UD10-22 isolated from red beet roots in North America (ATCC PTA-11857; System. Appl. Microbiol. 27, 372-379, 2004; US 2010/0260735; WO 2011/109395); *B. subtilis* GB07 (Phytopathol. 86(11), S36, 1996; Epic® from Gustafson, Inc., USA), *B. subtilis* QST-713 isolated from a California peach orchard in 1995 (NRRL B-21661; e. g. Rhapsody®, Serenade® MAX or Serenade® ASO from AgraQuest Inc., USA), *B. thuringiensis* ssp. *aizawai* ABTS-1857 (also called ABG-6346; ATCC SD-1372; e. g. XenTari® from BioFa AG, Münsingen, Germany), *B. t.* ssp. *aizawai* SAN 401 I, ABG-6305 (WO 2013/087709); *Bacillus t.* ssp. *israelensis* AM65-52 of Serotype H-14 (ATCC SD-1276; e. g. VectoBac® from Valent BioSciences, IL, USA), *Bacillus thuringiensis* ssp. *kurstaki* SB4 (NRRL B-50753; e. g. Beta Pro® from BASF Agricultural Specialities (Pty) Ltd., South Africa), *B. t.* ssp. *kurstakiABTS-351* identical to HD-1 (ATCC SD-1275; e. g. Dipel® DF from Valent BioSciences, IL, USA), *B. t.* ssp. *kurstaki* EG 2348 (NRRL B-18208; e. g. Lepinox® or Rapax® from CBC (Europe) S.r.l., Italy), *B. t.* ssp. *tenebrionis* DSM 2803 of Serotype H 8a, 8b (identical to NRRL B-15939; EP 0 585 215 B1; Mycogen Corp.), *B. t.* ssp. *tenebrionis* NB-125 (also referred to as SAN 418 I or ABG-6479; EP 0 585 215 B1; DSM 5526; former production strain of Novo-Nordisk), *B. t.* ssp. *tenebrionis* NB-176 (or NB-176-1; a gamma-irridated, induced high-yielding mutant of strain NB-125; EP 585 215 B1; DSM 5480; e. g. Novodor® from Valent BioSciences, Switzerland), *Beauveria bassiana* JW-1 (ATCC 74040; e. g. Naturalis® from CBC (Europe) S.r.l., Italy), *B. bassiana* DSM 12256 (US 200020031495; e. g. BioExpert® SC from Live Sytems Technology S.A., Colombia), *B. bassiana* GHA (ATCC 74250; e. g. BotaniGard® 22WGP from Laverlam Int. Corp., USA), *B. bassiana* PPRI 5339 (ARSEF 5339; NRRL 50757; e. g. BroadBand® from BASF Agricultural Specialities (Pty) Ltd., South Africa), *B. brongniartii* for control of cockchafer (J. Appl. Microbiol. 100(5),1063-72, 2006; e. g. Melocont® from Agrifutur, Agrianello, Italy), *Bradyrhizobium* sp. (e. g. Vault® from BASF Corp., USA), *B.* sp. (Arachis) CB1015 presumably originally collected in India (IITA 1006, USDA 3446; from Australian Inoculants Research Group; http://www.qaseeds.com.au/inoculant_applic.php). *B.* sp. (Arachis) strains deposited at SEMIA and known from FEMS Microbiol. Letters 303(2), 123-131, 2010; Revista Brasileira de Ciencia do Solo 35(3), 739-742, 2011, ISSN 0100-0683: SEMIA 6144, SEMIA 6462 (BR 3267) and SEMIA 6464 (BR 3262); *B.* sp. (Vigna) PNL01 (Bisson and Mason, April 29, 2010, Project report, Worcester Polytechnic Institute, Worcester, MA, USA: http://www.wpi.edu/Pubs/E-project/Available/E-project-042810-163614/; e. g. Vault® Peanut Liquid from BASF Corp., USA), *B. elkanii SEMIA* 587 (Appl. Environ. Microbiol. 73(8), 2635, 2007; e. g. GELFIX 5 from BASF Agricultural Specialties Ltd., Brazil), *B. elkanii* SEMIA 5019 (=29W; Appl. Environ. Microbiol. 73(8), 2635, 2007; e. g. GELFIX 5 from BASF Agricultural Specialties Ltd., Brazil), *B. elkanii* USDA 76, *B. elkanii* USDA 94*B*. *elkanii* USDA 3254, *B. elkanii* U-1301 and U-1302 (e. g. Nitragin® Optimize from Novozymes Bio As S.A., Brazil, or Nlitrasec for soybean from LAGE y Cia, Brazil), *B. japonicum* (e. g. VAULT® from BASF Corp., USA), *B. japonicum* 532c isolated from Wisconsin field (Nitragin 61A152; Can. J. Plant. Sci. 70, 661-666, 1990; e. g. in Rhizoflo®, Histick®, Hicoat® Super from BASF Agricultural Specialties Ltd., Canada), *B. japonicum* E-109 variant of strain USDA 138 (INTA E109, SEMIA 5085; Eur. J. Soil Biol. 45, 28-35, 2009; Biol. Fertil. Soils 47, 81-89, 2011), *B. japonicum* G49 (MSDJ G49; C. R. Acad. Agric. Fr. 73, 163-171, 1987); *B. japonicum* strains deposited at SEMIA known from Appl. Environ. Microbiol. 73(8), 2635, 2007: SEMIA 566 isolated from North American inoculant in 1966 and used in Brazilian commercial inoculants from 1966 to 1978, SEMIA 586 originally isolated in Maryland, USA, in 1961 but received from Australia in 1966 and used in Brazilian inoculants in 1977 (CB 1809, USDA 136, Nitragin 61A136, RCR 3407), SEMIA 5079 a natural variant of SEMIA 566 used in commercial inoculants since 1992 (CPAC 15; e. g. GELFIX 5 or ADHERE 60 from BASF Agricultural Specialties Ltd., Brazil), *B*. *japonicum* SEMIA 5080 a natural variant of SEMIA 586 used in commercial inoculants since 1992 (CPAC 7; e. g. GELFIX 5 or ADHERE 60 from BASF Agricultural Specialties Ltd., Brazil); *B. japonicum* TA-11 (TA11 NOD+) (NRRL B-18466; US 5,021,076; Appl. Environ. Microbiol. 56, 2399-2403, 1990; e. g. VAULT® NP, from BASF Corp., USA), *B. japonicum* strains deposited at USDA known from US 7,262,151 and Appl. Environ. Microbiol. 60, 940-94, 1994: USDA 3 isolated from *Glycine max* in Virginia (USA) in 1914, USDA 31 (= Nitragin 61A164) od Serogroup 31 isolated from *Glycine max* in Wisconsin (USA) in 1941, USDA 76 isolated from plant passage of strain USDA 74 (Serogroup 76) which has been isolated from *G. max* in California (USA) in 1956, USDA 110 (= IITA 2121, SEMIA 5032, RCR 3427, ARS I-110 and Nitragin 61A89; Serogroup 110) isolated from *G. max* in Florida in 1959, USDA 121 isolated from G. *max* in Ohio (USA) in 1965 (Crop Science 26(5), 911-916, 1986); *B. japonicum* WB74 (e. g. Eco-Rhiz Soya from Plant Health Products (Pty) Ltd, South Africa; or Soybean inoculant from Stimuplant CC, South Africa), *B. lupini* LL13 isolated from *Lupinus iuteus* nodules from French soils (deposited at INRA, France; http://agriculture.gouv.fr/ IMG/pdf/ch20060216.pdf), *B. lupini* strains from Australia and known from Palta J.A., Berger J.B. (eds), Proceed. 12th International Lupin Conference, 14-18 Sept. 2008, Fremantle, Western Australia, International Lupin Association, Canterbury, New Zealand, 47-50, http://www.lupins. org/pdf/conference/2008/Agronomy%20and%20Production/John%20Howieson%20and%20G% 20OHara.pdf; Appl. Environ. Microbiol. 71, 7041-7052, 2005; Australian J. Exp. Agricult. 36(1), 63-70, 1996: strains WU425 isolated in Esperance, Western Australia from a non-Australian legume *Ornithopus compressus,* WSM471 isolated *from Ornithopus pinnatus* in Oyster Harbour, Western Australia, and WSM4024 isolated from lupins in Australia by CRS during a 2005 survey; *Burkholderia sp.* A396 (NRRL B-50319; WO 2013/032693; Marrone Bio Innovations, Inc., USA), *Candida oleophila* I-182 (NRRL Y-18846; Phytoparasitica 23(3), 231-234, 1995; e. g. Aspire® from Ecogen Inc., USA;), *C. oleophila* strain O (NRRL Y-2317; Biological Control 51, 403-408, 2009), *Candida saitoana* (e. g. Biocure® [in mixture with lysozyme] and BioCoat® from Micro Flo Company, USA (BASF SE) and Arysta), chitosan (e. g. Armour-Zen® from BotriZen Ltd., NZ), *Clonostachys rosea* f. *catenulate* (also named *Gliocladium catenulatum*) J1446 isolated from Finnish field soil (NJF seminar No 389: Pest, disease and weed management in strawberry; Finland 8-9. Nov. 2006 in NJF Report 2(10), 15-15, 2006; DSM 9212; e. g. Primastop® or Prestop® from Verdera Oy, Finland), *Chromobacterium subtsugae* PRAA4-1 isolated from soil under an eastern hemlock (*Tsuga canadensis*) in the Catoctin Mountain region of central Maryland (NRRL B-30655; e. g. Grandevo® from Marrone Bio Innovations, USA), *Coniothyrium minitans* CON/M/91-08 (WO 1996/021358; DSM 9660; e. g. Contans® WG, Intercept® WG from Prophyta Biologischer Pflanzenschutz GmbH, Germany), *Cryphonectria parasitica* (hypovirulent strains; Microbiol. Reviews 56(4), 561-576, 1992; e. g. product Endothia parasitica from CNICM, France), *Cryptococcus albidus* (e. g. YIELD PLUS® from Anchor Bio-Technologies, South Africa), *Cryptophlebia leucotreta* granulovirus (CrIeGV) (e. g. CRYPTEX from Adermatt Biocontrol, Switzerland), *Cydia pomonella* granulovirus (CpGV) V03 (DSM GV-0006; e. g. Madex® Max from Andermatt Biocontrol, Switzerland), CpGV V22 (DSM GV-0014; e. g. Madex® Twin from Adermatt Biocontrol, Switzerland), *Delftia acidovorans* RAY209 (ATCC PTA-4249; WO 2003/57861; e. g. BioBoost® from Brett Young, Winnipeg, Canada), *Dilophosphora alopecuri* (FarmNote 396, Feb. 2010, Department of Agriculture and Food, Government of Western Australia; e.g. Twist Fungus from BASF Agricultural Specialties Pty Ltd, Australia), *Ecklonia maxima* (kelp) extract (J. Ecological Engineering 14(1), 48-52, 2013; e. g. KELPAK SL from Kelp Products Ltd, South Africa), *Flavobacterium sp.* H492 (ATCC B-505584; WO 2013/138398; e. g. MBI-302 from Marrone Bio Innovations, USA for soyean cyst nematode control), formononetin (US 5,002,603; e. g. Myconate® from Plant Health Care plc, U.K.), *Fusarium oxysporum* Fo47 (non-pathogenic strain isolated from a suppressive soil located at Châteaurenard, France; Appl. Environ. Microbiol 68(8), 4044-4060, 2002; Fusaclean® from Natural Plant Protection, N.P.P. (société anonyme) Route d'Artix F-64150 Nogueres, France), *F. oxysporum* 251/2RB (Prevention Today Vol. 2, n. 1-2, 47-62, 2006; e. g. Biofox® C from S.I.A.P.A., Italy); *Glomus intraradices* (e. g. Myc® 4000 from ITHEC, France), *Glomus intraradices* RTI-801 (e. g. MYKOS from Xtreme Gardening, USA or RTI Reforestation Technologies International; USA), grapefruit seeds and pulp extract (e. g. BC-1000 from Chemie S.A., Chile), harpin (alpha-beta) protein (Science 257, 85-88, 1992; e. g. Messenger™ or HARP-N-Tek from Plant Health Care plc, U.K.), *Helicoverpa armigera* nucleopolyhedrovirus (HearNPV) (J. Invertebrate Pathol. 107, 112-126, 2011; e. g. Helicovex® from Adermatt Biocontrol, Switzerland), *Heterorhabditis bacteriophora* (e. g. Nemasys® G from BASF Agricultural Specialities Limited, UK), *Isaria fumosorosea* Apopka-97 (ATCC 20874; Biocontrol Science Technol. 22(7), 747-761, 2012; e. g. PFR-97™ or PreFeRal® from Certis LLC, USA), *I. fumosorosea* FE 9901 (ARSEF 4490; Biocontrol Science Technol. 22(7), 747-761, 2012; e. g. blastospores in NoFly™ WP from Natural Industries, Inc., Houston, TX, USA or from Novozymes, U.S.A.), cis-jasmone (US 6,890,525; US 8,221,736; Plant Bioscience Limited, Norwich, U.K.), laminarin (e. g. in Vacciplant® from Laboratoires Goemar, St. Malo, France or Stähler SA, Switzerland), *Lecanicillium longisporum* KV42 and KV71 (e. g. Vertalec® from Koppert BV, Netherlands), *L. muscarium* Ve6 (also called KV01; IMI 19-79, CABI 268317, CBS 102071, ARSEF 5128; e. g. Mycotal® from Koppert BV, Netherlands), *Lysobacter antibioticus* 13-1 (Biological Control 45, 288-296, 2008), *L. antibioticus* HS124 (Curr. Microbiol. 59(6), 608-615, 2009), *L. enzymogenes* 3.1T8 (Microbiol. Res. 158, 107-115, 2003; Biological Control 31(2), 145-154, 2004); *Mesorhizobium* spp. strains known from Soil Biol. Biochem. 36(8), 1309-1317, 2004; Plant and Soil 348(1-2), 231-243, 2011: M. sp. WSM1271 collected in Sardinia, Italy, from plant host *Biserrula pelecinus,* M. sp. WSM 1497 collected in Mykonos, Greece, from *Biserrula pelecinus, Mesorhizobium ciceri CC1192* collected in Israel from Cicer arietinum nodules (UPM 848, CECT 5549; Can. J. Microbiol. 48, 279-284, 2002; from Horticultural Research Station, Gosford, Australia), *M. huakuii* HN3015 isolated from *Astralagus sinicus* in a rice-growing field of Southern China (World J. Microbiol. Biotechn. 23(6), 845-851, 2007, ISSN 0959-3993), *M. loti* CC829 isolated from *L. ulginosus* nodules in USA (NZP 2012; commerical inoculant for *Lotus pedunculatus* and *L. ulginosus* in Australia), and *M. loti* SU343 isolated from host nodules in USA (commercial inoculant for *Lotus corniculatus* in Australia); *Metarhizium anisopliae* FI-1045 (AGAL V10/0104285; WO 2012/018266; e. g. Biocane® from BASF Agricultural Specialties Pty Ltd, Australia), *M. anisopliae* var. *anisopliae* F52 also called 275 or V275 (DSM 3884, ATCC 90448; e. g. Met52® Novozymes Biologicals BioAg Group, Canada), *M. anisopliae* ICIPE 69 isolated from a soil sample obtained from the Democratic Republic of Congo (DRC) and using the Galleria bait method in 1990 (e. g. Metathripol from ICIPE, Nairobe, Kenya), *M. anisopliae* var. *acridum* IMI 330189 isolated from *Ornithacris cavroisi* in Niger (NRRL 50758; e. g. Green Muscle® from BASF Agricultural Specialities (Pty) Ltd., South Africa), *M. a.* var. *acridum* FI-985 isolated from a spur-throated locust, Austracris guttulosa (Walker), near Rockhampton, Queensland, Australia, in 1979 (ARSEF 324; Memoirs of the Entomological Society of Canada 171, 287-300, 1997; e. g. Green Guard® SC from BASF Agricultural Specialties Pty Ltd, Australia), *Metschnikowia fructicola* 277 isolated from the surface of grape berries (cv. Superior) grown in the central part of Israel (US 6,994,849; NRRL Y-30752; e. g. Shemer® from Agrogreen, Israel, now distributed by Bayer CropSciences, Germany), *Microdochium dimerum* L13 (CNCM I-3141; e. g. Antibot® from Agrauxine, France), *Microsphaeropsis ochracea* P130A isolated from apple leaves from an abandoned orchard, St-Joseph-du-Lac, Quebec, Canada in 1993 (ATCC 74412; Mycologia 94(2), 297-301, 2002), *Muscodor albus* QST 20799 also called 620 originally isolated from the bark of a cinnamon tree in Honduras (NRRL 30547; e. g. Muscudor™ or QRD300 from AgraQuest, USA), *Muscodor albus* SA-13 (NRRL B-50774; US 2014/0086879 A1; e. g. MBI-601-EP from Marrone Biolnnovations, Inc., USA), Neem oil (e. g. Trilogy®, Triact® 70 EC from Certis LLC, USA), *Nomuraea rileyi* strains SA86101, GU87401, SR86151, CG128 and VA9101 (Braz. Arch. Biol. Technol. 46(1), 13-19, 2003; WO 2013/110594), *Paecilomyces lilacinus* 251 isolated from infected nematode eggs in the Philippines (AGAL 89/030550; WO1991/02051; Crop Protection 27, 352-361, 2008; e. g. BioAct®/MeloCon® from Prophyta, Germany), *P. lilacinus* DSM 15169 (e. g. Nemata® SC from Live Systems Technology S.A., Colombia), *P. lilacinus* BCP2 (NRRL 50756; Acta agriculturae Slovenica, 101 - 2, 263-275, 2013; e. g. PL Gold from BASF Agricultural Specialities (Pty) Ltd., South Africa), *Paenibacillus alvei* NAS6G6 (WO 2014/029697; NRRL B-50755; e.g. BAC-UP from BASF Agricultural Specialities (Pty) Ltd., South Africa in mixture with *Bacillus pumilus* KFP9F), *P. polymyxa* PKB1 (ATCC 202127; Can. J. Microbiol. 48(2), 159-169, 2002), *Pantoea agglomerans* E325 (NRRL B-21856; Phytopathol. 101 (10), 1234-41, 2011; Trees 26, 227-238, 2012; Bloomtime Biological™ from Northwest Agricultural Products, Inc., USA), *Pantoea vagans* (formerly *agglomerans)* C9-1 originally isolated in 1994 from apple stem tissue for control of fire blight in apple (J. Bacteriol. 192(24), 6486-6487, 2010; e. g. BlightBan C9-1® from NuFrams America Inc., USA), *Pasteuria* sp. ATCC PTA-9643 (WO 2010/085795), *Pasteuria* sp. Ph3 isolated from turfgrass soil samples collected at the DeBary Golf Course in central Florida (ATCC SD-5832; WO 2012/064527; for control of *Hoplolaimus galeatus* nematode from Pasteuria Bioscience, Inc. now Syngenta Crop Protection, LLC, USA), *Pasteuria* sp. Pr3 isolated from soil samples collected in the southeastern United States (ATCC SD-5834; for control of *Rotylenchulus reniformis* nematode potentially of species *P. ramosa;* Naviva® ST from Syngenta Crop Protection, LLC, USA), *P. nishizawae* (WO 2010/80619), *P. nishizawae* Pn1 (Federal Register 76(22), 5808, February 2, 2011; ATCC SD-5833; e.g. Clariva™ PN from Syngenta Crop Protection, LLC, USA), *P. penetrans* (US 5,248,500; Del Monte Corp.), *P. ramosa* (WO 2010/080619), *P. thornea* (WO 2010/080619), *P. usgae* BL1 (ATCC SD-5835; J. Nematol. 42(2): 87-90, 2010; *ibid.* 43(2), 101-109, 2011; e. g. Econem™ for control of *Belonolaimus longicaudatus* from Pasteuria BioScience now Syngenta sold by Harell's LLC, Florida, USA for use on turf for management of *Belonolaimus longicaudatus), Penicillium bilaiae* (also called *P. bilaii*) strains ATCC 18309 (= ATCC 74319), ATCC 20851 and/or ATCC 22348 (= ATCC 74318) originally isolated from soil in southern Alberta (Fertilizer Res. 39, 97-103, 1994; Can. J. Plant Sci. 78(1), 91-102, 1998; US 5,026,417, WO 1995/017806; e. g. Jump Start®, Provide® from Novozymes Biologicals BioAg Group, Canada), *P. bilaiae* NRRL 50162 and NRRL 50169 (WO 2010/037228), *Phlebiopsis gigantea* (e. g. RotStop® from Verdera Oy, Finland), *Pichia anomala* WRL-076 (NRRL Y-30842; US 8,206,972), potassium bicarbonate (e. g. Amicarb® from Stähler SA, Switzerland), potassium silicate (e. g. Sil-MATRIX™ from Certis LLC, USA), *Pseudozyma flocculosa* PF-A22 UL (e. g. Sporodex® L from Plant Products Co. Ltd., Canada), *Pseudomonas* sp. Proradix (DSM 13134; WO 2001/40441, e. g. PRORADIX from Sourcon Padena GmbH & Co. KG, Hechinger Str. 262, 72072 Tübingen, Germany), *P. chloraphis* MA 342 (Microbiology Monographs 18, 21-43, 2011; e. g. Cerall® or Cedemon® from BioAgri AB, Uppsala, Sweden or Intrachem Bio Deutschland GmbH & Co. KG, Bad Camberg, Germany), *P. fluorescens* (e.g. in Bio Cure-B from T. Stanes & Company Limited, India; or in Blight-End from Agri Naturals, Mumbai, India), *P. fluorescens* A506 (Phytopathol 97(2), 244-249, 2007; ATCC 31948; e. g. BlightBan® from NuFarm Americas, Inc., Morrisville, NC, USA), *P. fluorescens* ATCC 13525 of biovar I = biotype A; originally isolated from pre-filter tanks in England (DSM 50090; registered for use in Canada), P. *fluorescens* CHA0 (Mol. Plant Microbe Interact. 5(1), 4-13, 1992), *P. fluorescens* CL 145A (J. Invertebr. Pathol. 113(1), 104-14, 2013; e. g. Zequanox® from Marrone Biolnnovations, Davis, CA, USA), *P. fluorescens* NCIB 12089 (EP 0210734 A!; Victus® from Mauri Laboratories, 9 Moorebank Ave., Moorebank, NSW 2170, Australia), *P. fluorescens* Pf-5 isolated from root surface of cotton (ATCC BAA-477), *P. putida* ATCC 202153 (EMBRAPA 63/88 4 B; WO 2004/0245865), *Pythium oligandrum* DV 74 (US 2013/0035230; ATCC 38472; e. g. Poyversum® from Remeslo SSRO, Biopreparaty, Czech Rep. and from Gowan, USA), *Reynoutria sachalinensis* extract (EP 0307510 B1; e. g. Regalia® SC from Marrone Biolnnovations, Davis, CA, USA or Milsana® from BioFa AG, Germany), *Rhizobium leguminosarum* bv. *phaseoli* (e. g. RHIZO-STICK from BASF Corp., USA), *R. leguminosarum bv. phaseoli* RG-B10 (USDA 9041; from Int. J. Syst. Bacteriol. 46(1), 240-244, 1996; Int. J. Syst. Evol. Microbiol. 50, 159-170, 2000; e. g. Nodulator® Dry Bean in Africa, HiStick NT Dry bean in US, and Nodulator® Dry Bean in Canada from BASF Corp., USA, or BASF Agricultural Specialties Ltd., Canada), R. *I.* bv. *trifolii* CB782 (Nodulaid® peat for Kenya white clover from BASF Agricultural Specialties Pty Ltd, Australia), R. *I.* bv. *trifolii* CC275e (Nodulaid® peat for NZ white clover from BASF Agricultural Specialties Pty Ltd, Australia), R. *I.* bv. *trifolii* CC283b (ICMP 4073b; Proc. New Zealand Grassland Assoc. 56, 101-105, 1994; Microbiol. 153, 3184-3195, 2007; Nodulaid® peat for Caucasian clover from BASF Agricultural Specialties Pty Ltd, Australia), R. *I.* bv. *trifolii* CC1099 (Inoculating Legumes: A Practical Guide, ed. Grain Research and Development Corporation, 2012, ISBN 978-1-921779-45-9; e. g. Nodulaid® peat for sainfoin from BASF Agricultural Specialties Pty Ltd, Australia), *R. I.* bv. *trifolii* RP113-7 (Appl. Environ. Microbiol. 44(5), 1096-1101, 1982; e. g. Dormal® from BASF Corp., USA), *R. I*. bv. *trifolii* TA1 (Appl. Environ. Microbiol. 49(1), 127-131, 1985; e. g. Nodulaid® peat for white clover from BASF Agricultural Specialties Pty Ltd, Australia), *R. I.* bv. *trifolii* strain WSM1325 isolated in 1993 from the Greek Island of Serifos (Stand. Genomic Sci. 2(3), 347-356, 2010; Inoculating Legumes: A Practical Guide, ed. Grain Research and Development Corporation, 2012, ISBN 978-1-921779-45-9; Nodulaid® peat for sub clover and Nodulator® granules for sub clover both from BASF Agricultural Specialties Pty Ltd, Australia, for a broad range of annual clovers of Mediterranean origin), *R. I.* bv. *trifolii* strain WSM2304 isolated from *Trifolium polymorphum* in Uruguay in 1998 (Stand. Genomic Sci. 2(1), 66-76, 2010), R. *I.* bv. *viciae* P1 NP3Cst being a Streptomycin-resistant mutant of P1 NP3C isolated from pea root nodules in Bretenière, France (also referred to as 1435; New Phytol. 176, 680-690, 2007; *ibid.* 179(1), 224-235, 2008; e. g. Nodulator® PL Peat Granule from BASF Corp., USA; or Nodulator® XL PL from BASF Agricultural Specialties Ltd., Canada), *R. I.* bv. *viciae* RG-P2 also called P2 isolated from pea root nodules in Sakatchewan, Canada (e. g RhizUP peat for peas and lentils in Canada from BASF Agricultural Specialties Ltd., Canada), *R. I.* bv. *viciae* SU303 (e. g. Nodulaid® Group E from BASF Agricultural Specialties Pty Ltd, Australia), *R. I.* bv. *viciae* WSM1455 (e. g. Nodulaid® Group F from BASF Agricultural Specialties Pty Ltd, Australia), *R. tropici* CC511 (Agronomy, N.Z. 36, 4-35, 2006; e. g. Nodulaid® peat for common bean from BASF Agricultural Specialties Pty Ltd, Australia)*R*. *tropici* CIAT 899 isolated in Colombia (SEMIA 4077; Rev. Ciênc. Agron. 44(4) Fortaleza Oct./Dec. 2013; e. g. Nitrafix® FEIJÃO peat for beans from BASF Agricultural Specialties Ltd., Brazil in mixture with strain SEMIA 4080), *R. tropici* H12 isolated in Planaltina, DF, Cerrados, Brazil (SEMIA 4088; Appl. Microbiol. Biotechnol. 93(5), 2035-49, 2012; e. g. Nitrafix® FEIJÃO from BASF Agricultural Specialties Ltd., Brazil), *R. tropici* PRF 81 isolated in Paraná, Brazil (SEMIA 4080; Soil Biology & Biochemistry 39, 867-876, 2007; BMC Microbiol. 12, 84, 2012; Nitrafix® FEIJÃO peat for beans from BASF Agricultural Specialties Ltd., Brazil in mixture with strain SEMIA 4077), *Sinorhizobium meliloti* RCR2011 also called 2011 or SU47 (MSDJ0848; Mol. Gen. Genomics 272, 1-17, 2004; e. g. Dormal® Alfalfa & Luzerne from BASF Corp., USA; Nitragin® Gold from Novozymes Biologicals BioAg Group, Canada), *Sphaerodes mycoparasitica* SMCD2220 also called SMCD2220-01 (IDAC 301008-01; WO 2011/022809), *Spodoptera littoralis* nucleopolyhedrovirus (SpliNPV) (e.g. in LITTOVIR from Adermatt Biocontrol, Switzerland), *Steinernema carpocapsae* (e. g. Millenium® from BASF Agricultural Specialities Limited, UK), S. *feltiae* (Nemashield® from BioWorks, Inc., USA; Nemasys® from BASF Agricultural Specialities Limited, UK), S. *kraussei* L137 (Nemasys® L from BASF Agricultural Specialities Limited, UK), *Streptomyces galbus* AQ6047 (NRRL 30232; WO 2012/135763; AgraQuest now Bayer CropScience LP, USA); S. *galbus* M1064 (NRRL 50334; WO 2012/135763; AgraQuest now Bayer CropScience LP, USA); S. *griseoviridis* K61 (Crop Protection 25, 468-475, 2006; e. g. Mycostop® from Verdera Oy, Espoo, Finland), S. *lydicus* WYEC 108 (US 5,403,584; e. g. Actinovate® from Natural Industries, Inc., USA), S. *vioiaceusniger* YCED-9 (US 5,968,503; e. g. DT-9® from Natural Industries, Inc., USA), *Talaromyces flavus* V117b isolated from soil (e. g. Protus® WG from Prophyta, Germany), *Trichoderma asperellum* SKT-1 isolated from the rhizosphere of Japanese lawngrass (FERM P-16510; J. Gen. Plant Pathol. 71(5), 351-356, 2005; e. g. Eco-Hope® from Kumiai Chemical Industry Co., Ltd., Japan), *T. asperellum* ICC 012 isolated from a soil in central Italy that was found to suppress plant disease (IMI 392716; e. g. Tenet WP, Remdier WP or Bioten WP from Isagro NC, USA, Bio-Tam™ from AgraQuest, USA), *T. asperellum* TV1 formerly *T. viride* (MUCL 43093; e. g. T. viride TV1 from Agribiotec srl, Italy or Xedavir from Xeda Italia, Italy), *T. atroviride* LC52 (e. g. Sentinel® from Agrimm Technologies Ltd, NZ), *T. atroviride* CNCM I-1237 (e. g. Esquive® WG from Agrauxine S.A., France, e. g. against pruning wound diseases on vine and plant root pathogens), *T. fertile* JM41 R (NRRL 50759; e. g. Trichoplus™ from BASF Agricultural Specialities (Pty) Ltd., South Africa), T. *gamsii* ICC 080 (IMI 392151; e. g. Tenet WP, Remdier WP, Bioten WP from Isagro NC, USA, Bio-Tam™ from AgraQuest, USA), *T. harzianum* T-22 also called KRL-AG2 (ATCC 20847; BioControl 57, 687-696, 2012; e. g. Plantshield® from BioWorks Inc., USA or SabrEx™ from Advanced Biological Marketing Inc., Van Wert, OH, USA), *T. harzianum* T-35 and T-315 (ATCC 20691; EP 0133878 B1; e. g. Root Pro® from Mycontrol Ltd., Israel), *T. harzianum* T-39 (CNCM I-952; EP 0466133 B2; e. g. Trichodex® or Trichoderma 2000® from Mycontrol Ltd., Israel and Makhteshim Ltd., Israel), mixture of *T. harzianum and T. viride* (e. g. Trichopel® from Agrimm Technologies Ltd, NZ), mixture of *T. harzianum* ICC012 and *T. viride* ICC080 (e. g. Remdier® WP from Isagro Ricerca, Italy), *T. polysporum* IMI 206039 (ATCC 20476; e. g. Binab® from BINAB Bio-Innovation AB, Sweden in mixture with *T. atroviride* IMI 206040), *T. stromaticum* (e. g. Tricovab® from C.E.P.L.A.C., Brazil), *T. virens* GI-3 also called G1-3 or GL-3 (CA 2471555 A1; ATCC 58678; e.g. QuickRoots™ from TJ Technologies, Watertown, SD, USA in mixture with *B. amyloliquefaciens* TJ1000), *T. virens* GL-21 also called G1-21 isolated from a sclerotium of *Sclerotinia minor* (US 7,429,477; e. g. Soilguard® 12G from Certis LLC, USA; EPA Registration Number: 70051-3 and EPA Establishment Number: 067250-IL-001), *T. virens* G-41 also called 041, #41 X or ABM 127 isolated from soil samples taken from *Aphanomyces-sup*pressive bean fields in Livingston County, New York (ATCC 20906; US 4,996,157; e. g. Root-shield® PLUS from BioWorks, Inc., USA), *T. viride* (J. Biological Control 23(1), 31-36, 2009; e. g. Trieco® from Ecosense Labs. (India) Pvt. Ltd., India; or Bio-Cure® F from T. Stanes & Co. Ltd., India), and *Ulocladium oudemansii* HRU3 (Agronomy 3, 632-647, 2013; e. g. Botry-Zen® from Botry-Zen Ltd, NZ).

Strains can be obtained from culture collections and deposition centers (listed by their acronym = strain prefix here: http://www.wfcc.info/ccinfo/collection/by_acronym/) such as strains with prefices AGAL or NMI from: National Measurement Institute, 1/153 Bertie Street, Port Melbourne, Victoria, Australia 3207; ATCC: American Type Culture Collection, 10801 University Blvd., Manassas, VA 20110-2209, USA; BR: Embrapa Agrobiology Diazothrophic Microbial Culture Collection, P.O.Box 74.505, Seropedica, Rio de Janeiro, 23.851-970, Brazil; CABI or IMI: CABI Europe - International Mycological Institute, Bakeham Lane, Egham, Surrey, TW20 9TYNRRL, UK; CB: The CB Rhizobium Collection, School of Environment and Agriculture, University of Western Sydney, Hawkesbury, Locked Bag 1797, South Penrith Distribution Centre, NSW 1797, Australia; CBS: Centraalbureau voor Schimmelcultures, Fungal Biodiversity Centre, Uppsalaan 8, PO Box 85167, 3508 AD Utrecht, Netherlands; CC: Division of Plant Industry, CSIRO, Canberra, Australia; CNCM: Collection Nationale de Cultures de Microorganismes, Institute Pasteur, 25 rue du Docteur Roux, F-75724 PARIS Cedex 15; CPAC: Embrapa-Cerrados, CX.Postal 08223,Planaltina,DF,73301-970, Brazil; DSM: Leibniz-Institut DSMZ-Deutsche Sammlung von Mikroorganismen und Zellkulturen GmbH, Inhoffenstraße 7 B, 38124 Braunschweig, Germany; IDAC: International Depositary Authority of Canada Collection, Canada; ICMP: Interntional Collection of Micro-organisms from Plants, Landcare Research, Private Bag 92170, Auckland Mail Centre, Auckland 1142, New Zealand; IITA: IITA, PMB 5320, Ibadan, Nigeria; INTA: Agriculture Collection Laboratory of the Instituto de Microbiologia y Zoologia Agncola (IMYZA), Instituto Nacional de Tecnologna Agropecuaria (INTA), Castelar, Argentina; MSDJ: Laboratoire de Microbiologie des Sols, INRA, Dijon, France; MUCL: Mycothèque de l'Université catholique de Louvain, Croix du Sud 2, box L7.05.06, 1348 Louvain-la-Neuve, Belgium; NCIMB or NICB: The National Collections of Industrial and Marine Bacteria Ltd., Torry Research Station, P.O. Box 31, 135 Abbey Road, Aberdeen, AB9 8DG, Scotland; Nitragin: Nitragin strain collection, The Nitragin Company, Milwaukee, Wisconsin, USA, NRRL or ARSEF (collection of entomopathogenic fungi): ARS Culture Collection of the National Center for Agricultural Utilization Research, Agricultural Research Service, U.S. Department of Agriculture, 1815 North University Street, Peoria, Illinois 61604, USA; NZP: Department of Scientific and Industrial Research Culture Collection, Applied Biochemistry Division, Palmerston North, New Zealand; PPRI: ARC-Plant Protection Research Institute, Private Bag X134, Queenswood Pretoria, Gauteng, 0121, South Africa; SEMIA: FEPAGRO-Fundação Estadual de Pesquisa Agropecuária, Rua Gonçalves Dias, 570, Bairro Menino Deus, Porto Alegre/RS, Brazil; SRDI: SARDI, Adelaide, South Australia; USDA: U.S. Department of Agriculture, Agricultural Research Service, Soybean and Alfalfa Research Laboratory, BARC-West, 10300 Baltimore Boulevard, Building 011, Beltsville, MD 20705, USA (Beltsville Rhiz. Cult. Catalog: http://pdf.usaid.gov/ pdf_docs/PNAAW891.pdf); and WSM: Murdoch University, Perth, Western Australia. Further strains may be found at: http://gcm.wfcc.info/; http://www.landcareresearch.co.nz/resources/ collections/ icmp.

Jasmonic acid, its salts (jasmonates) or derivatives include without limitation potassium, sodium, lithium, ammonium, dimethylammonium, isopropylammonium, diolammonium and diethtriethanolammonium jasmonate; and also jasmonic acid methyl ester, jasmonic acid amide, jasmonic acid methylamide, jasmonic acid-L-amino acid (amide-linked) conjugates (e. g. conjugates with L-isoleucine, L-valine, L-leucine, or L-phenylalanine), 12-oxo-phytodienoic acid, coronatine, coronalon, coronafacoyl-L-serine, coronafacoyl-L-threonine, methyl esters of 1-oxo-indanoyl-isoleucine, methyl esters of 1-oxo-indanoyl-leucine, cis-jasmone, linoleic acid or derivatives thereof, and combinations of any of the above.

Humates are humic and fulvic acids extracted from a form of lignite coal and clay, known as leonardite. Humic acids are organic acids that occur in humus and other organically derived materials such as peat and certain soft coal. They have been shown to increase fertilizer efficiency in phosphate and micro-nutrient uptake by plants as well as aiding in the development of plant root systems.

According to one embodiment of the inventive mixtures, the at least one pesticide II is selected from the groups L1) to L6):
L1) Microbial pesticides with fungicidal, bactericidal, viricidal and/or plant defense activator activity: *Ampelomyces quisqualis* M-10 (L.1.1), *Aspergillus flavus* NRRL 21882 (L1.2), *Aureobasidium pullulans* DSM 14940 (L1.3), A. *pullulans* DSM 14941 (L.1.4), *Bacillus altitudinis* 41 KF2b (L.1.5), *Bacillus amyloliquefaciens* AP-136 (L.1.6), *B. amyloliquefaciens* AP-188 (L.1.7), *B. amyloliquefaciens* AP-218 (L.1.8), *B. amyloliquefaciens AP-219* (L.1.9), *B. amyloliquefaciens* AP-295 (L.1.10), *B. amyloliquefaciens* I N937a (L.1.11), *B. amyloliquefaciens* IT-45 (L.1.12), *B. amyloliquefaciens* ssp. *plantarum* D747 (L.1.13), *B. amyloliquefaciens* ssp. *plantarum* FZB24 (L.1.14), *B. amyloliquefaciens* ssp. *plantarum* FZB42 (L.1.15), *B. amyloliquefaciens* ssp. *plantarum* GB03 (L.1.16), *B. amyloliquefaciens* ssp. *plantarum* MB1600 (NRRL B-50595) (L.1.17), *B. amyloliquefaciens* ssp. *plantarum* QST-713 (L.1.18), *B. amyloliquefaciens* ssp. *plantarum* TJ1000 (L.1.19), *B. mojavensis* AP-209 (L.1.20), *B. mycoides* AQ726 (L.1.21), *B. mycoides* strain J (L.1.22), *B. pumilus* INR-7 (L.1.23), *B. pumilus* KFP9F (L.1.24), B. *pumilus* QST 2808 (L.1.25), *B. pumilus* GHA 180 (L.1.26), *B. simplex* ABU 288 (L.1.27), *B. solisalsi* AP-217 (L.1.28), *B. subtilis* CX-9060 (L.1.29), *B. subtilis* FB17 (L.1.30), *B. subtilis* GB07 (L.1.31), *Candida oleophila* I-82 (L.1.32), *C. oleophila* O (L.1.33), *C. saitoana* (L.1.34), *Clavibacter michiganensis* (bacteriophages) (L.1.35), *Coniothyrium minitans* CON/M/91-08 (L.1.36), *Cryphonectria parasitica* (L.1.37), *Cryptococcus albidus* (L.1.38), *Dilophosphora alopecuri* (L.1.39), *Fusarium oxysporum* (L.1.40), *Clonostachys rosea* f. *catenulata* J 1446 (L.1.41), *Gliocladium roseum* 321 U (L.1.42), *Metschnikowia fructicola* NRRL Y-30752 (L.1.43), *Microdochium dimerum* (L.1.44), *Microsphaeropsis ochracea* P130A (L.1.45), *Muscodor albus* QST 20799 (L.1.46), *Muscodor albus* SA-13 (L.1.47), *Paenibacillus alvei* NAS6G6 (L.1.48), *Paenibacillus polymyxa* PKB1 (L.1.49), *Pantoea agglomerans* E325 (L.1.90), *Pantoea vagans* C9-1 (L.1.50), *Penicillium bilaiae* ATCC 22348 (L.1.51), *P. bilaiae* ATCC 20851 (L.1.52), *Penicillium bilaiae* ATCC 18309 (L.1.53), *Phlebiopsis gigantea* (L.1.54), *Pichia anomala* WRL-76 (L.1.55), *Pseudomonas* sp. Proradix (L.1.56), *Pseudomonas chloraphis* MA 342 (L.1.57), *P*. *fluorescens* A506 (L.1.58), *P. fluorescens* CL 145A (L.1.91), *P. fluorescens* NCIB 12089 (L.1.92), *P. fluorescens* Pf-5 (L.1.93), *P. fluorescens* WCS 374 (L.1.94), *P. fluorescens* ATCC 13525 (L.1.95), *P. fluorescens* CHA0 (L.1.96), *P. putida* ATCC 202153 (L.1.97), *Pseudozyma flocculosa* PF-A22 UL (L.1.59), *Pythium oligandrum* DV 74 (L.1.60), *Sphaerodes mycoparasitica* SMCD2220 (L.1.61), *Streptomyces griseoviridis* K61 (L.1.62), S. *lydicus* WYEC 108 (L.1.63), S. *violaceusniger* XL-2 (L.1.64), S. *violaceusniger* YCED-9 (L.1.65), *Talaromyces flavus* V117b (L.1.66), *Trichoderma asperellum* T34 (L.1.67), *T*. *asperellum* SKT-1 (L.1.68), *T. asperellum* ICC 012 (L.1.69), *T. atroviride* LC52 (L.1.70), *T*. *atroviride* CNCM 1-1237 (L.1.71), *T. fertile* JM41 R (L.1.72), *T. gamsii* ICC 080 (L.1.73), *T*. *harmatum* TH 382 (L.1.74), *T. harzianum* T-35 (L.1.75), *T. harzianum* T-22 (L.1.76), *T*. *harzianum* T-39 (L.1.77); mixture of *T. harzianum* ICC012 and *T. viride* ICC080 (L.1.78); *T*. *polysporum* (L.1.79); *T. stromaticum* (L.1.80), *T. virens* GI-3 (L.1.81), *T. virens* G-41 (L.1.82), *T. virens* GL-21 (L.1.83), *T. virens* G-41 (L.1.84), *T. viride* TV1 (L.1.85), *Typhula phacorrhiza* 94671 (L.1.86), *Ulocladium oudemansii* HRU3 (L.1.87), *Verticillium dahlia* (L.1.88), zucchini yellow mosaic virus (avirulent strain) (L.1.89);
L2) Biochemical pesticides with fungicidal, bactericidal, viricidal and/or plant defense activator activity: chitosan (hydrolysate) (L.2.1), harpin protein (L.2.2), laminarin (L.2.3), Menhaden fish oil (L.2.4), natamycin (L.2.5), Plum pox virus coat protein (L.2.6), potassium bicarbonate (L.2.7), *Reynoutria sachalinensis* extract (L.2.8), salicylic acid (L.2.9), potassium or sodium bicarbonate (L.2.10), tea tree oil (L.2.11);
L3) Microbial pesticides with insecticidal, acaricidal, molluscidal and/or nematicidal activity: Ag*robacterium radiobacter* K1026 (L.3.1), A. *radiobacter K84* (L.3.2), *Bacillus firmus* 1-1582 (L.3.3); *B. thuringiensis ssp. aizawai* strains: ABTS-1857 (L.3.4), SAN 401 I (L.3.5), ABG-6305 (L.3.6) and ABG-6346 (L.3.7); *B. t.* ssp. *israelensis* AM65-52 (L.3.8), *B. t.* ssp. *israelensis* SUM-6218 (L.3.9), *B. t.* ssp. *galleriae* SDS-502 (L.3.10), *B. t.* ssp. *kurstaki* EG 2348 (L.3.11), *B. t.* ssp. *kurstaki* SB4 (L.3.12), *B. t.* ssp. *kurstaki ABTS-351* (HD-1) (L.3.13), *Beauveria bassiana* ATCC 74040 (L.3.14), *B. bassiana* GHA (L.3.15), *B. bassiana* H123 (L.3.16), *B. bassiana* DSM 12256 (L.3.17), *B. bassiana* PPRI 5339 (L.3.18), *B. brongniartii* (L.3.19), *Burkholderia* sp. A396 (L.3.20), *Chromobacterium subtsugae* PRAA4-1 (L.3.21), *Cydia pomonella* granulosis virus V22 (L.3.22), *Cydia pomonella* granulosis virus V1 (L.3.23), *Cryptophlebia leucotreta* granulovirus (CrIeGV) (L.3.57), *Flavobacterium sp.* H492 (L.3.60), *Helicoverpa armigera* nucleopolyhedrovirus (HearNPV) (L.3.58), *Isaria fumosorosea* Apopka-97 (L.3.24), *Lecanicillium longisporum* KV42 (L.3.25), *L. longisporum* KV71 (L.3.26), *L. muscarium* KV01 (L.3.27), *Metarhizium anisopliae* FI-985 (L.3.28), *M. anisopliae* FI-1045 (L.3.29), *M. anisopliae* F52 (L.3.30), *M. anisopliae* ICIPE 69 (L.3.31), *M. anisopliae* var. *acridum* IMI 330189 (L.3.32); *Nomuraea rileyi* strains: SA86101 (L.3.33), GU87401 (L.3.34), SR86151 (L.3.35), CG128 (L.3.36) and VA9101 (L.3.37); *Paecilomyces fumosoroseus* FE 9901 (L.3.38), *P. lilacinus* 251 (L.3.39), *P. lilacinus* DSM 15169 (L.3.40), *P. lilacinus* BCP2 (L.3.41), *Paenibacillus popilliae* Dutky-1940 (NRRL B-2309 = ATCC 14706) (L.3.42), *P. popilliae* Dutky 1 (L.3.43), *P. popilliae* KLN 3 (L.3.56), *Pasteuria sp.* Ph3 (L.3.44), *Pasteuria sp.* ATCC PTA-9643 (L.3.45), *Pasteuria sp.* ATCC SD-5832 (L.3.46), *P*. *nishizawae* Pn1 (L.3.46), *P. penetrans* (L.3.47), *P. ramosa* (L.3.48), *P*. sp. Pr-3 (L.3.49), *P*. *thornea* (L.3.50), *P. usgae* (L.3.51), *Pseudomonas fluorescens* CL 145A (L.3.52), *Spodoptera littoralis* nucleopolyhedrovirus (SpliNPV) (L.3.59), *Steinernema carpocapsae* (L.3.53), S. *feltiae* (L.3.54), S. *kraussei* L137 (L.3.55);
L4) Biochemical pesticides with insecticidal, acaricidal, molluscidal, pheromone and/or nematicidal activity: L-carvone (L.4.1), citral (L.4.2), (E,Z)-7,9-dodecadien-1-yl acetate (L.4.3), ethyl formate (L.4.4), (E,Z)-2,4-ethyl decadienoate (pear ester) (L.4.5), (Z,Z,E)-7,11,13-hexadecatrienal (L.4.6), heptyl butyrate (L.4.7), isopropyl myristate (L.4.8), cis-jasmone (L.4.9), lavanulyl senecioate (L.4.10), 2-methyl 1-butanol (L.4.11), methyl eugenol (L.4.12), methyl jasmonate (L.4.13), (E,Z)-2,13-octadecadien-1-ol (L.4.14), (E,Z)-2,13-octa-decadien-1-ol acetate (L.4.15), (E,Z)-3,13-octadecadien-1-ol (L.4.16), R-1-octen-3-ol (L.4.17), pentatermanone (L.4.18), potassium silicate (L.4.19), sorbitol actanoate (L.4.20), (E,Z,Z)-3,8,11-tetradecatrienyl acetate (L.4.21), (Z,E)-9,12-tetradecadien-1-yl acetate (L.4.22), Z-7-tetradecen-2-one (L.4.23), Z-9-tetradecen-1-yl acetate (L.4.24), Z-11-tetra-decenal (L.4.25), Z-11-tetradecen-1-ol (L.4.26), Acacia negra extract (L.4.27), extract of grapefruit seeds and pulp (L.4.28), extract of *Chenopodium ambrosiodes* (L.4.29), Catnip oil (L.4.30), Neem oil (L.4.31), Quillay extract (L.4.32), Tagetes oil (L.4.33);
L5) Microbial pesticides with plant stress reducing, plant growth regulator, plant growth promoting and/or yield enhancing activity: *Azospirillum amazonense* BR 11140 (SpY2) (L.5.1), *A. brasilense* Ab-V5 (L.5.74), *A. brasilense Ab-V6* (L.5.75), A. *brasilense AZ39* (L.5.2), A. *brasilense* XOH (L.5.3), A. *brasilense* Sp245 (BR 11005) (L.5.4), A. *brasilense* BR 11002 (L.5.5), A. *lipoferum* BR 11646 (Sp31) (L.5.6), A. *irakense* (L.5.7), A. *halopraeferens* (L.5.8), *Bradyrhizobium sp.* PNL01 (L.5.9), *B. sp.* (Arachis) CB1015 (L.5.10), *B. sp.* (Arachis) USDA 3446 (L.5.11), B. sp. (Arachis) SEMIA 6144 (L.5.12), B. sp. (Arachis) SEMIA 6462 (L.5.13), *B. sp.* (Arachis) SEMIA 6464 (L.5.14), *B. sp.* (Vigna) (L.5.15), *B. elkanii* SEMIA 587 (L.5.16), *B. elkanii* SEMIA 5019 (L.5.17), *B. elkanii U-1301* (L.5.18), *B. elkanii* U-1302 (L.5.19), *B. elkanii* USDA 74 (L.5.20), *B. elkanii* USDA 76 (L.5.21), *B. elkanii* USDA 94 (L.5.22), *B. elkanii* USDA 3254 (L.5.23), *B. japonicum* 532c (L.5.24), *B. japonicum* CPAC 15 (L.5.25), *B. japonicum* E-109 (L.5.26), *B. japonicum* G49 (L.5.27), *B. japonicum* TA-11 (L.5.28), *B. japonicum* USDA 3 (L.5.29), *B. japonicum* USDA 31 (L.5.30), *B. japonicum* USDA 76 (L.5.31), *B. japonicum* USDA 110 (L.5.32), *B. japonicum* USDA 121 (L.5.33), *B. japonicum* USDA 123 (L.5.34), *B. japonicum* USDA 136 (L.5.35), B. japonicum SEMIA 566 (L.5.36), *B. japonicum* SEMIA 5079 (L.5.37), *B. japonicum* SEMIA 5080 (L.5.38), *B. japonicum* WB74 (L.5.39), *B. liaoningense* (L.5.40), *B. lupini* LL13 (L.5.41), *B. lupini* WU425 (L.5.42), *B. lupini WSM471* (L.5.43), *B. lupini* WSM4024 (L.5.44), *Glomus intraradices* RTI-801 (L.5.45), *Mesorhizobium sp.* WSM1271 (L.5.46), *M. sp.* WSM1497 (L.5.47), *M. ciceri* CC1192 (L.5.48), *M. huakii* (L.5.49), *M. loti* CC829 (L.5.50), *M. loti SU343* (L.5.51), *Rhizobium leguminosarum* bv. *phaseoli* RG-B10 (L.5.52), *R. I.* bv. *trifolii* RP113-7 (L.5.53), *R. I*. bv. *trifolii* 095 (L.5.57), *R. I*. bv. *trifolii* TA1 (L.5.58), *R. I*. bv. *trifolii CC283b* (L.5.59), *R. I*. bv. *trifolii* CC275e (L.5.60), *R. I*. bv. *trifolii* CB782 (L.5.61), *R. I*. bv. *trifolii* CC1099 (L.5.62), R. *I*. bv. *trifolii* WSM1325 (L.5.63), *R. I*. bv. *viciae* SU303 (L.5.64), *R. I*. bv. *viciae* WSM1455 (L.5.65), *R. I.* bv. *viciae* P1 NP3Cst (L.5.66), *R. I.* bv. *viciae* RG-P2 (L.5.67), *R. tropici* PRF 81 (L.5.68), *R. tropici* SEMIA 4077 (L.5.69), *R. tropici* CC511 (L.5.70), *Sinorhizobium meliloti* RCR2011 (L.5.71), *S. meliloti* NRG185 (L.5.72), S. *meliloti* RRI128 (L.5.73);
L6) Biochemical pesticides with plant stress reducing, plant growth regulator and/or plant yield enhancing activity: abscisic acid (L.6.1), aluminium silicate (kaolin) (L.6.2), 3-decen-2-one (L.6.3), formononectin (L.6.4), genistein (L.6.5), hesperetin (L.6.6), homobrassinolide (L.6.7), humates (L.6.8), methyl jasmonate (L.6.9), cis-jasmone (L.6.10), lysophosphatidyl ethanlamine (L.6.11), naringenin (L.6.12), polymeric polyhydroxy acid (L.6.13), salicylic acid (L.6.14), *Ascophyllum nodosum* (Norwegian kelp, Brown kelp) extract (L.6.15) and *Ecklonia maxima* (kelp) extract (L.6.16).

The present invention furthermore relates to agrochemical compositions comprising a mixture of at least one compound I (component 1) and at least one biopesticide selected from the group L) (component 2), in particular at least one further fungicidal biopesticide selected from the groups L1) and L2), as described above, and if desired at least one suitable auxiliary.

Preference is also given to mixtures comprising as pesticide II (component 2) a biopesticide from group L1), preferably selected from *Bacillus amyloliquefaciens* herein even more preferably from strains AP-136, AP-188, AP-218, AP-219, AP-295, IN937a, IT-45; *B. amyloliquefaciens* ssp. *plantarum* (formerly called *B. subtilis* or *B. subtilis* spp. *amyloliquefaciens*) herein even more preferably from strains MBI600, D747, FZB254, FZB42, GB03, QST-713 and TJ1000; *B. mojavensis* AP-209; *B. pumilus* herein even more preferably from strains GHA 180, INR-7, KFP9F and QST 2808; *B. simplex* herein more preferably strain ABU 288; *B. solisalsi* herein more preferably strain AP-217; *B. subtilis* herein even more preferably selected from strains CX-9060, FB17 and GB07; *Muscodor albus* herein more preferably strains QST 20799 and SA-13; *Paenibacillus alvei* herein more preferably strain NAS6G6, *Paenibacillus polymyxa* herein more preferably strain PKB1, *Penicillium bilaiae* herein more preferably strains ATCC 22348, ATCC 20581 and ATCC 18309; *Pseudomonas fluorescens* herein more preferably strain A506; *Sphaerodes mycoparasitica* herein more preferably strain SMCD2220; *Trichoderma fertile* herein more preferably strain JM41 R; *Trichoderma harzianum* herein more preferably strain T-22; *Trichoderma virens* herein more preferably strais GI-3 and G-41.

Preference is also given to mixtures comprising as pesticide II (component 2) a biopesticide from group L1), even more preferably selected from even more preferably from *B. amyloliquefaciens* AP-188, *B. amyloliquefaciens* ssp. *plantarum* MBI600, *B. amyloliquefaciens* ssp. *plantarum* QST-713, *B. pumilus* INR-7, *B. pumilus* QST 2808, *B. simplex* ABU 288, *B. subtilis* FB17, *Paenibacillus alvei* NAS6G6 and *Trichoderma fertile* JM41 R.
(3)According to one embodiment of the inventive mixtures, the at least one pesticide II is *Bacillus amyloliquefaciens* ssp. *plantarum* MBI600. These mixtures are particularly suitable in soybean.
(4) According to another embodiment of the inventive mixtures, the at least one pesticide II is *B. pumilus* INR-7. These mixtures are particularly suitable in soybean and corn.
(5) According to a further embodiment, the at least one pesticide II is Bacillus simplex, preferably *B. simplex* ABU 288. These mixtures are particularly suitable in soybean and corn.
(6) According to a further embodiment, the at least one pesticide II is *Bacillus subtilis,* preferably *B. subtilis* strain FB17.
(7) According to one embodiment of the inventive mixtures, the at least one pesticide II is selected from *Bacillus amyloliquefaciens* AP-136, *B. amyloliquefaciens* AP-188, *B. amyloliquefaciens* AP-218, *B. amyloliquefaciens* AP-219, *B. amyloliquefaciens* AP-295, *B. amyloliquefaciens* spp. *plantarum* FZB24, *B. amyloliquefaciens* ssp. *plantarum* FZB42, *B. amyloliquefaciens* ssp. *plantarum* TJ1000, *B. amyloliquefaciens* ssp. *plantarum* D747, *B. amyloliquefaciens* ssp. *plantarum* MBI600, *B. amyloliquefaciens* spp. *plantarum* GB03, *B. amyloliquefaciens* spp. *plantarum* QST-713, *B. mojavensis* AP-209, *B. pumilus* GB34, *B. pumilus* INR-7, *B. pumilus* KFP9F, *B. pumilus* QST 2808, *B. pumilus* GHA 180, *B. simplex* ABU 288, *B. solisalsi* AP-217, *B. subtilis* CX-9060, *B. subtilis* FB17 and *B. subtilis* GB07. These mixtures are particularly suitable in soybean and corn, in particular for seed treatment.
(8) According to a further embodiment, the at least one pesticide II is selected from *Streptomyces spp.,* preferably from S. *griseoviridis, S. lydicus* and S. *violaceusniger,* in particular from strains S. *griseoviridis* K61, S. *lydicus* WYEC 108, S. *violaceusniger* XL-2 and S. *violaceusniger* YCED-9.
(9) According to one embodiment of the inventive mixtures, the at least one pesticide II is selected from the following fungi *Coniothyrium minitans* CON/M/91-08, *Trichoderma fertile* JM41 R, *T. harzianum* T-22, *T. virens* GI-3, *T. virens* GL-21, *T. virens* G-41. These mixtures are particularly suitable for seed and/or soil treatment.
   The present invention also relates to mixtures wherein the at least one pesticide II is selected from the following yeasts and fungi: *Ampelomyces quisqualis,* in particular strain M-10; *Aureobasidium pullulans,* in particular blastospores of strain DSM14940 or blastospores of strain DSM 14941 or mixtures thereof; *Candida oleophila,* in particular strains I-182 and O; *Coniothyrium minitans,* in particular strain CON/M/91-8; *Dilophosphora alopecuri which* reduces annual ryegrass toxicity (ARGT), a disease of livestock resulting from the ingestion of annual ryegrass seed-heads that have been infected by the toxin producing bacterium *Rathayibacter toxicus; Clonostachys rosea f. catenulata,* in particular strain J1446; *Metschnikovia fructicola,* in particular strain 277, *Microsphaeropsis ochracea,* in particular strain P130A for control of apple scab; *Muscodor albus,* in particular strain QST 20799, *Pichia anomala,* in particular strain WRL-076, *Pseudozyma flocculosa,* in particular strain PF-A22 UL; *Pythium oligandrum,* in particular strain DV74.
(10) According to a further embodiment, the at least one pesticide II is selected from *Pseudomonas* spp., preferably selected from *P. chloraphis* herein more preferably strain MA 342 and *Pseudomonas sp.* DSM 13134; *P. fluorescens* herein more preferably selected from strains A506, WCS 374 and Pf-5; and *P. putida* herein more preferably strain ATCC 202153.

The present invention also relates to mixtures wherein the at least one pesticide II is selected from the fungal genus *Trichoderma,* preferably from the strains *T. asperellum* T34, *T. asperellum* SKT-1, *T. asperellum* ICC 012, *T. asperellum* TV1, *T. atroviride* LC52, *T. atroviride* CNCM I-1237, *T. fertile* JM41 R, *T. gamsii* ICC 080, *T. harmatum TH* 382, *T. harzianum* T-22, *T. harzianum* T-35, *T. harzianum* T-39, *T. harzianum* T-315; mixture of *T. harzianum* ICC012 and *T. gamsii* ICC080; mixture of *T. polysporum* and *T. harzianum; T. stromaticum, T. virens* GI-3, *T. virens* GL-21, *T. virens* G-41 and; in particular *T. fertile* JM41 R.

The present invention also relates to mixtures wherein the at least one pesticide II is selected from the fungal species *Muscodor albus* preferably from the strains SA-13 and QST 20799, which are particularly suiable for soil and seed treatment against soil-borne pathogens and/or nematodes.

Preference is also given to mixtures comprising as pesticide II (component 2) a biopesticide from group L2), preferably selected from chitosan (hydrolysate), methyl-jasmonate, cis-jasmone, laminarin, *Reynoutria sachalinensis* extract and tea tree oil; even more preferable from methyl jasmonate, cis-jasmone and laminarin.

Preference is also given to mixtures comprising as pesticide II (component 2) a biopesticide from group L3), preferably selected from *Agrobacterium radiobacter* herein preferably strain K1026, *Bacillus firmus* herein preferably strain I-1582, *Bacillus thuringiensis* ssp. *kurstaki* herein preferably strain SB4, *Beauveria bassiana* herein preferably selected from strains GHA, H123, DSM 12256 and PPRI 5339; *Burkholderia* sp. and herein preferably strain A396, *Metarhizium anisopliae* var. *acridum* herein preferably strain IMI 330189, *M. anisopliae* herein preferably selected from strains FI-985, FI-1045, F52 and ICIPE 69; *Paecilomyces lilacinus* herein preferably selected from strains 251, DSM 15169 and BCP2, *Paenibacillus popilliae* herein preferably selected from strains Dutky-1940, KLN 3 and Dutky 1; *Pasteuria nishazawa* and herein preferably strain Pn1.

Preference is also given to mixtures comprising as pesticide II (component 2) a biopesticide from group L3), even more preferably from *Bacillus thuringiensis* ssp. *kurstaki* SB4, *B. bassiana* DSM 12256, *B. bassiana* PPRI 5339, *Metarhizium anisopliae* var. *acridum* IMI 330189, M. *anisopliae* FI-985, *M. anisopliae* FI-1045, *Paecilomyces lilacinus* DSM 15169, *P. lilacinus* BCP2, *P. lilacinus* 251, *Paenibacillus popilliae* Dutky-1940, *P. popilliae* KLN 3 and *P. popilliae* Dutky 1.
(11) According to a further embodiment, the at least one pesticide II is *Beauveria brongniartii.*
(12) According to a further embodiment, the at least one pesticide II is *Metarhizium anisopliae* or M. *anisopliae* var. *acridium,* preferably selected from *M. anisopliae* FI-1045, M. *anisopliae* F52, *M. anisopliae* var. *acridum* strains FI-985 and IMI 330189; in particular strain IMI 330189. These mixtures are particularly suitable for control of arthropod pests in soybean and corn.
(13) According to a further embodiment, the at least one pesticide II is *Lecanicillium* sp., preferably selected from *Lecanicillium longisporum* KV42, *L. longisporum* KV71 and L. *muscarium* KV01.
(14) According to a further embodiment, the at least one pesticide II is *Paecilomyces fumosoroseus,* preferably strain FE 9901 especially for white fly control.
(15) According to a further embodiment, the at least one pesticide II is selected from *Nomuraea rileyi,* preferably strains SA86101, GU87401, SR86151, CG128 and VA9101; and *P*. *lilacinus,* preferably strains 251, DSM 15169 or BCP2, in particular BCP2, which strains especially control the growth of plant-pathogenic nematodes.
(16) According to a further embodiment, the at least one pesticide II is *Bacillus firmus,* preferably spores of strain CNCM I-1582, preferably useful for seed treatment of soybean and corn against nematodes and insects.
(17) According to a further embodiment, the at least one pesticide II is *Bacillus cereus,* preferably spores of CNCM I-1562, preferably useful for seed treatment of soybean and corn against nematodes and insects.
(18) According to a further embodiment, the at least one pesticide II is a mixture of spores of *B. firmus* and *B. cereus,* preferably mixtures spores of above mentioned strains CNCM I-1582 and CNCM I-1562, preferably useful for seed treatment of soybean and corn against nematodes and insects.
(19) According to a further embodiment, the at least one pesticide II is selected from *Bacillus t.* ssp. *kurstaki* preferably from strains EG 2348, SB4 and ABTS-351 (HD-1), in particular *B. t.* ssp. *kurstaki* SB4. These strains are used for control of lepidopteran larvae, but without noctuidae.
(20) According to one embodiment of the inventive mixtures, the at least one pesticide II is selected from *Bacillus firmus* CNCM I-1582, *Paecilomyces lilcinus* 251, *Pasteuria nishizawa* Pn1 and *Burkholderia sp.* A396 having nematicidal, acaricidal and/or insecticidal activity. These mixtures are particularly suitable in soybean and corn, in particular for seed treatment.

Preference is also given to mixtures comprising as pesticide II (component 2) a biopesticide from group L4), preferably selected from methyl jasmonate, *Acacia negra* extract, extract of grapefruit seeds and pulp, Catnip oil, Neem oil, Quillay extract and Tagetes oil, in particular methyl jasmonate or water-based Quillay extract.

Preference is also given to mixtures comprising as pesticide II (component 2) a biopesticide from group L5), preferably selected from *Azospirillum amazonense, A. brasilense, A. lipoferum, A. irakense, A. halopraeferens, Bradyrhizobium sp.* (Arachis), *Bradyrhizobium sp.* (Vigna), *B. elkanii, B. japonicum; Paenibacillus alvei, Penicillium bilaiae, Rhizobium leguminosarum* bv. *phaseoli,* R. *I.* bv. *trifolii,* R. *I.* bv. *viciae,* and *Sinorhizobium meliloti.*

Preference is also given to mixtures comprising as pesticide II (component 2) a biopesticide from group L5) selected from *Azospirillum amazonense* SpY2, A. *brasilense* XOH, A. *brasilense* Sp245, A. *brasilense* Cd, A. *brasilense* Ab-V5, A. *brasilense* Ab-V6, A. *lipoferum* Sp31, *Bradyrhizobium sp.* (Vigna) PNL1, *B. elkanii* SEMIA 587, *B. elkanii* SEMIA 5019, *B. japonicum* SEMIA 5079, *B. japonicum* SEMIA 5080, *B. japonicum* TA-11, *B. japonicum* 532c, *Paenibacillus alvei* NAS6G6, *Peniciillium bilaiae* strains ATCC 18309, ATCC 20851 and ATCC 22348; *Rhizobium leguminosarum* bv. *phaseoli* RG-B10, R. *I*. bv. *viciae* P1NP3Cst, R. *I*. bv. *viciae* R^{G}-P2, R. *I.* bv. *trifolii* RP113-7, R. *I*. bv. *viciae* SU303, R. *I*. bv. *viciae* WSM1455, *R. tropici* SEMIA 4077, *R. tropici* PRF 81 and *Sinorhizobium meliloti;* even more preferably selected from *Azospirillum brasilense* Sp245, *Bradyrhizobium sp.* (Vigna) PNL1, *B B. elkanii* SEMIA 587, *B. elkanii* SEMIA 5019, *B. japonicum* SEMIA 5079, *B. japonicum* SEMIA 5080, *B. japonicum* TA-11 and *B. japonicum* 532c.
(21) The present invention also relates to mixtures, wherein the at least one pesticide II is selected from *Azospirillum amazonense, A. brasilense, A. lipoferum, A. irakense* and A. *halopraeferens,* more preferably from A. *brasilense,* in particular selected from A. *brasilense* strains Sp245 and AZ39 which are both commercially used in Brazil and are obtainable from EMBRAPA-Agribiologia, Brazil, and strains Ab-V5 and Ab-V6; in particular mixtures of these strains Ab-V5 and Ab-V6. These mixtures are particularly suitable in soybean, especially as seed treatment.
(22) The present invention also relates to mixtures wherein the at least one pesticide II is selected from A. *amazonense, A. brasilense, A. lipoferum, A. irakense* and A. *halopraeferens,* more preferably A. *brasilense,* and further comprises a pesticide III, wherein pesticide III is selected from jasmonic acid, its salts and derivatives thereof, preferably methyl-jasmonate or cis-jasmone.
(23) According to another embodiment of the inventive mixtures, *Bradyrhizobium* spp. (meaning any *Bradyrhizobium* species and/or strain) as pesticide II is *B. japonicum.* These mixtures are particularly suitable in soybean. Certain *B. japonicum* strains have been re-classified as a novel species *B. elkanii,* e. g. strain USDA 76 (Can. J. Microbiol. 38, 501-505, 1992). *Bradyrhizobium* spp. are cultivated using media and fermentation techniques known in the art, e. g. in yeast extract-mannitol broth (YEM) at 27°C for about 5 days.
(24) The present invention also relates to mixtures, wherein the at least one pesticide II is selected from *Bradyrhizobium* spp., even more preferably from *B.* sp. (Arachis), *B. elkanii, B. japonicum, B. liaoningense* and *B. lupini,* and further comprises a pesticide III (component 3), wherein pesticide III is selected from jasmonic acid, its salts and derivatives thereof, preferably methyl-jasmonate or cis-jasmone.
   Preferably, *B. japonicum* is selected from strains E-109, SEMIA 5079, SEMIA 5080, TA-11 and 532c. According to a further embodiment, mixtures of *B. japonicum* strains TA-11 and 532c or *B. japonicum* strains SEMIA 5079 and 5080 are used. The strains having a prefix SEMIA are especially suitable for soybean grown in Australia or South America, in particular in Brazil. More preferably, mixtures of *B. japonicum* SEMIA 5079 and SEMIA 5080 are used. *B. japonicum* WB74 is especially suitable for soybean grown in South America and Africa, in particular in South Africa. Strain E-109 is especially suitable for soybean grown in South America, in particular in Argentina.
(25) The present invention also relates to mixtures, wherein the at least one pesticide II is selected from *B. japonicum* and further comprises a pesticide III, wherein pesticide III is selected from jasmonic acid, its salts and derivatives thereof, preferably methyl-jasmonate or cis-jasmone.
(26) The present invention also relates to mixtures, wherein the at least one pesticide II is selected from *Bradyrhizobium elkanii* and *Bradyrhizobium liaoningense,* more preferably from *B. elkanii* even more preferably *B. elkanii* strains SEMIA 587 and SEMIA 5019; in particular mixtures of both. These mixtures are particularly suitable in soybean in Australia or South America, in particular in Brazil.
(27) The present invention also relates to mixtures, wherein pesticide II is selected from *Bradyrhizobium* sp. (Arachis) and *B.* sp. (Vigna) which shall describe the cowpea miscellany cross-inoculation group which includes inter alia indigenous cowpea bradyrhizobia on cowpea (*Vigna unguiculata*), siratro (*Macroptilium atropurpureum*), lima bean *(Phaseolus lunatus*), and peanut *(Arachis hypogaea*), in particular in particular *B.* sp. (Vigna) strain PNL1. This mixture comprising as pesticide II *B.* sp. (Arachis) or *B.* sp. (Vigna) is especially suitable for use in peanut, cowpea, Mung bean, Moth bean, Dune bean, Rice bean, Snake bean and Creeping vigna, in particular peanut.
(28) The present invention also relates to mixtures, wherein the at least one pesticide II is selected from *Bradyrhizobium lupini* (also called *B.* sp. (Lupine), *B. lupines* or *Rhizobium lupini*). These mixtures are especially suitable for use in dry beans and lupins. Preferably, *B. lupini* is strain LL13. This strain is especially suitable for lupins grown in Australia, North America or Europe, in particular in Europe.
(29) The present invention also relates to mixtures wherein the at least one pesticide II is selected from *Rhizobium leguminosarum* bv. *phaseoli* especially for the legume common bean (*Phaseolus vulgaris*), but also for other for various legumes such as alfalfa, clover, peas, beans, lentils, soybeans, peanuts and other crops such as corn and lettuce, even more preferably strain RG-B10 thereof; *R*. *I.* bv. *trifolii,* especially strain RP113-7 thereof, *R*. *I.* bv. *viciae,* in particular strains RG-P2, SU303, WSM1455 and P1 NP3Cst thereof, in particular P1 NP3Cst; *R*. *tropici,* especially strains CC511, CIAT 899 and PRF 81 thereof; and *Sinorhizobium meliloti,* especially strain RCR2011 thereof. Further *R*. *I.* bv. *phaseoli* or *R*. *etli* strains are e. g. known from the above mentioned references and Appl. Environ. Microbiol. 45(3), 737-742, 1983; ibida 54(5), 1280-1283, 1988.
(30) According to a further embodiment, in the inventive mixtures pesticide II is selected from one compound II selected from *Sinorhizobium meliloti* more preferably from RCR2011, S. *meliloti* NRG185, *S*. *meliloti* RRI 128, *S*. *meliloti* SU277,
(31) *R. tropici* is useful for a range of legume crops especially all kind of clovers e. g. in tropical regions such as Brazil. Preferably, mixtures comprise as *R*. *tropici* at least one strain selected from CC511, CIAT899, H12 and PRF 81.
(32) The present invention also relates to mixtures wherein the at least one pesticide II is selected from R. leguminosarum bv. *phaseoli, R. I.* bv. *trifolii, R. I.* bv. *viciae, R. tropici* and *Sinorhizobium meliloti,* and further comprises a pesticide III, wherein pesticide III is selected from jasmonic acid, its salts and derivatives thereof, preferably methyl-jasmonate or cis-jasmone.
(33) According to a further embodiment, the at least one pesticide II is selected from *Delftia acidovorans,* in particular strain RAY209, especially in soybean and canola.

Accordingly, the present invention furthermore relates to compositions comprising one compound I (component 1) and one pesticide II (component 2), which pesticide II is selected from the column "Co. 2" of the lines C-1 to C-869 of Table C.

A further embodiment relates to the compositions C-1 to C-869 listed in Table C, where a row of Table C corresponds in each case to a fungicidal composition comprising as active components one of the in the present specification individualized compounds of formula I (component 1) and the respective pesticide II from groups A) to O) (component 2) stated in the row in question. Preferably, the compositions described comprise the active components in synergistically effective amounts.

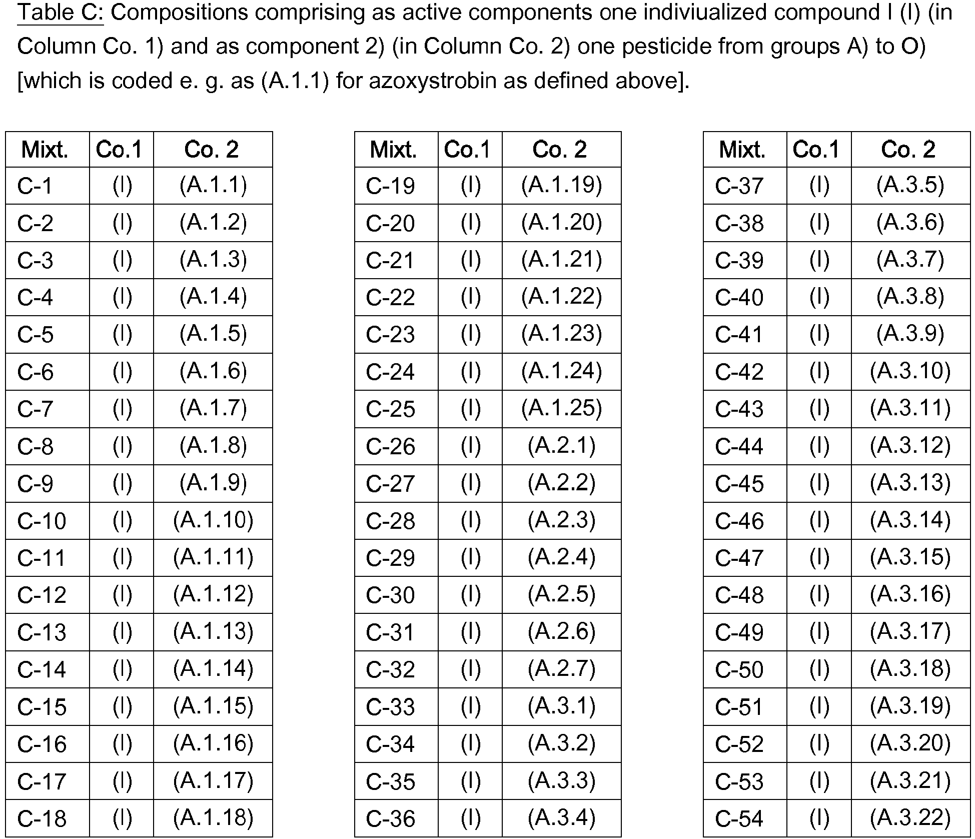

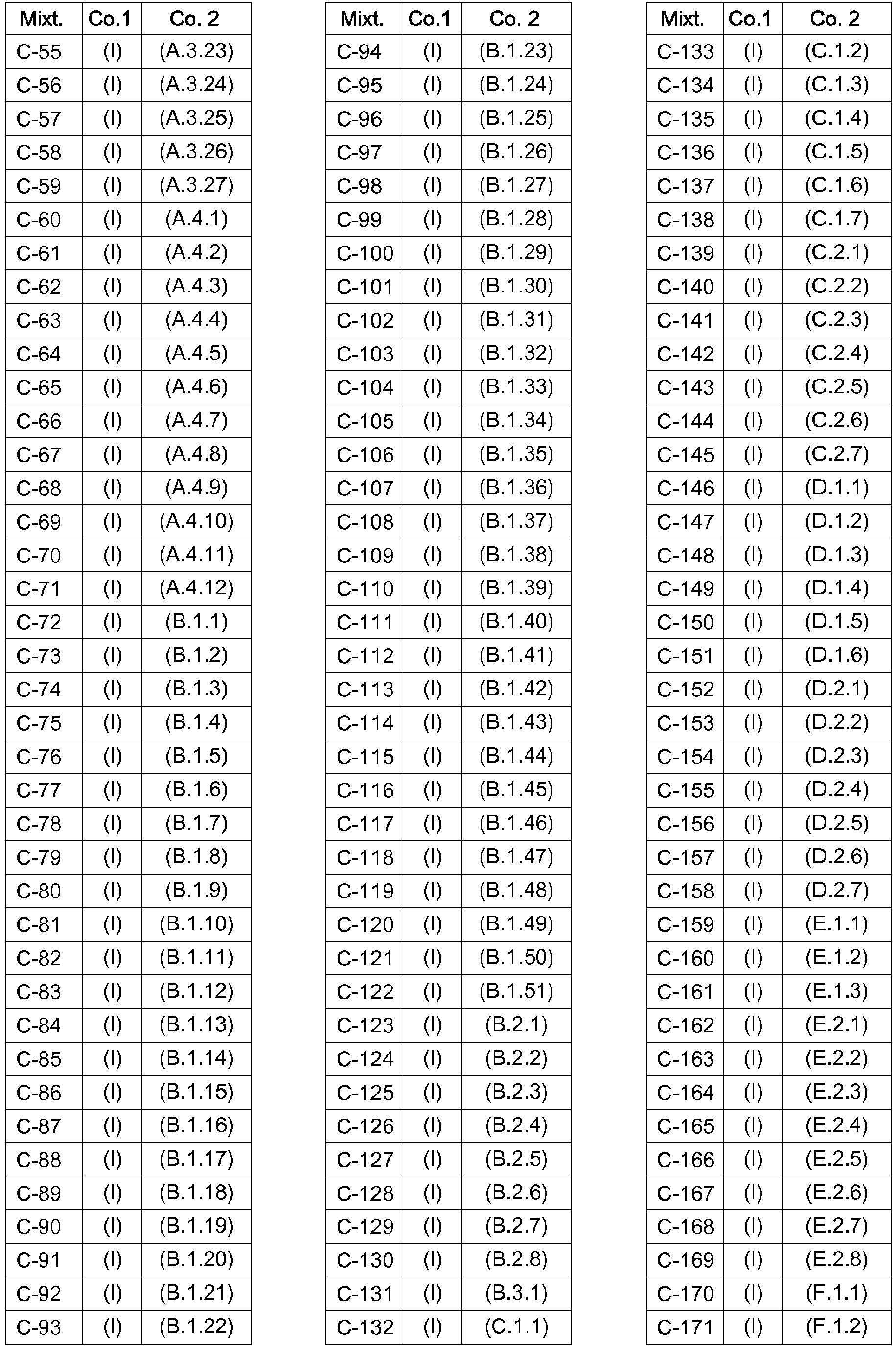

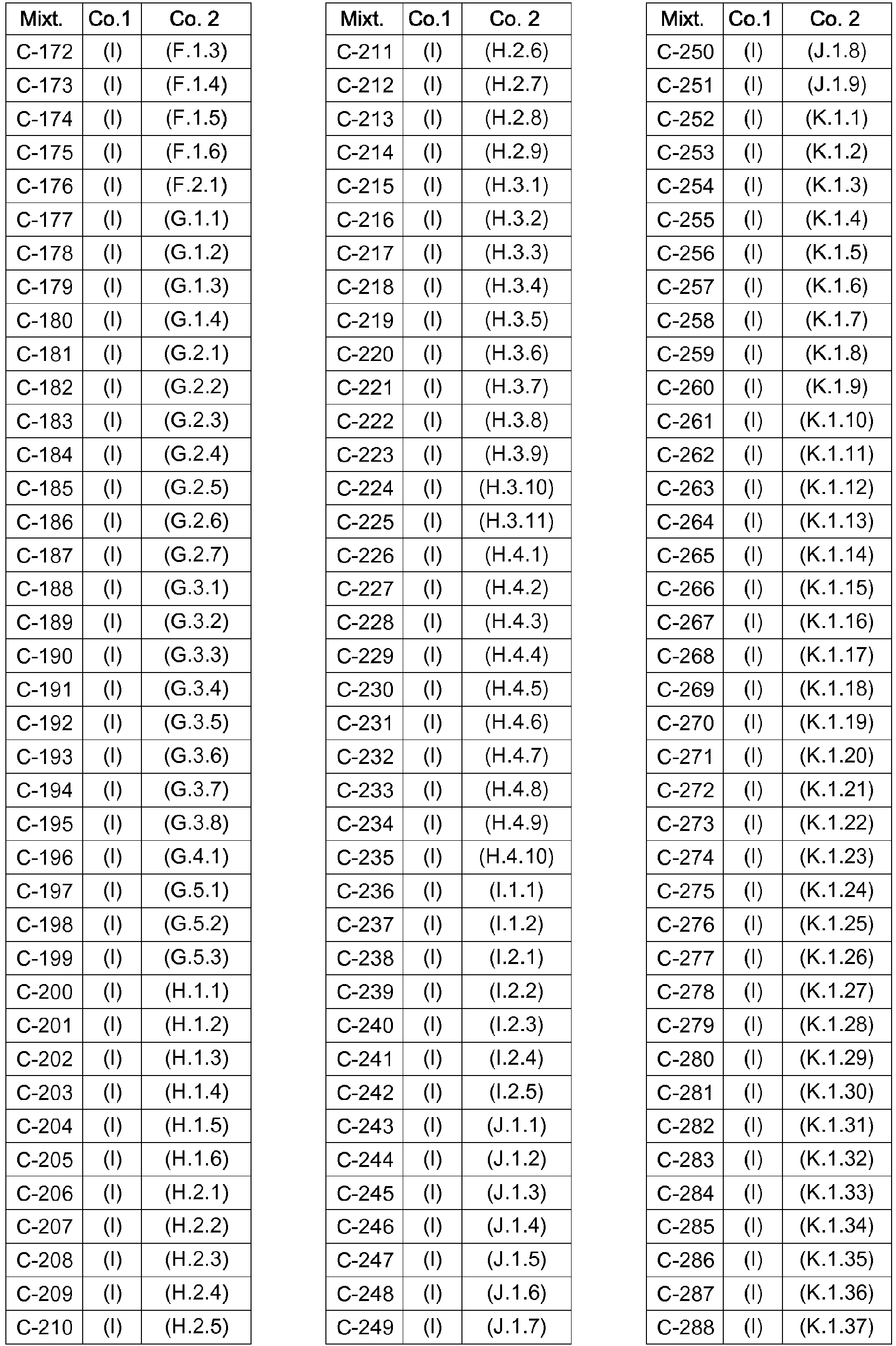

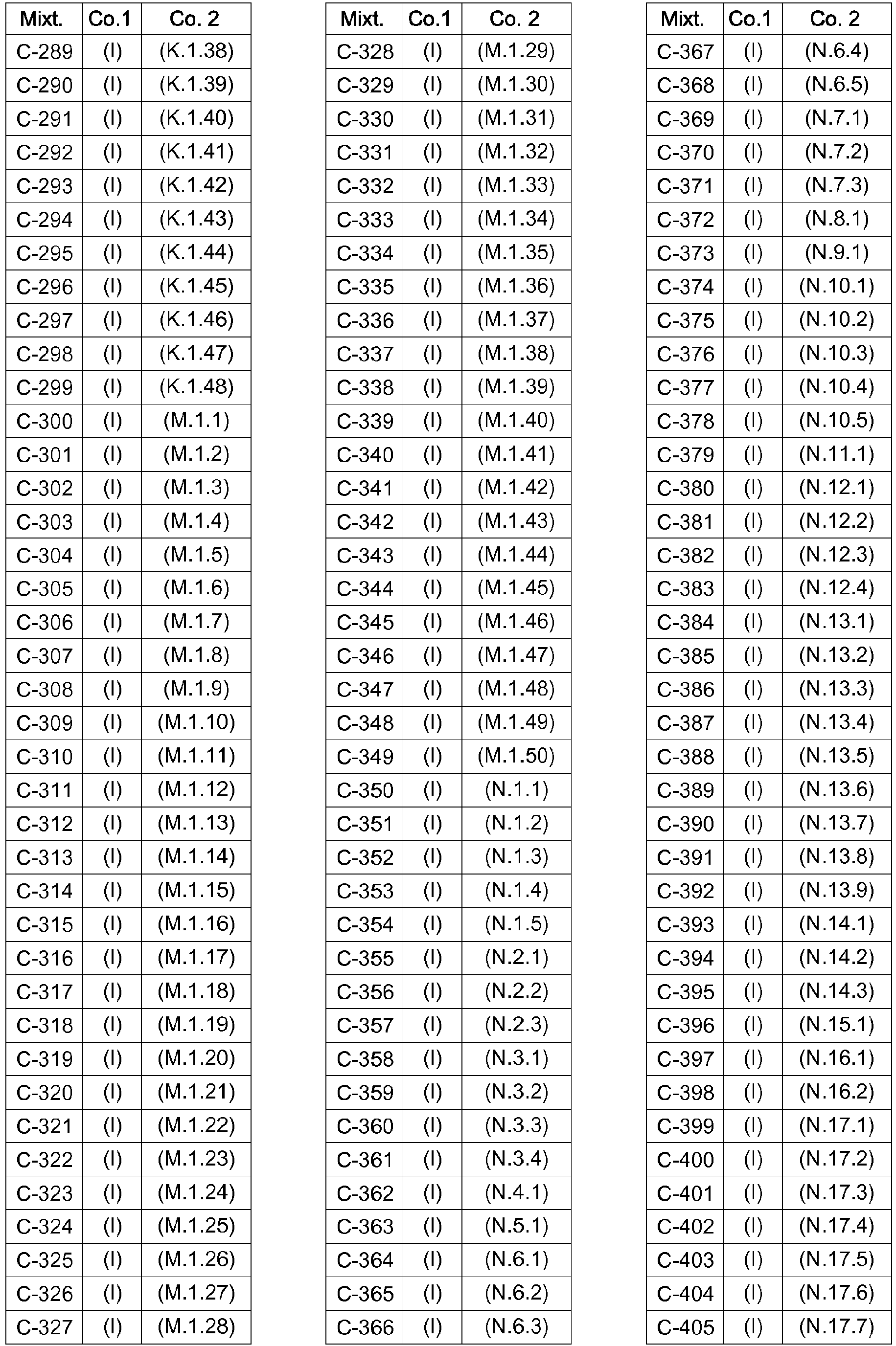

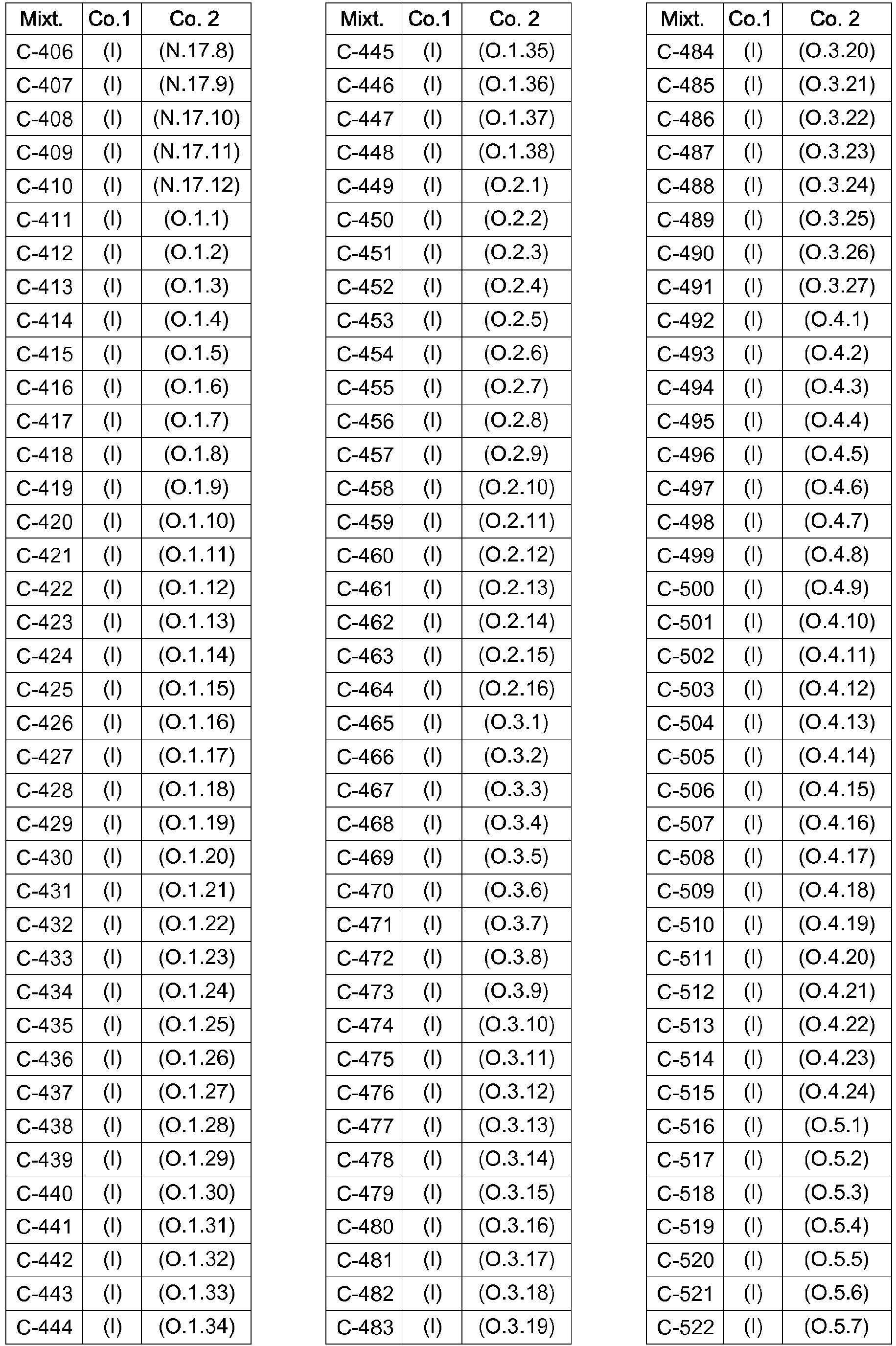

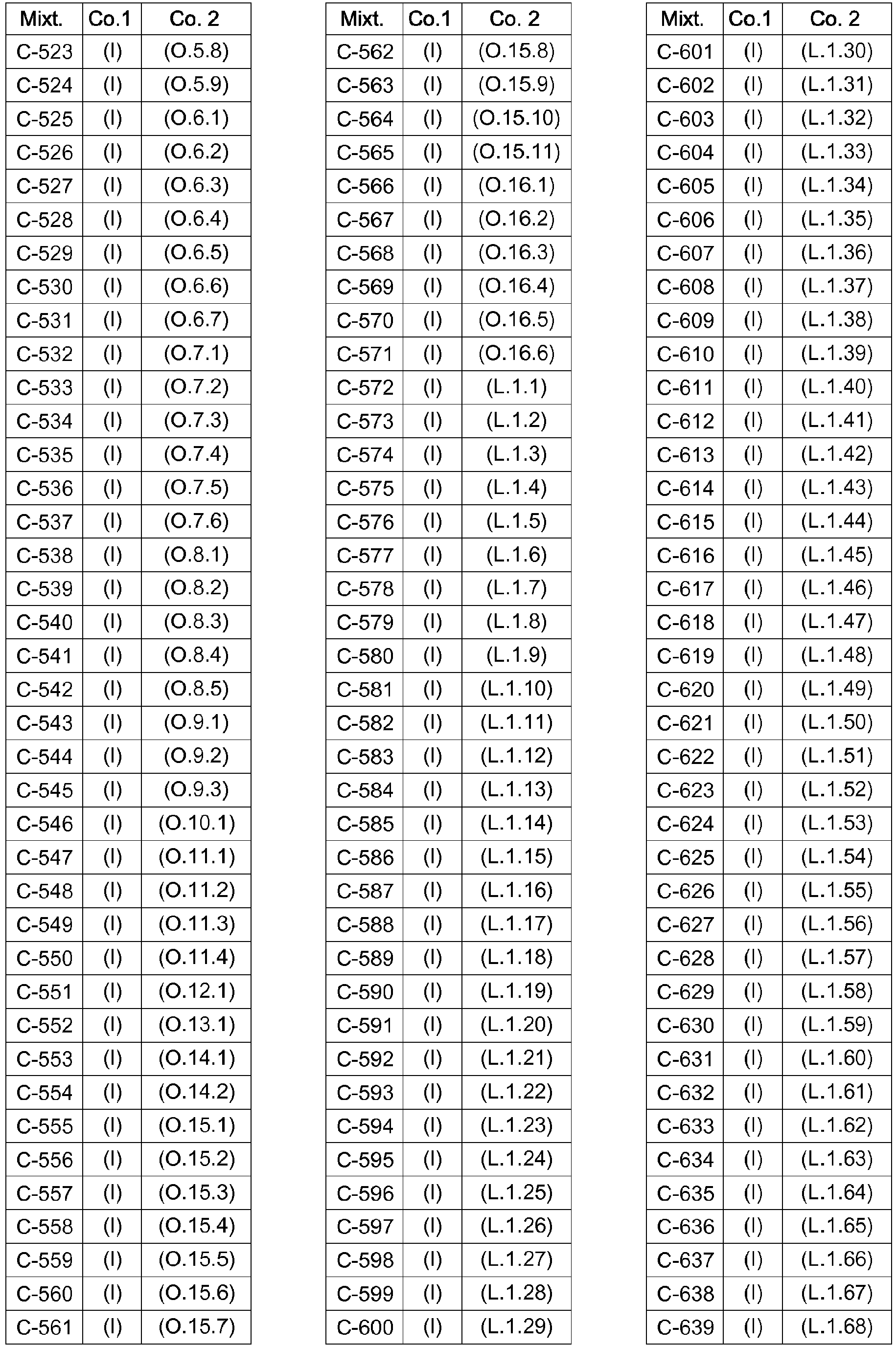

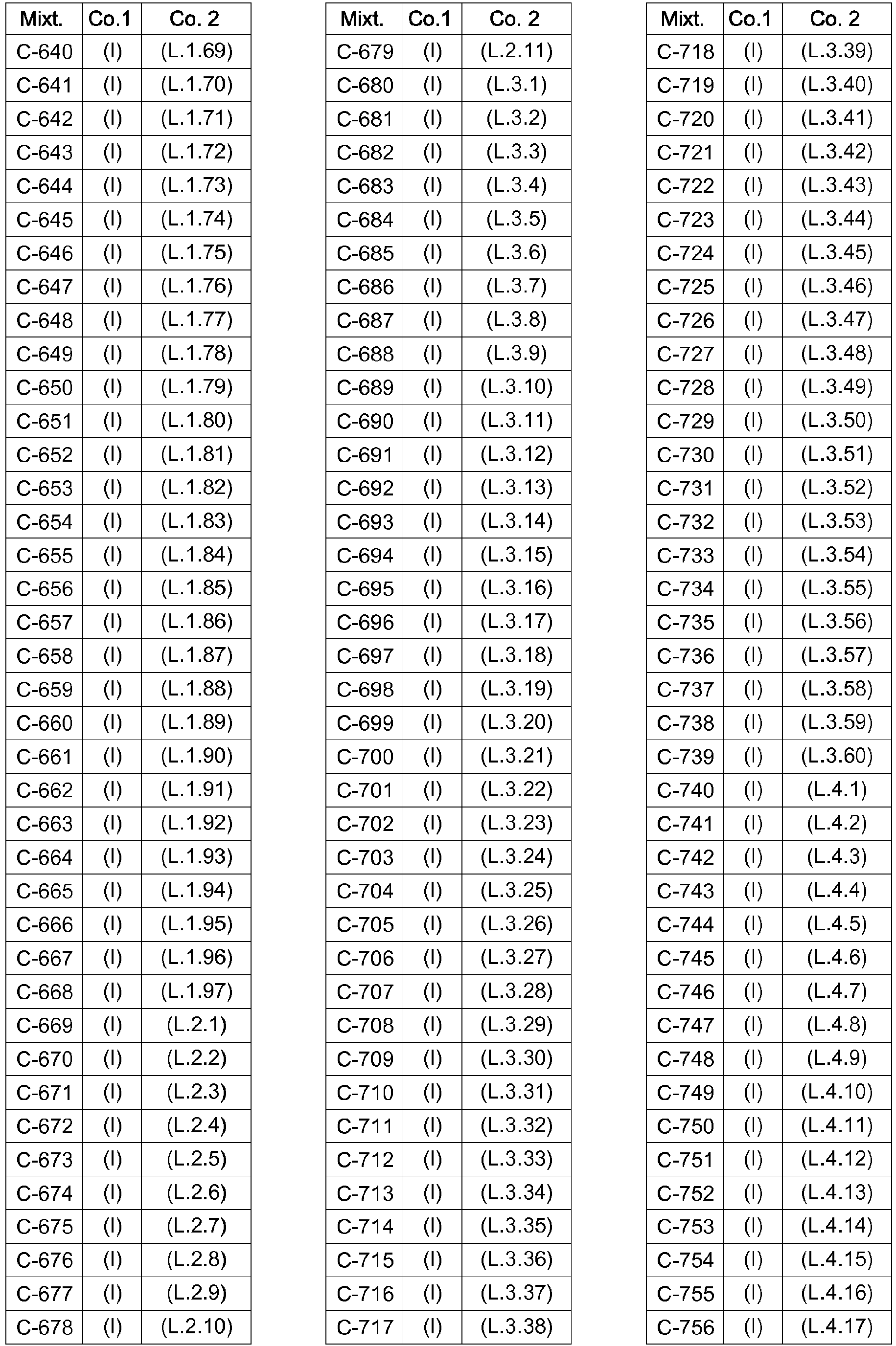

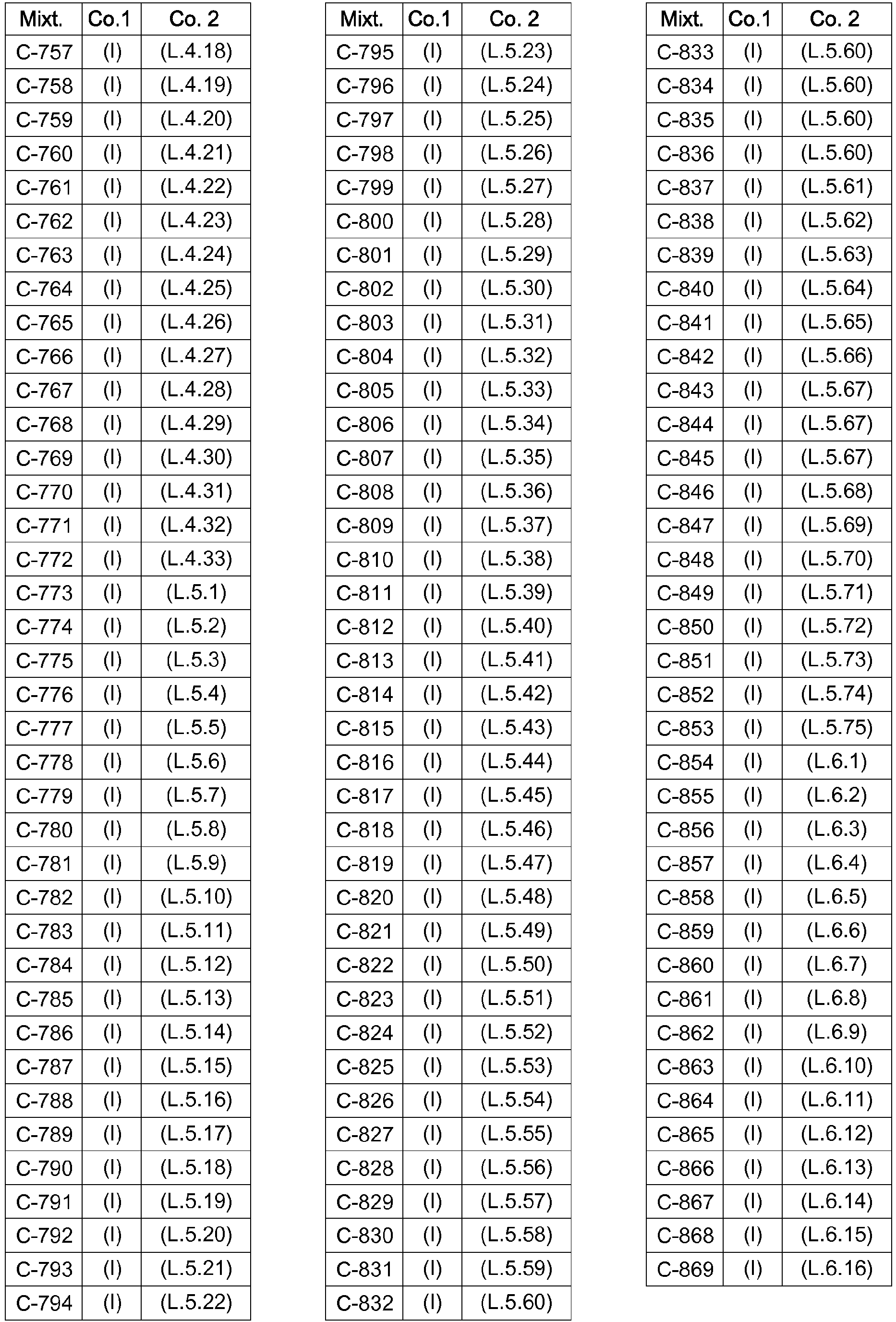

The active substances referred to as component 2, their preparation and their activity e. g. against harmful fungi is known (cf.: http://www.alanwood.net/pesticides/); these substances are commercially available. The compounds described by IUPAC nomenclature, their preparation and their pesticidal activity are also known (cf. Can. J. Plant Sci. 48(6), 587-94, 1968; EP-A 141 317; EP-A 152 031; EP-A 226 917; EP-A 243 970; EP-A 256 503; EP-A 428 941; EP-A 532 022; EP-A 1 028 125; EP-A 1 035 122; EP-A 1 201 648; EP-A 1 122 244, JP 2002316902; DE 19650197; DE 10021412; DE 102005009458; US 3,296,272; US 3,325,503; WO 98/46608; WO 99/14187; WO 99/24413; WO 99/27783; WO 00/29404; WO 00/46148; WO 00/65913; WO 01/54501; WO 01/56358; WO 02/22583; WO 02/40431; WO 03/10149; WO 03/11853; WO 03/14103; WO 03/16286; WO 03/53145; WO 03/61388; WO 03/66609; WO 03/74491; WO 04/49804; WO 04/83193; WO 05/120234; WO 05/123689; WO 05/123690; WO 05/63721; WO 05/87772; WO 05/87773; WO 06/15866; WO 06/87325; WO 06/87343; WO 07/82098; WO 07/90624, WO 11/028657, WO2012/168188, WO 2007/006670, WO 2011/77514; WO13/047749, WO 10/069882, WO 13/047441, WO 03/16303, WO 09/90181, WO 13/007767, WO 13/010862, WO 13/127704, WO 13/024009, WO 13/024010 and WO 13/047441, WO 13/162072, WO 13/092224).

The mixtures of active substances can be prepared as compositions comprising besides the active ingredients at least one inert ingredient (auxiliary) by usual means, e. g. by the means given for the compositions of compounds I.

Concerning usual ingredients of such compositions reference is made to the explanations given for the compositions containing compounds I.

The mixtures of active substances according to the present invention are suitable as fungicides, as are the compounds of formula I. They are distinguished by an outstanding effectiveness against a broad spectrum of phytopathogenic fungi, especially from the classes of the Ascomycetes, Basidiomycetes, Deuteromycetes and Peronosporomycetes (syn. Oomycetes). In addition, it is refered to the explanations regarding the fungicidal activity of the compounds and the compositions containing compounds I, respectively.

According to one embodiment, the microbial pesticides selected from groups L1), L3) and L5) embrace not only the isolated, pure cultures of the respective micro-organism as defined herein, but also its cell-free extract, its suspensions in a whole broth culture or as a metabolite-containing culture medium or a purified metabolite obtained from a whole broth culture of the microorganism or microorganism strain.

According to a further embodiment, the microbial pesticides selected from groups L1), L3 and L5) embraces not only the isolated, pure cultures of the respective micro-organism as defined herein, but also a cell-free extract thereof or at least one metabolite thereof, and/or a mutant of the respective micro-organism having all the identifying characteristics thereof and also a cell-free extract or at least one metabolite of the mutant.

As used herein, "whole culture broth" refers to a liquid culture of a microorganism containing vegetative cells and/or spores suspended in the culture medium and optionally metabolites produced by the respective microorganism.

As used herein, "culture medium", refers to a medium obtainable by culturing the microorganism in said medium, preferably a liquid broth, and remaining when cells grown in the medium are removed, e. g., the supernatant remaining when cells grown in a liquid broth are removed by centrifugation, filtration, sedimentation, or other means well known in the art; comprising e. g. metabolites produced by the respective microorganism and secreted into the culture medium. The "culture medium" sometimes also referred to as "supernatant" can be obtained e. g. by centrifugation at temperatures of about 2 to 30°C (more preferably at temperatures of 4 to 20°C) for about 10 to 60 min (more preferably about 15 to 30 min) at about 5,000 to 20,000 x g (more preferably at about 15,000 x g).

As used herein, "cell-free extract" refers to an extract of the vegetative cells, spores and/or the whole culture broth of a microorganism comprising cellular metabolites produced by the respective microorganism obtainable by cell disruption methods known in the art such as solvent-based (e. g. organic solvents such as alcohols sometimes in combination with suitable salts), temperature-based, application of shear forces, cell disruption with an ultrasonicator. The desired extract may be concentrated by conventional concentration techniques such as drying, evaporation, centrifugation or alike. Certain washing steps using organic solvents and/or water-based media may also be applied to the crude extract preferably prior to use.

As used herein, the term "metabolite" refers to any component, compound, substance or byproduct (including but not limited to small molecule secondary metabolites, polyketides, fatty acid synthase products, non-ribosomal peptides, ribosomal peptides, proteins and enzymes) produced by a microorganism (such as fungi and bacteria, in particular the strains of the invention) that has any beneficial effect as described herein such as pesticidal activity or improvement of plant growth, water use efficiency of the plant, plant health, plant appearance, or the population of beneficial microorganisms in the soil around the plant activity herein.

As used herein, "isolate" refers to a pure microbial culture separated from its natural origin, such an isolate obtained by culturing a single microbial colony. An isolate is a pure culture derived from a heterogeneous, wild population of microorganisms.

As used herein, "strain" refers to isolate or a group of isolates exhibiting phenotypic and/or genotypic traits belonging to the same lineage, distinct from those of other isolates or strains of the same species.

The term "mutant" refers a microorganism obtained by direct mutant selection but also includes microorganisms that have been further mutagenized or otherwise manipulated (e. g., via the introduction of a plasmid). Accordingly, embodiments include mutants, variants, and or derivatives of the respective microorganism, both naturally occurring and artificially induced mutants. For example, mutants may be induced by subjecting the microorganism to known mutagens, such as N-methyl-nitrosoguanidine, using conventional methods.

In the case of mixtures comprising microbial pesticides II selected from groups L1), L3) and L5), the microorganisms as used according to the invention can be cultivated continuously or discontinuously in the batch process or in the fed batch or repeated fed batch process. A review of known methods of cultivation will be found in the textbook by Chmiel (Bioprozesstechnik 1. Einführung in die Bioverfahrenstechnik (Gustav Fischer Verlag, Stuttgart, 1991)) or in the textbook by Storhas (Bioreaktoren und periphere Einrichtungen (Vieweg Verlag, Braunschweig/Wiesbaden, 1994)).

When living microorganisms, such as pesticides II from groups L1), L3) and L5), form part of the compositions, such compositions can be prepared as compositions comprising besides the active ingredients at least one auxiliary (inert ingredient) by usual means (see e. g. H.D. Burges: Formulation of Micobial Biopestcides, Springer, 1998). Suitable customary types of such compositions are suspensions, dusts, powders, pastes, granules, pressings, capsules, and mixtures thereof. Examples for composition types are suspensions (e. g. SC, OD, FS), capsules (e. g. CS, ZC), pastes, pastilles, wettable powders or dusts (e. g. WP, SP, WS, DP, DS), pressings (e. g. BR, TB, DT), granules (e. g. WG, SG, GR, FG, GG, MG), insecticidal articles (e. g. LN), as well as gel formulations for the treatment of plant propagation materials such as seeds (e. g. GF). Herein, it has to be taken into account that each formulation type or choice of auxiliary should not influence the viability of the microorganism during storage of thecomposition and when finally applied to the soil, plant or plant propagation material. Suitable formulations are e. g. mentioned in WO 2008/002371, US 6955,912, US 5,422,107.

Examples for suitable auxiliaries are those mentioned earlier herein, wherein it must be taken care that choice and amounts of such auxiliaries should not influence the viability of the microbial pesticides in the composition. Especially for bactericides and solvents, compatibility with the respective microorganism of the respective microbial pesticide has to be taken into account. In addition, compositions with microbial pesticides may further contain stabilizers or nutrients and UV protectants. Suitable stabilzers or nutrients are e. g. alpha-tocopherol, trehalose, glutamate, potassium sorbate, various sugars like glucose, sucrose, lactose and maltodextrine (H.D. Burges: Formulation of Micobial Biopestcides, Springer, 1998). Suitable UV protectants are e. g. inorganic compouns like titan dioxide, zinc oxide and iron oxide pigments or organic compounds like benzophenones, benzotriazoles and phenyltriazines. The compositions may in addition to auxiliaries mentioned for compositions comprising compounds I herein optionally comprise 0.1 - 80% stabilizers or nutrients and 0.1-10% UV protectants.

### I. Synthesis examples

### Example 1: Synthesis of 3-chloro-4-(1-cyclopropyl-1-hydroxy-2-(1H-1,2,4-triazol-1-yl)ethyl)benzonitrile

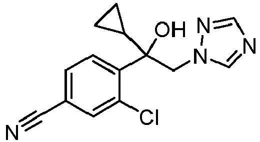

A round-bottom flask was charged with Cul (560 mg, 0.1 eq), Kl (970 mg, 0.2 eq), KCN (2.28 g, 1.2 eq) and *N,N*'-dimethylethylendiamine (2.57 g, 1.0 eq) before a solution of the aryl bromide 1-(4-bromo-2-chloro-phenyl)-1-cyclopropyl-2-(1,2,4-triazol-1-yl)ethanol (10.0 g, 1.0 eq) in toluene (50 mL) was added. The mixture was stirred overnight at room temperature and quenched with an aqueous solution of NH₃. The product was extracted into EtOAc and the combined organic extracts were washed with diluted HCl and water successively. After drying over MgSO₄, the solvent was removed under reduced pressure and the crude product was recrystallized from EtOAc/di-*iso*-propyl ether to afford the title compound as solid (6.39 g, 76%).
HPLC-MS: t_{R} = 0.801
NMR (400 MHz, CDCl₃): δ [ppm] = 0.20-0.30 (1 H), 0.35-0.50 (2H), 0.60-0.70 (1 H), 1.70-1.85 (1 H), 4.60 (1 H), 5.40 (1 H), 7.45 (1 H), 7.60 (1 H), 7.80-7.90 (2H), 8.00 (1 H).

### Example 2: Synthesis of 3-chloro-4-(1-cyclopropyl-1-hydroxy-2-(1H-1,2,4-triazol-1-yl)ethyl)benzaldehyde

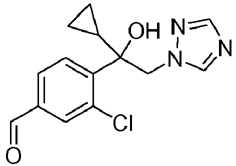

To a cold (-78 °C) solution of the nitrile prepared in Example 1 (30.0 g, 1.0 eq) in THF (500 mL) was added a solution of DiBAl (diisobutylaluminium hydride, 1.0 M in hexane, 332 mL, 3.2 eq) and it was stirred at this temperature for 0.5 h before it was warmed to room temperature over 1 h. The reaction mixture was quenched by careful addition of a saturated aqueous solution of AlCl₃ and diluted HCl was added until a clear solution was obtained. The aqueous phase was extracted with EtOAc and the combined organic extracts were washed with brine, dried over MgSO₄ and concentrated under reduced pressure. The residue was dissolved in a minimal amount of EtOAc and di-*iso*-propyl ether was added to precipitate the product which was collected after filtration. The title compound was isolated as solid after drying under vacuum (16.0 g, 48%).
NMR (400 MHz, CDCl₃): δ [ppm] = 0.20-0.30 (1 H), 0.40-0.45 (2H), 0.60-0.70 (1 H), 1.80-1.90 (1 H), 4.60 (1 H), 5.50 (1 H), 7.60 (1 H), 7.70-7.90 (3H), 8.00 (1 H), 9.95 (1 H).

### Example 3: Synthesis of methyl-3-chloro-4-(1-cyclopropyl-1-hydroxy-2-(1H-1,2,4-triazol-1-yl)ethyl)benzoate

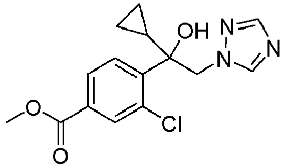

The nitrile as prepared in Example 1 (5.00 g) was dissolved in aqueous NaOH (1 M, 50 mL) and the solution was heated to reflux until HPLC showed complete conversion of the starting material. After cooling to room temperature, diluted HCl was added until a pH of about 2 was reached and the product was extracted with EtOAc. The combined organic layers were dried over MgSO₄ and concentrated under reduced pressure to afford the crude acid as yellow solid which was used without further purification.

The crude product was immediately dissolved in MeOH (20 mL) and treated with concentrated sulfuric acid (0.06 mL, 0.2 eq). The mixture was heated to reflux until no remaining starting material could be detected by HPLC. After cooling to room temperature, the reaction mixture was quenched by addition of a saturated solution of NaHCO₃ and the product was extracted into EtOAc. The combined organic layers were washed with brine, dried over MgSO₄ and freed from solvent under reduced pressure to afford the title compound as solid (1.60 g, 29% over 2 steps).

## Claims

1. Compounds of the formula I 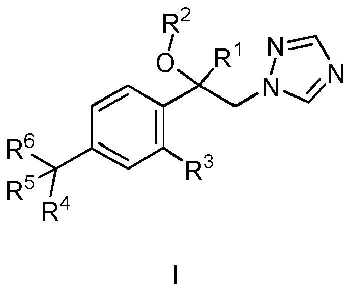 wherein
R¹ is C₁-C₆-alkyl, C₂-C₆-alkenyl, C₂-C₆-alkynyl or C₃-C₆-cycloalkyl;
wherein the aliphatic moieties of R¹ are unsubstituted or carry one, two, three or up to the maximum possible number of identical or different groups R^{1a}:
R^{1a} is independently of one another selected from halogen, OH, CN, C₁-C₄-alkoxy, C₃-C₆-cycloalkyl, C₃-C₆-halogencycloalkyl and C₁-C₄-halogenalkoxy;
wherein the cycloalkyl moieties of R¹ are unsubstituted or carry one, two, three, four, five or up to the maximum number of identical or different groups R^{1b}:
R^{1b} is independently of one another selected from halogen, OH, CN, C₁-C₄-alkyl, C₁-C₄-alkoxy, C₁-C₄-halogenalkyl, C₃-C₆-cycloalkyl, C₃-C₆-halogencycloalkyl and C₁-C₄-halogenalkoxy;
R² is hydrogen, C₁-C₄-alkyl, C₂-C₄-alkenyl or C₂-C₄-alkynyl;
wherein the aliphatic moieties of R² are unsubstituted or carry one, two, three or up to the maximum possible number of identical or different groups R^{2a}:
R^{2a} is independently of one another selected from halogen, OH, CN, C₁-C₄-alkoxy, C₃-C₆-cycloalkyl, C₃-C₆-halogencycloalkyl and C₁-C₄-halogenalkoxy;
R³ is selected from hydrogen, halogen, CN, C₁-C₄-alkyl, C₁-C₄-alkoxy, C₂-C₄-alkenyl, C₂-C₄-alkynyl, C₃-C₆-cycloalkyl and S(O)ₚ(C₁-C₄-alkyl), wherein p is 0, 1 or 2, and
wherein each of R³ is unsubstituted or further substituted by one, two, three or four R^{3a}:
R^{3a} is independently of one another selected from halogen, CN, OH, C₁-C₄-alkyl, C₁-C₄-halogenalkyl, C₃-C₆-cycloalkyl, C₃-C₆-halogencycloalkyl, C₁-C₄-alkoxy and C₁-C₄-halogenalkoxy;
R⁴, R⁵, and R⁶ are independently of one another selected from hydrogen, halogen, CN, -OCN, -SCN, -S(O)ₘ, -S(O)ₒ(C₁-C₄-alkyl), C₁-C₆-alkyl, C₂-C₆-alkenyl, C₂-C₆-alkynyl, C₁-C₄-alkoxy, C₂-C₄-alkenyloxy, C₃-C₆-cycloalkyl, C₃-C₈-cycloalkyl-C₁-C₄-alkyl, C₃-C₆-halogencycloalkyl, aryl, heteroaryl and aryloxy;
R⁴ and R⁵ together are =O, and R⁶ is as defined above;
R⁴ and R⁵ together are =NORⁿ, and R⁶ is as defined above; or
R⁴⁻⁶ together are ≡N;
wherein the aliphatic moieties of R⁴, R⁵, and R⁶ are unsubstituted or carry one, two, three, or up to the maximum number of identical or different groups R^{a}:
R^{a} is independently of one another selected from halogen, OH, CN, C₁-C₄-alkyl, C₁-C₄-halogenalkyl, C₃-C₆-cycloalkyl, C₃-C₆-halogencycloalkyl, C₁-C₄-alkoxy, C₁-C₄-halogenalkoxy and Si(R^{s})₃, wherein R^{s} is independently of one another C₁-C₄-alkyl;
wherein the cycloalkyl moieties of R⁴, R⁵, and R⁶ are unsubstituted or carry one, two, three, four, five or up to the maximum number of identical or different groups R^{b}:
R^{b} is independently of one another selected from halogen, OH, CN, C₁-C₄-alkyl, C₁-C₄-alkoxy, C₁-C₄-halogenalkyl, C₃-C₆-cycloalkyl, C₃-C₆-halogencycloalkyl and C₁-C₄-halogenalkoxy;
wherein the aryl, heteroaryl and aryloxy moieties of R⁴, R⁵, and R⁶ are unsubstituted or carry one, two, three, four, or up to the maximum number of identical or different groups R^{c}:
R^{c} is independently of one another selected from halogen, OH, CN, C₁-C₄-alkyl, C₁-C₄-halogenalkyl, C₃-C₆-cycloalkyl, C₃-C₆-halogencycloalkyl, C₁-C₄-alkoxy and C₁-C₄-halogenalkoxy;
and wherein
Rⁿ is C₁-C₄-alkyl, C₂-C₄-alkenyl or C₂-C₄-alkynyl;
wherein m is independently 0, 1 or 2;
wherein o is independently 0, 1 or 2;
provided that at least one of R⁴, R⁵ and R⁶ is not hydrogen;
provided that when R² and R³ are both hydrogen, and R⁴⁻⁶ is each F, then R¹ is not CH(OH)CH₃, CHCH₃CH=CH₂, cyclopropanecarbonitrile, CH₂CCl₃, CF₃, or CF₂CF₃;
provided that when R² and R³ are both hydrogen, and -CR⁴R⁵R⁶ is CH(CH₃)₂, then R¹ is not CHCH₃CHCH₂, or C(CH₃)₂CH₂OH;
provided that when R² and R³ are both hydrogen, and -CR⁴R⁵R⁶ is CH(CH₃)₂, then R¹ is not C(CH₃)₃;
provided that when R² and R³ are both hydrogen, and R⁴⁻⁶ is each CH₃, then R¹ is not CH₂CCl₃, or C(CH₃)₃;
provided that when R² and R³ are both hydrogen, R⁴⁻⁵ is =O and R⁶ is OCH₃, then R¹ is not C(CH₃)₃;
provided that when R² and R³ are both hydrogen, and R⁴⁻⁶ is ≡N, then R¹ is not C(CH₃)₃, C(CH₃)₂C(CH₃)₂OH, or CF₂C(CH₃)₂OH; and
provided that when R² is hydrogen, R³ is Cl, and R⁴⁻⁶ is ≡N, then R¹ is not CF₂C(CH₃)₂OH;
and the N-oxides and the agriculturally acceptable salts thereof.

2. The compounds of claim 1, wherein R⁴, R⁵, and R⁶ are independently selected from hydrogen, halogen, CN, C₁-C₄-alkyl, C₁-C₄-halogenalkyl, C₁-C₄-alkoxy, C₂-C₄-alkenyl, C₂-C₄-alkynyl, unsubstituted and substituted C₃-C₆-cycloalkyl, aryl, aryloxy, or heteroaryl, wherein the aliphatic moieties of R⁴, R⁵, and R⁶ are unsubstituted or further substituted by one, two, three or four R^{a},
wherein the cycloalkyl moieties of R⁴, R⁵, and R⁶ are unsubstituted or further substituted by one, two, three or four R^{b}, and
wherein the aryl, heteroaryl and aryloxy moieties of R⁴, R⁵, and R⁶ are unsubstituted or further substituted by one, two, three or four R^{c}.

3. The compound of any one of claims 1 to 2, wherein R⁴, R⁵, and R⁶ are independently selected from hydrogen, F, Cl, CN, C₁-C₃-alkyl, C₁-C₃-alkoxy, C₁-C₃-halogenalkyl, C₂-C₄-alkynyl, C₃-C₆-cycloalkyl, phenyl, phenoxy, p-chlorophenoxy, pyridyl or furyl,
wherein the aliphatic moieties of R⁴, R⁵, and R⁶ are unsubstituted or further substituted by one, two, three or four R^{a},
wherein the cycloalkyl moieties of R⁴, R⁵, and R⁶ are unsubstituted or further substituted by one, two, three or four R^{b}, and
wherein the aryl, heteroaryl and aryloxy moieties of R⁴, R⁵, and R⁶ are unsubstituted or further substituted by one, two, three or four R^{c}.

4. The compounds of any one of claims 1 to 3, wherein R^{a}, R^{b}, and R^{c} are independently selected from halogen, CN, and OH.

5. The compounds of any one of claims 1 to 4, wherein R³ is F, Cl, Br, CN, C₁-C₄-alkyl, C₁-C₄-halogenalkyl, C₁-C₄-alkoxy or S(C₁-C₄-alkyl).

6. The compounds of any one of claims 1 to 5, wherein R² is hydrogen.

7. The compounds of any one of claims 1 to 6, wherein R¹ is selected from methyl, ethyl, n-propyl, iso-propyl, CH₂C(CH₃)₃, CH₂CH(CH₃)₂, CF₃, C₂-C₆-alkenyl, C₂-C₆-alkynyl, C₃-C₆-cycloalkyl, 1-F-cyclopropyl and 1-Cl-cyclopropyl.

8. A composition, comprising one compound of formula I, as defined in any of the claims 1 to 7, an N-oxide or an agriculturally acceptable salt thereof.

9. The composition of claim 8, comprising additionally a further active substance.

10. A use of a compound of the formula I, as defined in any of the claims 1 to 7, and/or of an agriculturally acceptable salt thereof or of the compositions, as defined in any of the claims 8 or 9, for combating phytopathogenic fungi.

11. A method for combating harmful fungi, comprising treating the fungi or the materials, plants, the soil or seeds to be protected against fungal attack with an effective amount of at least one compound of formula I, as defined in any of the claims 1 to 7, or with a composition, as defined in any of the claims 8 or 9.

12. Seed, coated with at least one compound of the formula I, as defined in any of the claims 1 to 7, and/or an agriculturally acceptable salt thereof or with a composition, as defined in any of the claims 8 or 9, in an amount of from 0.1 to 10 kg per 100 kg of seed.
